# EUROPEAN PATENT APPLICATION

(11) **EP 4 768 585 A1**
(43) Date of publication of application: **01.07.2026**
(21) Application number: 24867296.6
(22) Date of filing: 03.09.2024
(51) Int. Cl.: C12N 15/113

(54) **SIRNAS FOR SIMULTANEOUSLY INHIBITING EXPRESSION OF TWO TARGET GENES, DRUG AND USE THEREOF**

(30) Priority: 18.09.2023 CN 202311209042; 07.02.2024 CN 202410174987; 13.06.2024 CN 202410762953
(71) Applicant: Bebetter Med Inc., Guangzhou, Guangdong 510660 (CN)
(72) Inventor: FAN, Fushun, Guangzhou, Guangdong 510660 (CN); LIU, Xinjian, Guangzhou, Guangdong 510660 (CN); WANG, Binjie, Guangzhou, Guangdong 510660 (CN); WEI, Yanli, Guangzhou, Guangdong 510660 (CN); QIAN, Changgeng, Guangzhou, Guangdong 510660 (CN)
(74) Representative: Fuchs Patentanwälte Partnerschaft mbB
(86) International application number: PCT/CN2024/116648
(87) International publication number: WO 2025/060884

(57) **Abstract**

The present invention relates to a dual-targeting siRNA agent comprising two distinct siRNAs targeting two different genes or their pharmaceutically acceptable salts, wherein the two distinct siRNAs or their salts are linked by a pharmaceutically acceptable ligand. The siRNA is a dsRNA composed of a sense strand and an antisense strand, and the two different genes are selected from a group consisting of angiotensinogen (AGT), proprotein convertase subtilisin/kexin type 9 (PCSK9), and human angiopoietin-like protein 3 (ANGPTL3). The present invention provides the application of the dual-targeting siRNA agent in the preparation of drugs for preventing or treating diseases associated with hypertension and/or dyslipidemia. The dual-targeting siRNA agent described in the present invention can effectively inhibit the expression of two target genes simultaneously *in vivo,* offering the advantages of strong non-antagonistic activity and high safety. The present invention also provides the siRNAs targeting corresponding genes for the aforementioned dual-targeting siRNA agent and their use for preventing or treating diseases associated with hypertension and/or dyslipidemia.

## Description

The present invention claims priority to Chinese Patent Application No. 202311209042.1 filed on September 18, 2023, Chinese Patent Application No. 202410174987.2 filed on February 07, 2024 and Chinese Patent Application No. 202410762953.5 filed on June 13, 2024, the entire contents of which are incorporated herein by reference.

### TECHNICAL FIELD

The present invention belongs to the field of biomedicine, and specifically relates to siRNA for simultaneously inhibiting the expression of two target genes, drug and use thereof.

### BACKGROUND ART

Chronic diseases such as hypertension, hyperlipidemia, and hypercholesterolemia are the main contributors to cardiovascular and cerebrovascular complications. As living standards improve, these chronic conditions have become important factors affecting human health and reducing the quality of life. In most hypertension guidelines, hypertension is defined as systolic blood pressure (SBP) or diastolic blood pressure (DBP) ≥140/90 mmHg without the use of antihypertensive drugs (Whelton, Paul K et al. Hypertension vol. 71, 6 (2018): 1269-1324.). According to statistics, the incidence of hypertension in China has risen significantly over the past 30 years, affecting the health of about a quarter of adults (Wang, Ji-Guang et al. Nature reviews. Cardiology vol. 20, 8 (2023): 531-545.). Hypertension often occurs together with other chronic conditions such as hyperlipidemia, hypercholesterolemia and diabetes. In a 2009 study involving 4942 outpatients with hypertension, 24.3% of them also had diabetes. Dyslipidemia is also prominent in Chinese hypertensive patients. In the above survey, the prevalence of hypertriglyceridemia was 18.9%, while that of hypercholesterolemia and low HDL cholesterol was 13.5% and 16.6%, respectively. The co-occurrence of these diseases not only increases the risk of cardiovascular and cerebrovascular complications but also complicates the management of such diseases (Miao, Chao-Ying et al. Journal of Clinical Hypertension vol. 23, 7 (2021): 1399-1404). It has been reported that antihypertensive treatment helps improve various patient indicators. For every 10 mmHg reduction in SBP, the risk of cardiovascular disease decreases by 20%; additionally, the risks of coronary heart disease, stroke and cardiac dysfunction decrease by 17%, 27% and 28%, respectively. Given shared risk factors and pathogenesis, the combined use of antihypertensive and lipid-lowering drugs exerts synergistic effects in treating these chronic conditions, and improvement in cardiovascular and cerebrovascular diseases may also serve as an endpoint for hypertension treatment (Ranasinghe, Priyanga et al. Journal of the American Heart Association vol. 11, 20 (2022): e027694.).

The renin-angiotensin-aldosterone system (RAAS) is an endocrine system that maintains blood pressure, blood volume, and water-salt balance in the human body. The angiotensin (ANG I/II) secreted by this system exerts a vasoconstrictive effect. Overactivation of RAAS is one of the key contributors to hypertension (Arendse, Lauren B et al. Pharmacological reviews vol. 71, 4 (2019): 539-570). The antihypertensive drugs currently used in clinical practice are mainly RAAS inhibitors, including angiotensin-converting enzyme inhibitors (ACEIs, e.g., prils) and angiotensin II receptor blockers (ARBs, e.g., sartans). Although the mechanisms of action for these RAAS inhibitors are not entirely identical, they are subject to certain compensatory mechanisms in the body and require daily administration without interruption. Consequently, some patients exhibit poor compliance. Notably, AGT produced by hepatocytes is the sole precursor of angiotensin II (Ang II). Studies have shown that the polymorphism of the AGT gene is closely related to the body's blood pressure regulation. Thus, AGT is an important target for gene silencing therapy of hypertension and related diseases. Inhibiting AGT effectively prevents RAAS overactivation, thereby lowering blood pressure and addressing common limitations of RAAS inhibitors (Ren, Liwei et al. Current Opinion in Nephrology and Hypertension vol. 29, 2 (2020): 180-189). Recent studies have shown that using the hepatocyte-specific GalNAc-conjugated siRNA to mediate AGT gene silencing can inhibit RAAS for weeks to months with a single administration, which offers better compliance and improvement in cardiovascular and cerebrovascular diseases compared to current RAAS inhibitors (Desai, Akshay S et al. The New England Journal of Medicine vol. 389, 3 (2023): 228-238).

As mentioned above, hypertension is closely related to hyperlipidemia. Hyperlipidemia typically refers to dyslipidemia caused by increased cholesterol and/or triglyceride levels in serum, serving as an important contributor to cardiovascular diseases such as atherosclerosis. Loss-of-function variants in the ANGPTL3 gene are associated with reduced plasma levels of triglycerides, low-density lipoprotein cholesterol (LDL-C) and high-density lipoprotein cholesterol (HDL-C). Human genetic studies have shown that heterozygous ANGPTL3 loss-of-function variant carriers exhibit significantly lower serum levels of triglycerides, HDL-C and LDL-C compared to those without this variant (Dewey, Frederick E et al. The New England Journal of Medicine vol. 377, 3 (2017): 211-221). In terms of function, ANGPTL3 produced by hepatocytes is secreted into the blood. After cleavage by PCSK3 or PCSK6, it forms active molecules that inhibit lipoprotein lipase (which catalyzes triglyceride hydrolysis) and endothelial lipase (which hydrolyzes lipoprotein phospholipids), thereby leading to increased plasma levels of triglycerides, HDL and phospholipids. Therefore, inhibiting ANGPTL3 expression represents an important target for treating hyperlipidemia (Kersten, Sander. Nature reviews. Endocrinology vol. 13, 12 (2017): 731-739.).

Additionally, the prevalence of hypercholesterolemia is also high in hypertensive patients, typically defined by LDL-C and non-HDL-C levels. Similarly, human genetics studies have shown that PCSK9 may be associated with familial hypercholesterolemia, and two common PCSK9 loss-of-function variants (PCSK9-679X and PCSK9-142X) are positively correlated with low LDL-C levels in the blood (Cohen, Jonathan C et al. The New England Journal of Medicine vol. 354, 12 (2006): 1264-72). PCSK9 is synthesized in the liver and secreted into the circulatory system; PCSK9 in cells binds to and guides newly generated LDL receptors to be transported from the Golgi apparatus to lysosomes for degradation; PCSK9 in the circulatory system specifically binds to LDL receptors on the surface of hepatocytes, mediating their entry into hepatocyte lysosomes for degradation; this ultimately reduces the liver's capacity to bind and clear LDL-C and increases LDL-C levels in the blood (Horton, Jay D et al. Journal of Lipid Research vol. 50 Suppl, Suppl (2009): S172-7). Therefore, inhibiting PCSK9 expression represents an important target for treating hypercholesterolemia and preventing LDL-C-related cardiovascular and cerebrovascular diseases. Current lipid-lowering therapies include monoclonal antibodies targeting ANGPTL3 and PCSK9, antisense oligonucleotides (ASOs), and siRNA drugs, which have demonstrated favorable therapeutic effects in reducing LDL-C or triglycerides (Chen, Ruoyu et al. Journal of Clinical Laboratory Analysis vol. 36, 7 (2022): e24552).

The discovery of RNA interference (RNAi) has brought RNA therapy to a new level. RNAi technology involves exogenous siRNAs utilizing the cell's endogenous system to degrade the target gene. In terms of mechanism, exogenously introduced double-stranded siRNAs can be recognized by the endoribonuclease Dicer in the cell and split into a single-stranded guide strand and its complementary strand in the RNA-induced silencing complex (RISC). The guide strand siRNA then binds to the Argonaute 2 protein (AGO2) and guides it to the target RNA, and AGO2 mediates the degradation of the target RNA. In addition to degrading RNAs in the cytoplasm, siRNAs also promote chromatin remodeling and histone modification in the nucleus, thereby inducing transcriptional silencing (Matzke et al. Nature Reviews Genetics vol. 6, 1 (2005): 24-35). Several siRNA drugs have been approved by the FDA, and many candidate siRNAs are currently undergoing phase III clinical trials (Zhu, Yiran et al. Cell Death & Disease vol. 13, 7 644. 23 Jul. 2022). siRNA is unstable in blood and tissues and is easily digested and degraded by nucleases in the body. To enhance the stability of the antisense and sense strands of siRNA, chemical modifications such as 2'-O-methylation and 2'-fluoro are commonly introduced when synthesizing siRNA drugs. The delivery of siRNA drugs is also an important guarantee for RNAi therapy. Currently, the most mature delivery system is GalNAc. GalNAc binds to the asialoglycoprotein receptor (ASGPR) specifically expressed by hepatocytes. When coupled with siRNA, it enables active delivery to hepatocytes, representing a significant breakthrough in the field of RNA therapy. The three genes of interest in this invention-AGT, ANGPTL3 and PCSK9-are all hepatocyte-specific or highly expressed proteins. Therefore, the GalNAc system enables liver delivery of single-targeting or dual-targeting siRNA drugs (Debacker, Alexandre J et al. Molecular therapy vol. 28, 8 (2020): 1759-1771).

RNAi drugs targeting the PCSK9 gene have already been approved for marketing, while those targeting the AGT and ANGPTL3 genes are still under development and both have shown promising therapeutic effects. However, further development of highly effective RNAi drugs with long-lasting efficacy remains of significant importance. Dual-targeting RNAi drugs formed by combining siRNAs targeting the three genes in pairs hold immense clinical potential for the combined treatment of chronic cardiovascular and cerebrovascular diseases such as hypertension, hyperlipidemia and hypercholesterolemia. For example, dual-targeting drugs formed by combining siRNAs targeting AGT and ANGPTL3 or targeting AGT and PCSK9 may exhibit synergistic therapeutic effects in lowering blood pressure and blood lipids; dual-targeting drugs formed by combining siRNAs targeting ANGPTL3 and PCSK9 may achieve unexpected efficacy persistence for treating hypercholesterolemia or hyperlipidemia, thereby enhancing therapeutic effects. However, few single-targeting siRNAs are truly suitable for clinical application, and it is even more difficult to combine different siRNAs targeting two target genes. In addition to effectively controlling the target genes, the two siRNAs must not antagonize each other *in vivo* and the duration of action at the two targets must be roughly consistent. Nevertheless, owing to its immense potential for medical applications, research on multi-targeting drugs remains highly sought after and anticipated.

### SUMMARY OF INVENTION

Based on this, the purpose of the present invention is to provide a dual-targeting siRNA agent for simultaneously inhibiting the expression of any two target genes of AGT, PCSK9 and ANGPTL3 in cells and use thereof, offering the advantages of simultaneously inhibiting the expression of two target genes *in vivo* without mutual antagonism, with high activity and high safety.

In the first aspect, the present invention provides a dual-targeting siRNA agent, which comprises two different siRNAs targeting two different genes or pharmaceutically acceptable salts thereof, wherein the two different siRNAs or salts thereof are connected to a ligand for delivering nucleic acid to form a single entity, each of the siRNAs is a dsRNA composed of a sense strand and an antisense strand, and the two different genes are selected from any two of AGT, PCSK9 and ANGPTL3;
wherein the base composition of the siRNA targeting AGT is selected from any one of the following dsRNA sequences, or a sequence having a sense strand or antisense strand that differs by no more than 1 nucleotide therefrom:
   A) the dsRNA duplex is 1167f, wherein the sense strand comprises UGACCAGCUUGUUUGUGAA (SEQ ID NO: 59), and the antisense strand comprises UUCACAAACAAGCUGGUCGGG (SEQ ID NO: 60);
   B) the dsRNA duplex is 1167b, wherein the sense strand comprises GCCGACCAGCUUGUUUGUGAA (SEQ ID NO: 51), and the antisense strand comprises UUCACAAACAAGCUGGUCGGC (SEQ ID NO: 52);
   C) the dsRNA duplex is 1165d, wherein the sense strand comprises GAGAACCAGUGUUUAGCGA (SEQ ID NO: 39), and the antisense strand comprises UCGCUAAACACUGGUUCUUGG (SEQ ID NO: 40);
   D) the dsRNA duplex is 1165f, wherein the sense strand comprises CCAAGAACCAGUGUUUAGCGA (SEQ ID NO: 43), and the antisense strand comprises UCGCUAAACACUGGUUCUUGG (SEQ ID NO: 44);
wherein the base composition of the siRNA targeting PCSK9 is selected from any one of the following dsRNA sequences, or a sequence having a sense strand or antisense strand that differs by no more than 1 nucleotide therefrom:
   E) the dsRNA duplex is 11040a, wherein the sense strand comprises CUUAUUCUGGGUUUUGUAGCA (SEQ ID NO: 375), and the antisense strand comprises UGCUACAAAACCCAGAAUAAG (SEQ ID NO: 376);
   F) the dsRNA duplex is 11002d, wherein the sense strand comprises GCAGCCAACUUUUCUAGAA (SEQ ID NO: 259), and the antisense strand comprises UUCUAGAAAAGUUGGCUGUGG (SEQ ID NO: 260);
   G) the dsRNA duplex is 11002a, wherein the sense strand comprises CUACAGCCAACUUUUCUAGAA (SEQ ID NO: 253), and the antisense strand comprises UUCUAGAAAAGUUGGCUGUAG (SEQ ID NO: 254);
wherein the base composition of the siRNA targeting ANGPTL3 is selected from any one of the following dsRNA sequences, or a sequence having a sense strand or antisense strand that differs by no more than 1 nucleotide therefrom:
   H) the dsRNA duplex is 7061f, wherein the sense strand comprises UACUUGAACUCAACUCAAA (SEQ ID NO: 579), and the antisense strand comprises UUUGAGUUGAGUUCAAGUGGG (SEQ ID NO: 580);
   I) the dsRNA duplex is 7061b, wherein the sense strand comprises GCCACUUGAACUCAACUCAAA (SEQ ID NO: 571), and the antisense strand comprises UUUGAGUUGAGUUCAAGUGGC (SEQ ID NO: 572).

In some embodiments, the dual-targeting siRNA agent targets genes AGT and PCSK9, AGT and ANGPTL3, and PCSK9 and ANGPTL3.

In some embodiments, the target genes are AGT and any one selected from PCSK9 and ANGPTL3, preferably AGT and PCSK9.

The invention also provides a method for modifying the nucleotides used in the aforementioned siRNA to enhance siRNA stability and activity *in vivo* and *in vitro* while reducing off-target activity. The modified nucleotides comprise at least one or more of the following: 2'-O-methyl modified nucleotides, 2'-fluoro modified nucleotides, 2'-deoxynucleotides, 2'3'-seco nucleotide mimics, locked nucleic acid (LNA), unlocked nucleic acid nucleotides (UNA), glycol nucleic acid nucleotides (GNA), 2'-F-arabinonucleotides, 2'-methoxyethyl nucleotides, abasic nucleotides, ribitol, inverted nucleotides, inverted abasic residues, inverted 2'-OMe nucleotides, inverted 2'-deoxynucleotides, 2'-amino modified nucleotides, 2'-alkyl modified nucleotides, morpholino nucleotides, 3'-OMe nucleotides, nucleotides containing a 5'-phosphorothioate group, terminal nucleotides linked to a cholesterol derivative or a dodecanoic acid bisdecylamide group, 2'-amino modified nucleotides, phosphoramidates, or unnatural bases containing nucleotides.

In some embodiments, the sense strand comprises no more than 3, 2, 1 or 0 unmodified nucleotides, wherein the modified nucleotides in the sense strand are selected from 2'-O-methyl modified nucleotides, 2'-deoxynucleotides, 2'-fluoro modified nucleotides and inverted abasic residues, and 5'-terminus and 3'-terminus of the sense strand each independently contains 0, 1, 2 or 3 phosphorothioate bonds; the antisense strand comprises no more than 3, 2, 1 or 0 unmodified nucleotides, wherein the modified nucleotides in the antisense strand are selected from 2'-O-methyl modified nucleotides, 2'-deoxynucleotides, 2'-fluoro modified nucleotides, VPU, VPU-S or other VPU derivatives, and 5'-terminus and 3'-terminus of the antisense strand each independently comprises 1-3 phosphorothioate bonds.

In some embodiments, the dsRNA duplex targeting the AGT gene is modified and selected from any one of the following sequences, or a sequence having a sense strand or antisense strand that differs by no more than 1 nucleotide therefrom:
a) the dsRNA duplex is 1167.25-19, wherein the 5'-3' sense strand comprises Invab*mU*mGmAmCmCmAfGfCfUfUmGfUmUmUmGmUmGmAmAInvab, and the 5'-3' antisense strand comprises VPU-S*fU*mCmAmCmAmAmAmCmAmAdGmCfUmGfGmUmCmG*mG*mG;
b) the dsRNA duplex is 1167.27-21, wherein the 5'-3' sense strand comprises Invab*mG*mCmCmGmAmCmCmAfGfCfUfUmGfUmUmUmGmUmGmAmAInvab, and the 5'-3' antisense strand comprises VPU-S*fU*mCmAmCmAmAmAmCmAmAdGmCfUmGfGmUmCmG*mG*mC;
c) the dsRNA duplex is 1165.5-14, wherein the 5'-3' sense strand comprises Invab*mG*mAmGmAmAmCfCfAfGfUmGfUmUmUmAmGmCmGmAInvab, and the 5'-3' antisense strand comprises VPU-S*fC*mGmCmUmAmAmAmCmAmCdTmGfGmUfUmCmUmU*mG*mG;
d) the dsRNA duplex is 1165.5-16, wherein the 5'-3' sense strand comprises Invab*mC*mCmAmAmGmAmAmCfCfAfGfUmGfUmUmUmAmGmCmGmAInvab, and the 5'-3' antisense strand comprises VPU-S*fC*mGmCmUmAmAmAmCmAmCdTmGfGmUfUmCmUmU*mG*mG;

In some embodiments, the siRNA targeting PCSK9 is modified and selected from any one of the following sequences, or a sequence having a sense strand or antisense strand that differs by no more than 1 nucleotide therefrom:
e) the dsRNA duplex is 11040.10-23, wherein the 5'-3' sense strand comprises Invab*mC*mUmUmAmUmUmCmUfGfGfGfUmUfUmUmGmUmAmGmCmAInvab, and the 5'-3' antisense strand comprises VPU-S*fG*mCmUmAmCmAmAmAmAmCdCmCfAmGfAmAmUmA*mA*mG;
f) the dsRNA duplex is 11002.17-21, wherein the 5'-3' sense strand comprises Invab*mG*mCmAmGmCmCfAfAfCfUmUfUmUmCmUmAmGmAmAInvab, and the 5'-3' antisense strand comprises VPU-S*fU*mCmUmAmGmAmAmAmAmGdTmUfGmGfCmUmGmU*mG*mG;
g) the dsRNA duplex is 11002.10-23, wherein the 5'-3' sense strand comprises Invab*mC*mUmAmCmAmGmCmCfAfAfCfUmUfUmUmCmUmAmGmAmAInvab, and the 5'-3' antisense strand comprises VPU-S*fU*mCmUmAmGmAmAmAmAmGdTmUfGmGfCmUmGmU*mA*mG;

In some embodiments, the dsRNA targeting the ANGPTL3 gene is modified and selected from any one of the following sequences, or a sequence having a sense strand or antisense strand that differs by no more than 1 nucleotide therefrom:
h) the dsRNA is 7061.18-14, wherein the 5'-3' sense strand comprises Invab*mU*mAmCmUmUmGfAfAfCfUmCfAmAmCmUmCmAmAmAInvab, and the 5'-3' antisense strand comprises VPU-S*fU*mUmGmAmGmUmUmGmAmGdTmUfCmAfAmGmUmG*mG*mG;
i) the dsRNA duplex is 7061.4-16, wherein the 5'-3' sense strand comprises Invab*mG*mCmCmAmCmUmUmGfAfAfCfUmCfAmAmCmUmCmAmAmAInvab, and the 5'-3' antisense strand comprises VPU-S*fU*mUmGmAmGmUmUmGmAmGdTmUfCmAfAmGmUmG*mG*mC;
wherein VPU-S is 2'-S-methyluridine-5'-(E)-vinylphosphonate-3'-phosphate, mA is 2'-O-methyladenosine-3'-phosphate, mU is 2'-O-methyluridine-3'-phosphate, mC is 2'-O-methylcytidine-3'-phosphate, mG is 2'-O-methylguanosine-3'-phosphate, fA is 2'-fluoroadenosine-3'-phosphate, fU is 2'-fluorouridine-3'-phosphate, fC is 2'-fluorocytidine-3'-phosphate, fG is 2'-fluoroguanosine-3'-phosphate, dA is 2'-deoxyadenosine-3'-phosphate, dT is 2'-deoxythymidine-3'-phosphate, dC is 2'-deoxycytidine-3'-phosphate, dG is 2'-deoxyguanosine-3'-phosphate, Invab is an inverted abasic residue, and * is a phosphorothioate bond.

In some embodiments, the ligand is GalNAc or a derivative thereof.

In some embodiments, the GalNAc or a derivative thereof is connected to the 3'-termini of the sense strands of two siRNAs via chemical bonds, preferably via phosphate bonds or phosphorothioate bonds, respectively.

In some embodiments, the structure of the ligand is as follows:

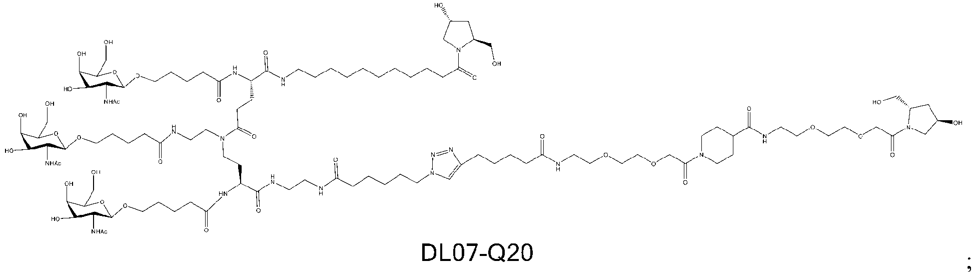

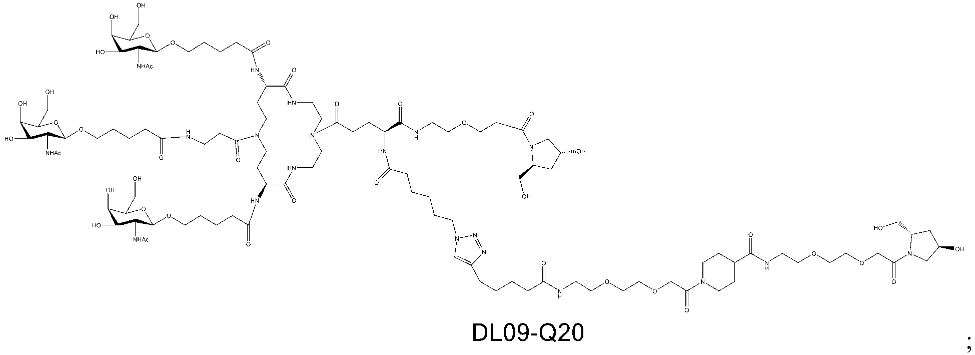

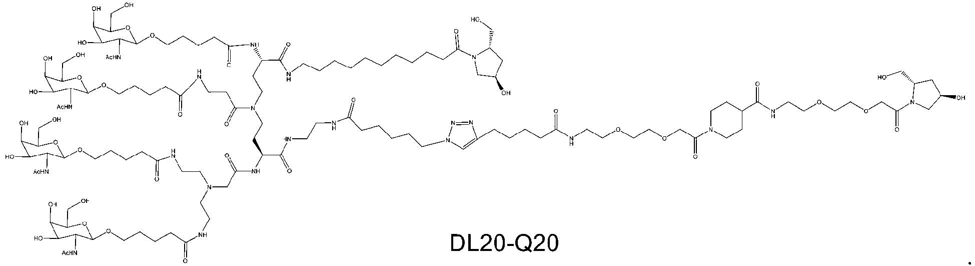

In some embodiments, the ligand is connected to the 3'-termini of the sense strands of siRNAs.

In some embodiments, the ligand is connected to the 3'-termini of the sense strands of siRNAs in the following mode: 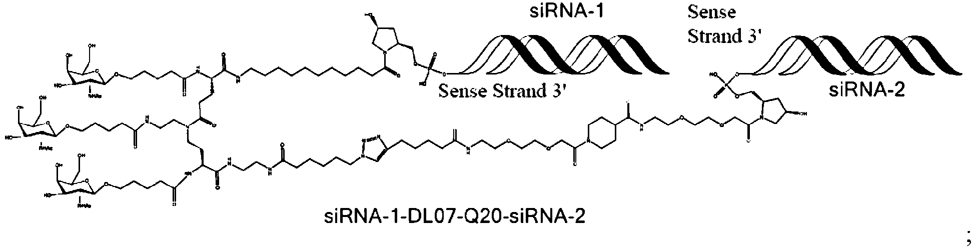 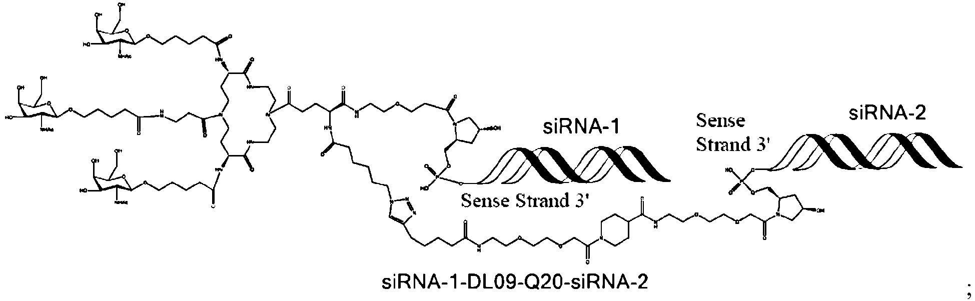 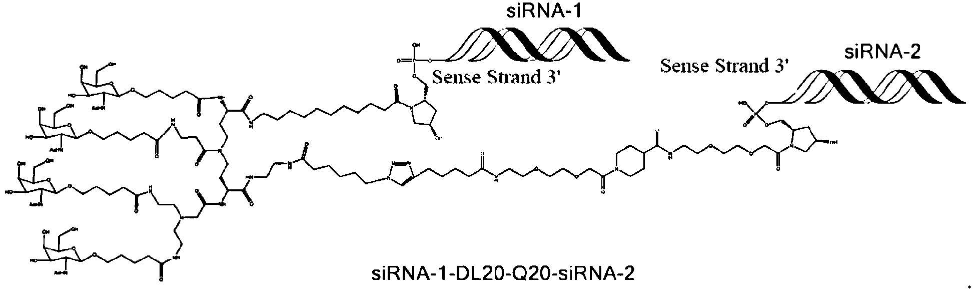 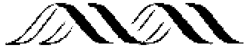 represents a modified siRNA sequence targeting AGT, PCSK9, or ANGPTL3.

In some embodiments, when targeting the AGT and PCSK9 genes, each siRNA of the dual-targeting siRNA agent is selected from any one of the sequences DT03081, DT03086, DT03103, DT03099, DT03085, DT03101, DT03082, DT03097, DT03100, or DT03102 shown in Table 45.

In some embodiments, when targeting the AGT and ANGPTL3 genes, each siRNA of the dual-targeting siRNA agent is selected from the sequences DT02043 or DT02047 shown in Table 45.

In some embodiments, when targeting the ANGPTL3 and PCSK9 genes, each siRNA of the dual-targeting siRNA agent is selected from any one of the sequences DT09001, DT09003 or DT09005 shown in Table 45.

In the second aspect, the present invention provides the use of any one of the above dual-targeting siRNA agents in the preparation of a double-stranded siRNA product for simultaneously inhibiting the expression of any two targets (target genes) selected from AGT, PCSK9, and ANGPTL3.

In some embodiments, the product is a biological preparation or a pharmaceutical preparation.

In the third aspect, the present invention provides the use of any one of the above dual-targeting siRNA agents in the preparation of a drug for preventing and/or treating hypertension and/or lipid disorder-related diseases.

In the fourth aspect, the present invention provides a biological preparation or pharmaceutical preparation for simultaneously inhibiting the expression of any two target genes selected from AGT, PCSK9 and ANGPTL3, wherein the active ingredient comprises any one of the above dual-targeting siRNA agents.

In the fifth aspect, the present invention provides a method for simultaneously inhibiting the expression of any two target genes selected from AGT, PCSK9 and ANGPTL3, comprising:
(a) exposing cells *in vivo* or *in vitro* to any one of the above dual-targeting siRNA agents or the above biological preparation or pharmaceutical preparation; and
(b) maintaining the cells generated in step (a) for a time sufficient to achieve degradation of mRNA transcripts expressed by any two targets selected from AGT, PCSK9 and ANGPTL3, thereby simultaneously inhibiting the expression of any two targets selected from AGT, PCSK9 and ANGPTL3 in the cells.

A method for treating a disease associated with the AGT gene and/or the PCSK9 gene and/or the ANGPTL3 gene, comprising administering to the subject a therapeutically effective amount of the above dual-targeting siRNA agent or the above biological preparation or pharmaceutical preparation, thereby treating the subject.

In some embodiments, the cell is *in vivo* or *in vitro* in the subject. Preferably, the subject is a mammal.

In some embodiments, the subject is a human.

In some embodiments, the expression of AGT, PCSK9 and ANGPTL3 is inhibited by at least approximately 30%, approximately 40%, approximately 50%, approximately 60%, approximately 70%, approximately 80%, approximately 90%, approximately 95%, approximately 98%, or approximately 100%.

Wherein, the hypertension-related diseases mediated by the AGT gene include but are not limited to borderline hypertension, primary hypertension, secondary hypertension, hypertensive crisis, hypertensive emergency, isolated systolic and diastolic hypertension, pregnancy-related hypertension, diabetic hypertension, intractable hypertension, refractory hypertension, paroxysmal hypertension, renovascular hypertension, Goldblatt hypertension, pulmonary hypertension, portal hypertension, systemic venous hypertension, systolic hypertension and unstable hypertension, or any other disease mediated by the AGT gene.

Wherein, the lipid disorder-related diseases mediated by the PCSK9 gene include but are not limited to hyperlipidemia, atherosclerosis, non-alcoholic steatohepatitis, non-alcoholic fatty liver disease, or any other diseases mediated by the PCSK9 gene.

Wherein, the lipid disorder-related diseases mediated by the ANGPTL3 gene include but are not limited to hyperlipidemia, hypertriglyceridemia, lipid and/or cholesterol metabolism disorders, homozygous and heterozygous familial hypercholesterolemia, statin-resistant hypercholesterolemia, cardiometabolic disease, obesity, atherosclerosis, type 2 diabetes, cardiovascular disease, coronary artery disease, non-alcoholic steatohepatitis, non-alcoholic fatty liver disease, hypertriglyceridemia-induced pancreatitis, or any other diseases mediated by the ANGPTL3 gene.

In the sixth aspect, the present invention provides an siRNA targeting the AGT gene, PCSK9 gene, or ANGPTL3 gene, which can be used as a single-targeting drug or a dual-targeting drug.

An siRNA targeting the AGT gene or a pharmaceutically acceptable salt thereof, wherein the sequence is selected from any one of the following, or a sense strand or antisense strand sequence that differs by no more than 1 nucleotide therefrom:
A) the dsRNA is 1167f, wherein the sense strand comprises UGACCAGCUUGUUUGUGAA (SEQ ID NO: 59), and the antisense strand comprises UUCACAAACAAGCUGGUCGGG (SEQ ID NO: 60);
B) the dsRNA is 1167b, wherein the sense strand comprises GCCGACCAGCUUGUUUGUGAA (SEQ ID NO: 51), and the antisense strand comprises UUCACAAACAAGCUGGUCGGC (SEQ ID NO: 52);
C) the dsRNA is 1165d, wherein the sense strand comprises GAGAACCAGUGUUUAGCGA (SEQ ID NO: 39), and the antisense strand comprises UCGCUAAACACUGGUUCUUGG (SEQ ID NO: 40);
D) the dsRNA is 1165f, wherein the sense strand comprises CCAAGAACCAGUGUUUAGCGA (SEQ ID NO: 43), and the antisense strand comprises UCGCUAAACACUGGUUCUUGG (SEQ ID NO: 44);

In some embodiments, the siRNA is modified and selected from any one of the following sequences, or a sense strand or antisense strand sequence that differs by no more than 1 nucleotide therefrom:
a) the dsRNA is 1167.25-19, wherein the 5'-3' sense strand comprises Invab*mU*mGmAmCmCmAfGfCfUfUmGfUmUmUmGmUmGmAmAInvab, and the 5'-3' antisense strand comprises VPU-S*fU*mCmAmCmAmAmAmCmAmAdGmCfUmGfGmUmCmG*mG*mG;
b) the dsRNA is 1167.27-21, wherein the 5'-3' sense strand comprises Invab*mG*mCmCmGmAmCmCmAfGfCfUfUmGfUmUmUmGmUmGmAmAInvab, and the 5'-3' antisense strand comprises VPU-S*fU*mCmAmCmAmAmAmCmAmAdGmCfUmGfGmUmCmG*mG*mC;
c) the dsRNA is 1165.5-14, wherein the 5'-3' sense strand comprises Invab*mG*mAmGmAmAmCfCfAfGfUmGfUmUmUmAmGmCmGmAInvab, and the 5'-3' antisense strand comprises VPU-S*fC*mGmCmUmAmAmAmCmAmCdTmGfGmUfUmCmUmU*mG*mG;
d) the dsRNA is 1165.5-16, wherein the 5'-3' sense strand comprises Invab*mC*mCmAmAmGmAmAmCfCfAfGfUmGfUmUmUmAmGmCmGmAInvab, and the 5'-3' antisense strand comprises VPU-S*fC*mGmCmUmAmAmAmCmAmCdTmGfGmUfUmCmUmU*mG*mG;

An siRNA targeting the PCSK9 gene or a pharmaceutically acceptable salt thereof, wherein the sequence is selected from any one of the following, or a sense strand or antisense strand sequence that differs by no more than 1 nucleotide therefrom:
E) the dsRNA is 11040a, wherein the sense strand comprises CUUAUUCUGGGUUUUGUAGCA (SEQ ID NO: 375), and the antisense strand comprises UGCUACAAAACCCAGAAUAAG (SEQ ID NO: 376);
F) the dsRNA is 11002d, wherein the sense strand comprises GCAGCCAACUUUUCUAGAA (SEQ ID NO: 259), and the antisense strand comprises UUCUAGAAAAGUUGGCUGUGG (SEQ ID NO: 260);
G) the dsRNA is 11002a, wherein the sense strand comprises CUACAGCCAACUUUUCUAGAA (SEQ ID NO: 253), and the antisense strand comprises UUCUAGAAAAGUUGGCUGUAG (SEQ ID NO: 254);

In some embodiments, the siRNA targeting the PCSK9 gene is modified and selected from any one of the following sequences, or a sense strand or antisense strand sequence that differs by no more than 1 nucleotide therefrom:
e) the dsRNA is 11040.10-23, wherein the 5'-3' sense strand comprises Invab*mC*mUmUmAmUmUmCmUfGfGfGfUmUfUmUmGmUmAmGmCmAInvab, and the 5'-3' antisense strand comprises VPU-S*fG*mCmUmAmCmAmAmAmAmCdCmCfAmGfAmAmUmA*mA*mG;
f) the dsRNA is 11002.17-21, wherein the 5'-3' sense strand comprises Invab*mG*mCmAmGmCmCfAfAfCfUmUfUmUmCmUmAmGmAmAInvab, and the 5'-3' antisense strand comprises VPU-S*fU*mCmUmAmGmAmAmAmAmGdTmUfGmGfCmUmGmU*mG*mG;
g) the dsRNA is 11002.10-23, wherein the 5'-3' sense strand comprises Invab*mC*mUmAmCmAmGmCmCfAfAfCfUmUfUmUmCmUmAmGmAmAInvab, and the 5'-3' antisense strand comprises VPU-S*fU*mCmUmAmGmAmAmAmAmGdTmUfGmGfCmUmGmU*mA*mG;

An siRNA targeting the ANGPTL3 gene or a pharmaceutically acceptable salt thereof, wherein the sequence is selected from any one of the following, or a sense strand or antisense strand sequence that differs by no more than 1 nucleotide therefrom:
H) the dsRNA is 7061f, wherein the sense strand comprises UACUUGAACUCAACUCAAA (SEQ ID NO: 579), and the antisense strand comprises UUUGAGUUGAGUUCAAGUGGG (SEQ ID NO: 580);
I) the dsRNA is 7061b, wherein the sense strand comprises GCCACUUGAACUCAACUCAAA (SEQ ID NO: 571), and the antisense strand comprises UUUGAGUUGAGUUCAAGUGGC (SEQ ID NO: 572);

In some embodiments, the siRNA is modified and selected from any one of the following sequences, or a sense strand or antisense strand sequence that differs by no more than 1 nucleotide therefrom:
h) the dsRNA is 7061.18-14, wherein the 5'-3' sense strand comprises Invab*mU*mAmCmUmUmGfAfAfCfUmCfAmAmCmUmCmAmAmAInvab, and the antisense strand comprises VPU-S*fU*mUmGmAmGmUmUmGmAmGdTmUfCmAfAmGmUmG*mG*mG;
i) the dsRNA is 7061.4-16, wherein the 5'-3' sense strand comprises Invab*mG*mCmCmAmCmUmUmGfAfAfCfUmCfAmAmCmUmCmAmAmAInvab, and the antisense strand comprises VPU-S*fU*mUmGmAmGmUmUmGmAmGdTmUfCmAfAmGmUmG*mG*mC;

In the seventh aspect, the present invention provides an siRNA agent targeting the AGT gene, PCSK9 gene, or ANGPTL3 gene, wherein the active ingredient is formed by connecting any one of the above siRNAs or a pharmaceutically acceptable salt thereof to a ligand.

In some embodiments, the ligand is a GalNAc derivative, preferably having the following structural formula: 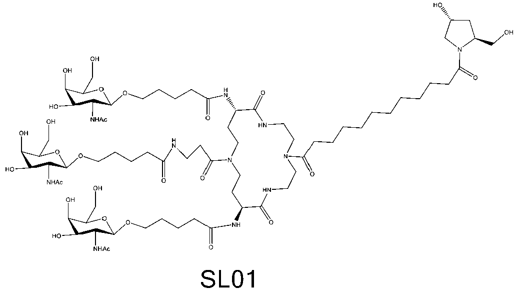 or

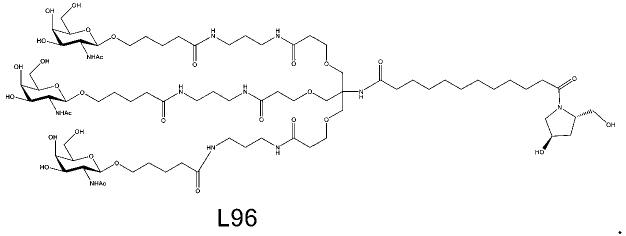

In some embodiments, the ligand is connected to the 3'-termini of sense strands via chemical bonds, preferably via phosphate bonds or phosphorothioate bonds, and the connection mode is as follows:

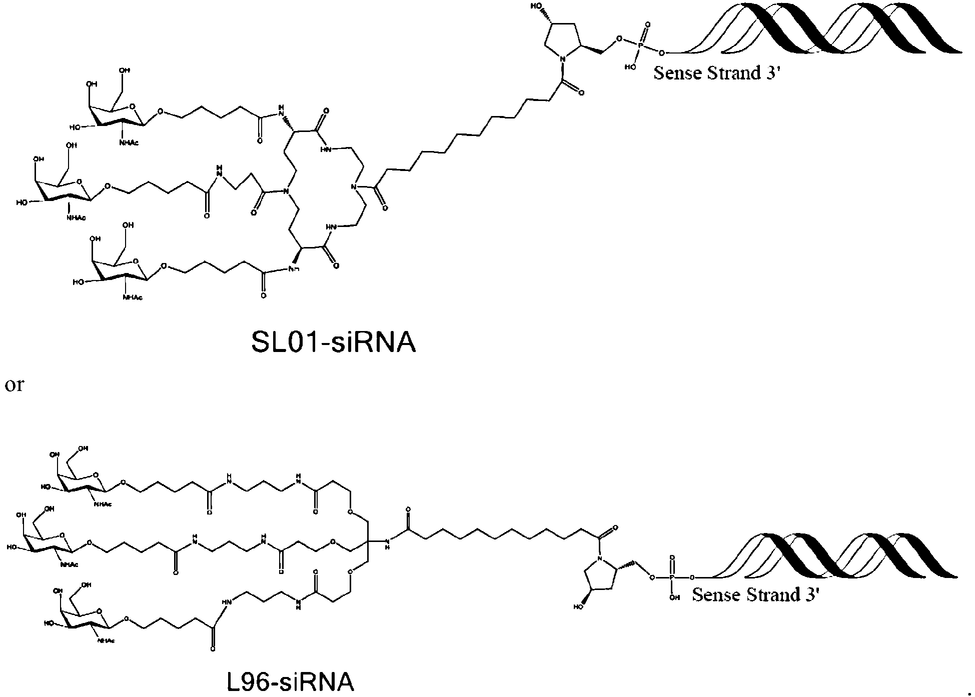

After studying siRNA sequences and conducting numerous experiments, we have screened multiple dual-targeting siRNA agents capable of simultaneously inhibiting the expression of any two targets selected from AGT, PCSK9, and ANGPTL3. On this basis, we have made appropriate modifications to enhance target silencing efficiency while reducing off-target activity. These agents can be used in single-targeting scenarios and are even more suitable for dual-targeting scenarios. Additionally, the siRNA agents described in the present invention, with suitable preferred combinations of two targets, can be simultaneously delivered to the liver via a delivery system to exert their effect, resulting in better activity. They are expected to be used clinically in hypertension and/or lipid disorder-related diseases related to any two targets selected from AGT, PCSK9 and ANGPTL3.

### BRIEF DESCRIPTION OF DRAWINGS

Figure 1 Curve Showing the Inhibition Effects of AGT/PCSK9 Dual-Targeting siRNA DT03081 on AGT Target in AAV8-hAGT/hPCSK9 Mice at a Dose of 60 nmol/kg over Time.
Figure 2 Curve Showing the Inhibition Effects of AGT/PCSK9 Dual-Targeting siRNA DT03081 on PCSK9 Target in AAV8-hAGT/hPCSK9 Mice at a Dose of 60 nmol/kg over Time.

### DESCRIPTION OF EMBODIMENTS

For ease of understanding of the present invention, the present invention will be described more fully below. The present invention may be implemented in many different forms and is not limited to the embodiments described herein. Rather, these embodiments are provided for a more thorough and complete understanding of the disclosure of the present invention.

The experimental methods in the following embodiments, where experimental conditions are not specified, are generally carried out under routine conditions, such as those described in Sambrook et al., Molecular Cloning: A Laboratory Manual (New York: Cold Spring Harbor Laboratory Press, 1989), or as recommended by the manufacturer. The common chemical reagents used in the embodiments are all commercially available products.

Unless otherwise defined, all technical and scientific terms used in the present invention have the same meaning as those commonly understood by those skilled in the art of the present invention. The terms used in the Description of the present invention are only for the purpose of describing specific embodiments and are not intended to limit the present invention. The term "and/or" as used herein encompasses any and all combinations of one or more of the related listed items.

The present invention provides a dual-targeting siRNA agent comprising two distinct siRNAs targeting two different genes or their pharmaceutically acceptable salts, wherein the siRNA comprises a double-stranded RNA (dsRNA) formed by a sense strand and an antisense strand, including the first dsRNA targeting the first gene and the second dsRNA targeting the second gene. The first and second dsRNAs are linked by a pharmaceutically acceptable ligand, wherein the first target gene and the second target gene are respectively selected from angiotensinogen (AGT), angiopoietin-like protein 3 (ANGPTL3), and proprotein convertase subtilisin/kexin type 9 (PCSK9); The expression of any two targets selected from AGT, ANGPTL3, and PCSK9 can be evaluated based on the levels of any variables associated with the expression of AGT, ANGPTL3, and PCSK9 genes, such as AGT, ANGPTL3, and PCSK9 mRNA levels in tissues or serum, AGT, ANGPTL3, and PCSK9 protein levels, and relevant lipid levels. Inhibition can be evaluated by the decrease in the absolute or relative level of one or more of these variables compared to the control level. This control level may be any type employed in the relevant technical field, such as baseline levels predose, levels measured from untreated or control-treated similar subjects, cells, or samples, and known population levels.

Some embodiments provide applications for the diseases of hypertension and/or dyslipidemia associated with the expression of any two targets selected from the group consisting of AGT, ANGPTL3, and PCSK9.

The application of the drug for the treatment and/or prevention of hypertension-related diseases, wherein said hypertension-related diseases are mediated by the AGT gene, including but not limited to borderline hypertension, essential hypertension, secondary hypertension, hypertensive crisis, hypertensive urgency, isolated systolic and diastolic hypertension, pregnancy-related hypertension, diabetic hypertension, refractory hypertension, paroxysmal hypertension, renovascular hypertension, Goldblatt hypertension, pulmonary hypertension, portal hypertension, systemic venous hypertension, systolic hypertension, and labile hypertension, or other diseases related to AGT gene mediation.

The application of the drug in treating and/or preventing dyslipidemia-related diseases, wherein said dyslipidemia-related diseases are mediated by the PCSK9 gene, including but not limited to hyperlipidemia, atherosclerosis, non-alcoholic steatohepatitis, non-alcoholic fatty liver disease, or other diseases associated with PCSK9 gene mediation.

The application of the drug in treating and/or preventing dyslipidemia-related diseases, wherein said dyslipidemia-related diseases are mediated by the ANGPTL3 gene, including but not limited to hyperlipidemia, hypertriglyceridemia, lipid and/or cholesterol metabolism disorders, homozygous and heterozygous familial hypercholesterolemia, statin-resistant hypercholesterolemia, cardiometabolic diseases, obesity, atherosclerosis, type 2 diabetes, cardiovascular diseases, coronary artery disease, non-alcoholic steatohepatitis, non-alcoholic fatty liver disease, pancreatitis induced by hypertriglyceridemia, or other diseases associated with ANGPTL3 gene mediation.

Delivery of the siRNA agent of the present invention to cells, such as cells within subjects (e.g., human subjects, such as those with conditions related to AGT, ANGPTL3, and PCSK9, including hemochromatosis), may be achieved through various pathways. For example, delivery may be effected by contacting cells with the siRNA of the present invention either *in vitro* or *in vivo. In vivo* delivery may also be achieved by administering compositions containing siRNA or its salts to subjects.

Generally, any method for delivering nucleic acid molecules (*in vitro* or *in vivo*) may refer to existing delivery technologies. For *in vivo* delivery, factors considered for delivering siRNA molecules include, for example, the biological stability of the delivered molecule, prevention of non-specific effects, and accumulation of the delivered molecule within the target tissue. The formulation can be administered locally (for example, by direct injection or implantation into tissue or topical application). Local administration to the treatment site maximizes the local concentration of the pharmaceuticals, limits exposure of the agent to systemic tissues that could be harmed by it or degrade it, and allows for a lower total dose of the siRNA molecules to be administered.

For systemic administration of siRNA to treat diseases, the RNA may be delivered either through modification or via drug delivery systems; both approaches function to prevent rapid degradation of dsRNA caused by endonucleases and exonucleases within the living organism. Modifications to the RNA or pharmaceutical carrier may also enable the siRNA composition to target specific tissues while avoiding off-target effects. siRNA molecules can be chemically conjugated to lipophilic groups (such as cholesterol) or encapsulated in lipid particles to enhance cellular uptake and prevent degradation.

The present invention also encompasses pharmaceutical compositions and formulations comprising any of the aforementioned siRNAs or their salts. In some embodiments, the pharmaceutical formulation comprises the siRNA or its salt described herein and a pharmaceutically acceptable carrier. Pharmaceutical compositions containing said siRNA are for treating diseases or conditions associated with AGT, ANGPTL3, and PCSK9, such as hemochromatosis. These pharmaceutical compositions are formulated based on the delivery mode.

In some embodiments, the compositions are formulated for systemic administration via non-intestinal routes such as intravenous (IV) or subcutaneous (SC) delivery. In the method of the present invention, the pharmaceutical formulation of the siRNA or its salt may be administered in solution, preferably in a sterile solution, such as by injection.

In the present invention, "therapeutically effective dose" includes, when administered to a patient for treating diseases related to AGT, ANGPTL3, and PCSK9, an amount of the siRNA or its salt sufficient to effect treatment of said disease (e.g., by attenuating, alleviating, or maintaining the existing disease or one or more symptoms thereof). The "therapeutically effective dose" may vary based on the pharmaceutical formulation of the siRNA or its salt, the method of administration of the formulation, the disease and its severity, the medical history, age, weight, family history, genetic makeup, stage of the pathological process mediated by the expression of AGT, ANGPTL3, and PCSK9, the type of prior or concomitant therapy (if any), and other independent characteristics of the patient to be treated.

In the present invention, "prophylactically effective dose" includes, when administered to subjects who have not yet experienced or exhibited symptoms of diseases related to AGT, ANGPTL3, and PCSK9 but may be susceptible to such diseases, an amount of a pharmaceutical formulation containing siRNA or its salts sufficient to prevent or mitigate the disease or one or more symptoms thereof. Alleviating the disease includes delaying its progression or reducing the severity of subsequent disease development. The "prophylactically effective dose" may vary based on the method of administration of the pharmaceutical formulation of the siRNA or its salt, the severity of disease risk, the medical history, age, weight, family history, genetic makeup, stage of the pathological process mediated by the expression of AGT, ANGPTL3, and PCSK9, the type of prior or concomitant therapy (if any), and other independent characteristics of the patient to be treated.

"Therapeutically effective dose" or "prophylactically effective dose" also includes the amount of siRNA that provides any reasonable benefit-risk ratio acceptable for therapeutic purposes.

The targeted delivery ligand or other types of delivery carriers of the present invention are preferably GalNAc derivatives (GalNAc carriers). Other types of delivery carriers, such as liposomes that specifically deliver siRNA to hepatocytes, may also be used in the present invention as long as they can achieve siRNA delivery.

In recent years, GalNAc carriers have been extensively studied. GalNAc-nucleic acid is a monoconjugate formed by a carbohydrate with a nucleic acid. N-acetylated galactosamine is covalently conjugated to the 3'- and/or 5'-terminus of the sense strand of RNA of different sequences in a trivalent state to form a GalNAc-siRNA drug, thereby achieving specific delivery to hepatocytes and allowing the drug to enter the cells and exert its function through endocytosis.

All siRNAs in the present invention target human AGT, PCSK9, or ANGPTL3. In some embodiments, the use of hAGT, hPCSK9, or hANGPTL3 to denote the targets is intended to emphasize that the genes or proteins detected in AAV8-transfected mice or humanized mice are human AGT, human PCSK9, or human ANGPTL3.

In some of the following embodiments, the dual-target GalNAc ligand structure is as follows:

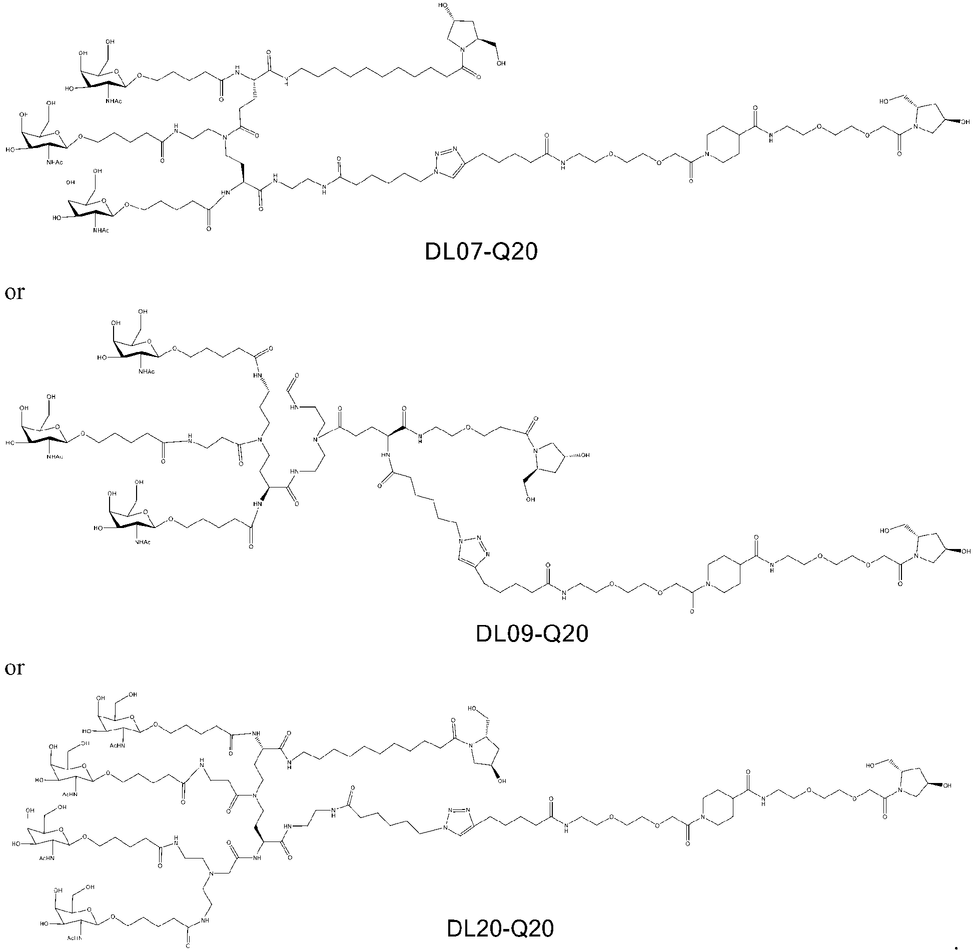

The single-target GalNAc ligand structure is as follows: 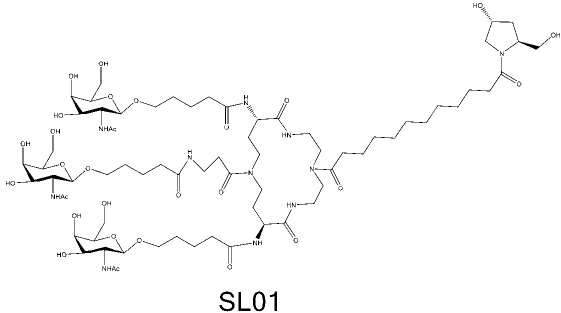 or

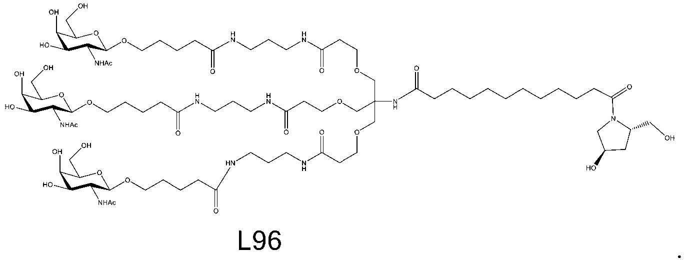

In some of the following embodiments, the structure of the dual-target GalNAc ligand conjugated to siRNA is as follows:

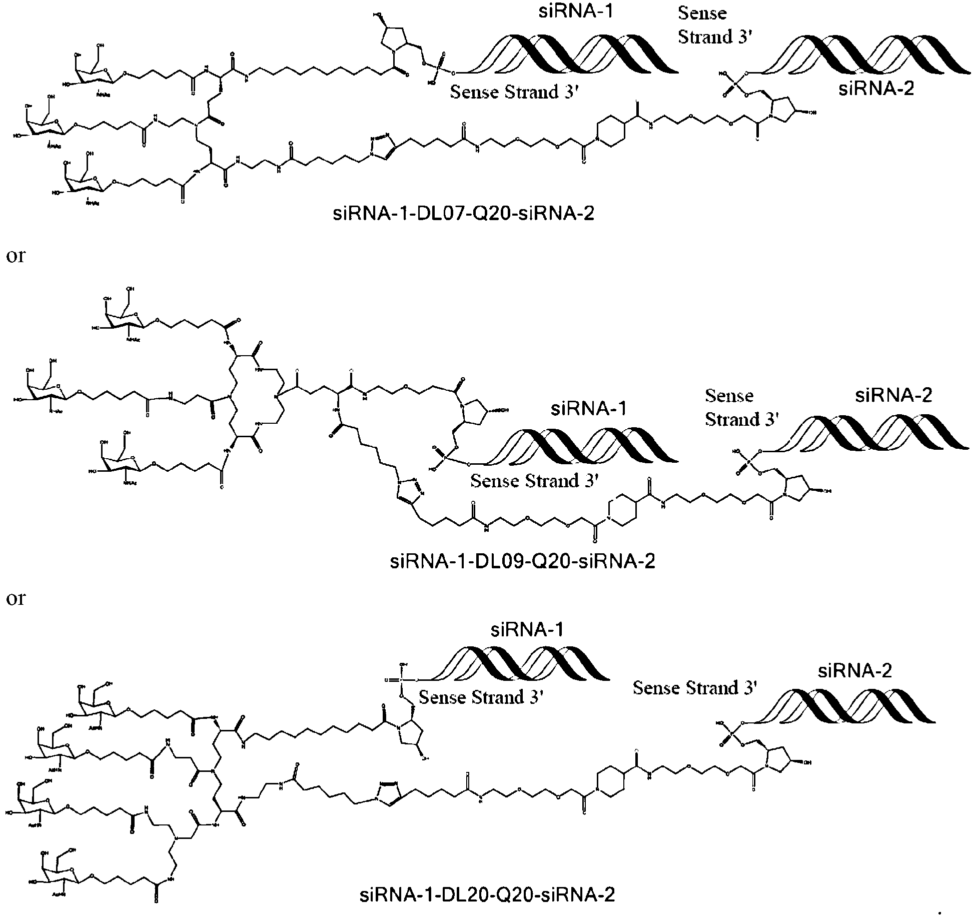

The structure of a single-target GalNAc ligand conjugated to siRNA is as follows:

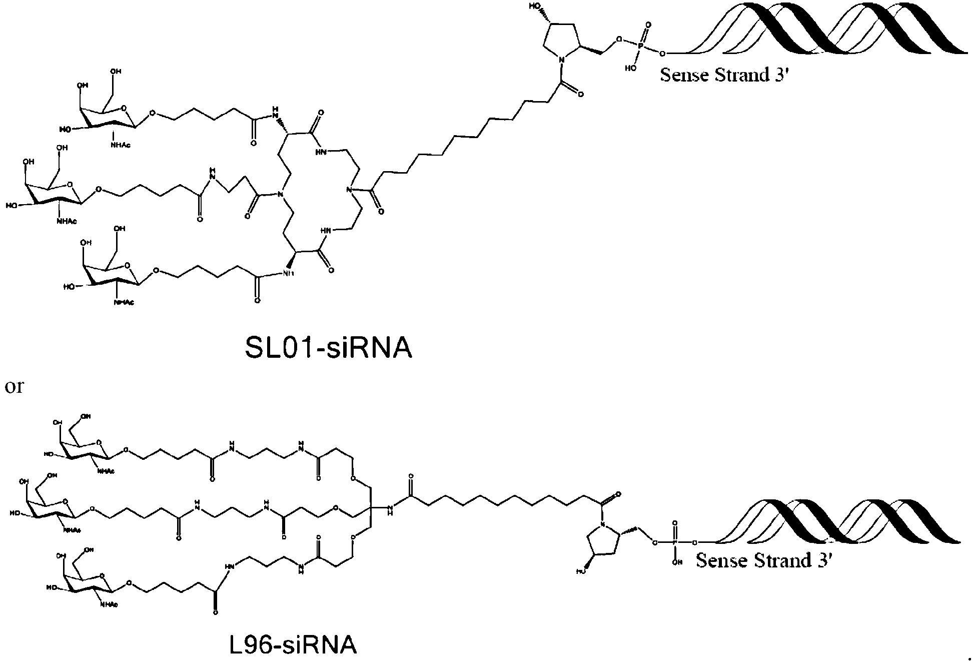

In the nucleic acid sequence listing of the present invention, the abbreviations and structures of the nucleotide monomers used are as follows:

| | |
|---|---|
| dA | 2'-Deoxyadenosine-3'-phosphate |
| dT | 2'-Deoxythymidine-3'-phosphate |
| dC | 2'-Deoxycytidine-3'-phosphate |
| dG | 2'-Deoxyguanosine-3'-phosphate |
| dU | 2'-Deoxyuridine-3'-phosphate |
| mA | 2'-O-methyladenosine-3'-phosphate |
| mC | 2'-O-Methylcytidine-3'-phosphate |
| mG | 2'-O-methylguanosine-3'-phosphate |
| mU | 2'-O-methyluridine-3'-phosphate |
| fA | 2'-Fluoroadenosine-3'-phosphate |
| fC | 2'-Fluorocytidine-3'-phosphate |
| fG | 2'-Fluoroguanosine-3'-phosphate |
| fU | 2'-Fluorouridine-3'-phosphate |
| VPU | 2'-O-methyluridine-5'-(E)-vinylphosphonate-3'-phosphate |
| VPU-S | 2'-S-methyluridine-5'-(E)-vinylphosphonate-3'-phosphate |
| Invab | Inverted abasic residue |
| * | Phosphorothioate modifications |
| isoGNA-A | Adenosine-isoglycerol nucleotide |
| isoGNA-T | Thymidine-isoglycerol nucleotide |
| isoGNA-C | Cytidine-isoglycerol nucleotide |
| isoGNA-G | Guanosine-isoglycerol nucleotide |

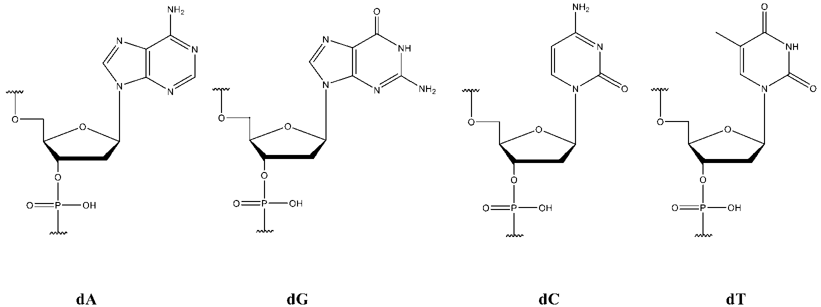

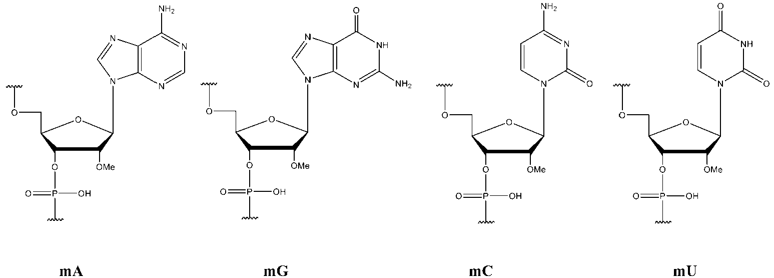

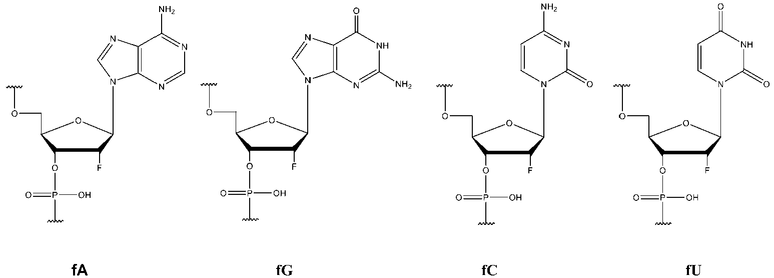

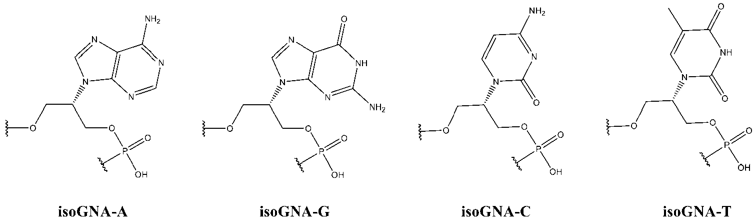

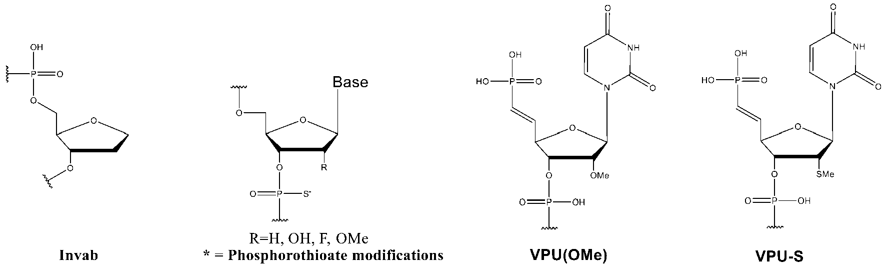

According to those skilled in the art, based on existing technology, the pharmaceutically acceptable salt of the siRNA may be a sodium salt or a potassium salt, such as the sodium salt generated during purification.

Human angiotensinogen (AGT) mRNA [NCBI Reference Sequence: NM_001384479.1].

Human proprotein convertase subtilisin/kexin type 9 (PCSK9) mRNA [NCBI Reference Sequence: NM_174936.4]:
Human angiopoietin-like protein 3 (ANGPTL3) mRNA [NCBI Reference Sequence: NM_014495.4].

The present invention will be further described in detail below with reference to specific embodiments.

### Embodiment 1 siRNA Targeting Human AGT (hAGT)

Oligonucleotide synthesis at a scale of 0.2-1 µmol was performed on a 12-channel nucleic acid synthesizer (Beijing Tsingke Biotech Co., Ltd.) using a solid-phase oligonucleotide synthesis protocol. The siRNA sequence needed to be synthesized on CPG pre-packed columns. Depending on requirements, either GalNAc-conjugated CPG or universal CPG was used. An ammonolysis reagent was added to the synthesized oligonucleotide and the mixture was incubated at 45-80°C to separate the oligonucleotide from the solid support, releasing it into solution. The crude oligonucleotide was then precipitated with ethanol. After high-speed centrifugation, the supernatant was discarded. This was repeated twice to give the crude oligonucleotide and the precipitate was resuspended in DEPC-treated water. The crude oligonucleotide was purified by ion-pairing HPLC, and the collected product was dried to powder in a vacuum centrifugal dryer. The purified product was dissolved in DEPC-treated water and analyzed by TOF LC-MS. The concentration of the oligonucleotide was determined. The volumes required for equimolar amounts of the sense and antisense strands were calculated. The equimolar amounts of the sense and antisense strands were mixed well and annealed (heated at 95°C for 5 min, then naturally cooled to room temperature) to prepare a duplex.

**Table 1: Sequences of Sense and Antisense Strands of Unmodified siRNA Targeting the AGT Gene**

| siRNA Name | Sense Strand Name | Sense Strand Sequence 5'-3' | Antisense Strand Name | Antisense Strand Sequence 5'-3' |
|---|---|---|---|---|
| 1131a | 1131a-SS | CGAGCUGAACCUGCAAAAAUU (SEQ ID NO: 1) | 1131a-AS | AAUUUUUGCAGGUUCAGCUCG GU (SEQ ID NO: 2) |
| 1158a | 1158a-SS | CUGGAGUGACAUCCAGGACAA (SEQ ID NO: 3) | 1158a-AS | UUGUCCUGGAUGUCACUCCAG UG (SEQ ID NO: 4) |
| 1159a | 1159a-SS | UGCUGUGUAUGAUCAAAGA (SEQ ID NO: 5) | 1159a-AS | UCUUUGAUCAUACACAGCAAA (SEQ ID NO: 6) |
| 1159b | 1159b-SS | CGCUGUGUAUGAUCAAAGA (SEQ ID NO: 7) | 1159b-AS | UCUUUGAUCAUACACAGCGAA (SEQ ID NO: 8 ) |
| 1159c | 1159c-SS | CUUGCUGUGUAUGAUCAAAGA (SEQ ID NO: 9 ) | 1159c-AS | UCUUUGAUCAUACACAGCAAG (SEQ ID NO: 10 ) |
| 1160a | 1160a-SS | CCAUUCCUGUUUGCUGUGA (SEQ ID NO: 11 ) | 1160a-AS | UCACAGCAAACAGGAAUGGGC (SEQ ID NO: 12 ) |
| 1161a | 1161a-SS | AAAACUCCCUCAACUGGAA (SEQ ID NO: 13) | 1161a-AS | UUCCAGUUGAGGGAGUUUUGC (SEQ ID NO: 14 ) |
| 1161b | 1161b-SS | GAAACUCCCUCAACUGGAA (SEQ ID NO: 15 ) | 1161b-AS | UUCCAGUUGAGGGAGUUUCGC (SEQ ID NO: 16 ) |
| 1161c | 1161c-SS | GCGAAACUCCCUCAACUGGAA (SEQ ID NO: 17) | 1161c-AS | UUCCAGUUGAGGGAGUUUCGC (SEQ ID NO: 18) |
| 1161d | 1161d-SS | GCAAAACUCCCUCAACUGGAA (SEQ ID NO: 19) | 1161d-AS | UUCCAGUUGAGGGAGUUUUGC(SEQ ID NO: 20 ) |
| 1161e | 1161e-SS | GAAACUCCCUCAACUGGAA (SEQ ID NO: 21 ) | 1161e-AS | UUCCAGUUGAGGGAGUUUUGG (SEQ ID NO: 22 ) |
| 1161f | 1161f-SS | GUAAAACUCCCUCAACUGGAA (SEQ ID NO: 23 ) | 1161f-AS | UUCCAGUUGAGGGAGUUUUGU (SEQ ID NO: 24 ) |
| 1161g | 1161g-SS | CCAAAACUCCCUCAACUGGAA (SEQ ID NO: 25 ) | 1161g-AS | UUCCAGUUGAGGGAGUUUUGG (SEQ ID NO: 26 ) |
| 1162a | 1162a-SS | UCCCUCAACUGGAUGAAGA (SEQ ID NO: 27 ) | 1162a-AS | UCUUCAUCCAGUUGAGGGAGU (SEQ ID NO: 28 ) |
| 1163a | 1163a-SS | CAUGCACAGUGAGCUAUGA (SEQ ID NO: 29 ) | 1163a-AS | UCAUAGCUCACUGUGCAUGUU (SEQ ID NO: 30 ) |
| 1164a | 1164a-SS | UAUAUGGCAUGCACAGUGA (SEQ ID NO: 31 ) | 1164a-AS | UCACUGUGCAUGCCAUAUAUA (SEQ ID NO: 32 ) |
| 1165a | 1165a-SS | AAGAACCAGUGUUUAGCGA (SEQ ID NO: 33 ) | 1165a-AS | UCGCUAAACACUGGUUCUUGC (SEQ ID NO: 34 ) |
| 1165b | 1165b-SS | GAGAACCAGUGUUUAGCGA (SEQ ID NO: 35 ) | 1165b-AS | UCGCUAAACACUGGUUCUCGC (SEQ ID NO: 36 ) |
| 1165c | 1165c-SS | GCGAGAACCAGUGUUUAGCGA (SEQ ID NO: 37 ) | 1165c-AS | UCGCUAAACACUGGUUCUCGC (SEQ ID NO: 38 ) |
| 1165d | 1165d-SS | GAGAACCAGUGUUUAGCGA (SEQ ID NO: 39 ) | 1165d-AS | UCGCUAAACACUGGUUCUUGG (SEQ ID NO: 40 ) |
| 1165e | 1165e-SS | GCAAGAACCAGUGUUUAGCGA (SEQ ID NO: 41 ) | 1165e-AS | UCGCUAAACACUGGUUCUUGC (SEQ ID NO: 42 ) |
| 1165f | 1165f-SS | CCAAGAACCAGUGUUUAGCGA (SEQ ID NO: 43 ) | 1165f-AS | UCGCUAAACACUGGUUCUUGG (SEQ ID NO: 44 ) |
| 1165g | 1165g-SS | GUAAGAACCAGUGUUUAGCGA (SEQ ID NO: 45 ) | 1165g-AS | UCGCUAAACACUGGUUCUUGU (SEQ ID NO: 46 ) |
| 1166a | 1166a-SS | GACUACUGUUCCAAAAAGA (SEQ ID NO: 47 ) | 1166a-AS | UCUUUUUGGAACAGUAGUCCC (SEQ ID NO: 48) |
| 1167a | 1167a-SS | CGACCAGCUUGUUUGUGAA (SEQ ID NO: 49 ) | 1167a-AS | UUCACAAACAAGCUGGUCGGU (SEQ ID NO: 50) |
| 1167b | 1167b-SS | GCCGACCAGCUUGUUUGUGAA (SEQ ID NO: 51) | 1167b-AS | UUCACAAACAAGCUGGUCGGC (SEQ ID NO: 52) |
| 1167c | 1167c-SS | GGACCAGCUUGUUUGUGAA (SEQ ID NO: 53) | 1167c-AS | UUCACAAACAAGCUGGUCCGU (SEQ ID NO: 54) |
| 1167d | 1167d-SS | GCCGACCAGCUUGUUUGUGAA (SEQ ID NO: 55) | 1167d-AS | UUCACAAACAAGCUGGUCGGC UG (SEQ ID NO: 56) |
| 1167e | 1167e-SS | GGACCAGCUUGUUUGUGAA (SEQ ID NO: 57) | 1167e-AS | UUCACAAACAAGCUGGUCCGG (SEQ ID NO: 58) |
| 1167f | 1167f-SS | UGACCAGCUUGUUUGUGAA (SEQ ID NO: 59) | 1167f-AS | UUCACAAACAAGCUGGUCGGG (SEQ ID NO: 60) |
| 1167g | 1167g-SS | UGACCAGCUUGUUUGUGAA (SEQ ID NO: 61) | 1167g-AS | UUCACAAACAAGCUGGUCGCC (SEQ ID NO: 62) |
| 1167h | 1167h-SS | UGACCAGCUUGUUUGUGAA (SEQ ID NO: 63) | 1167h-AS | UUCACAAACAAGCUGGUCGUU (SEQ ID NO: 64) |
| 1168a | 1168a-SS | CUGGGUUUAUUUUAGAGAA (SEQ ID NO: 65) | 1168a-AS | UUCUCUAAAAUAAACCCAGCA (SEQ ID NO: 66) |
| 1168b | 1168b-SS | CGCUGGGUUUAUUUUAGAGAA (SEQ ID NO: 67) | 1168b-AS | UUCUCUAAAAUAAACCCAGCG (SEQ ID NO: 68) |
| 1168c | 1168c-SS | CCGGGUUUAUUUUAGAGAA (SEQ ID NO: 69) | 1168c-AS | UUCUCUAAAAUAAACCCGGCA (SEQ ID NO: 70) |
| 1169a | 1169a-SS | UCUUGGGCUUCCGUAUAUA (SEQ ID NO: 71) | 1169a-AS | UAUAUACGGAAGCCCAAGAAG (SEQ ID NO: 72) |
| 1170a | 1170a-SS | CAGCAAAACUCCCUCAACA (SEQ ID NO: 73) | 1170a-AS | UGUUGAGGGAGUUUUGCUGGA (SEQ ID NO: 74) |
| 1171a | 1171a-SS | GUCUCACUUUCCAGCAAAA (SEQ ID NO: 75) | 1171a-AS | UUUUGCUGGAAAGUGAGACCC (SEQ ID NO: 76) |
| 1171b | 1171b-SS | GCCUCACUUUCCAGCAAAA(SEQ ID NO: 77) | 1171b-AS | UUUUGCUGGAAAGUGAGGCCC(SEQ ID NO: 78) |
| 1171c | 1171c-SS | CGGUCUCACUUUCCAGCAAAA (SEQ ID NO: 79) | 1171c-AS | UUUUGCUGGAAAGUGAGACCG (SEQ ID NO: 80) |
| 1172a | 1172a-SS | AUUCCUGUUUGCUGUGUAA (SEQ ID NO: 81) | 1172a-AS | UUACACAGCAAACAGGAAUGG (SEQ ID NO: 82) |
| 1172b | 1172b-SS | GUUCCUGUUUGCUGUGUAA (SEQ ID NO: 83) | 1172b-AS | UUACACAGCAAACAGGAACGG (SEQ ID NO: 84) |
| 1172c | 1172c-SS | CCACUCCUGUUUGCUGUGUAA (SEQ ID NO: 85) | 1172c-AS | UUACACAGCAAACAGGAGUGG (SEQ ID NO: 86) |
| 1172d | 1172d-SS | CCGUUCCUGUUUGCUGUGUAA (SEQ ID NO: 87) | 1172d-AS | UUACACAGCAAACAGGAGUGG (SEQ ID NO: 88) |
| 1172e | 1172e-SS | CCAUUCCUGUUUGCUGUGUAA (SEQ ID NO: 89) | 1172e-AS | UUACACAGCAAACAGGAAUGG (SEQ ID NO: 90) |
| 1172f | 1172f-SS | CCGUUCCUGUUUGCUGUGUAA (SEQ ID NO: 91) | 1172fAS | UUACACAGCAAACAGGAACGG (SEQ ID NO: 92) |
| 1172g | 1172g-SS | ACUCCUGUUUGCUGUGUAA (SEQ ID NO: 93) | 1172gAS | UUACACAGCAAACAGGAGUGG (SEQ ID NO: 94) |
| 1172k | 1172k-SS | CCGUUCCUGUUUGCUGUGUAA (SEQ ID NO: 95) | 1172k-AS | UUACACAGCAAACAGGAACGG GC (SEQ ID NO: 96) |
| 11721 | 11721-SS | GUUCCUGUU~GCUGUGUAA (SEQ ID NO: 97) | 11721-AS | UUACACAGCAAACAGGAACGG (SEQ ID NO: 98) |
| 1173a | 1173a-SS | CUUUUCAAGUUGAGAACAA (SEQ ID NO: 99) | 1173a-AS | UUGUUCUCAACUUGAAAAGGG (SEQ ID NO: 100) |
| 1174a | 1174a-SS | GGGUCUCACUUUCCAGCAA (SEQ ID NO: 101) | 1174a-AS | UUGCUGGAAAGUGAGACCCUC (SEQ ID NO: 102) |
| 1175a | 1175a-SS | GGUCUCACUUUCCAGCAAA (SEQ ID NO: 103) | 1175a-AS | UUUGCUGGAAAGUGAGACCCU (SEQ ID NO: 104) |
| 1176a | 1176a-SS | GGUGGAGGGUCUCACUUUA (SEQ ID NO: 105) | 1176a-AS | UAAAGUGAGACCCUCCACCUU (SEQ ID NO: 106) |
| 1176b | 1176b-SS | CGUGGAGGGUCUCACUUUA (SEQ ID NO: 107) | 1176b-AS | UAAAGUGAGACCCUCCACGUU (SEQ ID NO: 108) |
| 1176c | 1176c-SS | GAGGUGGAGGGUCUCACUUUA (SEQ ID NO: 109) | 1176c-AS | UAAAGUGAGACCCUCCACCUC (SEQ ID NO: 110) |
| 1177a | 1177a-SS | CCCUUGGUCUAAGUGUGCA (SEQ ID NO: 111) | 1177a-AS | UGCACACUUAGACCAAGGGGA (SEQ ID NO: 112) |
| 1177b | 1177b-SS | CCUCCUUGGUCUAAGUGUGCA (SEQ ID NO: 113) | 1177b-AS | UGCACACUUAGACCAAGGAGG (SEQ ID NO: 114) |
| 1178a | 1178a-SS | CGGUCUAAGUGUGCUGCAA (SEQ ID NO: 115) | 1178a-AS | UUGCAGCACACUUAGACCGAG (SEQ ID NO: 116) |
| 1178b | 1178b-SS | CCUGGUCUAAGUGUGCUGCAA (SEQ ID NO: 117) | 1178b-AS | UUGCAGCACACUUAGACCAGG (SEQ ID NO: 118) |
| 1179a | 1179a-SS | GGCAGCCGUUUCUCCUUGA (SEQ ID NO: 119) | 1179a-AS | UCAAGGAGAAACGGCUGCCUU (SEQ ID NO: 120) |
| 1179b | 1179b-SS | GAAGCAGCCGUUUCUCCUUGA (SEQ ID NO: 121) | 1179b-AS | UCAAGGAGAAACGGCUGCUUC (SEQ ID NO: 122) |
| 1180a | 1180a-SS | CCUAAUGAGUCGACUUUGA (SEQ ID NO: 123) | 1180a-AS | UCAAAGUCGACUCAUUAGGAG (SEQ ID NO: 124) |
| 1180b | 1180b-SS | CCUCUAAUGAGUCGACUUUGA (SEQ ID NO: 125) | 1180b-AS | UCAAAGUCGACUCAUUAGAGG (SEQ ID NO: 126) |
| 1181a | 1181a-SS | GAUUCCUGUUUGCUGUGUA (SEQ ID NO: 127) | 1181a-AS | UACACAGCAAACAGGAAUCGG (SEQ ID NO: 128) |
| 1181b | 1181b-SS | GCCAUUCCUGUUUGCUGUGUA (SEQ ID NO: 129) | 1181b-AS | UACACAGCAAACAGGAAUGGC (SEQ ID NO: 130) |
| 1182a | 1182a-SS | CUCUUCUAAUGAGUCGACA (SEQ ID NO: 131) | 1182a-AS | UGUCGACUCAUUAGAAGAGAA (SEQ ID NO: 132) |
| 1182b | 1182b-SS | CUUUCUUCUAAUGAGUCGACA (SEQ ID NO: 133) | 1182b-AS | UGUCGACUCAUUAGAAGAAAG (SEQ ID NO: 134) |
| 1182c | 1182c-SS | CUCUUCUAAUGAGUCGACA(SEQ ID NO: 135) | 1182c-AS | UGUCGACUCAUUAGAAGAGGG(SEQ ID NO: 136) |
| 1183a | 1183a-SS | CAGCGCGGGACUACUGUUCCA (SEQ ID NO: 137) | 1183a-AS | UGGAACAGUAGUCCCGCGCUG AA (SEQ ID NO: 138) |
| 1183b | 1183b-SS | CAGCGCGGGACUACUGUUCCA (SEQ ID NO: 139) | 1183b-AS | UGGAACAGUAGUCCCGCGCUG (SEQ ID NO: 140) |
| 1184a | 1184a-SS | CACUACUGUUCCAAAAAGA (SEQ ID NO: 141) | 1184a-AS | UCUUUUUGGAACAGUAGUGCC (SEQ ID NO: 142) |
| 1184b | 1184b-SS | CGGACUACUGUUCCAAAAAGA (SEQ ID NO: 143) | 1184b-AS | UCUUUUUGGAACAGUAGUCCG (SEQ ID NO: 144) |
| 1185a | 1185a-SS | GCGUUCCCUUUUCAAGUUGAA (SEQ ID NO: 145) | 1185a-AS | UUCAACUUGAAAGGAACGC UU (SEQ ID NO: 146) |
| 1185b | 1185b-SS | GCGUUCCCUUUUCAAGUUGAA (SEQ ID NO: 147) | 1185b-AS | UUCAACUUGAAAAGGGAACGC (SEQ ID NO: 148) |
| 1186a | 1186a-SS | GAGUGUUCCCUUUUCAAGUUA (SEQ ID NO: 149) | 1186a-AS | UAACUUGAAAGGAACACUC UU (SEQ ID NO: 150) |
| 1186b | 1186b-SS | GAGUGUUCCCUUUUCAAGUUA (SEQ ID NO: 151) | 1186b-AS | UAACUUGAAAAGGGAACACUC (SEQ ID NO: 152) |
| 1187a | 1187a-SS | GCGUUCCCUUUUCAAGUUA (SEQ ID NO: 153) | 1187a-AS | UAACUUGAAAAGGGAACGCUU (SEQ ID NO: 154) |
| 1188a | 1188a-SS | UUUUGAGCUUGAAGCGGAA (SEQ ID NO: 155) | 1188a-AS | UUCCGCUUCAAGCUCAAAAGG (SEQ ID NO: 156) |
| 1188b | 1188b-SS | CUUUUUGAGCUUGAAGCGGAA (SEQ ID NO: 157) | 1188b-AS | UUCCGCUUCAAGCUCAAAAAG (SEQ ID NO: 158) |
| 1189a | 1189a-SS | CGCAAAAAUUGAGCAAUGA (SEQ ID NO: 159) | 1189a-AS | UCAUUGCUCAAUUUUUGCGGG (SEQ ID NO: 160) |
| 1190a | 1190a-SS | GCUUUGAGCUGGAAAGCAA (SEQ ID NO: 161) | 1190a-AS | UUGCUUUCCAGCUCAAAGUGG (SEQ ID NO: 162) |
| 1191a | 1191a-SS | CUUUCAAGUUGAGAACAAA (SEQ ID NO: 163) | 1191a-AS | UUUGUUCUCAACUUGAAAGGG (SEQ ID NO: 164) |
| 1191b | 1191b-SS | CCCUUUCAAGUUGAGAACAAA (SEQ ID NO: 165) | 1191b-AS | UUUGUUCUCAACUUGAAAGGG (SEQ ID NO: 166) |
| 1191c | 1191c-SS | CCUUCAAGUUGAGAACAAA (SEQ ID NO: 167) | 1191c-AS | UUUGUUCUCAACUUGAAGG (SEQ ID NO: 168) |
| 1192a | 1192a-SS | CUCGGUUUGUAUUUAGUGA (SEQ ID NO: 169) | 1192a-AS | UCACUAAAUACAAACCGAGGG (SEQ ID NO: 170) |
| 1193a | 1193a-SS | GCCUUCGGUUUGUAUUUAA (SEQ ID NO: 171) | 1193a-AS | UUAAAUACAAACCGAAGGCGG (SEQ ID NO: 172) |
| 1194a | 1194a-SS | GCAUUGCCUUCGGUUUGUA (SEQ ID NO: 173) | 1194a-AS | UACAAACCGAAGGCAAUGCGG (SEQ ID NO: 174) |
| 1194b | 1194b-SS | CUGCAUUGCCUUCGGUUUGUA (SEQ ID NO: 175) | 1194b-AS | UACAAACCGAAGGCAAUGCAG (SEQ ID NO: 176) |
| 1195a | 1195a-SS | CUGUGUUAGUAAUAAACGA (SEQ ID NO: 177) | 1195a-AS | UCGUUUAUUACUAACACAGGG (SEQ ID NO: 178) |
| 1196a | 1196a-SS | GCGGAACCAUAGCUGGUUA (SEQ ID NO: 179) | 1196a-AS | UAACCAGCUAUGGUUCCGCGG (SEQ ID NO: 180) |
| 1197a | 1197a-SS | CCCGUGUAGUGUCUGUAAA (SEQ ID NO: 181) | 1197a-AS | UUUACAGACACUACACGGGGG (SEQ ID NO: 182) |
| 1198a | 1198a-SS | GCGACCAGCUUGUUUGUGA (SEQ ID NO: 183) | 1198a-AS | UCACAAACAAGCUGGUCGCUU (SEQ ID NO: 184) |
| 1199a | 1199a-SS | GCGGACAAAUCAGCGAUGA (SEQ ID NO: 185) | 1199a-AS | UCAUCGCUGAUUUGUCCGCGG (SEQ ID NO: 186) |
| 1200a | 1200a-SS | CCUAAUGAGUCGACUUUGA (SEQ ID NO: 187) | 1200a-AS | UCAAAGUCGACUCAUUAGGGG (SEQ ID NO: 188) |
| 1201a | 1201a-SS | CGCUGUGUAUGAUCAAAGA (SEQ ID NO: 189) | 1201a-AS | UCUUUGAUCAUACACAGCGGG (SEQ ID NO: 190) |
| 1202a | 1202a-SS | CUUUUGAGCUUGAAGCGGA (SEQ ID NO: 191) | 1202a-AS | UCCGCUUCAAGCUCAAAAGGG (SEQ ID NO: 192) |
| 1203a | 1203a-SS | GCGAGGAUCUUAUGACCUA(SEQ ID NO: 193) | 1203a-AS | UAGGUCAUAAGAUCCUUGCGG(SEQ ID NO: 194) |
| 1204a | 1204a-SS | AACUGGUGCUGCAAGGAUA (SEQ ID NO: 195) | 1204a-AS | UAUCCUUGCAGCACCAGUUGG (SEQ ID NO: 196) |
| 1205a | 1205a-SS | CGCUGGGUUUAUUUUAGAA (SEQ ID NO: 197) | 1205a-AS | UUCUAAAAUAAACCCAGCGGG (SEQ ID NO: 198) |
| 1206a | 1206a-SS | GCCCAUUCCUGUUUGCUGA (SEQ ID NO: 199) | 1206a-AS | UCAGCAAACAGGAAUGGGCGG (SEQ ID NO: 200) |
| 1207a | 1207a-SS | GACCGCCCAUUCCUGUUUA (SEQ ID NO: 201) | 1207a-AS | UAAACAGGAAUGGGCGGUUGG (SEQ ID NO: 202) |
| 1208a | 1208a-SS | GAGUGCCCUUCACUGAGAA (SEQ ID NO: 203) | 1208a-AS | UUCUCAGUGAAGGGCACUUGG (SEQ ID NO: 204) |
| 1209a | 1209a-SS | GCUUCUCGGUGACUCAAGA (SEQ ID NO: 205) | 1209a-AS | UCUUGAGUCACCGAGAAGUGG (SEQ ID NO: 206) |
| 1210a | 1210a-SS | GUGGCAGGAUGGAAGACUA (SEQ ID NO: 207) | 1210a-AS | UAGUCUUCCAUCCUGUCACGG (SEQ ID NO: 208) |
| 1211a | 1211a-SS | ACCCCGUCAUCCACAAUGA (SEQ ID NO: 209) | 1211a-AS | UCAUUGUGGAUGACGGGGUGG (SEQ ID NO: 210) |
| 1212a | 1212a-SS | GACCCCACCUUCAUACCUA (SEQ ID NO: 211) | 1212a-AS | UAGGUAUGAAGGUGGGGUCUU (SEQ ID NO: 212) |
| 1213a | 1213a-SS | GUCUGGACUUCACAGAACA (SEQ ID NO: 213) | 1213a-AS | UGUUCUGUGAAGUCCAGACGG (SEQ ID NO: 214) |
| 1214a | 1214a-SS | CCGCAAAAAUUGAGCAAUA (SEQ ID NO: 215) | 1214a-AS | UAUUGCUCAAUUUUUGCGGGU (SEQ ID NO: 216) |
| 1215a | 1215a-SS | CGAAGCGGAUGAGAGAGAA (SEQ ID NO: 217) | 1215a-AS | UUCUCUCUCAUCCGCUUCGGG (SEQ ID NO: 218) |
| 1216a | 1216a-SS | CGAGUCGACUUUGAGCUGA (SEQ ID NO: 219) | 1216a-AS | UCAGCUCAAAGUCGACUCGUU (SEQ ID NO: 220) |
| 1217a | 1217a-SS | CGGUCUAAGUGUGCUGCAA (SEQ ID NO: 221) | 1217a-AS | UUGCAGCACACUUAGACCGGG (SEQ ID NO: 222) |
| 1218a | 1218a-SS | GCUCCCUUUUCAAGUUGAA (SEQ ID NO: 223) | 1218a-AS | UUCAACUUGAAAAGGGAGCGG (SEQ ID NO: 224) |
| 1219a | 1219a-SS | CUCCACAGAUGCUUGUGAA (SEQ ID NO: 225) | 1219a-AS | UUCACAAGCAUCUGUGGAGGG (SEQ ID NO: 226) |
| 1220a | 1220a-SS | CGUGAUUUUUGAACAAUAA (SEQ ID NO: 227) | 1220a-AS | UUAUUGUUCAAAAAUCACGGG (SEQ ID NO: 228) |
| 1221a | 1221a-SS | GGAUUUCUGUUUGAAUGCA (SEQ ID NO: 229) | 1221a-AS | UGCAUUCAAACAGAAAUUCGG (SEQ ID NO: 230) |
| 1222a | 1222a-SS | GACCAGCUUGUUUGUGAAA (SEQ ID NO: 231) | 1222a-AS | UUUCACAAACAAGCUGGUCGG (SEQ ID NO: 232) |
| 1223a | 1223a-SS | ACCAGCUUGUUUGUGAAAA (SEQ ID NO: 233) | 1223a-AS | UUUUCACAAACAAGCUGGUCG (SEQ ID NO: 234) |
| 1224a | 1224a-SS | ACCGACCAGCUUGUUUGUA (SEQ ID NO: 235) | 1224a-AS | UACAAACAAGCUGGUCGGUUG (SEQ ID NO: 236) |
| 1225a | 1225a-SS | CUCCGUAUAUAUGGCAUGCAA (SEQ ID NO: 237) | 1225a-AS | UUGCAUGCCAUAUAUACGGAG (SEQ ID NO: 238) |
| 1226a | 1226a-SS | CCGUAUAUAUGGCAUGCAA (SEQ ID NO: 239) | 1226a-AS | UUGCAUGCCAUAUAUACGGGG (SEQ ID NO: 240) |
| 1227a | 1227a-SS | AGUUCUGGGUGGACAACAA (SEQ ID NO: 241) | 1227a-AS | UUGUUGUCCACCCAGAACUUU (SEQ ID NO: 242) |
| 1228a | 1228a-SS | CGCAUUGCCUUCGGUUUGA (SEQ ID NO: 243) | 1228a-AS | UCAAACCGAAGGCAAUGCGGG (SEQ ID NO: 244) |
| 1229a | 1229a-SS | CUUGCAUUGCCUUCGGUUUGA (SEQ ID NO: 245) | 1229a-AS | UCAAACCGAAGGCAAUGCAAG (SEQ ID NO: 246) |
| 1230a | 1230a-SS | GCGUUCCCUUUUCAAGUUGAA (SEQ ID NO: 247) | 1230a-AS | UUCAACUUGAAAAGGGAACGC (SEQ ID NO: 248) |
| 1231a | 1231a-SS | AGUGUUCCCUUUUCAAGUUGA (SEQ ID NO: 249) | 1231a-AS | UCAACUUGAAAAGGGAACACU (SEQ ID NO: 250) |

Next, we modified the siRNA to enhance its stability both *in vivo* and *in vitro,* increase its activity at the target site, and reduce its activity at non-target sites. Unless otherwise specified, L96 delivery was employed for both *in vivo* and *in vitro* screening of single-target sequences to more accurately reflect the efficacy of liver-targeted siRNA. The siRNA was connected to L96 via the 3'-terminus of the sense strand.

The dsRNA 1000PM (AD-85481), the most advanced AGT-targeting drug Zilebesiran, is currently undergoing Phase II clinical studies. Under patent No. US11015201B2, it served as the positive control sequence.

**Table 2: Sequences of Modified siRNA Targeting the AGT Gene**

| siRN A Nam e | Modified Sense Strand Name | Modified Sense Strand Sequence 5'-3' | Modified Antisense Strand Name | Modified Antisense Strand Sequence 5'-3' |
|---|---|---|---|---|
| 1000 PM | 1000PM-SM | mG*mU*mCmAmUmCfCmAfCfA fAmUmGmAmGmAmGmUmAm CmA | 1000PM-AM | mU*fG*mUmAmCGNA- TmCmUmCmAmUmUmGfUmGfG mAmUmGmAmC*mG*mA |
| 1131 .21-11 | 1131SM11 | mC*mG*mAmGmCmUfGmAfAf CdCmUmGmCmAmAmAmAmA mUmU | 1131AM21 | VPA*fA*mUmUmUmUmUmGmC mAmGdGmUfUmCfAmGmCmUm CmG*mG*mU |
| 1158 .1-1 | 1158SM1 | mC*mU*mGmGmAmGfUmGfAf CdAmUmCmCmAmGmGmAmC mAmA | 1158AM1 | VPU*fU*mGmUmCmCmUmGmG mAmUdGmUfCmAfCmUmCmCm AmG*mU*mG |
| 1159 .1-1 | 1159SM1 | mU*mG*mCmUfGmUfGfUdAmU mGmAmUmCmAmAmAmGmA | 1159AM1 | VPU*fC*mUmUmUmGmAmUmC mAmUdAmCfAmCfAmGmCmA* mA*mA |
| 1159 .2-2 | 1159SM2 | Invab*mC*mGmCmUfGmUfGfUd AmUmGmAmUmCmAmAmAmG mA | 1159AM2 | VPU*fC*mUmUmUmGmAmUmC mAmUdAmCfAmCfAmGmCmG* mA*mA |
| 1159 .4-10 | 1159SM10 | Invab*mC*mGmCmUmGmUfGfU dAmUfGmAmUmCmAmAmAmG mAInvab | 1159AM4 | VPU*fC*mUmUmUmGmAfUmCm AmUdAmCfAmCfAmGmCmG*m A*mA |
| 1159 .3-11 | 1159SM11 | Invab*mC*mUmUmGmCmUmGm UfGfUdAmUfGmAmUmCmAmA mAmGmAInvab | 1159AM3 | VPU*fC*mUmUmUmGmAmUmC mAmUdAmCfAmCfAmGmCmA* mA*mG |
| 1159 .5-11 | 1159SM11 | Invab*mC*mUmUmGmCmUmGm UfGfUdAmUfGmAmUmCmAmA mAmGmAInvab | 1159AM5 | VPU*fC*mUmUmUmGmAfUmCm AmUdAmCfAmCfAmGmCmA*m A*mG |
| 1160 .1-1 | 1160SM1 | mC*mC*mAmUfUmCfCfUdGmU mUmUmGmCmUmGmUmGmA | 1160AM1 | VPU*fC*mAmCmAmGmCmAmA mAmCdAmGfGmAfAmUmGmG* mG*mC |
| 1161 .1-1 | 1161SM1 | mA*mA*mAmAfCmUfCfCdCmU mCmAmAmCmUmGmGmAmA | 1161AM1 | VPU*fU*mCmCmAmGmUmUmG mAmGdGmGfAmGfUmUmUmU* mG*mC |
| 1161 .2-2 | 1161SM2 | Invab*mG*mAmAmAfCmUfCfCd CmUmCmAmAmCmUmGmGmA mA | 1161AM2 | VPU*fU*mCmCmAmGmUmUmG mAmGdGmGfAmGfUmUmUmC* mG*mC |
| 1161 .2-4 | 1161SM4 | Invab*mG*mCmGmAmAmAfCm UfCfCdCmUmCmAmAmCmUmG mGmAmA | 1161AM2 | VPU*fU*mCmCmAmGmUmUmG mAmGdGmGfAmGfUmUmUmC* mG*mC |
| 1161 .2-6 | 1161SM6 | mG*mA*mAmAmCmUfCmCfCm UfCmAmAmCmUmGmGmAmA | 1161AM2 | VPU*fU*mCmCmAmGmUmUmG mAmGdGmGfAmGfUmUmUmC* mG*mC |
| 1161 .2-3 | 1161SM3 | Invab*mG*mAmAmAfCmUfCfCd CmUmCmAmAmCmUmGmGmA mAInvab | 1161AM2 | VPU*fU*mCmCmAmGmUmUmG mAmGdGmGfAmGfUmUmUmC* mG*mC |
| 1161 .3-7 | 1161SM7 | Invab*mG*mAmAmAmCmUfCfC fCmUfCmAmAmCmUmGmGmA mAInvab | 1161AM3 | VPU*fU*mCmCmAmGmUfUmGm AmGdGmGfAmGfUmUmUmC*m G*mC |
| 1161 .2-7 | 1161SM7 | Invab*mG*mAmAmAmCmUfCfC fCmUfCmAmAmCmUmGmGmA mAInvab | 1161AM2 | VPU*fU*mCmCmAmGmUmUmG mAmGdGmGfAmGfUmUmUmC* mG*mC |
| 1161 .2-8 | 1161SM8 | Invab*mG*mAmAmAmCmUfCm CfCmUfCmAmAmCmUmGmGm AmAInvab | 1161AM2 | VPU*fU*mCmCmAmGmUmUmG mAmGdGmGfAmGfUmUmUmC* mG*mC |
| 1161 .3-8 | 1161SM8 | Invab*mG*mAmAmAmCmUfCm CfCmUfCmAmAmCmUmGmGm AmAInvab | 1161AM3 | VPU*fU*mCmCmAmGmUfUmGm AmGdGmGfAmGfUmUmUmC*m G*mC |
| 1161 .3-3 | 1161SM3 | Invab*mG*mAmAmAfCmUfCfCd CmUmCmAmAmCmUmGmGmA mAInvab | 1161AM3 | VPU*fU*mCmCmAmGmUfUmGm AmGdGmGfAmGfUmUmUmC*m G*mC |
| 1161 .2-5 | 1161SM5 | Invab*mG*mCmGmAmAmAfCm UfCfCdCmUmCmAmAmCmUmG mGmAmAInvab | 1161AM2 | VPU*fU*mCmCmAmGmUmUmG mAmGdGmGfAmGfUmUmUmC* mG*mC |
| 1161 .2-9 | 1161SM9 | Invab*mG*mCmGmAmAmAmCm UfCfCfCmUfCmAmAmCmUmGm GmAmAInvab | 1161AM2 | VPU*fU*mCmCmAmGmUmUmG mAmGdGmGfAmGfUmUmUmC* mG*mC |
| 1161 .2-10 | 1161SM10 | Invab*mG*mCmGmAmAmAmCm UfCmCfCmUfCmAmAmCmUmG mGmAmAInvab | 1161AM2 | VPU*fU*mCmCmAmGmUmUmG mAmGdGmGfAmGfUmUmUmC* mG*mC |
| 1161 .3-9 | 1161SM9 | Invab*mG*mCmGmAmAmAmCm UfCfCfCmUfCmAmAmCmUmGm GmAmAInvab | 1161AM3 | VPU*fU*mCmCmAmGmUfUmGm AmGdGmGfAmGfUmUmUmC*m G*mC |
| 1161 .3-10 | 1161SM10 | Invab*mG*mCmGmAmAmAmCm UfCmCfCmUfCmAmAmCmUmG mGmAmAInvab | 1161AM3 | VPU*fU*mCmCmAmGmUfUmGm AmGdGmGfAmGfUmUmUmC*m G*mC |
| 1161 .3-5 | 1161SM5 | Invab*mG*mCmGmAmAmAfCm UfCfCdCmUmCmAmAmCmUmG mGmAmAInvab | 1161AM3 | VPU*fU*mCmCmAmGmUfUmGm AmGdGmGfAmGfUmUmUmC*m G*mC |
| 1161 .2-11 | 1161SM11 | Invab*mG*mAmAmAmCmUfCfC dCmUfCmAmAmCmUmGmGmA mAInvab | 1161AM2 | VPU*fU*mCmCmAmGmUmUmG mAmGdGmGfAmGfUmUmUmC* mG*mC |
| 1161 .3-11 | 1161SM11 | Invab*mG*mAmAmAmCmUfCfC dCmUfCmAmAmCmUmGmGmA mAInvab | 1161AM3 | VPU*fU*mCmCmAmGmUfUmGm AmGdGmGfAmGfUmUmUmC*m G*mC |
| 1161 .2-12 | 1161SM12 | Invab*mG*mCmGmAmAmAmCm UfCfCdCmUfCmAmAmCmUmG mGmAmAInvab | 1161AM2 | VPU*fU*mCmCmAmGmUmUmG mAmGdGmGfAmGfUmUmUmC* mG*mC |
| 1161 .3-12 | 1161SM12 | Invab*mG*mCmGmAmAmAmCm UfCfCdCmUfCmAmAmCmUmG mGmAmAInvab | 1161AM3 | VPU*fU*mCmCmAmGmUfUmGm AmGdGmGfAmGfUmUmUmC*m G*mC |
| 1161 .4-13 | 1161SM13 | Invab*mG*mAmAmAmCmUfCfC fCfUmCfAmAmCmUmGmGmAm AInvab | 1161AM4 | VPU- S*fU*mCmCmAmGmUmUmGmA mGdGmGfAmGfUmUmUmC*mG* mC |
| 1161 .5-14 | 1161SM14 | Invab*mG*mCmAmAmAmAmCm UfCfCfCfUmCfAmAmCmUmGm GmAmAInvab | 1161AM5 | VPU- S*fU*mCmCmAmGmUmUmGmA mGdGmGfAmGfUmUmUmU*mG* mC |
| 1161 .6-15 | 1161SM15 | Invab*mG*mAmAmAmCmUfCfC fCfUmCfAmAmCmUmGmGmAm AInvab | 1161AM6 | mU*fU*mCmCmAmGmUmUmGm AmGdGmGfAmGfUmUmUmU*m G*mG |
| 1161 .7-15 | 1161SM15 | Invab*mG*mAmAmAmCmUfCfC fCfUmCfAmAmCmUmGmGmAm AInvab | 1161AM7 | VPU- S*fU*mCmCmAmGmUmUmGmA mGdGmGfAmGfUmUmUmU*mG* mG |
| 1161.8-16 | 1161SM16 | Invab*mG*mUmAmAmAmAmC mUfCfCfCfUmCfAmAmCmUmG mGmAmAInvab | 1161AM8 | VPU- S*fU*mCmCmAmGmUmUmGmA mGdGmGfAmGfUmUmUmU*mG* mU |
| 1161 .9-16 | 1161SM16 | Invab*mG*mUmAmAmAmAmC mUfCfCfCfUmCfAmAmCmUmG mGmAmAInvab | 1161AM9 | mU*fU*mCmCmAmGmUmUmGm AmGdGmGfAmGfUmUmUmU*m G*mU |
| 1161 .7-17 | 1161SM17 | Invab*mC*mCmAmAmAmAmCm UfCfCfCfUmCfAmAmCmUmGm GmAmAInvab | 1161AM7 | VPU- S*fU*mCmCmAmGmUmUmGmA mGdGmGfAmGfUmUmUmU*mG* mG |
| 1161 .6-17 | 1161SM17 | Invab*mC*mCmAmAmAmAmCm UfCfCfCfUmCfAmAmCmUmGm GmAmAInvab | 1161AM6 | mU*fU*mCmCmAmGmUmUmGm AmGdGmGfAmGfUmUmUmU*m G*mG |
| 1161 .10-13 | 1161SM13 | Invab*mG*mAmAmAmCmUfCfC fCfUmCfAmAmCmUmGmGmAm AInvab | 1161AM10 | mU*fU*mCmCmAmGmUmUmGm AmGdGmGfAmGfUmUmUmC*m G*mC |
| 1162 .1-1 | 1162SM1 | mU*mC*mCmCfUmCfAfAdCmU mGmGmAmUmGmAmAmGmA | 1162AM1 | VPU*fC*mUmUmCmAmUmCmC mAmGdTmUfGmAfGmGmGmA* mG*mU |
| 1163 .1-1 | 1163SM1 | mC*mA*mUmGfCmAfCfAdGmU mGmAmGmCmUmAmUmGmA | 1163AM1 | VPU*fC*mAmUmAmGmCmUmC mAmCdTmGfUmGfCmAmUmG* mU*mU |
| 1164 .1-1 | 1164SM1 | mU*mA*mUmAfUmGfGfCdAmU mGmCmAmCmAmGmUmGmA | 1164AM1 | VPU*fC*mAmCmUmGmUmGmC mAmUdGmCfCmAfUmAmUmA* mU*mA |
| 1165 .1-1 | 1165SM1 | mA*mA*mGmAfAmCfCfAdGmU mGmUmUmUmAmGmCmGmA | 1165AM1 | VPU*fC*mGmCmUmAmAmAmC mAmCdTmGfGmUfUmCmUmU* mG*mC |
| 1165 .2-2 | 1165SM2 | Invab*mG*mAmGmAfAmCfCfAd GmUmGmUmUmUmAmGmCmG mA | 1165AM2 | VPU*fC*mGmCmUmAmAmAmC mAmCdTmGfGmUfUmCmUmC*m G*mC |
| 1165 .2-3 | 1165SM3 | Invab*mG*mAmGmAfAmCfCfAd GmUmGmUmUmUmAmGmCmG mAInvab | 1165AM2 | VPU*fC*mGmCmUmAmAmAmC mAmCdTmGfGmUfUmCmUmC*m G*mC |
| 1165 .2-4 | 1165SM4 | Invab*mG*mCmGmAmGmAfAm CfCfAdGmUmGmUmUmUmAmG mCmGmA | 1165AM2 | VPU*fC*mGmCmUmAmAmAmC mAmCdTmGfGmUfUmCmUmC*m G*mC |
| 1165 .2-5 | 1165SM5 | Invab*mG*mCmGmAmGmAfAm CfCfAdGmUmGmUmUmUmAmG mCmGmAInvab | 1165AM2 | VPU*fC*mGmCmUmAmAmAmC mAmCdTmGfGmUfUmCmUmC*m G*mC |
| 1165 .2-6 | 1165SM6 | mG*mA*mGmAmAmCfCmAfGm UfGmUmUmUmAmGmCmGmA | 1165AM2 | VPU*fC*mGmCmUmAmAmAmC mAmCdTmGfGmUfUmCmUmC*m G*mC |
| 1165 .3-7 | 1165SM7 | Invab*mG*mAmGmAmAmCfCfA fGmUfGmUmUmUmAmGmCmG mAInvab | 1165AM3 | VPU*fC*mGmCmUmAmAfAmCm AmCdTmGfGmUfUmCmUmC*mG *mC |
| 1165 .2-7 | 1165SM7 | Invab*mG*mAmGmAmAmCfCfA fGmUfGmUmUmUmAmGmCmG mAInvab | 1165AM2 | VPU*fC*mGmCmUmAmAmAmC mAmCdTmGfGmUfUmCmUmC*m G*mC |
| 1165 .2-8 | 1165SM8 | Invab*mG*mAmGmAmAmCfCm AfGmUfGmUmUmUmAmGmCm GmAInvab | 1165AM2 | VPU*fC*mGmCmUmAmAmAmC mAmCdTmGfGmUfUmCmUmC*m G*mC |
| 1165 .3-8 | 1165SM8 | Invab*mG*mAmGmAmAmCfCm AfGmUfGmUmUmUmAmGmCm GmAInvab | 1165AM3 | VPU*fC*mGmCmUmAmAfAmCm AmCdTmGfGmUfUmCmUmC*mG *mC |
| 1165 .3-3 | 1165SM3 | Invab*mG*mAmGmAfAmCfCfAd GmUmGmUmUmUmAmGmCmG mAInvab | 1165AM3 | VPU*fC*mGmCmUmAmAfAmCm AmCdTmGfGmUfUmCmUmC*mG *mC |
| 1165.2-9 | 1165SM9 | Invab*mG*mCmGmAmGmAmA | 1165AM2 | VPU*fC*mGmCmUmAmAmAmC |
| | | mCfCfAfGmUfGmUmUmUmAm GmCmGmAInvab | | mAmCdTmGfGmUfUmCmUmC*m G*mC |
| 1165 .2-10 | 1165SM10 | Invab*mG*mCmGmAmGmAmA mCfCmAfGmUfGmUmUmUmAm GmCmGmAInvab | 1165AM2 | VPU*fC*mGmCmUmAmAmAmC mAmCdTmGfGmUfUmCmUmC*m G*mC |
| 1165 .3-9 | 1165SM9 | Invab*mG*mCmGmAmGmAmA mCfCfAfGmUfGmUmUmUmAm GmCmGmAInvab | 1165AM3 | VPU*fC*mGmCmUmAmAfAmCm AmCdTmGfGmUfUmCmUmC*mG *mC |
| 1165 .3-10 | 1165SM10 | Invab*mG*mCmGmAmGmAmA mCfCmAfGmUfGmUmUmUmAm GmCmGmAInvab | 1165AM3 | VPU*fC*mGmCmUmAmAfAmCm AmCdTmGfGmUfUmCmUmC*mG *mC |
| 1165 .3-5 | 1165SM5 | Invab*mG*mCmGmAmGmAfAm CfCfAdGmUmGmUmUmUmAmG mCmGmAInvab | 1165AM3 | VPU*fC*mGmCmUmAmAfAmCm AmCdTmGfGmUfUmCmUmC*mG *mC |
| 1165 .2-11 | 1165SM11 | Invab*mG*mAmGmAmAmCfCfA dGmUfGmUmUmUmAmGmCmG mAInvab | 1165AM2 | VPU*fC*mGmCmUmAmAmAmC mAmCdTmGfGmUfUmCmUmC*m G*mC |
| 1165 .3-11 | 1165SM11 | Invab*mG*mAmGmAmAmCfCfA dGmUfGmUmUmUmAmGmCmG mAInvab | 1165AM3 | VPU*fC*mGmCmUmAmAfAmCm AmCdTmGfGmUfUmCmUmC*mG *mC |
| 1165 .2-12 | 1165SM12 | Invab*mG*mCmGmAmGmAmA mCfCfAdGmUfGmUmUmUmAm GmCmGmAInvab | 1165AM2 | VPU*fC*mGmCmUmAmAmAmC mAmCdTmGfGmUfUmCmUmC*m G*mC |
| 1165 .3-12 | 1165SM12 | Invab*mG*mCmGmAmGmAmA mCfCfAdGmUfGmUmUmUmAm GmCmGmAInvab | 1165AM3 | VPU*fC*mGmCmUmAmAfAmCm AmCdTmGfGmUfUmCmUmC*mG *mC |
| 1165 .4-13 | 1165SM13 | Invab*mG*mAmGmAfAmCfCfAd GmUmGmUmUmUmAmGmCmG mAInvab | 1165AM4 | VPU*fC*mGmCmUmAmAmAmC mAmCdTmGfGmUfUmCmUmU* mG*mG |
| 1165 .5-14 | 1165SM14 | Invab*mG*mAmGmAmAmCfCfA fGfUmGfUmUmUmAmGmCmGm AInvab | 1165AM5 | VPU- S*fC*mGmCmUmAmAmAmCmA mCdTmGfGmUfUmCmUmU*mG* mG |
| 1165 .6-15 | 1165SM15 | Invab*mG*mCmAmAmGmAmA mCfCfAfGfUmGfUmUmUmAmG mCmGmAInvab | 1165AM6 | VPU- S*fC*mGmCmUmAmAmAmCmA mCdTmGfGmUfUmCmUmU*mG* mC |
| 1165 .7-14 | 1165SM14 | Invab*mG*mAmGmAmAmCfCfA fGfUmGfUmUmUmAmGmCmGm AInvab | 1165AM7 | mU*fC*mGmCmUmAmAmAmCm AmCdTmGfGmUfUmCmUmU*mG *mG |
| 1165 .5-16 | 1165SM16 | Invab*mC*mCmAmAmGmAmAm CfCfAfGfUmGfUmUmUmAmGm CmGmAInvab | 1165AM5 | VPU- S*fC*mGmCmUmAmAmAmCmA mCdTmGfGmUfUmCmUmU*mG* mG |
| 1165 .7-16 | 1165SM16 | Invab*mC*mCmAmAmGmAmAm CfCfAfGfUmGfUmUmUmAmGm CmGmAInvab | 1165AM7 | mU*fC*mGmCmUmAmAmAmCm AmCdTmGfGmUfUmCmUmU*mG *mG |
| 1165 .8-17 | 1165SM17 | Invab*mG*mUmAmAmGmAmA mCfCfAfGfUmGfUmUmUmAmG mCmGmAInvab | 1165AM8 | VPU- S*fC*mGmCmUmAmAmAmCmA mCdTmGfGmUfUmCmUmU*mG* mU |
| 1165 .9-17 | 1165SM17 | Invab*mG*mUmAmAmGmAmA mCfCfAfGfUmGfUmUmUmAmG mCmGmAInvab | 1165AM9 | mU*fC*mGmCmUmAmAmAmCm AmCdTmGfGmUfUmCmUmU*mG *mU |
| 1166 .1-1 | 1166SM1 | mG*mA*mCmUfAmCfUfGdTmU mCmCmAmAmAmAmAmGmA | 1166AM1 | VPU*fC*mUmUmUmUmUmGmG mAmAdCmAfGmUfAmGmUmC* mC*mC |
| 1167 .1-1 | 1167SM1 | mC*mG*mAmCfCmAfGfCdTmU mGmUmUmUmGmUmGmAmA | 1167AM1 | VPU*fU*mCmAmCmAmAmAmC mAmAdGmCfUmGfGmUmCmG* mG*mU |
| 1167 .2-2 | 1167SM2 | Invab*mG*mCmCmGmAmCfCm AfGfCdTmUmGmUmUmUmGmU mGmAmA | 1167AM2 | VPU*fU*mCmAmCmAmAmAmC mAmAdGmCfUmGfGmUmCmG* mG*mC |
| 1167 .3-3 | 1167SM3 | Invab*mG*mGmAmCfCmAfGfCd TmUmGmUmUmUmGmUmGmA mA | 1167AM3 | VPU*fU*mCmAmCmAmAmAmC mAmAdGmCfUmGfGmUmCmC* mG*mU |
| 1167 .2-4 | 1167SM4 | Invab*mG*mCmCmGmAmCfCm AfGfCdTmUmGmUmUmUmGmU mGmAmAInvab | 1167AM2 | VPU*fU*mCmAmCmAmAmAmC mAmAdGmCfUmGfGmUmCmG* mG*mC |
| 1167 .3-5 | 1167SM5 | Invab*mG*mGmAmCfCmAfGfCd TmUmGmUmUmUmGmUmGmA mAInvab | 1167AM3 | VPU*fU*mCmAmCmAmAmAmC mAmAdGmCfUmGfGmUmCmC* mG*mU |
| 1167 .3-6 | 1167SM6 | mG*mG*mAmCmCmAfGmCfUm UfGmUmUmUmGmUmGmAmA | 1167AM3 | VPU*fU*mCmAmCmAmAmAmC mAmAdGmCfUmGfGmUmCmC* mG*mU |
| 1167 .4-7 | 1167SM7 | Invab*mG*mGmAmCmCmAfGfC fUmUfGmUmUmUmGmUmGmA mAInvab | 1167AM4 | VPU*fU*mCmAmCmAmAfAmCm AmAdGmCfUmGfGmUmCmC*m G*mU |
| 1167 .3-7 | 1167SM7 | Invab*mG*mGmAmCmCmAfGfC fUmUfGmUmUmUmGmUmGmA mAInvab | 1167AM3 | VPU*fU*mCmAmCmAmAmAmC mAmAdGmCfUmGfGmUmCmC* mG*mU |
| 1167 .3-9 | 1167SM9 | Invab*mG*mGmAmCmCmAfGm CfUmUfGmUmUmUmGmUmGm AmAInvab | 1167AM3 | VPU*fU*mCmAmCmAmAmAmC mAmAdGmCfUmGfGmUmCmC* mG*mU |
| 1167 .4-9 | 1167SM9 | Invab*mG*mGmAmCmCmAfGm CfUmUfGmUmUmUmGmUmGm AmAInvab | 1167AM4 | VPU*fU*mCmAmCmAmAfAmCm AmAdGmCfUmGfGmUmCmC*m G*mU |
| 1167 .4-5 | 1167SM5 | Invab*mG*mGmAmCfCmAfGfCd TmUmGmUmUmUmGmUmGmA mAInvab | 1167AM4 | VPU*fU*mCmAmCmAmAfAmCm AmAdGmCfUmGfGmUmCmC*m G*mU |
| 1167 .2-12 | 1167SM12 | Invab*mG*mCmCmGmAmCmCm AfGfCfUmUfGmUmUmUmGmU mGmAmAInvab | 1167AM2 | VPU*fU*mCmAmCmAmAmAmC mAmAdGmCfUmGfGmUmCmG* mG*mC |
| 1167 .2-13 | 1167SM13 | Invab*mG*mCmCmGmAmCmCm AfGmCfUmUfGmUmUmUmGmU mGmAmAInvab | 1167AM2 | VPU*fU*mCmAmCmAmAmAmC mAmAdGmCfUmGfGmUmCmG* mG*mC |
| 1167 .5-12 | 1167SM12 | Invab*mG*mCmCmGmAmCmCm AfGfCfUmUfGmUmUmUmGmU mGmAmAInvab | 1167AM5 | VPU*fU*mCmAmCmAmAfAmCm AmAdGmCfUmGfGmUmCmG*m G*mC |
| 1167 .5-13 | 1167SM13 | Invab*mG*mCmCmGmAmCmCm AfGmCfUmUfGmUmUmUmGmU mGmAmAInvab | 1167AM5 | VPU*fU*mCmAmCmAmAfAmCm AmAdGmCfUmGfGmUmCmG*m G*mC |
| 1167 .5-4 | 1167SM4 | Invab*mG*mCmCmGmAmCfCm AfGfCdTmUmGmUmUmUmGmU mGmAmAInvab | 1167AM5 | VPU*fU*mCmAmCmAmAfAmCm AmAdGmCfUmGfGmUmCmG*m G*mC |
| 1167 .3-14 | 1167SM14 | Invab*mG*mGmAmCmCmAfGfC dTmUfGmUmUmUmGmUmGmA mAInvab | 1167AM3 | VPU*fU*mCmAmCmAmAmAmC mAmAdGmCfUmGfGmUmCmC* mG*mU |
| 1167 .4-14 | 1167SM14 | Invab*mG*mGmAmCmCmAfGfC dTmUfGmUmUmUmGmUmGmA mAInvab | 1167AM4 | VPU*fU*mCmAmCmAmAfAmCm AmAdGmCfUmGfGmUmCmC*m G*mU |
| 1167 .2-15 | 1167SM15 | Invab*mG*mCmCmGmAmCmCm AfGfCdTmUfGmUmUmUmGmU mGmAmAInvab | 1167AM2 | VPU*fU*mCmAmCmAmAmAmC mAmAdGmCfUmGfGmUmCmG* mG*mC |
| 1167 .5-15 | 1167SM15 | Invab*mG*mCmCmGmAmCmCm AfGfCdTmUfGmUmUmUmGmU mGmAmAInvab | 1167AM5 | VPU*fU*mCmAmCmAmAfAmCm AmAdGmCfUmGfGmUmCmG*m G*mC |
| 1167.6-14 | 1167SM14 | Invab*mG*mGmAmCmCmAfGfC dTmUfGmUmUmUmGmUmGmA mAInvab | 1167AM6 | VPU*fU*mCmAmCmAmAfAmCm AmAfGmCfUmGfGmUmCmC*mG *mU |
| 1167 .7-14 | 1167SM14 | Invab*mG*mGmAmCmCmAfGfC dTmUfGmUmUmUmGmUmGmA mAInvab | 1167AM7 | VPU*fU*mCmAmCmAmAfAmCm AmAdGmCfUmGfGmUfCmC*mG *mU |
| 1167 .9-14 | 1167SM14 | Invab*mG*mGmAmCmCmAfGfC dTmUfGmUmUmUmGmUmGmA mAInvab | 1167AM9 | VPU- S*fU*mCmAmCmAmAmAmCmA mAdGmCfUmGfGmUmCmC*mG* mU |
| 1167 .3-16 | 1167SM16 | Invab*mG*mGmAmCmCmAfGfC fUfUmGfUmUmUmGmUmGmAm AInvab | 1167AM3 | VPU*fU*mCmAmCmAmAmAmC mAmAdGmCfUmGfGmUmCmC* mG*mU |
| 1167 .11-15 | 1167SM15 | Invab*mG*mCmCmGmAmCmCm AfGfCdTmUfGmUmUmUmGmU mGmAmAInvab | 1167AM11 | VPU*fU*mCmAmCmAmAmAmC mAmAdGmCfUmGfGmUmCmGm GmC*mU*mG |
| 1167 .12-14 | 1167SM14 | Invab*mG*mGmAmCmCmAfGfC dTmUfGmUmUmUmGmUmGmA mAInvab | 1167AM12 | mU*fU*mCmAmCmAmAmAmCm AmAdGmCfUmGfGmUmCmC*m G*mU |
| 1167 .16-5 | 1167SM5 | Invab*mG*mGmAmCfCmAfGfCd TmUmGmUmUmUmGmUmGmA mAInvab | 1167AM16 | VPU*fU*mCmAmCmAmAmAmC mAmAdGmCfUmGfGmUmCmC* mG*mG |
| 1167 .16-14 | 1167SM14 | Invab*mG*mGmAmCmCmAfGfC dTmUfGmUmUmUmGmUmGmA mAInvab | 1167AM16 | VPU*fU*mCmAmCmAmAmAmC mAmAdGmCfUmGfGmUmCmC* mG*mG |
| 1167 .12-5 | 1167SM5 | Invab*mG*mGmAmCfCmAfGfCd TmUmGmUmUmUmGmUmGmA mAInvab | 1167AM12 | mU*fU*mCmAmCmAmAmAmCm AmAdGmCfUmGfGmUmCmC*m G*mU |
| 1167 .9-5 | 1167SM5 | Invab*mG*mGmAmCfCmAfGfCd TmUmGmUmUmUmGmUmGmA mAInvab | 1167AM9 | VPU- S*fU*mCmAmCmAmAmAmCmA mAdGmCfUmGfGmUmCmC*mG* mU |
| 1167 .17-17 | 1167SM17 | Invab*mU*mGmAmCfCmAfGfCd TmUmGmUmUmUmGmUmGmA mAInvab | 1167AM17 | VPU*fU*mCmAmCmAmAmAmC mAmAdGmCfUmGfGmUmCmG* mG*mG |
| 1167 .18-18 | 1167SM18 | Invab*mC*mGmAmCfCmAfGfCd TmUmGmUmUmUmGmUmGmA mAInvab | 1167AM18 | mU*fU*mCmAmCmAmAmAmCm AmAdGmCfUmGfGmUmCmG*m G*mU |
| 1167 .11-4 | 1167SM4 | Invab*mG*mCmCmGmAmCfCm AfGfCdTmUmGmUmUmUmGmU mGmAmAInvab | 1167AM11 | VPU*fU*mCmAmCmAmAmAmC mAmAdGmCfUmGfGmUmCmGm GmC*mU*mG |
| 1167 .16-16 | 1167SM16 | Invab*mG*mGmAmCmCmAfGfC fUfUmGfUmUmUmGmUmGmAm AInvab | 1167AM16 | VPU*fU*mCmAmCmAmAmAmC mAmAdGmCfUmGfGmUmCmC* mG*mG |
| 1167 .19-5 | 1167SM5 | Invab*mG*mGmAmCfCmAfGfCd TmUmGmUmUmUmGmUmGmA mAInvab | 1167AM19 | VPU*fU*mCmAmCmAUNA- AmAmCmAmAdGmCfUmGfGmU mCmC*mG*mU |
| 1167 .17-19 | 1167SM19 | Invab*mU*mGmAmCmCmAfGfC fUfUmGfUmUmUmGmUmGmAm AInvab | 1167AM17 | VPU*fU*mCmAmCmAmAmAmC mAmAdGmCfUmGfGmUmCmG* mG*mG |
| 1167 .20-5 | 1167SM5 | Invab*mG*mGmAmCfCmAfGfCd TmUmGmUmUmUmGmUmGmA mAInvab | 1167AM20 | mU*fU*mCmAmCmAmAmAmCm AmAdGmCfUmGfGmUmCmC*m G*mG |
| 1167 .21-5 | 1167SM5 | Invab*mG*mGmAmCfCmAfGfCd TmUmGmUmUmUmGmUmGmA mAInvab | 1167AM21 | VPU- S*fU*mCmAmCmAmAmAmCmA mAdGmCfUmGfGmUmCmC*mG* mG |
| 1167 .22-19 | 1167SM19 | Invab*mU*mGmAmCmCmAfGfC fUfUmGfUmUmUmGmUmGmAm AInvab | 1167AM22 | VPU*fU*mCmAmCmAmAmAmC mAmAdGmCfUmGfGmUmCmG* mC*mC |
| 1167 .23-19 | 1167SM19 | Invab*mU*mGmAmCmCmAfGfC fUfUmGfUmUmUmGmUmGmAm AInvab | 1167AM23 | VPU*fU*mCmAmCmAmAmAmC mAmAdGmCfUmGfGmUmCmG* mU*mU |
| 1167 .24-19 | 1167SM19 | Invab*mU*mGmAmCmCmAfGfC fUfUmGfUmUmUmGmUmGmAm AInvab | 1167AM24 | VPU*fU*mCmAmCmAmAmAmC mAmAdGmCfUmGfGmUmCmG*d T*dT |
| 1167 .25-19 | 1167SM19 | Invab*mU*mGmAmCmCmAfGfC fUfUmGfUmUmUmGmUmGmAm AInvab | 1167AM25 | VPU- S*fU*mCmAmCmAmAmAmCmA mAdGmCfUmGfGmUmCmG*mG* mG |
| 1167 .17-20 | 1167SM20 | Invab*mU*mGmAmCfCmAfGfCf UmUmGmUmUmUmGmUmGmA mAInvab | 1167AM17 | VPU*fU*mCmAmCmAmAmAmC mAmAdGmCfUmGfGmUmCmG* mG*mG |
| 1167 .26-19 | 1167SM19 | Invab*mU*mGmAmCmCmAfGfC fUfUmGfUmUmUmGmUmGmAm AInvab | 1167AM26 | mU*fU*mCmAmCmAmAmAmCm AmAdGmCfUmGfGmUmCmG*m G*mG |
| 1167 .27-21 | 1167SM21 | Invab*mG*mCmCmGmAmCmCm AfGfCfUfUmGfUmUmUmGmUm GmAmAInvab | 1167AM27 | VPU- S*fU*mCmAmCmAmAmAmCmA mAdGmCfUmGfGmUmCmG*mG* mC |
| 1168 .1-1 | 1168SM1 | mC*mU*mGmGfGmUfUfUdAmU mUmUmUmAmGmAmGmAmA | 1168AM1 | VPU*fU*mCmUmCmUmAmAmA mAmUdAmAfAmCfCmCmAmG* mC*mA |
| 1168 .2-2 | 1168SM2 | Invab*mC*mGmCmUmGmGfGm UfUfUdAmUmUmUmUmAmGm AmGmAmA | 1168AM2 | VPU*fU*mCmUmCmUmAmAmA mAmUdAmAfAmCfCmCmAmG* mC*mG |
| 1168 .3-3 | 1168SM3 | Invab*mC*mCmGmGfGmUfUfUd AmUmUmUmUmAmGmAmGmA mA | 1168AM3 | VPU*fU*mCmUmCmUmAmAmA mAmUdAmAfAmCfCmCmGmG* mC*mA |
| 1169 .1-1 | 1169SM1 | mU*mC*mUmUfGmGfGfCdTmU mCmCmGmUmAmUmAmUmA | 1169AM1 | VPU*fA*mUmAmUmAmCmGmG mAmAdGmCfCmCfAmAmGmA* mA*mG |
| 1170 .1-1 | 1170SM1 | mC*mA*mGmCfAmAfAfAdCmU mCmCmCmUmCmAmAmCmA | 1170AM1 | VPU*fG*mUmUmGmAmGmGmG mAmGdTmUfUmUfGmCmUmG* mG*mA |
| 1171 .1-1 | 1171SM1 | mG*mU*mCmUfCmAfCfUdTmU mCmCmAmGmCmAmAmAmA | 1171AM1 | VPU*fU*mUmUmGmCmUmGmG mAmAdAmGfUmGfAmGmAmC* mC*mC |
| 1171 .2-2 | 1171SM2 | Invab*mG*mCmCmUfCmAfCfUd TmUmCmCmAmGmCmAmAmA mA | 1171AM2 | VPU*fU*mUmUmGmCmUmGmG mAmAdAmGfUmGfAmGmGmC* mC*mC |
| 1171 .4-11 | 1171SM11 | Invab*mG*mCmCmUmCmAfCfU dTmUfCmCmAmGmCmAmAmA mAInvab | 1171AM4 | VPU*fU*mUmUmGmCmUfGmGm AmAdAmGfUmGfAmGmGmC*m C*mC |
| 1172 .1-1 | 1172SM1 | mA*mU*mUmCfCmUfGfUdTmU mGmCmUmGmUmGmUmAmA | 1172AM1 | VPU*fU*mAmCmAmCmAmGmC mAmAdAmCfAmGfGmAmAmU* mG*mG |
| 1172 .2-2 | 1172SM2 | mG*mU*mUmCfCmUfGfUdTmU mGmCmUmGmUmGmUmAmA | 1172AM2 | VPU*fU*mAmCmAmCmAmGmC mAmAdAmCfAmGfGmAmAmC* mG*mG |
| 1172 .2-9 | 1172SM9 | Invab*mG*mUmUmCfCmUfGfUd TmUmGmCmUmGmUmGmUmA mA | 1172AM2 | VPU*fU*mAmCmAmCmAmGmC mAmAdAmCfAmGfGmAmAmC* mG*mG |
| 1172 .3-13 | 1172SM13 | Invab*mC*mCmGmUmUmCfCm UfGfUdTmUmGmCmUmGmUmG mUmAmA | 1172AM3 | VPU*fU*mAmCmAmCmAmGmC mAmAdAmCfAmGfGmAmGmU* mG*mG |
| 1172 .2-17 | 1172SM17 | Invab*mG*mUmUmCfCmUfGfUd TmUmGmCmUmGmUmGmUmA mAInvab | 1172AM2 | VPU*fU*mAmCmAmCmAmGmC mAmAdAmCfAmGfGmAmAmC* mG*mG |
| 1172 .9-28 | 1172SM28 | Invab*mG*mUmUmCmCmUfGfU dTmUfGmCmUmGmUmGmUmA mAInvab | 1172AM9 | VPU*fU*mAmCmAmCmAfGmCm AmAdAmCfAmGfGmAmAmC*m G*mG |
| 1173 .1-1 | 1173SM1 | mC*mU*mUmUfUmCfAfAdGmU mUmGmAmGmAmAmCmAmA | 1173AM1 | VPU*fU*mGmUmUmCmUmCmA mAmCdTmUfGmAfAmAmAmG* mG*mG |
| 1174 .1-1 | 1174SM1 | mG*mG*mGmUfCmUfCfAdCmU mUmUmCmCmAmGmCmAmA | 1174AM1 | VPU*fU*mGmCmUmGmGmAmA mAmGdTmGfAmGfAmCmCmC*m U*mC |
| 1175 .1-1 | 1175SM1 | mG*mG*mUmCfUmCfAfCdTmU mUmCmCmAmGmCmAmAmA | 1175AM1 | VPU*fU*mUmGmCmUmGmGmA mAmAdGmUfGmAfGmAmCmC* mC*mU |
| 1176 .1-1 | 1176SM1 | mG*mG*mUmGfGmAfGfGdGmU mCmUmCmAmCmUmUmUmA | 1176AM1 | VPU*fA*mAmAmGmUmGmAmG mAmCdCmCfUmCfCmAmCmC*m U*mU |
| 1177 .1-1 | 1177SM1 | Invab*mC*mCmCmUfUmGfGfUd CmUmAmAmGmUmGmUmGmC mAInvab | 1177AM1 | VPU*fG*mCmAmCmAmCmUmU mAmGdAmCfCmAfAmGmGmG* mG*mA |
| 1178 .1-1 | 1178SM1 | Invab*mC*mGmGmUfCmUfAfAd GmUmGmUmGmCmUmGmCmA mAInvab | 1178AM1 | VPU*fU*mGmCmAmGmCmAmC mAmCdTmUfAmGfAmCmCmG*m A*mG |
| 1179 .1-1 | 1179SM1 | Invab*mG*mGmCmAfGmCfCfGd TmUmUmCmUmCmCmUmUmG mAInvab | 1179AM1 | VPU*fC*mAmAmGmGmAmGmA mAmAdCmGfGmCfUmGmCmC* mU*mU |
| 1180 .1-1 | 1180SM1 | Invab*mC*mCmUmAfAmUfGfAd GmUmCmGmAmCmUmUmUmG mAInvab | 1180AM1 | VPU*fC*mAmAmAmGmUmCmG mAmCdTmCfAmUfUmAmGmG* mA*mG |
| 1181 .1-1 | 1181SM1 | Invab*mG*mAmUmUfCmCfUfGd TmUmUmGmCmUmGmUmGmU mAInvab | 1181AM1 | VPU*fA*mCmAmCmAmGmCmA mAmAdCmAfGmGfAmAmUmC* mG*mG |
| 1182 .1-1 | 1182SM1 | Invab*mC*mUmCmUfUmCfUfAd AmUmGmAmGmUmCmGmAmC mAInvab | 1182AM1 | VPU*fG*mUmCmGmAmCmUmC mAmUdTmAfGmAfAmGmAmG* mA*mA |
| 1182 .5-10 | 1182SM10 | Invab*mC*mUmCmUfUmCfUfAd AmUmGmAmGmUmCmGmAmC mAInvab | 1182AM5 | VPU*fG*mUmCmGmAmCmUmC mAmUdTmAfGmAfAmGmAmG* mG*mG |
| 1183 .1-1 | 1183SM1 | Invab*mC*mAmGmCmGmCfGm GfGfAdCmUmAmCmUmGmUmU mCmCmAInvab | 1183AM1 | VPU*fG*mGmAmAmCmAmGmU mAmGdTmCfCmCfGmCmGmCm UmG*mA*mA |
| 1184 .1-1 | 1184SM1 | Invab*mC*mAmCmUfAmCfUfGd TmUmCmCmAmAmAmAmAmG mAInvab | 1184AM1 | VPU*fC*mUmUmUmUmUmGmG mAmAdCmAfGmUfAmGmUmG* mC*mC |
| 1184 .1-2 | 1184SM2 | mC*mA*mCmUmAmCfUmGfUm UfCmCmAmAmAmAmAmGmA | 1184AM1 | VPU*fC*mUmUmUmUmUmGmG mAmAdCmAfGmUfAmGmUmG* mC*mC |
| 1184 .2-3 | 1184SM3 | Invab*mC*mAmCmUmAmCfUfG fUmUfCmCmAmAmAmAmAmG mAInvab | 1184AM2 | VPU*fC*mUmUmUmUmUfGmGm AmAdCmAfGmUfAmGmUmG*m C*mC |
| 1185 .1-1 | 1185SM1 | Invab*mG*mCmGmUmUmCfCm CfUfUdTmUmCmAmAmGmUmU mGmAmAInvab | 1185AM1 | VPU*fU*mCmAmAmCmUmUmG mAmAdAmAfGmGfGmAmAmCm GmC*mU*mU |
| 1186 .1-1 | 1186SM1 | Invab*mG*mAmGmUmGmUfUm CfCfCdTmUmUmUmCmAmAmG mUmUmAInvab | 1186AM1 | VPU*fA*mAmCmUmUmGmAmA mAmAdGmGfGmAfAmCmAmCm UmC*mU*mU |
| 1187 .1-1 | 1187SM1 | Invab*mG*mCmGmUfUmCfCfCd TmUmUmUmCmAmAmGmUmU mAInvab | 1187AM1 | VPU*fA*mAmCmUmUmGmAmA mAmAdGmGfGmAfAmCmGmC* mU*mU |
| 1188 .1-1 | 1188SM1 | Invab*mU*mUmUmUfGmAfGfCd TmUmGmAmAmGmCmGmGmA mAInvab | 1188AM1 | VPU*fU*mCmCmGmCmUmUmC mAmAdGmCfUmCfAmAmAmA* mG*mG |
| 1188 .2-1 | 1188SM1 | Invab*mU*mUmUmUfGmAfGfCd TmUmGmAmAmGmCmGmGmA mAInvab | 1188AM2 | mU*fU*mCmCmGmCmUmUmCm AmAdGmCfUmCfAmAmAmA*m G*mG |
| 1188 .3-2 | 1188SM2 | Invab*mU*mUmUmUmGmAfGfC fUfUmGfAmAmGmCmGmGmAm AInvab | 1188AM3 | VPU- S*fU*mCmCmGmCmUmUmCmA mAdGmCfUmCfAmAmAmA*mG* mG |
| 1188 .4-3 | 1188SM3 | Invab*mC*mUmUmUmUmUmG mAfGfCfUfUmGfAmAmGmCmG mGmAmAInvab | 1188AM4 | VPU- S*fU*mCmCmGmCmUmUmCmA mAdGmCfUmCfAmAmAmA*mA* mG |
| 1189 .1-1 | 1189SM1 | Invab*mC*mGmCmAfAmAfAfAd TmUmGmAmGmCmAmAmUmG mAInvab | 1189AM1 | VPU*fC*mAmUmUmGmCmUmC mAmAdTmUfUmUfUmGmCmG* mG*mG |
| 1190 .1-1 | 1190SM1 | Invab*mG*mCmUmUfUmGfAfGd CmUmGmGmAmAmAmGmCmA mAInvab | 1190AM1 | VPU*fU*mGmCmUmUmUmCmC mAmGdCmUfCmAfAmAmGmU* mG*mG |
| 1191 .1-1 | 1191SM1 | Invab*mC*mUmUmUfCmAfAfGd TmUmGmAmGmAmAmCmAmA mAInvab | 1191AM1 | VPU*fU*mUmGmUmUmCmUmC mAmAdCmUfUmGfAmAmAmG* mG*mG |
| 1191 .2-1 | 1191SM1 | Invab*mC*mUmUmUfCmAfAfGd TmUmGmAmGmAmAmCmAmA mAInvab | 1191AM2 | mU*fU*mUmGmUmUmCmUmCm AmAdCmUfUmGfAmAmAmG*m G*mG |
| 1191 .4-1 | 1191SM1 | Invab*mC*mUmUmUfCmAfAfGd TmUmGmAmGmAmAmCmAmA mAInvab | 1191AM4 | VPU- S*fU*mUmGmUmUmCmUmCmA mAdCmUfUmGfAmAmAmG*mG* mG |
| 1191 .1-2 | 1191SM2 | Invab*mC*mCmCmUmUmUfCm AfAfGdTmUmGmAmGmAmAmC mAmAmAInvab | 1191AM1 | VPU*fU*mUmGmUmUmCmUmC mAmAdCmUfUmGfAmAmAmG* mG*mG |
| 1191 .2-2 | 1191SM2 | Invab*mC*mCmCmUmUmUfCm AfAfGdTmUmGmAmGmAmAmC mAmAmAInvab | 1191AM2 | mU*fU*mUmGmUmUmCmUmCm AmAdCmUfUmGfAmAmAmG*m G*mG |
| 1191 .5-3 | 1191SM3 | Invab*mC*mCmUmUfCmAfAfGd TmUmGmAmGmAmAmCmAmA mAInvab | 1191AM5 | VPU*fU*mUmGmUmUmCmUmC mAmAdCmUfUmGfAmA*mG*mG |
| 1191 .1-4 | 1191SM4 | Invab*mC*mUmUmUmCmAfAfG fUfUmGfAmGmAmAmCmAmAm AInvab | 1191AM1 | VPU*fU*mUmGmUmUmCmUmC mAmAdCmUfUmGfAmAmAmG* mG*mG |
| 1191 .1-5 | 1191SM5 | Invab*mC*mUmUmUfCmAfAfGf UmUmGmAmGmAmAmCmAmA mAInvab | 1191AM1 | VPU*fU*mUmGmUmUmCmUmC mAmAdCmUfUmGfAmAmAmG* mG*mG |
| 1191 .2-4 | 1191SM4 | Invab*mC*mUmUmUmCmAfAfG fUfUmGfAmGmAmAmCmAmAm AInvab | 1191AM2 | mU*fU*mUmGmUmUmCmUmCm AmAdCmUfUmGfAmAmAmG*m G*mG |
| 1191 .4-4 | 1191SM4 | Invab*mC*mUmUmUmCmAfAfG fUfUmGfAmGmAmAmCmAmAm AInvab | 1191AM4 | VPU- S*fU*mUmGmUmUmCmUmCmA mAdCmUfUmGfAmAmAmG*mG* mG |
| 1191 .4-6 | 1191SM6 | Invab*mC*mCmCmUmUmUmCm AfAfGfUfUmGfAmGmAmAmCm AmAmAInvab | 1191AM4 | VPU- S*fU*mUmGmUmUmCmUmCmA mAdCmUfUmGfAmAmAmG*mG* mG |
| 1191 .4-2 | 1191SM2 | Invab*mC*mCmCmUmUmUfCm AfAfGdTmUmGmAmGmAmAmC mAmAmAInvab | 1191AM4 | VPU- S*fU*mUmGmUmUmCmUmCmA mAdCmUfUmGfAmAmAmG*mG* mG |
| 1191.6-4 | 1191SM4 | Invab*mC*mUmUmUmCmAfAfG fUfUmGfAmGmAmAmCmAmAm AInvab | 1191AM6 | VPU-S*fU*mUfGmUisoGNA- TmCfUmCfAmAfCmUfUmGfAmA mAmG*mG*mG |
| 1191 .6-6 | 1191SM6 | Invab*mC*mCmCmUmUmUmCm AfAfGfUfUmGfAmGmAmAmCm AmAmAInvab | 1191AM6 | VPU-S*fU*mUfGmUisoGNA- TmCfUmCfAmAfCmUfUmGfAmA mAmG*mG*mG |
| 1192 .1-1 | 1192SM1 | Invab*mC*mUmCmGfGmUfUfUd GmUmAmUmUmUmAmGmUmG mAInvab | 1192AM1 | VPU*fC*mAmCmUmAmAmAmU mAmCdAmAfAmCfCmGmAmG* mG*mG |
| 1193 .1-1 | 1193SM1 | Invab*mG*mCmCmUfUmCfGfGd TmUmUmGmUmAmUmUmUmA mAInvab | 1193AM1 | VPU*fU*mAmAmAmUmAmCmA mAmAdCmCfGmAfAmGmGmC* mG*mG |
| 1194 .1-1 | 1194SM1 | Invab*mG*mCmAmUfUmGfCfCd TmUmCmGmGmUmUmUmGmU mAInvab | 1194AM1 | VPU*fA*mCmAmAmAmCmCmG mAmAdGmGfCmAfAmUmGmC* mG*mG |
| 1194 .2-1 | 1194SM1 | Invab*mG*mCmAmUfUmGfCfCd TmUmCmGmGmUmUmUmGmU mAInvab | 1194AM2 | mU*fA*mCmAmAmAmCmCmGm AmAdGmGfCmAfAmUmGmC*m G*mG |
| 1194 .4-2 | 1194SM2 | Invab*mG*mCmAmUmUmGfCfC fUfUmCfGmGmUmUmUmGmUm AInvab | 1194AM4 | VPU- S*fA*mCmAmAmAmCmCmGmA mAdGmGfCmAfAmUmGmC*mG* mG |
| 1194 .5-3 | 1194SM3 | Invab*mC*mUmGmCmAmUmUm GfCfCfUfUmCfGmGmUmUmUm GmUmAInvab | 1194AM5 | VPU- S*fA*mCmAmAmAmCmCmGmA mAdGmGfCmAfAmUmGmC*mA* mG |
| 1195 .1-1 | 1195SM1 | Invab*mC*mUmGmUfGmUfUfAd GmUmAmAmUmAmAmAmCmG mAInvab | 1195AM1 | VPU*fC*mGmUmUmUmAmUmU mAmCdTmAfAmCfAmCmAmG*m G*mG |
| 1196 .1-1 | 1196SM1 | Invab*mG*mCmGmGfAmAfCfCd AmUmAmGmCmUmGmGmUmU mAInvab | 1196AM1 | VPU*fA*mAmCmCmAmGmCmU mAmUdGmGfUmUfCmCmGmC* mG*mG |
| 1197 .1-1 | 1197SM1 | Invab*mC*mCmCmGfUmGfUfAd GmUmGmUmCmUmGmUmAmA mAInvab | 1197AM1 | VPU*fU*mUmAmCmAmGmAmC mAmCdTmAfCmAfCmGmGmG*m G*mG*mG |
| 1198 .1-1 | 1198SM1 | Invab*mG*mCmGmAfCmCfAfGd CmUmUmGmUmUmUmGmUmG mAInvab | 1198AM1 | VPU*fC*mAmCmAmAmAmCmA mAmGdCmUfGmGfUmCmGmC* mU*mU |
| 1199 .1-1 | 1199SM1 | Invab*mG*mCmGmGfAmCfAfAd AmUmCmAmGmCmGmAmUmG mAInvab | 1199AM1 | VPU*fC*mAmUmCmGmCmUmG mAmUdTmUfGmUfCmCmGmC*m G*mG |
| 1200 .1-1 | 1200SM1 | Invab*mC*mCmUmAfAmUfGfAd GmUmCmGmAmCmUmUmUmG mAInvab | 1200AM1 | VPU*fC*mAmAmAmGmUmCmG mAmCdTmCfAmUfUmAmGmG* mG*mG |
| 1201 .1-1 | 1201SM1 | Invab*mC*mGmCmUfGmUfGfUd AmUmGmAmUmCmAmAmAmG mAInvab | 1201AM1 | VPU*fC*mUmUmUmGmAmUmC mAmUdAmCfAmCfAmGmCmG* mG*mG |
| 1202 .1-1 | 1202SM1 | Invab*mC*mUmUmUfUmGfAfGd CmUmUmGmAmAmGmCmGmG mAInvab | 1202AM1 | VPU*fC*mCmGmCmUmUmCmA mAmGdCmUfCmAfAmAmAmG* mG*mG |
| 1203 .1-1 | 1203SM1 | Invab*mG*mCmGmAfGmGfAfUd CmUmUmAmUmGmAmCmCmU mAInvab | 1203AM1 | VPU*fA*mGmGmUmCmAmUmA mAmGdAmUfCmCfUmUmGmC* mG*mG |
| 1204 .1-1 | 1204SM1 | Invab*mA*mAmCmUfGmGfUfGd CmUmGmCmAmAmGmGmAmU mAInvab | 1204AM1 | VPU*fA*mUmCmCmUmUmGmC mAmGdCmAfCmCfAmGmUmU* mG*mG |
| 1205 .1-1 | 1205SM1 | Invab*mC*mGmCmUfGmGfGfUd TmUmAmUmUmUmUmAmGmA mAInvab | 1205AM1 | VPU*fU*mCmUmAmAmAmAmU mAmAdAmCfCmCfAmGmCmG* mG*mG |
| 1206.1-1 | 1206SM1 | Invab*mG*mCmCmCfAmUfUfCd CmUmGmUmUmUmGmCmUmG mAInvab | 1206AM1 | VPU*fC*mAmGmCmAmAmAmC mAmGdGmAfAmUfGmGmGmC* mG*mG |
| 1207 .1-1 | 1207SM1 | Invab*mG*mAmCmCfGmCfCfCd AmUmUmCmCmUmGmUmUmU mAInvab | 1207AM1 | VPU*fA*mAmAmCmAmGmGmA mAmUdGmGfGmCfGmGmUmU* mG*mG |
| 1208 .1-1 | 1208SM1 | Invab*mG*mAmGmUfGmCfCfCd TmUmCmAmCmUmGmAmGmA mAInvab | 1208AM1 | VPU*fU*mCmUmCmAmGmUmG mAmAdGmGfGmCfAmCmUmU* mG*mG |
| 1209 .1-1 | 1209SM1 | Invab*mG*mCmUmUfCmUfCfGd GmUmGmAmCmUmCmAmAmG mAInvab | 1209AM1 | VPU*fC*mUmUmGmAmGmUmC mAmCdCmGfAmGfAmAmGmU* mG*mG |
| 1210 .1-1 | 1210SM1 | Invab*mG*mUmGmGfCmAfGfGd AmUmGmGmAmAmGmAmCmU mAInvab | 1210AM1 | VPU*fA*mGmUmCmUmUmCmC mAmUdCmCfUmGfUmCmAmC* mG*mG |
| 1211 .1-1 | 1211SM1 | Invab*mA*mCmCmCfCmGfUfCd AmUmCmCmAmCmAmAmUmG mAInvab | 1211AM1 | VPU*fC*mAmUmUmGmUmGmG mAmUdGmAfCmGfGmGmGmU* mG*mG |
| 1212 .1-1 | 1212SM1 | Invab*mG*mAmCmCfCmCfAfCd CmUmUmCmAmUmAmCmCmU mAInvab | 1212AM1 | VPU*fA*mGmGmUmAmUmGmA mAmGdGmUfGmGfGmGmUmC* mU*mU |
| 1213 .1-1 | 1213SM1 | Invab*mG*mUmCmUfGmGfAfCd TmUmCmAmCmAmGmAmAmC mAInvab | 1213AM1 | VPU*fG*mUmUmCmUmGmUmG mAmAdGmUfCmCfAmGmAmC* mG*mG |
| 1214 .1-1 | 1214SM1 | Invab*mC*mCmGmCfAmAfAfAd AmUmUmGmAmGmCmAmAmU mAInvab | 1214AM1 | VPU*fA*mUmUmGmCmUmCmA mAmUdTmUfUmUfGmCmGmG* mG*mU |
| 1215 .1-1 | 1215SM1 | Invab*mC*mGmAmAfGmCfGfGd AmUmGmAmGmAmGmAmGmA mAInvab | 1215AM1 | VPU*fU*mCmUmCmUmCmUmC mAmUdCmCfGmCfUmUmCmG* mG*mG |
| 1216 .1-1 | 1216SM1 | Invab*mC*mGmAmGfUmCfGfAd CmUmUmUmGmAmGmCmUmG mAInvab | 1216AM1 | VPU*fC*mAmGmCmUmCmAmA mAmGdTmCfGmAfCmUmCmG*m U*mU |
| 1217 .1-1 | 1217SM1 | Invab*mC*mGmGmUfCmUfAfAd GmUmGmUmGmCmUmGmCmA mAInvab | 1217AM1 | VPU*fU*mGmCmAmGmCmAmC mAmCdTmUfAmGfAmCmCmG*m G*mG |
| 1218 .1-1 | 1218SM1 | Invab*mG*mCmUmCfCmCfUfUd TmUmCmAmAmGmUmUmGmA mAInvab | 1218AM1 | VPU*fU*mCmAmAmCmUmUmG mAmAdAmAfGmGfGmAmGmC* mG*mG |
| 1219 .1-1 | 1219SM1 | Invab*mC*mUmCmCfAmCfAfGd AmUmGmCmUmUmGmUmGmA mAInvab | 1219AM1 | VPU*fU*mCmAmCmAmAmGmC mAmUdCmUfGmUfGmGmAmG* mG*mG |
| 1220 .1-1 | 1220SM1 | Invab*mC*mGmUmGfAmUfUfUd TmUmGmAmAmCmAmAmUmA mAInvab | 1220AM1 | VPU*fU*mAmUmUmGmUmUmC mAmAdAmAfAmUfCmAmCmG* mG*mG |
| 1221 .1-1 | 1221SM1 | Invab*mG*mGmAmUfUmUfCfUd GmUmUmUmGmAmAmUmGmC mAInvab | 1221AM1 | VPU*fG*mCmAmUmUmCmAmA mAmCdAmGfAmAfAmUmUmC* mG*mG |
| 1222 .1-1 | 1222SM1 | Invab*mG*mAmCmCfAmGfCfUd TmGmUmUmUmGmUmGmAmA mAInvab | 1222AM1 | VPU*fU*mUmCmAmCmAmAmA mCmAdAmGfCmUfGmGmUmC* mG*mG |
| 1223 .1-1 | 1223SM1 | Invab*mA*mCmCmAfGmCfUfUd GmUmUmUmGmUmGmAmAmA mAInvab | 1223AM1 | VPU*fU*mUmUmCmAmCmAmA mAmCdAmAfGmCfUmGmGmU* mC*mG |
| 1224 .1-1 | 1224SM1 | Invab*mA*mCmCmGfAmCfCfAd GmCmUmUmGmUmUmUmGmU mAInvab | 1224AM1 | VPU*fA*mCmAmAmAmCmAmA mGmCdTmGfGmUfCmGmGmU* mU*mG |
| 1225 .1-1 | 1225SM1 | Invab*mC*mUmCmCmGmUfAm UfAfUdAmUmGmGmCmAmUm GmCmAmAInvab | 1225AM1 | VPU*fU*mGmCmAmUmGmCmC mAmUdAmUfAmUfAmCmGmG* mA*mG |
| 1225 .2-2 | 1225SM2 | Invab*mC*mUmCmCmGmUmAm UfAfUfAfUmGfGmCmAmUmGm CmAmAInvab | 1225AM2 | VPU- S*fU*mGmCmAmUmGmCmCmA mUdAmUfAmUfAmCmGmG*mA* mG |
| 1226 .1-1 | 1226SM1 | Invab*mC*mCmGmUfAmUfAfUd AmUmGmGmCmAmUmGmCmA mAInvab | 1226AM1 | VPU*fU*mGmCmAmUmGmCmC mAmUdAmUfAmUfAmCmGmG* mG*mG |
| 1226 .2-2 | 1226SM2 | Invab*mC*mCmGmUmAmUfAfU fAfUmGfGmCmAmUmGmCmAm AInvab | 1226AM2 | VPU- S*fU*mGmCmAmUmGmCmCmA mUdAmUfAmUfAmCmGmG*mG* mG |
| 1227 .1-1 | 1227SM1 | Invab*mA*mGmUmUfCmUfGfGd GmUmGmGmAmCmAmAmCmA mAInvab | 1227AM1 | VPU*fU*mGmUmUmGmUmCmC mAmCdCmCfAmGfAmAmCmU* mU*mU |
| 1228 .1-1 | 1228SM1 | Invab*mC*mGmCmAfUmUfGfCd CmUmUmCmGmGmUmUmUmG mAInvab | 1228AM1 | VPU*fC*mAmAmAmCmCmGmA mAmGdGmCfAmAfUmGmCmG* mG*mG |
| 1228 .2-2 | 1228SM2 | Invab*mC*mGmCmAmUmUfGfC fCfUmUfCmGmGmUmUmUmGm AInvab | 1228AM2 | VPU- S*fC*mAmAmAmCmCmGmAmA mGdGmCfAmAfUmGmCmG*mG* mG |
| 1229 .1-1 | 1229SM1 | Invab*mC*mUmUmGmCmAfUm UfGfCdCmUmUmCmGmGmUmU mUmGmAInvab | 1229AM1 | VPU*fC*mAmAmAmCmCmGmA mAmGdGmCfAmAfUmGmCmA* mA*mG |
| 1229 .2-2 | 1229SM2 | Invab*mC*mUmUmGmCmAmUm UfGfCfCfUmUfCmGmGmUmUm UmGmAInvab | 1229AM2 | VPU- S*fC*mAmAmAmCmCmGmAmA mGdGmCfAmAfUmGmCmA*mA* mG |
| 1230 .1-1 | 1230SM1 | Invab*mG*mCmGmUmUmCfCm CfUfUdTmUmCmAmAmGmUmU mGmAmAInvab | 1230AM1 | VPU*fU*mCmAmAmCmUmUmG mAmAdAmAfGmGfGmAmAmC* mG*mC |
| 1231 .1-1 | 1231SM1 | Invab*mA*mGmUmGmUmUfCm CfCfUdTmUmUmCmAmAmGmU mUmGmAInvab | 1231AM1 | VPU*fC*mAmAmCmUmUmGmA mAmAdAmGfGmGfAmAmCmA* mC*mU |

### Embodiment 2 In Vitro Screening of siRNAs Targeting AGT

### Experimental Method 1: In Vitro siRNA Screening Using Liposome-mediated Transfection in Hep3B Cells

Cell culture and 96-well plate transfection: *In vitro* experiments were conducted in Hep3B cells using MEM + 10% FBS + 1X penicillin-streptomycin + 1X non-essential amino acids medium. When cells reached 80% confluence, they were digested with trypsin. Cell density was determined using a Scepter automated cell counter (Millipore, #PHCC00000). Concurrently, siRNA, Opti-MEM, and INTERFERin (Polyplus) were mixed in a 96-well plate, and the plate was incubated at room temperature for 10 min. Then, complete medium containing Hep3B cells was added to each well. The 96-well plate was incubated at 37°C in a 5% CO₂ incubator for 24 h.

96-well plate RNA extraction and reverse transcription: mRNA was extracted from cells in the 96-well plate using Oligo d(T)25 Magnetic Beads reagent (NEB). The culture medium was aspirated from the 96-well plate, and the wells were washed once with DPBS. Then, 100 µL of cell lysate was added to each well, followed by 20 µL of beads. The plate was shaken on an oscillator and then placed on a magnetic separation rack. The lysate was aspirated from the wells. Then, 100 µL of wash buffer A was added to each well, pipetted up and down, and the plate was placed back on the magnetic separation rack. Wash buffer A was aspirated. The beads were then pipetted up with 100 µL of wash buffer B and transferred to a new 96-well plate. The plate was placed on the magnetic separation rack, wash buffer B was aspirated, and the beads were pipetted up with 100 µL of low-salt buffer and transferred to a 96-well PCR plate. The 96-well PCR plate was placed on the magnetic separation rack, low-salt buffer was aspirated, and 10 µL of elution buffer was added to each well to pipette up the beads. The plate was incubated at 50°C for 2 min to elute mRNA from the beads. The reverse transcription system was prepared using the StarScript Pro One-Tube Degenomic Reverse Transcription Premix (Genstar); 5 µL was dispensed into each well of a 96-well PCR plate, 5 µL of the mRNA solution obtained in the previous step was added, the mixture was mixed thoroughly, briefly centrifuged, and the plate was sealed with a sealing film. 9) The plate was incubated on the PCR instrument at 37°C for 3 min, then at 50°C for 50 min, followed by 85°C for 2 min. Then, it was cooled to 4°C to complete reverse transcription.

Real-time fluorescent quantitative PCR: After reverse transcription, the 96-well plate was placed on the magnetic separation rack until all beads were adsorbed to the bottom. The reverse transcription reagent was aspirated. The prepared QPCR system was added to the 96-well PCR plate. The plate was sealed with sealing film, and PCR was performed on a StepOnePlus Real-Time PCR System (Applied Biosystems). Data were analyzed using the ΔΔCt method and normalized using cells transfected with the negative control sequence at the same concentration.

The negative control AD-1955 sequence is as follows:
Sense strand: CUUACGCUGAGUACUUCGAdTdT (SEQ ID NO: 703)
Antisense strand: UCGAAGUACUCAGCGUAAGdTdT (SEQ ID NO: 704).

The primers for detecting AGT are as follows:
Forward primer: ATTCTGCACACCGAGCTGAA (SEQ ID NO: 705)
Reverse primer: TCAAGCTCAAAAAAAATGCTGTTC (SEQ ID NO: 706)
Probe: CTGCAAAAATTGAGCAATGACCGCATC (SEQ ID NO: 707) (Reporter gene 5'FAM, Quencher group 3'MGB)

Experimental Method 2: *In vitro* screening was performed in primary hepatocytes from humanized AGT mice (purchased from Gempharmatech) or mice infected with AAV8 virus expressing both human AGT (hAGT) and ANGPTL3 (hANGPTL3).

Generation of mice infected with AAV8 virus stably expressing both hAGT and hANGPTL3 (AAV8-hAGT/hANGPTL3 mice): Transgenic mice stably expressing both hAGT and hANGPTL3 were generated by infecting mice with 1×10¹¹ titer ultra-purified recombinant AAV8-hAGT and 1×10¹¹ titer ultra-purified recombinant AAV8-hANGPTL3 viral particles.

Isolation of mouse primary hepatocytes: Mouse hepatocytes were isolated by collagenase digestion via inferior vena cava perfusion. Mouse primary hepatocytes with good viability were obtained by filtration through a cell strainer (BIOLOGIX, 15-1070). Cells were resuspended in DMEM supplemented with 10% FBS and 1X penicillin-streptomycin, and cell density was determined using a Scepter automated cell counter.

siRNA transfection, RNA extraction, reverse transcription, and real-time fluorescent quantitative PCR methods are identical to Method 1. siRNA delivery via free uptake does not require the addition of INTERFERin.

**Table 3: Experimental Results of 0.1 nM Modified siRNA Transfection via Liposome in Hep3B Cells**

| siRNA | Residual AGT mRNA Level (%) | |
|---|---|---|
| | Mean | SD |
| 1000PM | 8.4 | 1.1 |
| 1131.21-11 | 5.3 | 0.6 |
| 1158.1-1 | 79.7 | 1.9 |
| 1159.1-1 | 17.2 | 1.8 |
| 1160.1-1 | 32.6 | 2.4 |
| 1161.1-1 | 11.3 | 1.2 |
| 1162.1-1 | 52.6 | 7.8 |
| 1163.1-1 | 12.1 | 0.7 |
| 1164.1-1 | 44.6 | 5.5 |
| 1165.1-1 | 10.5 | 1.4 |
| 1166.1-1 | 16.9 | 1.0 |
| 1167.1-1 | 9.3 | 1.0 |
| 1168.1-1 | 7.9 | 0.9 |
| 1169.1-1 | 79.3 | 13.5 |
| 1170.1-1 | 21.7 | 5.0 |
| 1171.1-1 | 13.2 | 2.3 |
| 1172.1-1 | 4.4 | 0.5 |
| 1173.1-1 | 22.4 | 2.8 |
| 1174.1-1 | 22.3 | 2.4 |
| 1175.1-1 | 17.4 | 3.5 |
| 1176.1-1 | 23.3 | 2.1 |

We conducted activity screening of some sequences targeting AGT using Hep3B cells. As shown in Table 3, at a concentration of 0.1 nM, multiple sequences, including 1131.21-11, 1165.1-1, 1167.1-1, and 1172.1-1, exhibited activity comparable to or superior to that of the positive control.

**Table 4: Experimental Results of 1 nM Modified siRNA Delivered via L96 in Humanized AGT Mouse Primary Hepatocytes**

| siRNA | Residual AGT mRNA Level (%) | |
|---|---|---|
| | Mean | SD |
| 1000PM | 25.9 | 12.1 |
| 1131.21-11 | 5.5 | 1.8 |
| 1158.1-1 | 104.8 | 13.8 |
| 1159.1-1 | 13.2 | 2.8 |
| 1160.1-1 | 34.1 | 7.7 |
| 1161.1-1 | 13.2 | 3.0 |
| 1162.1-1 | 45.6 | 11.1 |
| 1163.1-1 | 22.1 | 10.8 |
| 1164.1-1 | 57.3 | 5.8 |
| 1165.1-1 | 14.8 | 7.5 |
| 1166.1-1 | 22.0 | 3.4 |
| 1167.1-1 | 8.3 | 1.0 |
| 1168.1-1 | 14.4 | 2.3 |
| 1169.1-1 | 72.7 | 2.4 |
| 1170.1-1 | 24.2 | 3.7 |
| 1171.1-1 | 12.3 | 4.8 |
| 1172.1-1 | 13.5 | 5.6 |
| 1173.1-1 | 23.1 | 2.5 |
| 1174.1-1 | 34.1 | 6.9 |
| 1175.1-1 | 19.9 | 5.8 |
| 1176.1-1 | 30.1 | 1.6 |

We further employed humanized AGT mouse primary hepatocytes to evaluate the *in vitro* activity of the modified siRNA. Table 4 showed that multiple siRNA sequences, including 1131.21-11 and 1167.1-1, demonstrated superior activity compared to the positive control sequence when delivered via L96.

**Table 5: Experimental Results of 0.1 nM Modified siRNA Delivered via L96 in AAV8-hAGT/hANGPTL3 Mouse Primary Hepatocytes**

| siRNA | Residual AGT mRNA Level (%) | |
|---|---|---|
| | Mean | SD |
| 1000PM | 39.3 | 6.3 |
| 1161.2-3 | 36.6 | 4.9 |
| 1165.2-3 | 37.6 | 6.3 |
| 1165.4-13 | 25.6 | 5.4 |
| 1182.5-10 | 10.8 | 2.2 |
| 1187.1-1 | 15.5 | 4.2 |
| 1189.1-1 | 8.3 | 2.5 |
| 1190.1-1 | 61.8 | 9.1 |
| 1191.1-1 | 11.7 | 1.9 |
| 1192.1-1 | 13.7 | 2.4 |
| 1193.1-1 | 11.0 | 0.1 |
| 1197.1-1 | 13.2 | 1.1 |
| 1198.1-1 | 34.7 | 8.0 |
| 1199.1-1 | 62.0 | 7.2 |
| 1200.1-1 | 4.9 | 2.1 |
| 1201.1-1 | 14.1 | 1.9 |
| 1202.1-1 | 27.1 | 1.1 |
| 1203.1-1 | 47.8 | 6.2 |
| 1204.1-1 | 46.3 | 6.5 |
| 1205.1-1 | 54.3 | 5.0 |
| 1208.1-1 | 60.6 | 4.5 |
| 1209.1-1 | 43.6 | 8.5 |
| 1210.1-1 | 38.3 | 4.3 |
| 1167.2-4 | 19.8 | 0.8 |
| 1167.3-14 | 8.1 | 0.3 |
| 1172.2-17 | 2.1 | 0.3 |
| 1188.1-1 | 15.4 | 1.6 |
| 1194.1-1 | 10.3 | 1.4 |
| 1195.1-1 | 9.4 | 1.1 |
| 1196.1-1 | 46.9 | 4.6 |
| 1206.1-1 | 21.4 | 1.1 |
| 1207.1-1 | 52.3 | 1.3 |
| 1212.1-1 | 71.8 | 4.2 |
| 1213.1-1 | 88.9 | 5.8 |
| 1214.1-1 | 6.3 | 2.0 |
| 1215.1-1 | 36.0 | 4.0 |
| 1216.1-1 | 41.4 | 3.6 |
| 1217.1-1 | 23.7 | 3.7 |
| 1218.1-1 | 22.9 | 1.9 |
| 1219.1-1 | 86.1 | 6.9 |
| 1220.1-1 | 14.2 | 0.8 |
| 1221.1-1 | 28.1 | 1.1 |

From the experimental data in Table 5, we observed that multiple siRNAs, including 1165.4-13 and 1167.3-14, exhibited superior activity compared to the positive control 1000PM at a concentration of 0.1 nM. This superiority was demonstrated in AAV8-hAGT/hANGPTL3 mouse primary hepatocytes under L96 delivery conditions. Moreover, we found that even with identical seed regions, the activity of 1165.4-13 surpassed that of 1165.2-3, while the 1167.3-14 sequence with 19/21 base pairing exhibited superior activity compared to 1167.2-4 with 21/21 base pairing. This demonstrates that alterations in the terminal bases of siRNA can significantly impact sequence activity. We then evaluated the sequences with higher activity in the aforementioned *in vitro* experiments in mouse models.

### Embodiment 3 Evaluation of the Effect of Preferred Sequences on AGT Protein Expression in AAV8-hAGT Mice

### Experimental Method:

### 1. Adenovirus integration of hAGT

Transgenic mice stably expressing hAGT were generated by infecting mice with 1×10¹¹ titer of ultra-purified recombinant AAV8 viral particles.

### 2. Grouping and Administration

Blood was collected from the submandibular vein of mice 14 days after virus injection. Samples were allowed to stand at room temperature for 30 min, then centrifuged at 1000 × g for 10 min. The supernatant serum was collected, aliquoted, and frozen at -80°C. Serum samples were diluted 1000-fold, and hAGT expression in mouse serum was analyzed using the Human Angiotensinogen/AGT/SerpinA8 ELISA Kit (Lianke Bio, Cat. No.: EK1202-96). Mice were equally divided into groups based on hAGT expression levels, with 4 mice per group. siRNA was dissolved in PBS to a concentration of 0.2 mg/mL, and mice were administered the siRNA solution subcutaneously at a dose of 1 mg/kg.

### 3. ELISA Test

Following siRNA injection, blood samples were collected from the submandibular vein at regular intervals. Samples were allowed to stand at room temperature for 30 min and centrifuged at 1000 × g for 10 min. The supernatant serum was collected, diluted 1000-fold, and analyzed for hAGT expression by ELISA. Residual AGT (%) = hAGT Protein Concentration on Day N / hAGT Protein Concentration on Day 0 × 100%.

**Table 6: Experimental Results of hAGT Protein Expression Level over Time after Delivery of hAGT-targeted siRNA into AAV8-hAGT Mice via L96 at 1 mg/kg**

| siRNA | AGT Protein Expression Level (%) (Relative to the predose level) | | | | | |
|---|---|---|---|---|---|---|
| | Day 6 | SD | Day 30 | SD | Day 56 | SD |
| 1000PM | 9.5 | 0.3 | 25.1 | 4 | 77.2 | 17.9 |
| 1159.4-10 | 12 | 1 | 36.3 | 3.6 | NA | NA |
| 1161.3-11 | 13.4 | 0.5 | 45.1 | 3 | NA | NA |
| 1161.3-7 | 10.4 | 0.2 | 24 | 1.1 | NA | NA |
| 1165.3-11 | 10.9 | 1.2 | 22.6 | 2.7 | 42 | 7 |
| 1167.4-14 | 8.7 | 0.5 | 14.3 | 3.6 | 35.3 | 4.5 |
| 1171.4-11 | 10 | 0.9 | 31.2 | 1.6 | NA | NA |
| 1172.2-17 | 8.6 | 0.7 | 18.9 | 2.9 | 47.2 | 6.7 |
| 1172.9-28 | 8.6 | 1.2 | 17.9 | 4.8 | 41.6 | 11.7 |
| 1184.2-3 | 12 | 1.6 | 37.5 | 5.2 | NA | NA |

| | | | | | | |
|---|---|---|---|---|---|---|
| NA means not tested | | | | | | |

Table 6 showed that some sequences with relatively high *in vitro* activity did not perform as well as the positive control *in vivo.* The activity of several siRNA sequences, including 1161.3-7, 1165.3-11, 1167.4-14, 1172.2-17, and 1172.9-28, was comparable or superior to that of the positive control (1000PM). Particularly on Day 56, a marked rebound in hAGT protein expression levels was clearly observed in the 1000PM group of the positive control sequence, while the hAGT protein expression levels in the 1165.3-11, 1167.4-14, 1172.2-17, and 1172.9-28 groups remained below 50%. Furthermore, AGT expression in 1161.3-7 was reduced to only 24% by Day 30, whereas that in 1161.3-11 retained 45.1% at the same time point. This demonstrates that the same naked sequence exhibits significant activity differences after undergoing different modifications, further confirming that modifications profoundly impact siRNA activity.

### Embodiment 4 siRNA Safety Testing

The 7-8 week-old mice (Vital River) were subcutaneously administered 400 mg/kg siRNA every two weeks for 4 consecutive weeks, totaling 3 injections. Mice in the control group received subcutaneous injections of normal saline every two weeks for 4 consecutive weeks, totaling 3 injections. Each group consisted of 4-6 mice. Serum samples were collected 24 h after the final injection for the detection of alanine aminotransferase (ALT) and aspartate aminotransferase (AST) levels.

**Table 7: Safety Experimental Results for AGT-Targeting siRNA**

| siRNA | ALT (U/L) | AST (U/L) |
|---|---|---|
| Normal saline | 42 | 135 |
| 1167.3-14 | 31 | 75 |
| 1172.2-17 | 221 | 219 |
| 1191.1-1 | 325 | 347 |

Table 7 showed that different siRNA sequences exerted varying effects on mouse liver function. 1167.3-14 had no effect on mouse liver function, whereas 1172.2-17 and 1191.1-1 had a significant impact.

### Embodiment 5 Effects of Different Modifications on the Activity and Stability of AGT-Targeting Preferred Sequences

Refer to Embodiment 2 for the sequence activity method.

The experimental methods for siRNA stability studies are as follows:
The test compound sequence was diluted precisely using DEPC-treated water to a working solution concentration of 10 µM.

Preparation of liver homogenate: A total of 200 mg of mouse liver was weighed, with 1 mL of potassium phosphate buffer solution and 5 magnetic beads added. The sample was homogenized three times in a homogenizer at 60 Hz for 30 seconds per cycle to obtain a 200 mg/mL liver homogenate.

Preparation of sample at 72 h: A total of 17 µL of the 10 µM working solution of the test/positive compound was taken, with 153 µL of liver homogenate added. The mixture was vortexed for 30 s. Then, 75 µL of the compound-liver homogenate mixture was transferred to an EP tube. The sample was prepared in duplicate and incubated at 37°C for 72 h. After incubation, 50 µL was withdrawn. Preparation of sample at 0 h: A total of 75 µL of liver homogenate was transferred into an EP tube.

The sample was prepared in duplicate and incubated at 37°C for 72 h. After incubation, 45 µL of liver homogenate was withdrawn, and 5 µL of 10 µM test/positive compound was added. Then, the mixture was mixed thoroughly.

Each tube containing the incubated sample was added with 5 µL of 50 µg/mL internal standard and mixed well. Then, 100 µL of lysate was added to each tube, and the mixture was vortexed for 30 s and allowed to stand for 20 min.

After all samples were treated with appropriate pretreatment methods, the supernatant was collected for instrumental analysis. Subsequently, the relative residual rate (%) was calculated as: (Compound content at 72 h / Compound content at 0 h) × 100%.

We then investigated whether sequences near 1167 all exhibit high activity. To this end, we selected sequences differing by 1-2 nucleotides from 1167 and evaluated their activity in humanized AGT mouse primary hepatocytes. The results are shown in Table 8.

**Table 8: Experimental Results of 1 nM Modified siRNA Delivered via L96 in Mouse Primary Hepatocytes**

| siRNA | Residual AGT mRNA Level (%) | |
|---|---|---|
| | Mean | SD |
| 1000PM | 31.1 | 4.0 |
| 1167.3-5 | 17.5 | 1.0 |
| 1167.2-4 | 34.9 | 1.3 |
| 1167.11-15 | 33.7 | 3.3 |
| 1198.1-1 | 30.9 | 3.7 |
| 1222.1-1 | 30.8 | 1.0 |
| 1223.1-1 | 30.6 | 1.2 |
| 1224.1-1 | 52.5 | 4.5 |

We found that sequences 1198.1-1, 1222.1-1, 1223.1-1, 1224.1-1, etc., exhibited significantly reduced activity compared to 1167.3-5. We also observed that even within the same seed region, 1167.3-5 with 19/21 base pairing exhibited significantly higher activity than 1167.2-4 and 1167.11-15 with 21/21 base pairing.

We next examined the effects of different modifications on the activity of 1167, with the results shown in Table 9.

**Table 9: Experimental Results of Different Concentrations of Modified siRNA Using Different Transfection Methods in Various Cells**

| siRNA | Residual AGT mRNA Level (%) | | | | | | | |
|---|---|---|---|---|---|---|---|---|
| | Hep3B | | AAV8-hAGT/hPCSK9 Mouse Primary Hepatocytes | | Humanized AGT Mouse Primary Hepatocytes | | | |
| | Mean at 0.1 nM/Liposome Transfection | SD | Mean at 1 nM/Free Uptake | SD | Mean at 1 nM/Free Uptake | SD | Mean at 0.1 nM/Liposom e Transfection | SD |
| 1000PM | 22 | 1 | 12.3 | 2.7 | 46.1 | 2.4 | 45.2 | 3.5 |
| 1167.3-5 | 13.1 | 0.8 | 1.4 | 0.2 | 17.7 | 0.4 | 12.4 | 0.9 |
| 1167.3-16 | 11.1 | 1.7 | 0.7 | 0.1 | 10.1 | 0.7 | 7 | 2.2 |
| 1167.16-5 | 18.5 | 1.6 | 2.1 | 0.3 | 21 | 0.2 | 18.9 | 8.2 |
| 1167.16-16 | 10.9 | 1.9 | 1.9 | 0.4 | 19.4 | 2.2 | 18.1 | 3.8 |
| 1167.17-17 | 7.8 | 2 | 1 | 0.2 | 10.2 | 2 | 16.7 | 6.2 |
| 1167.17-19 | 4.5 | 0.3 | 0.5 | 0.2 | 9.5 | 1.6 | 8.4 | 3.4 |
| 1167.19-5 | 13.5 | 1.8 | 5.3 | 0.7 | NA | NA | NA | NA |
| 1167.4-14 | 10.2 | 1 | 1.4 | 0.2 | NA | NA | NA | NA |
| 1167.6-14 | 23.6 | 5.8 | 3.7 | 0.2 | NA | NA | NA | NA |
| 1167.18-18 | 32.2 | 2.7 | 7.4 | 0.6 | NA | NA | NA | NA |
| 1167.12-14 | 43.5 | 1.9 | 21.2 | 3.1 | NA | NA | NA | NA |
| 1167.11-4 | 27.5 | 1.5 | 5.1 | 0.8 | NA | NA | NA | NA |
| 1167.2-4 | 14.7 | 1.3 | 2.8 | 0.9 | NA | NA | NA | NA |

| | | | | | | | | |
|---|---|---|---|---|---|---|---|---|
| NA means not tested | | | | | | | | |

For the preferred sequence 1167, different modified siRNAs were designed. Their *in vitro* activity was compared in Hep3B cells, AAV8-hAGT/hPCSK9 mouse primary hepatocytes, and humanized AGT mouse primary hepatocytes via both liposomal transfection and free uptake methods. The experimental method was based on Embodiment 2. As can be seen from Table 9, different modifications significantly impacted sequence activity. The activity of sequence 1167.17-19 (with the highest activity) far exceeded that of the positive control, while sequence 1167.12-14 (with the lowest activity) showed markedly reduced activity compared to the positive control. This further underscores the critical importance of modifications for siRNA activity. Next, we conducted liver homogenate stability testing on the highly active sequences to evaluate their stability *in vivo.*

**Table 10: Experimental Results of Stability in Liver Homogenate for Different Modified siRNAs**

| siRNA | Residual Percentage of Antisense Strand at 72 h (%) |
|---|---|
| 1167.3-5 | 70.74 |
| 1167.3-16 | 58.98 |
| 1167.16-5 | 79.27 |
| 1167.16-16 | 68.00 |
| 1167.17-17 | 90.89 |
| 1167.17-19 | 91.16 |

As shown in Table 10, different modifications resulted in varying degrees of sequence stability in liver homogenate. Besides, the most active 1167.17-19 was more stable in liver homogenate than other modified 1167 sequences.

**Table 11: Experimental Results of 0.1 nM Modified siRNA Delivered via Liposomes in Humanized AGT Mouse Primary Hepatocytes**

| siRNA | Residual AGT mRNA Level (%) | |
|---|---|---|
| | Mean | SD |
| 1167.17-19 | 41.4 | 8.6 |
| 1167.22-19 | 48.7 | 13.5 |
| 1167.23-19 | 50.0 | 5.5 |
| 1167.24-19 | 62.0 | 2.8 |

**Table 12: Experimental Results of Stability in Liver Homogenate for Different Modified siRNAs**

| siRNA | Residual Percentage of Antisense Strand at 72 h (%) |
|---|---|
| 1167.17-19 | 81.96 |
| 1167.22-19 | 86.08 |
| 1167.23-19 | 68.25 |
| 1167.24-19 | 59.74 |

Based on Tables 11 and 12, we observed that different terminals significantly impacted the activity and stability of the 1167 sequence. When the 1167 sequence employed the mGmG terminal, both its activity and stability were markedly higher than those achieved with mCmC, dTdT, or mAmA.

**Table 13: Experimental Results of 0.3 nM Modified siRNA Delivered via L96 in Primary Hepatocytes from AGT/PCSK9 Double-humanized Mice**

| siRNA | Residual AGT mRNA Level (%) | |
|---|---|---|
| | Mean | SD |
| 1000PM | 61.8 | 4.9 |
| 1167.17-19 | 8.6 | 0.7 |
| 1167.25-19 | 7.9 | 0.2 |

As shown in Table 13, the activity of the VPU-S-modified 1167.25-19 was comparable to that of the VPU-modified 1167.17-19.

We further optimized the 1165 and 1161 sequences, which exhibited satisfactory activity and safety, and examined changes in *in vitro* activity when different modifications were introduced or bases were altered.

**Table 14: Experimental Results of 0.3 nM Modified Double-stranded siRNA Delivered via L96 in Primary Hepatocytes from AGT/PCSK9 Double-humanized Mice**

| siRNA | Residual AGT mRNA Level (%) | |
|---|---|---|
| | Mean | SD |
| 1000PM | 61.8 | 4.9 |
| 1161.4-13 | 17.3 | 1.0 |
| 1161.10-13 | 64.5 | 0.8 |
| 1161.5-14 | 15.6 | 1.7 |
| 1161.6-15 | 62.4 | 1.6 |
| 1161.7-15 | 14.8 | 2.9 |
| 1161.8-16 | 25.1 | 2.3 |
| 1161.9-16 | 70.2 | 1.8 |
| 1161.7-17 | 23.7 | 2.2 |
| 1161.6-17 | 75.4 | 2.9 |
| 1165.5-14 | 9.5 | 0.2 |
| 1165.7-14 | 67.2 | 0.4 |
| 1165.6-15 | 12.3 | 0.4 |
| 1165.5-16 | 8.7 | 0.6 |
| 1165.7-16 | 74.7 | 2.4 |
| 1165.8-17 | 6.6 | 0.7 |
| 1165.9-17 | 45.0 | 0.3 |

Preferred sequences 1161 and 1165 were further optimized by altering bases and introducing modifications. It was discovered that VPU-S modification significantly impacted the activity of both sequences. Among these, for sequence 1161, the modified sequences 1161.4-13, 1161.5-14, and 1161.7-15 exhibited the best activity. For sequence 1165, the modified sequences 1165.8-17, 1165.5-14, and 1165.5-16 exhibited excellent activity. Moreover, the activity of these preferred sequences was significantly higher than that of the positive control 1000PM.

**Table 15: Experimental Results of Different Concentrations of Modified siRNA Delivered via SL01 in AAV8-AGT/ANGPTL3 Mouse Primary Hepatocytes**

| siRNA | Residual AGT mRNA Level (%) | | | |
|---|---|---|---|---|
| | Mean at 1 nM | SD | Mean at 0.1 nM | SD |
| 1000PM | 11 | 1.3 | 68.4 | 2.5 |
| 1161.7-15 | 2.3 | 0.4 | 43.2 | 3.3 |
| 1161.7-17 | 3 | 0.6 | 39 | 1 |
| 1165.5-14 | 1.2 | 0 | 26.6 | 0.9 |
| 1165.5-16 | 1.3 | 0.4 | 28.6 | 0.9 |
| 1167.25-19 | 0.9 | 0 | 30.7 | 1.6 |
| 1167.27-21 | 2.4 | 0.3 | 46.7 | 2.6 |

*In vitro* activity evaluation of double-stranded siRNA delivered by SL01 was conducted at concentrations of 1 nM and 0.1 nM for the preferred sequences 1161.7-15, 1161.7-17, 1165.5-14, 1165.5-16, 1167.25-19, and 1167.27-21. As can be seen from Table 15, at 1 nM and 0.1 nM, the preferred sequences all exhibited higher activity than the Alnylam positive control, with the highest activity observed in 1167.25-19, 1165.5-14, and 1165.5-16.

Next, we evaluated multiple preferred sequences *in vivo.*

**Table 16: Experimental Results of hAGT Protein Expression Level after Delivery of AGT-targeted siRNA into AAV8-hAGT Mice via L96 at 1 mg/kg**

| siRNA | Mean hAGT Protein Expression Level (%) (relative to the predose level) | | | |
|---|---|---|---|---|
| | Day 31/Mean | SD | Day 74/Mean | SD |
| 1000PM | 19.3 | 7.1 | 80.4 | 12.2 |
| 1167.25-19 | 12.7 | 4.2 | 52.1 | 11 |
| 1167.27-21 | 13.2 | 1.7 | 51.3 | 8.6 |
| 1165.5-16 | 12.4 | 6.3 | 50.5 | 25.2 |

As can be seen from Table 16, the *in vivo* activity of 1167.25-19, 1167.27-21, and 1165.5-16 significantly outperformed the positive control, particularly in terms of long-acting properties. On Day 74, the positive control 1000PM exhibited only 20% inhibition of AGT expression, whereas the preferred sequences 1167.25-19, 1167.27-21, and 1165.5-16 maintained approximately 50% inhibition of AGT expression. Next, we tested the *in vivo* activity of the preferred sequence delivered using SL01

**Table 17: Experimental Results of hAGT Protein Expression Level after Delivery of hAGT-targeted siRNA into AAV8-hAGT/hPCSK9 Mice via SL01 at 1 mg/kg**

| siRNA | Mean hAGT Protein Expression Level (%) (relative to the predose level) | | | | | |
|---|---|---|---|---|---|---|
| | Day 7/Mean | SD | Day 14/Mean | SD | Day 28/Mean | SD |
| 1000PM | 17.5 | 1.1 | 21.5 | 3.9 | 34.1 | 5 |
| 1167.25-19-SL01 | 11.2 | 1 | 12.2 | 1.8 | 15.1 | 4.3 |
| 1165.5-16-SL01 | 12.1 | 0.6 | 11 | 1.1 | 13 | 1.6 |

For the two sequences with the highest activity, their *in vivo* activities were further validated. Among them, the positive control was delivered using L96, while 1167.25-19 and 1165.5-16 were delivered using SL01. As can be seen, the activity of the two preferred sequences was significantly superior to that of the positive control.

### Embodiment 6 Synthesis of siRNA Targeting PCSK9

The synthesis method was based on that in Embodiment 1. The dsRNA 11000PM corresponded to the approved drug inclisiran (Patent No.: CN108220295B) and served as the positive control sequence.

**Table 18: Sequences of Sense and Antisense Strands of Unmodified siRNA Targeting PCSK9**

| siRNA Name | Sense Strand Name | Sense Strand Sequence 5'-3' | Antisense Strand Name | Antisense Strand Sequence 5'-3' |
|---|---|---|---|---|
| 11001a | 11001a-SS | CUUUCUAGACCUGUUUG CUA (SEQ ID NO: 251) | 11001a-AS | UAGCAAAACAGGUCUAGAAAG (SEQ ID NO: 252) |
| 11002a | 11002a-SS | CUACAGCCAACUUUUCUA GAA (SEQ ID NO: 253) | 11002a-AS | UUCUAGAAAAGUUGGCUGUA G (SEQ ID NO: 254) |
| 11002b | 11002b-SS | ACAGCCAACUUUUCUAGA A (SEQ ID NO: 255) | 11002b-AS | UUCUAGAAAAGUUGGCUGUA A (SEQ ID NO: 256) |
| 11002c | 11002c-SS | GCAGCCAACUUUUCUAGA A (SEQ ID NO: 257) | 11002c-AS | UUCUAGAAAAGUUGGCUGCAA (SEQ ID NO: 258) |
| 11002d | 11002d-SS | GCAGCCAACUUUUCUAGA A (SEQ ID NO: 259) | 11002d-AS | UUCUAGAAAAGUUGGCUGUG G (SEQ ID NO: 260) |
| 11003a | 11003a-SS | CCUGGGUUUGUAGCAU UUUA (SEQ ID NO: 261) | 11003a-AS | UAAAAUGCUACAAAACCCAGG (SEQ ID NO: 262) |
| 11003b | 11003b-SS | CGGGUUUUGUAGCAUUU UA (SEQ ID NO: 263) | 11003b-AS | UAAAAUGCUACAAAACCCGGG (SEQ ID NO: 264) |
| 11004a | 11004a-SS | CUUUGUAACUUGAAGAU AA (SEQ ID NO: 265) | 11004a-AS | UUAUCUUCAAGUUACAAAGGC (SEQ ID NO: 266) |
| 11004b | 11004b-SS | GCCUUUGUAACUUGAAG AUAA (SEQ ID NO: 267) | 11004b-AS | UUAUCUUCAAGUUACAAAGGC (SEQ ID NO: 268) |
| 11005a | 11005a-SS | CCGUUGCCUUUUUACAGC CAA (SEQ ID NO: 269) | 11005a-AS | UUGGCUGUAAAAAGGCAACGG (SEQ ID NO: 270) |
| 11006a | 11006a-SS | CCUGUUGCCUUUUUACAG CCA (SEQ ID NO: 271) | 11006a-AS | UGGCUGUAAAAAGGCAACAGG (SEQ ID NO: 272) |
| 11007a | 11007a-SS | CUUGUAACUUGAAGAUA UA (SEQ ID NO: 273) | 11007a-AS | UAUAUCUUCAAGUUACAAGAG (SEQ ID NO: 274) |
| 11007b | 11007b-SS | CCUUUGUAACUUGAAGA UAUA (SEQ ID NO: 275) | 11007b-AS | UAUAUCUUCAAGUUACAAAGG (SEQ ID NO: 276) |
| 11007c | 11007c-SS | CCCUUGUAACUUGAAGAU AUA (SEQ ID NO: 277) | 11007c-AS | UAUAUCUUCAAGUUACAAGGG (SEQ ID NO: 278) |
| 11007d | 11007d-SS | CCUGUAACUUGAAGAUA UA (SEQ ID NO: 279) | 11007d-AS | UAUAUCUUCAAGUUACAGGGG (SEQ ID NO: 280) |
| 11008a | 11008a-SS | CUGAAGAUAUUUAUUCU GA (SEQ ID NO: 281) | 11008a-AS | UCAGAAUAAAUAUCUUCAGGU (SEQ ID NO: 282) |
| 11008b | 11008b-SS | GCUUGAAGAUAUUUAUU CUGA (SEQ ID NO: 283) | 11008b-AS | UCAGAAUAAAUAUCUUCAAGC (SEQ ID NO: 284) |
| 11009a | 11009a-SS | CACAGCCAACUUUUCUAG ACA (SEQ ID NO: 285) | 11009a-AS | UGUCUAGAAAAGUUGGCUGU G (SEQ ID NO: 286) |
| 11010a | 11010a-SS | CCUGUUGCCUUUUUACAG A (SEQ ID NO: 287) | 11010a-AS | UCUGUAAAAAGGCAACAGGGA (SEQ ID NO: 288) |
| 11010b | 11010b-SS | CCUCUGUUGCCUUUUUAC AGA (SEQ ID NO: 289) | 11010b-AS | UCUGUAAAAAGGCAACAGAGG (SEQ ID NO: 290) |
| 11011a | 11011a-SS | CGUCCUCUCUGUUGCCUU A (SEQ ID NO: 291) | 11011a-AS | UAAGGCAACAGAGAGGACGGA (SEQ ID NO: 292) |
| 11011b | 11011b-SS | CCUGUCCUCUCUGUUGCC UUA (SEQ ID NO: 293) | 11011b-AS | UAAGGCAACAGAGAGGACAGG (SEQ ID NO: 294) |
| 11012a | 11012a-SS | CGGCGGAGAUGCUUCUAA A (SEQ ID NO: 295) | 11012a-AS | UUUAGAAGCAUCUCCGCCGGG (SEQ ID NO: 296) |
| 11012b | 11012b-SS | CCCGGCGGAGAUGCUUCU AAA (SEQ ID NO: 297) | 11012b-AS | UUUAGAAGCAUCUCCGCCGGG (SEQ ID NO: 298) |
| 11013a | 11013a-SS | GGCAGACAUUUAUCUUU UA (SEQ ID NO: 299) | 11013a-AS | UAAAAGAUAAAUGUCUGCCUG (SEQ ID NO: 300) |
| 11013b | 11013b-SS | CGAGCAGACAUUUAUCUU UUA (SEQ ID NO: 301) | 11013b-AS | UAAAAGAUAAAUGUCUGCUCG (SEQ ID NO: 302) |
| 11014a | 11014a-SS | GGCAUGGAACUUUUUCCG UUA (SEQ ID NO: 303) | 11014a-AS | UAACGGAAAAAGUUCCAUGCC (SEQ ID NO: 304) |
| 11015a | 11015a-SS | CCGCAGGCAUGGAACUUU A (SEQ ID NO: 305) | 11015a-AS | UAAAGUUCCAUGCCUGCGGGC (SEQ ID NO: 306) |
| 11015b | 11015b-SS | CCCUGCAGGCAUGGAACU UUA (SEQ ID NO: 307) | 11015b-AS | UAAAGUUCCAUGCCUGCAGGG (SEQ ID NO: 308) |
| 11016a | 11016a-SS | GCCAACUUUCUAGACCU GUA (SEQ ID NO: 309) | 11016a-AS | UACAGGUCUAGAAAAGUUGGC (SEQ ID NO: 310) |
| 11017a | 11017a-SS | GAGCCAACUUUUCUAGAC CUA (SEQ ID NO: 311) | 11017a-AS | UAGGUCUAGAAAGUUGGCUC (SEQ ID NO: 312) |
| 11018a | 11018a-SS | GCAGCCAACUUUUCUAGA | 11018a-AS | UGGUCUAGAAAAGUUGGCUGC |
| | | CCA (SEQ ID NO: 313) | | GG (SEQ ID NO: 314) |
| 11019a | 11019a-SS | CGUUGCCUUUUUACAGCC AAA (SEQ ID NO: 315) | 11019a-AS | UUUGGCUGUAAAAAGGCAACG (SEQ ID NO: 316) |
| 11020a | 11020a-SS | CCCUGUUGCCUUUUUACA GCA (SEQ ID NO: 317) | 11020a-AS | UGCUGUAAAAAGGCAACAGGG (SEQ ID NO: 318) |
| 11021a | 11021a-SS | CGUCCUCUCUGUUGCCUU UUA (SEQ ID NO: 319) | 11021a-AS | UAAAAGGCAACAGAGAGGACG (SEQ ID NO: 320) |
| 11022a | 11022a-SS | CCCUCUCUGUUGCCUUUU A (SEQ ID NO: 321) | 11022a-AS | UAAAAGGCAACAGAGAGGGCA (SEQ ID NO: 322) |
| 11023a | 11023a-SS | CGAAGAUAUUUAUUCUG GA (SEQ ID NO: 323) | 11023a-AS | UCCAGAAUAAAUAUCUUCGAG (SEQ ID NO: 324) |
| 11023b | 11023b-SS | CCUGAAGAUAUUUAUUC UGGA (SEQ ID NO: 325) | 11023b-AS | UCCAGAAUAAAUAUCUUCAGG (SEQ ID NO326) |
| 11024a | 11024a-SS | CGGGCAGAAUGACUUUU AA (SEQ ID NO: 327) | 11024a-AS | UUAAAAGUCAUUCUGCCCGCG (SEQ ID NO: 328) |
| 11024b | 11024b-SS | CGCGGGCAGAAUGACUUU UAA (SEQ ID NO: 329) | 11024b-AS | UUAAAAGUCAUUCUGCCCGCG (SEQ ID NO: 330) |
| 11025a | 11025a-SS | CGUGGGCAUUUCACCAU UCA (SEQ ID NO: 331) | 11025a-AS | UGAAUGGUGAAAUGCCCCACG (SEQ ID NO: 332) |
| 11026a | 11026a-SS | CUGGGUCUGUCCUCUCUG A (SEQ ID NO: 333) | 11026a-AS | UCAGAGAGGACAGACCCAGAA (SEQ ID NO: 334) |
| 11026b | 11026b-SS | CUUUGGGUCUGUCCUCUC UGA (SEQ ID NO: 335) | 11026b-AS | UCAGAGAGGACAGACCCAAAG (SEQ ID NO: 336) |
| 11027a | 11027a-SS | CGGAGUUUAUUCGGAAA AA (SEQ ID NO: 337) | 11027a-AS | UUUUUCCGAAUAAACUCCGGG (SEQ ID NO: 338) |
| 11027b | 11027b-SS | CCCGGAGUUUAUUCGGAA AAA (SEQ ID NO: 339) | 11027b-AS | UUUUUCCGAAUAAACUCCGGG (SEQ ID NO: 340) |
| 11028a | 11028a-SS | GUAGACAUUUAUCUUUU GA (SEQ ID NO: 341) | 11028a-AS | UCAAAAGAUAAAUGUCUGUU U (SEQ ID NO: 342) |
| 11028b | 11028b-SS | GAGCAGACAUUUAUCUU UUGA (SEQ ID NO: 343) | 11028b-AS | UCAAAAGAUAAAUGUCUGCUC (SEQ ID NO: 344) |
| 11029a | 11029a-SS | CCUGCCAAAGAUGUCAUC AAA (SEQ ID NO: 345) | 11029a-AS | UUUGAUGACAUCUUGGCAGG GA (SEQ ID NO: 346) |
| 11030a | 11030a-SS | CUAAUGGAGGCUUAGCU UUCA (SEQ ID NO: 347) | 11030a-AS | UGAAAGCUAAGCCUCCAUUAG UC (SEQ ID NO: 348) |
| 11031a | 11031a-SS | CAAUGGAGGCUUAGCUU UCUA (SEQ ID NO: 349) | 11031a-AS | UAGAAAGCUAAGCCUCCAUUG AU (SEQ ID NO: 350) |
| 11032a | 11032a-SS | CGAUCCACUUCUCUGCCA AAA (SEQ ID NO: 351) | 11032a-AS | UUUUGGCAGAGAAGUGGAUC GGU (SEQ ID NO: 352) |
| 11033a | 11033a-SS | GUUGAGCUUUAAAAUGG UA (SEQ ID NO: 353) | 11033a-AS | UACCAUUUUAAAGCUCAGCUU (SEQ ID NO: 354) |
| 11033b | 11033b-SS | CGGCUGAGCUUUAAAU GGUA (SEQ ID NO: 355) | 11033b-AS | UACCAUUUUAAAGCUCAGCCG (SEQ ID NO: 356) |
| 11034a | 11034a-SS | CGGAAUGACUUUUAUUG AA (SEQ ID NO: 357) | 11034a-AS | UUCAAUAAAAGUCAUUCUGUU (SEQ ID NO: 358) |
| 11034b | 11034b-SS | CGCAGAAUGACUUUUAU UGAA (SEQ ID NO: 359) | 11034b-AS | UUCAAUAAAAGUCAUUCUGCG (SEQ ID NO: 360) |
| 11035a | 11035a-SS | CGACUUUUAUUGAGCUCU A (SEQ ID NO: 361) | 11035a-AS | UAGAGCUCAAUAAAAGUCGUU (SEQ ID NO: 362) |
| 11035b | 11035b-SS | GAUGACUUUUAUUGAGC UCUA (SEQ ID NO: 363) | 11035b-AS | UAGAGCUCAAUAAAAGUCAUC (SEQ ID NO: 364) |
| 11036a | 11036a-SS | CUUUAUUGAGCUCUUGU UCCA (SEQ ID NO: 365) | 11036a-AS | UGGAACAAGAGCUCAAUAAAG GU (SEQ ID NO: 366) |
| 11037a | 11037a-SS | CGGGAUUCUUCCCAUGGA A (SEQ ID NO: 367) | 11037a-AS | UUCCAUGGGAAGAAUCCUGUU (SEQ ID NO: 368) |
| 11037b | 11037b-SS | CGCAGGAUUCUUCCCAUG GAA (SEQ ID NO: 369) | 11037b-AS | UUCCAUGGGAAGAAUCCUGCG (SEQ ID NO: 370) |
| 11038a | 11038a-SS | CGAGCCACCUUUACUCUG CUA (SEQ ID NO: 371) | 11038a-AS | UAGCAGAGUAAAGGUGGCUCG (SEQ ID NO: 372) |
| 11039a | 11039a-SS | CCCCUGAUUAAUGGAGGC UUA (SEQ ID NO: 373) | 11039a-AS | UAAGCCUCCAUUAAUCAGGGG GC (SEQ ID NO: 374) |
| 11040a | 11040a-SS | CUUAUUCUGGGUUUUGU AGCA (SEQ ID NO: 375) | 11040a-AS | UGCUACAAAACCCAGAAUAAG (SEQ ID NO: 376) |
| 11040b | 11040b-SS | CAUUCUGGGUUUUGUAG CA (SEQ ID NO: 377) | 11040b-AS | UGCUACAAAACCCAGAAUGAA (SEQ ID NO: 378) |
| 11040c | 11040c-SS | CAUUCUGGGUUUUGUAG CA (SEQ ID NO: 379) | 11040c-AS | UGCUACAAAACCCAGAAUGGG (SEQ ID NO: 380) |
| 11040d | 11040d-SS | UAUUCUGGGUUUUGUAG CA (SEQ ID NO: 381) | 11040d-AS | UGCUACAAAACCCAGAAUAAA (SEQ ID NO: 382) |
| 11041a | 11041a-SS | CGUUUUGCUUUUGUAAC UUGA (SEQ ID NO: 383) | 11041a-AS | UCAAGUUACAAAAGCAAAACG (SEQ ID NO: 384) |
| 11042a | 11042a-SS | CUUUGGGUCUGUCCUCUC A (SEQ ID NO: 385) | 11042a-AS | UGAGAGGACAGACCCAAAGGA (SEQ ID NO: 386) |
| 11042b | 11042b-SS | CCUUUUGGGUCUGUCCUC UCA (SEQ ID NO: 387) | 11042b-AS | UGAGAGGACAGACCCAAAAGG (SEQ ID NO: 388) |
| 11043a | 11043a-SS | CCUUUUGGGUCUGUCCUC A (SEQ ID NO: 389) | 11043a-AS | UGAGGACAGACCCAAAAGGUA (SEQ ID NO: 390) |
| 11043b | 11043b-SS | CAUCUUUGGUCUGUCC UCA (SEQ ID NO: 391) | 11043b-AS | UGAGGACAGACCCAAAAGAUG (SEQ ID NO: 392) |
| 11044a | 11044a-SS | CACAUUUAUCUUUUGGG UCUA (SEQ ID NO: 393) | 11044a-AS | UAGACCCAAAAGAUAAAUGUG (SEQ ID NO: 394) |
| 11045a | 11045a-SS | GGACAUUUAUCUUUUGG GUCA (SEQ ID NO: 395) | 11045a-AS | UGACCCAAAAGAUAAAUGUCC (SEQ ID NO: 396) |
| 11046a | 11046a-SS | GGACAUUUAUCUUUUGG GA (SEQ ID NO: 397) | 11046a-AS | UCCCAAAAGAUAAAUGUCCGC (SEQ ID NO: 398) |
| 11046b | 11046b-SS | GCGGACAUUUAUCUUUU GGGA (SEQ ID NO: 399) | 11046b-AS | UCCCAAAAGAUAAAUGUCCGC (SEQ ID NO: 400) |
| 11047a | 11047a-SS | GCCCUCUCUGUUGCCUUU UUA (SEQ ID NO: 401) | 11047a-AS | UAAAAAGGCAACAGAGAGGGC (SEQ ID NO: 402) |
| 11048a | 11048a-SS | ACUCUGGGUUUUGUAGC AA (SEQ ID NO: 403) | 11048a-AS | UUGCUACAAAACCCAGAGUGG (SEQ ID NO: 404) |
| 11049a | 11049a-SS | CUCUGGGUUUUGUAGCA UA (SEQ ID NO: 405) | 11049a-AS | UAUGCUACAAAACCCAGAGUU I (SEQ ID NO: 406) |
| 11050a | 11050a-SS | CCUGGGUUUUGUAGCAU UA (SEQ ID NO: 407) | 11050a-AS | UAAUGCUACAAAACCCAGGUU (SEQ ID NO: 408) |

Next, we modified the siRNA to enhance its stability both *in vivo* and *in vitro,* increase its activity at the target site, and reduce its activity at non-target sites. Unless otherwise specified, L96 delivery was employed for both *in vivo* and *in vitro* screening of single-target sequences to more accurately reflect the efficacy of liver-targeted siRNA. The siRNA was connected to L96 via the 3'-terminus of the sense strand.

**Table 19: Sequences of Modified siRNA Targeting PCSK9**

| siRNA Name | Modified Sense Strand Name | Modified Sense Strand Sequence 5'-3' | Modified Antisense Strand Name | Modified Antisense Strand Sequence 5'-3' |
|---|---|---|---|---|
| 11000P M | 11000PM-SM | mC*mU*mAmGmAmC fCmUfGmUdTmUmUm GmCmUmUmUmUmG mU | 11000PM-AM | mA*fC*mAfAfAfAmGfCm AfAmAfAmCfAmGfGmUfC mUmAmG*mA*mA |
| 11001.1-1 | 11001SM1 | mC*mU*mUmUmCmU fAmGfAfCdCmUmGm UmUmUmUmGmCmU mA | 11001AM1 | VPU*fA*mGmCmAmAmA mAmCmAmGdGmUfCmUf AmGmAmA*mA*mG |
| 11002.1-1 | 11002SM1 | mC*mU*mAmCmAmG fCmCfAfAdCmUmUm UmUmCmUmAmGmA mA | 11002AM1 | VPU*fU*mCmUmAmGmA mAmAmAmAmGdTmUfGmGf CmUmGmU*mA*mG |
| 11002.1-8 | 11002SM8 | Invab*mC*mUmAmCm AmGmCmCfAfAdCmU fUmUmUmCmUmAmG mAmAInvab | 11002AM1 | VPU*fU*mCmUmAmGmA mAmAmAmGdTmUfGmGf CmUmGmU*mA*mG |
| 11002.3-10 | 11002SM1 0 | Invab*mA*mCmAmGm CmCfAfAdCmUfUmU mUmCmUmAmGmAm AInvab | 11002AM3 | VPU*fU*mCmUmAmGmA mAmAmAmGdTmUfGmGf CmUmGmU*mA*mA |
| 11002.5-14 | 11002SM1 4 | Invab*mG*mCmAmGm CmCfAfAdCmUfUmU mUmCmUmAmGmAm AInvab | 11002AM5 | VPU*fU*mCmUmAmGmA mAmAmAmGdTmUfGmGf CmUmGmC*mA*mA |
| 11002.7-14 | 11002SM1 4 | Invab*mG*mCmAmGm CmCfAfAdCmUfUmU mUmCmUmAmGmAm AInvab | 11002AM7 | VPU*fU*mCmUmAmGmA mAmAmAmGdTmUfGmGf CmUmGmU*mG*mG |
| 11002.7-21 | 11002SM2 1 | Invab*mG*mCmAmGm CmCfAfAfCfUmUfUm UmCmUmAmGmAmAI nvab | 11002AM7 | VPU*fU*mCmUmAmGmA mAmAmAmGdTmUfGmGf CmUmGmU*mG*mG |
| 11002.9-8 | 11002SM8 | Invab*mC*mUmAmCm AmGmCmCfAfAdCmU fUmUmUmCmUmAmG mAmAInvab | 11002AM9 | mU*fU*mCmUmAmGmAm AmAmAmGdTmUfGmGfC mUmGmU*mA*mG |
| 11002.1 0-8 | 11002SM8 | Invab*mC*mUmAmCm AmGmCmCfAfAdCmU fUmUmUmCmUmAmG mAmAInvab | 11002AM10 | VPU- S*fU*mCmUmAmGmAmA mAmAmGdTmUfGmGfCm UmGmU*mA*mG |
| 11002.1 0-23 | 11002SM2 3 | Invab*mC*mUmAmCm AmGmCmCfAfAfCfUm UfUmUmCmUmAmGm AmAInvab | 11002AM10 | VPU- S*fU*mCmUmAmGmAmA mAmAmGdTmUfGmGfCm UmGmU*mA*mG |
| 11002.1 6-21 | 11002SM2 1 | Invab*mG*mCmAmGm CmCfAfAfCfUmUfUm UmCmUmAmGmAmAI nvab | 11002AM16 | mU*fU*mCmUmAmGmAm AmAmAmGdTmUfGmGfC mUmGmU*mG*mG |
| 11002.1 7-21 | 11002SM2 1 | Invab*mG*mCmAmGm CmCfAfAfCfUmUfUm UmCmUmAmGmAmAI nvab | 11002AM17 | VPU- S*fU*mCmUmAmGmAmA mAmAmGdTmUfGmGfCm UmGmU*mG*mG |
| 11002.2 3-22 | 11002SM2 2 | Invab*mA*mCmAmGm CmCfAfAfCfUmUfUm UmCmUmAmGmAmAI nvab | 11002AM23 | VPU- S*fU*mCmUmAmGmAmA mAmAmGdTmUfGmGfCm UmGmU*mA*mA |
| 11003.1-1 | 11003SM1 | mC*mC*mUmGmGmG fUmUfUfUdGmUmAm GmCmAmUmUmUmU mA | 11003AM1 | VPU*fA*mAmAmAmUmG mCmUmAmCdAmAfAmAf CmCmCmA*mG*mG |
| 11003.1-8 | 11003SM8 | Invab*mC*mCmUmGm GmGmUmUfUfUdGmU fAmGmCmAmUmUmU mUmAInvab | 11003AM1 | VPU*fA*mAmAmAmUmG mCmUmAmCdAmAfAmAf CmCmCmA*mG*mG |
| 11004.1-1 | 11004SM1 | mC*mU*mUmUfGmUf AfAdCmUmUmGmAm AmGmAmUmAmA | 11004AM1 | VPU*fU*mAmUmCmUmU mCmAmAmGdTmUfAmCfA mAmAmG*mG*mC |
| 11004.1-2 | 11004SM2 | mG*mC*mCmUmUmU fGmUfAfAdCmUmUm GmAmAmGmAmUmA mA | 11004AM1 | VPU*fU*mAmUmCmUmU mCmAmAmGdTmUfAmCfA mAmAmG*mG*mC |
| 11005.1-1 | 11005SM1 | mC*mC*mGmUmUmG fCmCfUfUdTmUmUm AmCmAmGmCmCmA mA | 11005AM1 | VPU*fU*mGmGmCmUmG mUmAmAmAdAmAfGmGf CmAmAmC*mG*mG |
| 11006.1-1 | 11006SM1 | mC*mC*mUmGmUmU fGmCfCfUdTmUmUm UmAmCmAmGmCmC mA | 11006AM1 | VPU*fG*mGmCmUmGmU mAmAmAmAdAmGfGmCf AmAmCmA*mG*mG |
| 11007.1-1 | 11007SM1 | mC*mU*mUmGfUmAf AfCdTmUmGmAmAm GmAmUmAmUmA | 11007AM1 | VPU*fA*mUmAmUmCmU mUmCmAmAdGmUfUmAf CmAmAmG*mA*mG |
| 11007.2-2 | 11007SM2 | mC*mC*mUmUmUmG fUmAfAfCdTmUmGm AmAmGmAmUmAmU mA | 11007AM2 | VPU*fA*mUmAmUmCmU mUmCmAmAdGmUfUmAf CmAmAmA*mG*mG |
| 11008.1-1 | 11008SM1 | mC*mU*mGmAfAmGf AfUdAmUmUmUmAm UmUmCmUmGmA | 11008AM1 | VPU*fC*mAmGmAmAmU mAmAmAmUdAmUfCmUf UmCmAmG*mG*mU |
| 11008.2-2 | 11008SM2 | mG*mC*mUmUmGmA fAmGfAfUdAmUmUm UmAmUmUmCmUmG mA | 11008AM2 | VPU*fC*mAmGmAmAmU mAmAmAmUdAmUfCmUf UmCmAmA*mG*mC |
| 11009.1-1 | 11009SM1 | mC*mA*mCmAmGmC fCmAfAfCdTmUmUm UmCmUmAmGmAmC mA | 11009AM1 | VPU*fG*mUmCmUmAmG mAmAmAmAdGmUfUmGf GmCmUmG*mU*mG |
| 11010.1-1 | 11010SM1 | mC*mC*mUmGfUmUf GfCdCmUmUmUmUm UmAmCmAmGmA | 11010AM1 | VPU*fC*mUmGmUmAmA mAmAmAmGdGmCfAmAf CmAmGmG*mG*mA |
| 11010.2-2 | 11010SM2 | mC*mC*mUmCmUmG fUmUfGfCdCmUmUm UmUmUmAmCmAmG mA | 11010AM2 | VPU*fC*mUmGmUmAmA mAmAmAmGdGmCfAmAf CmAmGmA*mG*mG |
| 11011.1-1 | 11011SM1 | mC*mG*mUmCfCmUf CfUdCmUmGmUmUm GmCmCmUmUmA | 11011AM1 | VPU*fA*mAmGmGmCmA mAmCmAmGdAmGfAmGf GmAmCmG*mG*mA |
| 11011.2-2 | 11011SM2 | mC*mC*mUmGmUmC fCmUfCfUdCmUmGm UmUmGmCmCmUmU mA | 11011AM2 | VPU*fA*mAmGmGmCmA mAmCmAmGdAmGfAmGf GmAmCmA*mG*mG |
| 11012.1-1 | 11012SM1 | mC*mG*mGmCfGmGf AfGdAmUmGmCmUm UmCmUmAmAmA | 11012AM1 | VPU*fU*mUmAmGmAmA mGmCmAmUdCmUfCmCfG mCmCmG*mG*mG |
| 11013.1-1 | 11013SM1 | mG*mG*mCmAfGmAf CfAdTmUmUmAmUm CmUmUmUmUmA | 11013AM1 | VPU*fA*mAmAmAmGmA mUmAmAmAdTmGfUmCf UmGmCmC*mU*mG |
| 11013.2-2 | 11013SM2 | mC*mG*mAmGmCmA fGmAfCfAdTmUmUm AmUmCmUmUmUmU mA | 11013AM2 | VPU*fA*mAmAmAmGmA mUmAmAmAdTmGfUmCf UmGmCmU*mC*mG |
| 11014.1-1 | 11014SM1 | mG*mG*mCmAmUmG fGmAfAfCdTmUmUm UmUmCmCmGmUmU mA | 11014AM1 | VPU*fA*mAmCmGmGmA mAmAmAmAmAdGmUfUmCf CmAmUmG*mC*mC |
| 11015.1-1 | 11015SM1 | mC*mC*mGmCfAmGf GfCdAmUmGmGmAm AmCmUmUmUmA | 11015AM1 | VPU*fA*mAmAmGmUmU mCmCmAmUdGmCfCmUfG mCmGmG*mG*mC |
| 11015.2-2 | 11015SM2 | mC*mC*mCmUmGmCf AmGfGfCdAmUmGmG mAmAmCmUmUmUm A | 11015AM2 | VPU*fA*mAmAmGmUmU mCmCmAmUdGmCfCmUfG mCmAmG*mG*mG |
| 11016.1-1 | 11016SM1 | mG*mC*mCmAmAmC fUmUfUfUdCmUmAm GmAmCmCmUmGmU mA | 11016AM1 | VPU*fA*mCmAmGmGmU mCmUmAmGdAmAfAmAf GmUmUmG*mG*mC |
| 11017.1-1 | 11017SM1 | mG*mA*mGmCmCmA fAmCfUfUdTmUmCm UmAmGmAmCmCmU mA | 11017AM1 | VPU*fA*mGmGmUmCmU mAmGmAmAdAmAfGmUf UmGmGmC*mU*mC |
| 11018.1-1 | 11018SM1 | mG*mC*mAmGmCmC fAmAfCfUdTmUmUm CmUmAmGmAmCmC mA | 11018AM1 | VPU*fG*mGmUmCmUmA mGmAmAmAdAmGfUmUf GmGmCmUmGmC*mG*mG |
| 11019.1-1 | 11019SM1 | mC*mG*mUmUmGmC fCmUfUfUdTmUmAm CmAmGmCmCmAmA mA | 11019AM1 | VPU*fU*mUmGmGmCmU mGmUmAmAdAmAfAmGf GmCmAmA*mC*mG |
| 11020.1-1 | 11020SM1 | mC*mC*mCmUmGmU fUmGfCfCdTmUmUm UmUmAmCmAmGmC mA | 11020AM1 | VPU*fG*mCmUmGmUmA mAmAmAmAmAdGmGfCmAf AmCmAmG*mG*mG |
| 11021.1-1 | 11021SM1 | mC*mG*mUmCmCmU fCmUfCfUdGmUmUm GmCmCmUmUmUmU mA | 11021AM1 | VPU*fA*mAmAmAmGmG mCmAmAmCdAmGfAmGf AmGmGmA*mC*mG |
| 11022.1-1 | 11022SM1 | mC*mC*mCmUfCmUf CfUdGmUmUmGmCm CmUmUmUmUmA | 11022AM1 | VPU*fA*mAmAmAmGmG mCmAmAmCdAmGfAmGf AmGmGmG*mC*mA |
| 11023.1-1 | 11023SM1 | mC*mG*mAmAfGmAf UfAdTmUmUmAmUm UmCmUmGmGmA | 11023AM1 | VPU*fC*mCmAmGmAmA mUmAmAmAdTmAfUmCf UmUmCmG*mA*mG |
| 11023.2-2 | 11023SM2 | mC*mC*mUmGmAmA fGmAfUfAdTmUmUm AmUmUmCmUmGmG mA | 11023AM2 | VPU*fC*mCmAmGmAmA mUmAmAmAdTmAfUmCf UmUmCmA*mG*mG |
| 11024.1-1 | 11024SM1 | mC*mG*mGmGfCmAf GfAdAmUmGmAmCm UmUmUmUmAmA | 11024AM1 | VPU*fU*mAmAmAmAmG mUmCmAmUdTmCfUmGfC mCmCmG*mC*mG |
| 11024.1-15 | 11024SM1 5 | Invab*mC*mGmGmGm CmAfGfAdAmUfGmA mCmUmUmUmUmAm AInvab | 11024AM1 | VPU*fU*mAmAmAmAmG mUmCmAmUdTmCfUmGfC mCmCmG*mC*mG |
| 11024.1-16 | 11024SM1 6 | Invab*mC*mGmCmGm GmGmCmAfGfAdAmU fGmAmCmUmUmUmU mAmAInvab | 11024AM1 | VPU*fU*mAmAmAmAmG mUmCmAmUdTmCfUmGfC mCmCmG*mC*mG |
| 11025.1-1 | 11025SM1 | mC*mG*mUmGmGmG fGmCfAfUdTmUmCm AmCmCmAmUmUmC mA | 11025AM1 | VPU*fG*mAmAmUmGmG mUmGmAmAdAmUfGmCf CmCmCmA*mC*mG |
| 11026.1-1 | 11026SM1 | mC*mU*mGmGfGmUf CfUdGmUmCmCmUm CmUmCmUmGmA | 11026AM1 | VPU*fC*mAmGmAmGmA mGmGmAmCdAmGfAmCf CmCmAmG*mA*mA |
| 11026.2-2 | 11026SM2 | mC*mU*mUmUmGmG fGmUfCfUdGmUmCm CmUmCmUmCmUmG mA | 11026AM2 | VPU*fC*mAmGmAmGmA mGmGmAmCdAmGfAmCf CmCmAmA*mA*mG |
| 11027.1-1 | 11027SM1 | mC*mG*mGmAfGmUf UfUdAmUmUmCmGm GmAmAmAmAmA | 11027AM1 | VPU*fU*mUmUmUmCmC mGmAmAmUdAmAfAmCf UmCmCmG*mG*mG |
| 11027.1-2 | 11027SM2 | mC*mC*mCmGmGmA fGmUfUfUdAmUmUm CmGmGmAmAmAmA mA | 11027AM1 | VPU*fU*mUmUmUmCmC mGmAmAmUdAmAfAmCf UmCmCmG*mG*mG |
| 11028.1-1 | 11028SM1 | mG*mU*mAmGfAmCf AfUdTmUmAmUmCm UmUmUmUmGmA | 11028AM1 | VPU*fC*mAmAmAmAmG mAmUmAmAdAmUfGmUf CmUmGmU*mU*mU |
| 11028.2-2 | 11028SM2 | mG*mA*mGmCmAmG fAmCfAfUdTmUmAm UmCmUmUmUmUmG mA | 11028AM2 | VPU*fC*mAmAmAmAmG mAmUmAmAdAmUfGmUf CmUmGmC*mU*mC |
| 11029.1-1 | 11029SM1 | mC*mC*mUmGmCmCf AmAfAfGdAmUmGmU mCmAmUmCmAmAm A | 11029AM1 | VPU*fU*mUmGmAmUmG mAmCmAmUdCmUfUmUf GmGmCmAmGmG*mG*m A |
| 11030.1-1 | 11030SM1 | mC*mU*mAmAmUmG fGmAfGfGdCmUmUm AmGmCmUmUmUmC mA | 11030AM1 | VPU*fG*mΛmΛmΛmGmC mUmAmAmGdCmCfUmCfC mAmUmUmAmG*mU*mC |
| 11031.1-1 | 11031SM1 | mC*mA*mAmUmGmG fAmGfGfCdTmUmAm GmCmUmUmUmCmU mA | 11031AM1 | VPU*fA*mGmAmAmAmG mCmUmAmAdGmCfCmUfC mCmAmUmUmG*mA*mU |
| 11032.1-1 | 11032SM1 | mC*mG*mAmUmCmC fAmCfUfUdCmUmCm UmGmCmCmAmAmA mA | 11032AM1 | VPU*fU*mUmUmGmGmC mAmGmAmGdAmAfGmUf GmGmAmUmCmG*mG*m U |
| 11033.1-1 | 11033SM1 | mG*mU*mUmGfAmGf CfUdTmUmAmAmAm AmUmGmGmUmA | 11033AM1 | VPU*fA*mCmCmAmUmU mUmUmAmAdAmGfCmUf CmAmGmC*mU*mU |
| 11033.2-2 | 11033SM2 | mC*mG*mGmCmUmG fAmGfCfUdTmUmAm AmAmAmUmGmGmU mA | 11033AM2 | VPU*fA*mCmCmAmUmU mUmUmAmAdAmGfCmUf CmAmGmC*mC*mG |
| 11034.1-1 | 11034SM1 | mC*mG*mGmAfAmUf GfAdCmUmUmUmUm AmUmUmGmAmA | 11034AM1 | VPU*fU*mCmAmAmUmA mAmAmAmGdTmCfAmUf UmCmUmG*mU*mU |
| 11034.2-2 | 11034SM2 | mC*mG*mCmAmGmA fAmUfGfAdCmUmUm UmUmAmUmUmGmA mA | 11034AM2 | VPU*fU*mCmAmAmUmA mAmAmAmGdTmCfAmUf UmCmUmG*mC*mG |
| 11035.1-1 | 11035SM1 | mC*mG*mAmCfUmUf UfUdAmUmUmGmAm GmCmUmCmUmA | 11035AM1 | VPU*fA*mGmAmGmCmU mCmAmAmUdAmAfAmAf GmUmCmG*mU*mU |
| 11035.2-2 | 11035SM2 | mG*mA*mUmGmAmC fUmUfUfUdAmUmUm GmAmGmCmUmCmU mA | 11035AM2 | VPU*fA*mGmAmGmCmU mCmAmAmUdAmAfAmAf GmUmCmA*mU*mC |
| 11036.1-1 | 11036SM1 | mC*mU*mUmUmAmU fUmGfAfGdCmUmCm UmUmGmUmUmCmC mA | 11036AM1 | VPU*fG*mGmAmAmCmA mAmGmAmGdCmUfCmAf AmUmAmAmAmG*mG*m U |
| 11037.1-1 | 11037SM1 | mC*mG*mGmGfAmUf UfCdTmUmCmCmCm AmUmGmGmAmA | 11037AM1 | VPU*fU*mCmCmAmUmG mGmGmAmAdGmAfAmUf CmCmUmG*mU*mU |
| 11037.2-2 | 11037SM2 | mC*mG*mCmAmGmG fAmUfUfCdTmUmCmC mCmAmUmGmGmAm A | 11037AM2 | VPU*fU*mCmCmAmUmG mGmGmAmAdGmAfAmUf CmCmUmG*mC*mG |
| 11038.1-1 | 11038SM1 | mC*mG*mAmGmCmC fAmCfCfUdTmUmAmC mUmCmUmGmCmUm A | 11038AM1 | VPU*fA*mGmCmAmGmA mGmUmAmAdAmGfGmUf GmGmCmU*mC*mG |
| 11039.1-1 | 11039SM1 | mC*mC*mCmCmUmGf AmUfUfAdAmUmGmG mAmGmGmCmUmUm A | 11039AM1 | VPU*fA*mAmGmCmCmU mCmCmAmUdTmAfAmUfC mAmGmGmGmG*mG*mC |
| 11040.1-1 | 11040SM1 | mC*mU*mUmAmUmU fCmUfGfGdGmUmUm UmUmGmUmAmGmC mA | 11040AM1 | VPU*fG*mCmUmAmCmA mAmAmAmCdCmCfAmGf AmAmUmA*mA*mG |
| 11040.1-6 | 11040SM6 | Invab*mC*mUmUmAm UmUmCmUfGmGfGm UfUmUmUmGmUmAm GmCmAInvab | 11040AM1 | VPU*fG*mCmUmAmCmA mAmAmAmCdCmCfAmGf AmAmUmA*mA*mG |
| 11040.1-8 | 11040SM8 | Invab*mC*mUmUmAm UmUmCmUfGfGdGmU fUmUmUmGmUmAmG mCmAInvab | 11040AM1 | VPU*fG*mCmUmAmCmA mAmAmAmCdCmCfAmGf AmAmUmA*mA*mG |
| 11040.1-23 | 11040SM2 3 | Invab*mC*mUmUmAm UmUmCmUfGfGfGfU mUfUmUmGmUmAmG mCmAInvab | 11040AM1 | VPU*fG*mCmUmAmCmA mAmAmAmCdCmCfAmGf AmAmUmA*mA*mG |
| 11040.3-10 | 11040SM1 0 | Invab*mC*mAmUmUm CmUfGfGdGmUfUmU mUmGmUmAmGmCm AInvab | 11040AM3 | VPU*fG*mCmUmAmCmA mAmAmAmCdCmCfAmGf AmAmUmG*mA*mA |
| 11040.5-10 | 11040SM1 0 | Invab*mC*mAmUmUm CmUfGfGdGmUfUmU mUmGmUmAmGmCm AInvab | 11040AM5 | VPU*fG*mCmTmAmCmAm AmAmAmCdCmCfAmGfA mAmTmG*mG*mG |
| 11040.7-18 | 11040SM1 8 | Invab*mU*mAmUmUm CmUfGfGdGmUfUmU mUmGmUmAmGmCm AInvab | 11040AM7 | VPU*fG*mCmUmAmCmA mAmAmAmCdCmCfAmGf AmAmUmA*mA*mA |
| 11040.9-6 | 11040SM6 | Invab*mC*mUmUmAm UmUmCmUfGmGfGm UfUmUmUmGmUmAm GmCmAInvab | 11040AM9 | mU*fG*mCmUmAmCmAm AmAmAmCdCmCfAmGfA mAmUmA*mA*mG |
| 11040.9-23 | 11040SM2 3 | Invab*mC*mUmUmAm UmUmCmUfGfGfGfU mUfUmUmGmUmAmG mCmAInvab | 11040AM9 | mU*fG*mCmUmAmCmAm AmAmAmCdCmCfAmGfA mAmUmA*mA*mG |
| 11040.1 0-6 | 11040SM6 | Invab*mC*mUmUmAm UmUmCmUfGmGfGm UfUmUmUmGmUmAm GmCmAInvab | 11040AM10 | VPU- S*fG*mCmUmAmCmAmA mAmAmCdCmCfAmGfAm AmUmA*mA*mG |
| 11040.1 0-23 | 11040SM2 3 | Invab*mC*mUmUmAm UmUmCmUfGfGfGfU mUfUmUmGmUmAmG mCmAInvab | 11040AM10 | VPU- S*fG*mCmUmAmCmAmA mAmAmCdCmCfAmGfAm AmUmA*mA*mG |
| 11040.1 7-25 | 11040SM2 5 | Invab*mC*mAmUmUm CmUfGfGfGfUmUfUm UmGmUmAmGmCmAI nvab | 11040AM17 | VPU- S*fG*mCmUmAmCmAmA mAmAmCdCmCfAmGfAm AmUmG*mG*mG |
| 11040.2 5-24 | 11040SM2 4 | Invab*mU*mAmUmUm CmUfGfGfGfUmUfUm UmGmUmAmGmCmAI nvab | 11040AM25 | VPU- S*fG*mCmUmAmCmAmA mAmAmCdCmCfAmGfAm AmUmA*mA*mA |
| 11041.1-1 | 11041SM1 | mC*mG*mUmUmUmU fGmCfUfUdTmUmGm UmAmAmCmUmUmG mA | 11041AM1 | VPU*fC*mAmAmGmUmU mAmCmAmAdAmAfGmCf AmAmAmA*mC*mG |
| 11042.1-1 | 11042SM1 | mC*mU*mUmUfGmGf GfUdCmUmGmUmCm CmUmCmUmCmA | 11042AM1 | VPU*fG*mAmGmAmGmG mAmCmAmGdAmCfCmCfA mAmAmG*mG*mA |
| 11042.1-15 | 11042SM1 5 | Invab*mC*mUmUmUm GmGfGfUdCmUfGmU mCmCmUmCmUmCm AInvab | 11042AM1 | VPU*fG*mAmGmAmGmG mAmCmAmGdAmCfCmCfA mAmAmG*mG*mA |
| 11042.1-17 | 11042SM1 7 | Invab*mC*mUmUmUm GmGfGfUfCfUmGfUm CmCmUmCmUmCmAI nvab | 11042AM1 | VPU*fG*mAmGmAmGmG mAmCmAmGdAmCfCmCfA mAmAmG*mG*mA |
| 11042.2-2 | 11042SM2 | mC*mC*mUmUmUmU fGmGfGfUdCmUmGm UmCmCmUmCmUmC mA | 11042AM2 | VPU*fG*mAmGmAmGmG mAmCmAmGdAmCfCmCfA mAmAmA*mG*mG |
| 11043.1-1 | 11043SM1 | mC*mC*mUmUfUmUf GfGdGmUmCmUmGm UmCmCmUmCmA | 11043AM1 | VPU*fG*mAmGmGmAmC mAmGmAmCdCmCfAmAf AmAmGmG*mU*mA |
| 11043.2-2 | 11043SM2 | mC*mA*mUmCmUmU fUmUfGfGdGmUmCm UmGmUmCmCmUmC mA | 11043AM2 | VPU*fG*mAmGmGmAmC mAmGmAmCdCmCfAmAf AmAmGmA*mU*mG |
| 11044.1-1 | 11044SM1 | mC*mA*mCmAmUmU fUmAfUfCdTmUmUm UmGmGmGmUmCmU mA | 11044AM1 | VPU*fA*mGmAmCmCmC mAmAmAmAdGmAfUmAf AmAmUmG*mU*mG |
| 11045.1-1 | 11045SM1 | mG*mG*mAmCmAmU fUmUfAfUdCmUmUm UmUmGmGmGmUmC mA | 11045AM1 | VPU*fG*mAmCmCmCmA mAmAmAmGdAmUfAmAf AmUmGmU*mC*mC |
| 11046.1-1 | 11046SM1 | mG*mG*mAmCfAmUf UfUdAmUmCmUmUm UmUmGmGmGmA | 11046AM1 | VPU*fC*mCmCmAmAmA mAmGmAmUdAmAfAmUf GmUmCmC*mG*mC |
| 11046.1-2 | 11046SM2 | mG*mC*mGmGmAmC fAmUfUfUdAmUmCm UmUmUmUmGmGmG mA | 11046AM1 | VPU*fC*mCmCmAmAmA mAmGmAmUdAmAfAmUf GmUmCmC*mG*mC |
| 11047.1-1 | 11047SM1 | mG*mC*mCmCmUmCf UmCfUfGdTmUmGmC mCmUmUmUmUmUm A | 11047AM1 | VPU*fA*mAmAmAmAmG mGmCmAmAdCmAfGmAf GmAmGmG*mG*mC |
| 11048.1-1 | 11048SM1 | Invab*mA*mCmUmCf UmGfGfGdTmUmUmU mGmUmAmGmCmAm AInvab | 11048AM1 | VPU*fU*mGmCmUmAmC mAmAmAmAdCmCfCmAf GmAmGmU*mG*mG |
| 11048.1-2 | 11048SM2 | Invab*mA*mCmUmCm UmGfGfGdTmUfUmU mGmUmAmGmCmAm AInvab | 11048AM1 | VPU*fU*mGmCmUmAmC mAmAmAmAdCmCfCmAf GmAmGmU*mG*mG |
| 11049.1-1 | 11049SM1 | Invab*mC*mUmCmUf GmGfGfUdTmUmUmG mUmAmGmCmAmUm AInvab | 11049AM1 | VPU*fA*mUmGmCmUmA mCmAmAmAdAmCfCmCfA mGmAmG*mU*mU |
| 11049.1-2 | 11049SM2 | Invab*mC*mUmCmUm GmGfGfUdTmUfUmG mUmAmGmCmAmUm AInvab | 11049AM1 | VPU*fA*mUmGmCmUmA mCmAmAmAdAmCfCmCfA mGmAmG*mU*mU |
| 11050.1-1 | 11050SM1 | Invab*mC*mCmUmGf GmGfUfUdTmUmGmU mAmGmCmAmUmUm AInvab | 11050AM1 | VPU*fA*mAmUmGmCmU mAmCmAmAdAmAfCmCfC mAmGmG*mU*mU |
| 11050.1-2 | 11050SM2 | Invab*mC*mCmUmGm GmGfUfUdTmUfGmU mAmGmCmAmUmUm AInvab | 11050AM1 | VPU*fA*mAmUmGmCmU mAmCmAmAdAmAfCmCfC mAmGmG*mU*mU |

### Embodiment 7 Experimental Results of In Vitro Activity and Stability of siRNA Targeting PCSK9

Humanized PCSK9 mice were purchased from Shanghai Model Organisms Center, Inc. The experimental method for extracting primary hepatocytes and the qPCR assay for detecting target genes in cells were based on Embodiment 2.

The primers for detecting PCSK9 are as follows:
Forward primer: ACGTGGCTGGCATTGCA (SEQ ID NO: 708)
Reverse primer: AAGTGGATCAGTCTCTGCCTCAA (SEQ ID NO: 709)
Probe: CATGATGCTGTCTGCCGAGCCG (SEQ ID NO: 710) (Reporter gene 5'NED, Quencher group 3'BHQ1)

**Table 20: Experimental Results of Different Concentrations of Modified siRNA Transfection via Liposome in Hep3B Cells**

| siRNA | Residual PCSK9 mRNA Level (%) | | | |
|---|---|---|---|---|
| | Mean at 1 nM | SD | Mean at 0.1 nM | SD |
| 11000PM | 12.6 | 1.7 | 29.5 | 4.8 |
| 11001.1-1 | 9.2 | 0.5 | 26.9 | 5.6 |
| 11002.1-1 | 8.8 | 0.6 | 22.4 | 8.2 |
| 11003.1-1 | 8.3 | 0.4 | 19.9 | 3.9 |
| 11004.1-1 | 6.2 | 0.7 | 25.6 | 12.8 |
| 11005.1-1 | 16.7 | 1.4 | 70.3 | 23.4 |
| 11006.1-1 | 14.7 | 1.5 | 64.2 | 5.6 |
| 11007.1-1 | 7.5 | 1.3 | 15.2 | 5.1 |
| 11008.1-1 | 7.3 | 2.7 | 26.1 | 12.1 |
| 11009.1-1 | 9 | 1.6 | 15.5 | 1.6 |
| 11010.1-1 | 9.5 | 1.9 | 27.1 | 1.8 |
| 11011.1-1 | 8.6 | 1.6 | 23.6 | 3.2 |
| 11012.1-1 | NA | NA | 30.2 | 4 |
| 11014.1-1 | 14.7 | 2.4 | 57.1 | 11.4 |
| 11015.1-1 | 46.7 | 2.9 | NA | NA |
| 11016.1-1 | 11.7 | 1.3 | 44.6 | 13.4 |
| 11017.1-1 | 19.4 | 2.6 | 69.5 | 14.7 |
| 11018.1-1 | 10.2 | 1.7 | 17.8 | 3.5 |
| 11019.1-1 | 10.4 | 1.3 | 47.5 | 3.3 |
| 11020.1-1 | 13.3 | 2.9 | 40.8 | 5.2 |
| 11021.1-1 | 8.7 | 0.2 | 17.9 | 2.2 |
| 11022.1-1 | 12.2 | 0.6 | 31.2 | 5.1 |
| 11023.1-1 | 9.2 | 0.7 | 30.6 | 4.6 |
| 11024.1-1 | 9.3 | 1.9 | 19.9 | 4.7 |
| 11025.1-1 | NA | NA | 80.3 | 13.5 |
| 11026.1-1 | 12.2 | 1.2 | 36.7 | 2.6 |
| 11027.1-1 | 16.8 | 1 | 63.2 | 1 |
| 11028.1-1 | 15.1 | 2.1 | 51 | 3.5 |
| 11030.1-1 | NA | NA | 28.2 | 4.2 |
| 11031.1-1 | 8.1 | 0.5 | 31.9 | 1.7 |
| 11032.1-1 | NA | NA | 95.2 | 7.7 |
| 11033.1-1 | NA | NA | 76.9 | 10.3 |
| 11034.1-1 | NA | NA | 21.4 | 3.5 |
| 11035.1-1 | NA | NA | 20.7 | 1 |
| 11036.1-1 | NA | NA | 23.5 | 2.8 |
| 11037.1-1 | NA | NA | 86.1 | 9.8 |
| 11038.1-1 | NA | NA | 73.4 | 9.3 |
| 11039.1-1 | NA | NA | 106 | 14.7 |
| 11040.1-1 | NA | NA | 16.4 | 4.5 |
| 11041.1-1 | NA | NA | 17.2 | 2.3 |
| 11042.1-1 | NA | NA | 23.5 | 2.4 |
| 11043.1-1 | NA | NA | 42.7 | 10.1 |
| 11044.1-1 | NA | NA | 86.4 | 14.6 |
| 11045.1-1 | NA | NA | 83.6 | 8.7 |
| 11046.1-1 | NA | NA | 83.1 | 4.6 |

| | | | | |
|---|---|---|---|---|
| NA means not tested | | | | |

**Table 21: Experimental Results of Different Concentrations of Modified siRNA in Primary Hepatocytes from Humanized PCSK9 Mice Transfected by Liposome or Delivered via L96**

| siRNA | Residual PCSK9 mRNA Level (%) | | | |
|---|---|---|---|---|
| | Mean at 0.1 nM/Liposome Transfection | SD | Mean at 1 nM/Free Uptake | SD |
| 11000PM | 50.4 | 0.6 | 70.7 | 7.3 |
| 11001.1-1 | 37.4 | 4.3 | 63.9 | 5.9 |
| 11002.1-1 | 26.4 | 2.7 | 43.3 | 6.2 |
| 11003.1-1 | 17.7 | 2.1 | 39.9 | 6 |
| 11004.1-1 | 29.6 | 4.4 | 57.6 | 7.8 |
| 11007.1-1 | 19.7 | 0.7 | 43.8 | 1.7 |
| 11008.1-1 | 44.3 | 8.9 | 65.7 | 5.3 |
| 11009.1-1 | 36.3 | 7 | 60.3 | 5.4 |
| 11010.1-1 | 32 | 2.6 | 58.9 | 2.1 |
| 11011.1-1 | 28.9 | 4 | 47.2 | 1.1 |
| 11012.1-1 | 43 | 1.4 | 65.7 | 9.9 |
| 11016.1-1 | 67.1 | 5.6 | 90.1 | 7.1 |
| 11018.1-1 | 40.5 | 1.4 | 73.6 | 8.9 |
| 11019.1-1 | 55.3 | 0.7 | 84 | 5.7 |
| 11020.1-1 | 71.3 | 6.2 | 94.3 | 7.1 |
| 11021.1-1 | 32 | 2.6 | 66.2 | 8.9 |
| 11022.1-1 | 38.3 | 1.1 | 68.9 | 11 |
| 11023.1-1 | 30.6 | 4.6 | 50.3 | 3.2 |
| 11024.1-1 | 29.2 | 1.2 | 55.8 | 2.4 |
| 11026.1-1 | 40.6 | 1.5 | 61.6 | 5.1 |
| 11028.1-1 | 46.8 | 2.3 | 66.7 | 2.8 |

**Table 22: Experimental Results of 10 nM Modified siRNA Delivered via L96 in Humanized PCSK9 Mouse Primary Hepatocytes**

| siRNA | Residual PCSK9 mRNA Level (%) | |
|---|---|---|
| | Mean | SD |
| 11000PM | 26.3 | 7.8 |
| 11029.1-1 | 44.6 | 6 |
| 11030.1-1 | 32.2 | 3.6 |
| 11031.1-1 | 46 | 6.7 |
| 11034.1-1 | 26.2 | 2.5 |
| 11035.1-1 | 21.1 | 2.2 |
| 11036.1-1 | 31.2 | 3.4 |
| 11040.1-1 | 16.6 | 5.6 |
| 11041.1-1 | 20.3 | 5.4 |
| 11042.1-1 | 21.1 | 5 |
| 11043.1-1 | 32.8 | 1 |
| 11047.1-1 | 46.9 | 7.9 |

*In vitro* activity data from Hep3B and humanized PCSK9 mouse primary hepatocytes showed that multiple sequences, including 11002.1-1, 11040.1-1, and 11042.1-1, exhibited superior activity compared to the positive control. Next, we tested the activity of these sequences in humanized PCSK9 mice.

### Embodiment 8 Evaluation of the Effect of a Single Subcutaneous Injection of 1 mg/kg siRNA on PCSK9 Protein Expression in Humanized PCSK9 Mice

### Experimental Method:

### 1. Grouping and Administration

Blood samples were collected from mice via submandibular vein puncture before the experiment. Whole blood was obtained, allowed to stand at room temperature for 30 min, then centrifuged at 1000 × g for 10 min. The supernatant serum was collected, aliquoted, and stored frozen at -80°C. Serum samples were diluted 1000-fold, and PCSK9 expression in mouse serum was analyzed using the Human PCSK9 ELISA Kit (Proteintech, Cat. No: KE00278). Mice were equally divided into groups based on PCSK9 expression levels, with 4 mice per group. siRNA was dissolved in normal saline and adjusted to a concentration of 0.1 mg/mL. Mice received a single subcutaneous injection of the siRNA solution at a dose of 1 mg/kg.

### 2. ELISA Test

Following siRNA injection, a small amount of blood samples was collected from the submandibular vein at regular intervals. Samples were allowed to stand at room temperature for 30 min and centrifuged at 1000 × g for 10 min. The supernatant serum was collected, diluted 1000-fold, and analyzed for PCSK9 expression by ELISA.

The experimental results are shown in Table 23.

**Table 23: Experimental Results of PCSK9-targeting siRNA Inhibition of PCSK9 Protein Expression Level in Humanized PCSK9 Mice at 1 mg/kg**

| siRNA | Mean PCSK9 Protein Expression Level (%) (relative to the predose level) | | | | | | | | | |
|---|---|---|---|---|---|---|---|---|---|---|
| | Day 14 | SD | Day 19 | SD | Day 30 | SD | Day 40 | SD | Day 55 | SD |
| 11000PM | 48.6 | 13.8 | 43.3 | 6.6 | 35.4 | 3.6 | 89.7 | 5.7 | 90.3 | 19.2 |
| 11002.1-8 | 37.2 | 11.3 | 37 | 6.7 | 32.9 | 7.2 | 53.2 | 11.3 | 52.4 | 8.8 |
| 11003.1-8 | 45.9 | 5.2 | 39.7 | 0.4 | 42.3 | 2.2 | 76.9 | 4.3 | 82.3 | 2.9 |
| 11024.1-15 | 52.3 | 4 | 49.4 | 0.8 | 53.1 | 6.1 | 75.8 | 11.6 | 84.8 | 11.1 |
| 11040.1-6 | 32.1 | 5 | 30.7 | 5.8 | 25.2 | 1.2 | 43.3 | 4.3 | 51.4 | 5.1 |
| 11040.1-8 | 32.9 | 6.3 | 33.1 | 3.8 | 34.1 | 5.5 | 57.5 | 9 | 67.4 | 18.4 |
| 11042.1-15 | 34.9 | 3.5 | 33.4 | 9.4 | 29.1 | 8.1 | 45.6 | 9.3 | 50 | 7.2 |

Data showed that serum PCSK9 protein inhibition reached its peak at Day 30 postdose, with subsequent recovery. The positive control group retained approximately 89.7% of PCSK9 protein at Day 40, with an inhibition rate of about 10.3%. All other experimental groups exhibited higher inhibition rates than the positive control group, particularly groups 11040.1-6 and 11042.1-15, both achieving inhibition rates exceeding 50%, while group 11002.1-8 achieved 46.8% inhibition. By Day 55, group 11042.1-15 maintained 50% inhibition. The activity of 11040.1-6 was higher than that of 11040.1-8, indicating that even with the same sequence, different modifications exhibit varying activities *in vivo.*

### Embodiment 9 Safety Testing of siRNA Targeting PCSK9

The safety testing method for siRNA is detailed in Embodiment 4, with experimental results presented in Table 24

**Table 24: Safety Experimental Results of siRNA Targeting PCSK9**

| siRNA | ALT (U/L) | AST (U/L) |
|---|---|---|
| Normal saline | 47 | 154 |
| 11002.7-14 | 47 | 172 |
| 11040.3-10 | 39 | 85 |
| 11042.1-15 | 526 | 320 |

| | | |
|---|---|---|
| Note: Data for 11042.1-15 corresponds to testing conducted 10 days after the second injection. | | |

Table 24 showed that 11002.7-14 and 11040.3-10 exhibited excellent safety profiles in mice, whereas 11042.1-15 significantly impacted mouse liver function.

### Embodiment 10 Effects of Different Modifications and Base Alterations on the Activity and Stability of Sequences 1002 and 11040

**Table 25: Experimental Results of 1 nM Modified siRNA Delivered via L96 in Humanized PCSK9 Mouse Primary Hepatocytes**

| siRNA | Residual PCSK9 mRNA Level (%) | |
|---|---|---|
| | Mean | SD |
| 11000PM | 19.9 | 5.3 |
| 11040.1-6 | 11.8 | 0.6 |
| 11040.1-8 | 11.4 | 2.1 |
| 11040.1-23 | 7.7 | 1.1 |

Table 25 showed that for the same sequence, different sense strand modifications significantly impacted activity. The activity of the modified sequence 11040.1-23 was optimal.

**Table 26: Experimental Results of 3 nM Modified siRNA Delivered via L96 in Humanized PCSK9 Mouse Primary Hepatocytes**

| siRNA | Residual PCSK9 mRNA Level (%) | |
|---|---|---|
| | Mean | SD |
| 11002.1-8 | 10.2 | 3.5 |
| 11002.3-10 | 11.0 | 1.8 |
| 11002.5-14 | 9.8 | 2.9 |
| 11002.7-14 | 9.5 | 3.6 |
| 11040.1-6 | 13.4 | 5.4 |
| 11040.3-10 | 8.9 | 3.3 |
| 11040.5-10 | 11.7 | 2.2 |
| 11040.7-18 | 10.1 | 0.7 |

Table 26 showed that base and modification alterations in different sequences resulted in varying changes in activity.

**Table 27: Metabolic Stability Data in Liver Homogenates for Different Modified siRNAs**

| siRNA | Residual Percentage of Antisense Strand at 72 h (%) |
|---|---|
| 11000PM | 68.26 |
| 11002.1-1 | 73.45 |
| 11002.3-10 | 70.31 |
| 11002.5-14 | 63.18 |
| 11002.7-14 | 86.41 |
| 11040.1-1 | 107.97 |
| 11040.3-10 | 48.69 |
| 11040.5-10 | 24.93 |
| 11040.7-18 | 25.32 |

Table 27 showed that different sequences exhibited varying degrees of effects on stability following modification and base alterations. Among these, 11002.7-14 and 11040.1-1 demonstrated exceptional stability in liver homogenate, whereas 11002.5-14, 11040.5-10, and others showed significantly reduced stability.

**Table 28: Experimental Results of Different Concentrations of Modified siRNA in Primary Hepatocytes from Humanized PCSK9 Mice Delivered via L96 or Transfected by Liposome**

| siRNA | Residual PCSK9 mRNA Level (%) | | | |
|---|---|---|---|---|
| | Mean at 10 nM/Free Uptake | SD | Mean at 1 nM/Liposome Transfection | SD |
| 11002.7-14 | 13.2 | 4.1 | 13.2 | 4.1 |
| 11002.7-21 | 10.9 | 2.2 | 10.9 | 2.2 |
| 11040.1-8 | 15.5 | 1.2 | 15.5 | 1.2 |
| 11040.1-23 | 12.5 | 2.3 | 12.5 | 2.3 |

**Table 29: Experimental Results of Different Concentrations of Modified siRNA in Primary Hepatocytes from Humanized PCSK9 Mice Delivered via L96 or Transfected by Liposome**

| siRNA | Residual PCSK9 mRNA Level (%) | | | |
|---|---|---|---|---|
| | Mean at 3 nM/Free Uptake | SD | Mean at 0.3 nM/Liposome Transfection | SD |
| 11002.9-8 | 14.4 | 3.7 | 15.3 | 1.9 |
| 11002.1-8 | 6.9 | 0.6 | 8.4 | 1.0 |
| 11002.10-8 | 5.9 | 0.5 | 3.9 | 0.7 |
| 11040.9-6 | 35.1 | 4.1 | 30.3 | 10.5 |
| 11040.1-6 | 12.0 | 1.1 | 3.9 | 1.0 |
| 11040.10-6 | 6.3 | 1.4 | 3.4 | 0.4 |

**Table 30: Experimental Results of 0.3 nM Modified siRNA Delivered via Liposomes in AVV8-hPCSK9 Mouse Primary Henatocytes**

| siRNA | Residual PCSK9 mRNA Level (%) | |
|---|---|---|
| | Mean | SD |
| 11000PM | 51.5 | 8.2 |
| 11002.17-21 | 19.9 | 4.2 |
| 11002.16-21 | 55.0 | 8.5 |
| 11002.23-22 | 23.2 | 4.0 |
| 11002.10-23 | 23.8 | 5.7 |

**Table 31: Experimental Results of 0.1 nM Modified siRNA Transfected via Liposomes in AVV8-hPCSK9 Mouse Primary Hepatocytes**

| siRNA | Residual PCSK9 mRNA Level (%) | |
|---|---|---|
| | Mean | SD |
| 11000PM | 62.0 | 4.9 |
| 11040.10-23 | 30.6 | 2.2 |
| 11040.9-23 | 61.3 | 5.0 |

**Table 32: Experimental Results of Different Concentrations of Modified siRNA Delivered via L96 in AVV8-hPCSK9 Mouse Primary Hepatocytes**

| siRNA | Residual PCSK9 mRNA Level (%) | | | |
|---|---|---|---|---|
| | Mean at 1 nM | SD | Mean at 0.1 nM | SD |
| 11000PM | 21.9 | 2.3 | 43.3 | 1.4 |
| 11002.17-21 | 14.1 | 1.1 | 22.9 | 2.6 |
| 11002.16-21 | 20.2 | 4.1 | 46.0 | 5.1 |
| 11002.10-23 | 16.6 | 3.9 | 23.5 | 2.7 |
| 11040.10-23 | 15.3 | 1.7 | 27.9 | 7.5 |
| 11040.9-23 | 23.0 | 0.2 | 68.0 | 2.2 |

The experimental data in Tables 28-32 demonstrate that we optimized the 11002 and 11040 sequences in multiple aspects, including both sense and antisense strands. Results showed that the activity of the preferred sequences 11002.17-21, 11002.10-23, and 11040.10-23 all outperformed the positive control 11000PM. Next, we validated the activity of preferred sequences *in vivo.*

**Table 33: Experimental Results of PCSK9-targeting siRNA Inhibition of PCSK9 Protein Expression Level in Humanized PCSK9 Mice at 1 mg/kg**

| siRNA | Mean PCSK9 Protein Expression Level (%) (relative to the predose level) | | | | | |
|---|---|---|---|---|---|---|
| | Day 13/Mean | SD | Day 22/Mean | SD | Day 36/Mean | SD |
| 11000PM | 40.7 | 10.5 | 53.1 | 14.5 | 74.8 | 15.4 |
| 11002.17-21-SL01 | 21.8 | 2.1 | 27.5 | 3.5 | 51.2 | 4.6 |
| 11040.10-23-SL01 | 20.5 | 4.3 | 29.7 | 6.8 | 50.0 | 18.2 |

Table 33 showed that 11002.17-21 and 11040.10-23 exhibited significantly superior *in vivo* activity compared to the positive control 1000PM.

### Embodiment 11 Synthesis of siRNA Targeting ANGPTL3

The specific synthesis method was based on that in Embodiment 1.

**Table 34: Sequences of Sense and Antisense Strands of Unmodified siRNA Targeting ANGPTL3**

| siRNA Name | Sense Strand Name | Sense Strand Sequence 5'-3' | Antisense Strand Name | Antisense Strand Sequence 5'-3' |
|---|---|---|---|---|
| 7001a | 7001a-SS | UCCAGAAUUGAUCAAGACAAA (SEQ ID NO: 409) | 7001a-AS | UUUGUCUUGAUCAAUUCUGGAGG (SEQ ID NO: 410) |
| 7002a | 7002a-SS | UUCCUCCAGAAUUGAUCAAGA (SEQ ID NO: 411) | 7002a-AS | UCUUGAUCAAUUCUGGAGGAAAU (SEQ ID NO: 412) |
| 7003a | 7003a-SS | CAGAGCCAAAAUCAAGAUUUA (SEQ ID NO: 413) | 7003a-AS | UAAAUCUUGAUUUUGGCUCUGGA (SEQ ID NO: 414) |
| 7003b | 7003b-SS | CGGAGCCAAAAUCAAGAUUUA (SEQ ID NO: 415) | 7003b-AS | UAAAUCUUGAUUUUGGCUUUGGA (SEQ ID NO: 416) |
| 7003c | 7003c-SS | CGGAGCCAAAAUCAAGAUUUA (SEQ ID NO: 417) | 7003c-AS | UAAAUCUUGAUUUUGGCUCCG (SEQ ID NO: 418) |
| 7004a | 7004a-SS | GAUCAAGACAAUUCAUCAUUA (SEQ ID NO: 419) | 7004a-AS | UAAUGAUGAAUUGUCUUGAUCAA (SEQ ID NO: 420) |
| 7005a | 7005a-SS | UAGUUAUUUCCUCCAGAAUUA (SEQ ID NO: 421) | 7005a-AS | UAAUUCUGGAGGAAAUAACUAGA (SEQ ID NO: 422) |
| 7006a | 7006a-SS | AAGGGCCAAAUUAAUGACAUA (SEQ ID NO: 423) | 7006a-AS | UAUGUCAUUAAUUUGGCCCUUCG (SEQ ID NO: 424) |
| 7007a | 7007a-SS | AAGAUUUGCUAUGUUAGACGA (SEQ ID NO: 425) | 7007a-AS | UCGUCUAACAUAGCAAAUCUUGA (SEQ ID NO: 426) |
| 7008a | 7008a-SS | AUGUUAGACGAUGUAAAAAUA (SEQ ID NO: 427) | 7008a-AS | UAUUUUUACAUCGUCUAACAUAG (SEQ ID NO: 428) |
| 7009a | 7009a-SS | GACUUUGUCCAUAAGACGAAA (SEQ ID NO: 429) | 7009a-AS | UUUCGUCUUAUGGACAAAGUCUU (SEQ ID NO: 430) |
| 7010a | 7010a-SS | UUGGGACAUGGUCUUAAAGAA (SEQ ID NO: 431) | 7010a-AS | UUCUUUAAGACCAUGUCCCAACU (SEQ ID NO: 432) |
| 7011a | 7011a-SS | CUUAAAACUUUUGUAGAAAAA (SEQ ID NO: 433) | 7011a-AS | UUUUUCUACAAAAGUUUUAAGUG (SEQ ID NO434) |
| 7012a | 7012a-SS | CUGCAAACCAGUGAAAUCAAA (SEQ ID NO: 435) | 7012a-AS | UUUGAUUUCACUGGUUUGCAGCG (SEQ ID NO: 436) |
| 7013a | 7013a-SS | UUUUUAUGAUCUAUCGCUGCA (SEQ ID NO: 437) | 7013a-AS | UGCAGCGAUAGAUCAUAAAAAGA (SEQ ID NO: 438) |
| 7014a | 7014a-SS | AACCAGUGAAAUCAAAGAAGA (SEQ ID NO: 439) | 7014a-AS | UCUUCUUUGAUUUCACUGGUUUG (SEQ ID NO: 440) |
| 7014b | 7014b-SS | GACCAGUGAAAUCAAAGAAGA (SEQ ID NO: 441) | 7014b-AS | UCUUCUUUGAUUUCACUGGUCUG (SEQ ID NO: 442) |
| 7014c | 7014c-SS | AACCAGUGAAAUCAAAGAAGA (SEQ ID NO: 443) | 7014c-AS | UCUUCUUUGAUUUCACUGGUU (SEQ ID NO: 444) |
| 7014d | 7014d-SS | GACCAGUGAAAUCAAAGAAGA (SEQ ID NO: 445) | 7014d-AS | UCUUCUUUGAUUUCACUGGUC (SEQ ID NO: 446) |
| 7015a | 7015a-SS | AGAAAAGGAACUGAGAAGAAA (SEQ ID NO: 447) | 7015a-AS | UUUCUUCUCAGUUCCUUUUCUUC (SEQ ID NO: 448) |
| 7016a | 7016a-SS | CAAGUCAAAAAUGAAGAGGUA (SEQ ID NO: 449) | 7016a-AS | UACCUCUUCAUUUUUGACUUGUA (SEQ ID NO: 450) |
| 7017a | 7017a-SS | UAAACUACAAGUCAAAAAUGA (SEQ ID NO: 451) | 7017a-AS | UCAUUUUUGACUUGUAGUUUAUA (SEQ ID NO: 452) |
| 7018a | 7018a-SS | GAGGUAAAGAAUAUGUCACUA (SEQ ID NO: 453) | 7018a-AS | UAGUGACAUAUUCUUUACCUCUU (SEQ ID NO: 454) |
| 7019a | 7019a-SS | GGUAAAGAAUAUGUCACUUGA (SEQ ID NO: 455) | 7019a-AS | UCAAGUGACAUAUUCUUUACCUC (SEQ ID NO: 456) |
| 7020a | 7020a-SS | UAAAGAAUAUGUCACUUGAAA (SEQ ID NO: 457) | 7020a-AS | UUUCAAGUGACAUAUUCUUUACC (SEQ ID NO: 458) |
| 7021a | 7021a-SS | UUGAACUCAACUCAAAACUUA (SEQ ID NO: 459) | 7021a-AS | UAAGUUUUGAGUUGAGUUCAAGU (SEQ ID NO: 460) |
| 7021b | 7021b-SS | CUGAACUCAACUCAAAACUUA (SEQ ID NO: 461) | 7021b-AS | UAAGUUUUGAGUUGAGUUCAG (SEQ ID NO: 462) |
| 7021c | 7021c-SS | UCGAACUCAACUCAAAACUUA (SEQ ID NO: 463) | 7021c-AS | UAAGUUUUGAGUUGAGUUCGA (SEQ ID NO: 464) |
| 7022a | 7022a-SS | AGAAGAAAAAAUUCUACUUCA (SEQ ID NO: 465) | 7022a-AS | UGAAGUAGAAUUUUUUCUUCUAG (SEQ ID NO: 466) |
| 7023a | 7023a-SS | GAAGAAAAAAUUCUACUUCAA (SEQ ID NO: 467) | 7023a-AS | UUGAAGUAGAAUUUUUUCUUCUA (SEQ ID NO: 468) |
| 7024a | 7024a-SS | CAAAAUCAACCUGAAACUCCA (SEQ ID NO: 469) | 7024a-AS | UGGAGUUUCAGGUUGAUUUUGAA (SEQ ID NO: 470) |
| 7025a | 7025a-SS | CAACCUGAAACUCCAGAACAA (SEQ ID NO: 471) | 7025a-AS | UUGUUCUGGAGUUUCAGGUUGAU (SEQ ID NO: 472) |
| 7026a | 7026a-SS | GAAAAACAAGAUAAUAGCAUA (SEQ ID NO: 473) | 7026a-AS | UAUGCUAUUAUCUUGUUUUUCUA (SEQ ID NO: 474) |
| 7027a | 7027a-SS | GAAGACCAAUAUAAACAAUUA (SEQ ID NO: 475) | 7027a-AS | UAAUUGUUUAUAUUGGUCUUCCA (SEQ ID NO: 476) |
| 7028a | 7028a-SS | UGGAAGACCAAUAUAAACAAA (SEQ ID NO: 477) | 7028a-AS | UUUGUUUAUAUUGGUCUUCCACG (SEQ ID NO: 478) |
| 7029a | 7029a-SS | CUCAGAAGGACUAGUAUUCAA (SEQ ID NO: 479) | 7029a-AS | UUGAAUACUAGUCCUUCUGAGCU (SEQ ID NO: 480) |
| 7030a | 7030a-SS | AGAAGGACUAGUAUUCAAGAA (SEQ ID NO: 481) | 7030a-AS | UUCUUGAAUACUAGUCCUUCUGA (SEQ ID NO: 482) |
| 7031a | 7031a-SS | AUUUCUCUAUCUUCCAAGCCA (SEQ ID NO: 483) | 7031a-AS | UGGCUUGGAAGAUAGAGAAAUUU (SEQ ID NO: 484) |
| 7032a | 7032a-SS | UUUCUCUAUCUUCCAAGCCAA (SEQ ID NO: 485) | 7032a-AS | UUGGCUUGGAAGAUAGAGAAAUU (SEQ ID NO: 486) |
| 7033a | 7033a-SS | CCUUUCUUCAGUUGAAUGAAA (SEQ ID NO: 487) | 7033a-AS | UUUCAUUCAACUGAAGAAAGGGA (SEQ ID NO: 488) |
| 7034a | 7034a-SS | GUUGAAUGAAAUAAGAAAUGA (SEQ ID NO: 489) | 7034a-AS | UCAUUUCUUAUUUCAUUCAACUG (SEQ ID NO: 490) |
| 7035a | 7035a-SS | AUGUACCACCAUUUAUAACAA (SEQ ID NO: 491) | 7035a-AS | UUGUUAUAAAUGGUGGUACAUUC (SEQ ID NO: 492) |
| 7036a | 7036a-SS | UGUACCACCAUUUAUAACAGA (SEQ ID NO: 493) | 7036a-AS | UCUGUUAUAAAUGGUGGUACAUU (SEQ ID NO: 494) |
| 7037a | 7037a-SS | GUCCAUGGACAUUAAUUCAAA (SEQ ID NO: 495) | 7037a-AS | UUUGAAUUAAUGUCCAUGGACUA (SEQ ID NO: 496) |
| 7038a | 7038a-SS | CGUGGGAGAACUACAAAUAUA (SEQ ID NO: 497) | 7038a-AS | UAUAUUUGUAGUUCUCCCACGUU (SEQ ID NO: 498) |
| 7039a | 7039a-SS | GUUUUACGAAUUGAGUUGGAA (SEQ ID NO: 499) | 7039a-AS | UUCCAACUCAAUUCGUAAAACAU (SEQ ID NO: 500) |
| 7040a | 7040a-SS | CAACUAUACGCUACAUCUAGA (SEQ ID NO: 501) | 7040a-AS | UCUAGAUGUAGCGUAUAGUUGGU (SEQ ID NO: 502) |
| 7041a | 7041a-SS | UACGCUACAUCUAGUUGCGAA (SEQ ID NO: 503) | 7041a-AS | UUCGCAACUAGAUGUAGCGUAUA (SEQ ID NO: 504) |
| 7042a | 7042a-SS | UCUAGUUGCGAUUACUGGCAA (SEQ ID NO: 505) | 7042a-AS | UUGCCAGUAAUCGCAACUAGAUG (SEQ ID NO: 506) |
| 7043a | 7043a-SS | CUAGUUGCGAUUACUGGCAAA (SEQ ID NO: 507) | 7043a-AS | UUUGCCAGUAAUCGCAACUAGAU (SEQ ID NO: 508) |
| 7044a | 7044a-SS | UCUACUUGGGAUCACAAAGCA (SEQ ID NO: 509) | 7044a-AS | UGCUUUGUGAUCCCAAGUAGAAA (SEQ ID NO: 510) |
| 7045a | 7045a-SS | UCUUGGAAGUCUCAAAAUGGA (SEQ ID NO: 511) | 7045a-AS | UCCAUUUUGAGACUUCCAAGAUA (SEQ ID NO: 512) |
| 7046a | 7046a-SS | ACUCUAUAAAAUCAACCAAAA (SEQ ID NO: 513) | 7046a-AS | UUUUGGUUGAUUUUAUAGAGUAU (SEQ ID NO: 514) |
| 7047a | 7047a-SS | UAUCUUGGAAGUCUCAAAAUA (SEQ ID NO: 515) | 7047a-AS | UAUUUUGAGACUUCCAAGAUAAU (SEQ ID NO: 516) |
| 7048a | 7048a-SS | CAUAUUUGAUCAGUCUUUA (SEQ ID NO: 517) | 7048a-AS | UAAAGACUGAUCAAAUAUGUU (SEQ ID NO: 518) |
| 7049a | 7049a-SS | AUCAAGAUUUGCUAUGUUA (SEQ ID NO: 519) | 7049a-AS | UAACAUAGCAAAUCUUGAUUU (SEQ ID NO: 520) |
| 7049b | 7049b-SS | GUCAAGAUUUGCUAUGUUA (SEQ ID NO: 521) | 7049b-AS | UAACAUAGCAAAUCUUGACUU (SEQ ID NO: 522) |
| 7049c | 7049c-SS | AAAUCAAGAUUUGCUAUGUUA (SEQ ID NO: 523) | 7049c-AS | UAACAUAGCAAAUCUUGAUUU (SEQ ID NO: 524) |
| 7049d | 7049d-SS | GAAUCAAGAUUUGCUAUGUUA (SEQ ID NO: 525) | 7049d-AS | UAACAUAGCAAAUCUUGAUUC (SEQ ID NO: 526) |
| 7050a | 7050a-SS | AAUCAAGAUUUGCUAUGUA (SEQ ID NO: 527) | 7050a-AS | UACAUAGCAAAUCUUGAUUUU (SEQ ID NO: 528) |
| 7050b | 7050b-SS | GAUCAAGAUUUGCUAUGUA (SEQ ID NO: 529) | 7050b-AS | UACAUAGCAAAUCUUGAUCUU (SEQ ID NO: 530) |
| 7050c | 7050c-SS | GAAAUCAAGAUUUGCUAUGUA (SEQ ID NO: 531) | 7050c-AS | UACAUAGCAAAUCUUGAUUUC (SEQ ID NO: 532) |
| 7051a | 7051a-SS | AAAUCAAGAUUUGCUAUGA (SEQ ID NO: 533) | 7051a-AS | UCAUAGCAAAUCUUGAUUUUG (SEQ ID NO: 534) |
| 7051b | 7051b-SS | GAAUCAAGAUUUGCUAUGA (SEQ ID NO: 535) | 7051b-AS | UCAUAGCAAAUCUUGAUUCUG (SEQ ID NO: 536) |
| 7051c | 7051c-SS | CGAAAUCAAGAUUUGCUAUGA (SEQ ID NO: 537) | 7051c-AS | UCAUAGCAAAUCUUGAUUUCG (SEQ ID NO: 538) |
| 7052a | 7052a-SS | AACUCAACAUAUUUGAUCA (SEQ ID NO: 539) | 7052a-AS | UGAUCAAAUAUGUUGAGUUUU (SEQ ID NO: 540) |
| 7053a | 7053a-SS | UUUGAUCAGUCUUUUUAUA (SEQ ID NO: 541) | 7053a-AS | UAUAAAAAGACUGAUCAAAUA (SEQ ID NO: 542) |
| 7054a | 7054a-SS | CCAGUGAAAUCAAAGAAGA (SEQ ID NO: 543) | 7054a-AS | UCUUCUUUGAUUUCACUGGUU (SEQ ID NO: 544) |
| 7055a | 7055a-SS | GAUUUGCUAUGUUAGACGA (SEQ ID NO: 545) | 7055a-AS | UCGUCUAACAUAGCAAAUCUU (SEQ ID NO: 546) |
| 7056a | 7056a-SS | UCAACAUAUUUGAUCAGUCUA (SEQ ID NO: 547) | 7056a-AS | UAGACUGAUCAAAUAUGUUGAGU (SEQ ID NO: 548) |
| 7057a | 7057a-SS | GAGCCAAAAUCAAGAUUUGCA (SEQ ID NO: 549) | 7057a-AS | UGCAAAUCUUGAUUUUGGCUCUG (SEQ ID NO: 550) |
| 7058a | 7058a-SS | GGAUAGAUGGAUCACAAAACA (SEQ ID NO: 551) | 7058a-AS | UGUUUUGUGAUCCAUCUGUUCGA (SEQ ID NO: 552) |
| 7058b | 7058b-SS | GGAUAGAUGGAUCACAAAACA (SEQ ID NO: 553) | 7058b-AS | UGUUUUGUGAUCCAUCUGUCCGA (SEQ ID NO: 554) |
| 7058c | 7058c-SS | GGAUAGAUGGAUCACAAAACA (SEQ ID NO: 555) | 7058c-AS | UGUUUUGUGAUCCAUCUGUCC (SEQ ID NO: 556) |
| 7059a | 7059a-SS | CCUAAAGACUUUGUCCAUAAA (SEQ ID NO: 557) | 7059a-AS | UUUAUGGACAAAGUCUUUAAGAC (SEQ ID NO: 558) |
| 7059b | 7059b-SS | CCUAAAGACUUUGUCCAUAAA (SEQ ID NO: 559) | 7059b-AS | UUUAUGGACAAAGUCUUUAGGAC (SEQ ID NO: 560) |
| 7059c | 7059c-SS | CCUAAAGACUUUGUCCAUAAA (SEQ ID NO: 561) | 7059c-AS | UUUAUGGACAAAGUCUUUAGG (SEQ ID NO: 562) |
| 7060a | 7060a-SS | ACAUCAGGUAGUCCAUGGACA (SEQ ID NO: 563) | 7060a-AS | UGUCCAUGGACUACCUGAUAUAA (SEQ ID NO: 564) |
| 7060b | 7060b-SS | ACAUCAGGUAGUCCAUGGACA (SEQ ID NO: 565) | 7060b-AS | UGUCCAUGGACUACCUGAUGUAA (SEQ ID NO: 566) |
| 7060c | 7060c-SS | ACAUCAGGUAGUCCAUGGACA (SEQ ID NO: 567) | 7060c-AS | UGUCCAUGGACUACCUGAUGU (SEQ ID NO: 568) |
| 7061a | 7061a-SS | CGCUUGAACUCAACUCAAA (SEQ ID NO: 569) | 7061a-AS | UUUGAGUUGAGUUCAAGUGGC (SEQ ID NO: 570) |
| 7061b | 7061b-SS | GCCACUUGAACUCAACUCAAA (SEQ ID NO: 571) | 706lb-AS | UUUGAGUUGAGUUCAAGUGGC (SEQ ID NO: 572) |
| 7061c | 7061c-SS | CGCUUGAACUCAACUCAAA (SEQ ID NO: 573) | 7061c-AS | UUUGAGUUGAGUUCAAGCGAC (SEQ ID NO: 574) |
| 7061d | 7061d-SS | CGCUUGAACUCAACUCAAA (SEQ ID NO: 575) | 7061d-AS | UUUGAGUUGAGUUCAAGCG (SEQ ID NO: 576) |
| 7061e | 7061e-SS | CGCUUGAACUCAACUCAAA (SEQ ID NO: 577) | 7061e-AS | UUUGAGUUGAGUUCAAGCGGG (SEQ ID NO: 578) |
| 7061f | 7061f-SS | UACUUGAACUCAACUCAAA (SEQ ID NO: 579) | 7061f-AS | UUUGAGUUGAGUUCAAGUGGG (SEQ ID NO: 580) |
| 7062a | 7062a-SS | CACUACAUAUAAACUACAA (SEQ ID NO: 581) | 7062a-AS | UUGUAGUUUAUAUGUAGUGCU (SEQ ID NO: 582) |
| 7062b | 7062b-SS | GGAACUACAUAUAAACUACAA (SEQ ID NO: 583) | 7062b-AS | UUGUAGUUUAUAUGUAGUUCC (SEQ ID NO: 584) |
| 7063a | 7063a-SS | CGUAUAAACUACAAGUCAA (SEQ ID NO: 585) | 7063a-AS | UUGACUUGUAGUUUAUGUGUA (SEQ ID NO: 586) |
| 7063b | 7063b-SS | CGCAUAUAAACUACAAGUCAA (SEQ ID NO: 587) | 7063b-AS | UUGACUUGUAGUUUAUAUGCG (SEQ ID NO: 588) |
| 7064a | 7064a-SS | CCACAAAACUUCAAUGAAA (SEQ ID NO: 589) | 7064a-AS | UUUCAUUGAAGUUUUGUGGUC (SEQ ID NO: 590) |
| 7064b | 7064b-SS | GGUCACAAAACUUCAAUGAAA (SEQ ID NO: 591) | 7064b-AS | UUUCAUUGAAGUUUUGUGACC (SEQ ID NO: 592) |
| 7064c | 7064c-SS | CCACAAAACUUCAAUGAAA (SEQ ID NO: 593) | 7064c-AS | UUUCAUUGAAGUUUUGUGGUU (SEQ ID NO: 594) |
| 7065a | 7065a-SS | GGGUUAUACUCUAUAAAAA (SEQ ID NO: 595) | 7065a-AS | UUUUUAUAGAGUAUAACUUUC (SEQ ID NO: 596) |
| 7065b | 7065b-SS | GGAGGUUAUACUCUAUAAAAA (SEQ ID NO: 597) | 7065b-AS | UUUUUAUAGAGUAUAACCUCC (SEQ ID NO: 598) |
| 7066a | 7066a-SS | GGGAGGUUAUACUCUAUAA (SEQ ID NO: 599) | 7066a-AS | UUAUAGAGUAUAACCUUUCAU (SEQ ID NO: 600) |
| 7066b | 7066b-SS | ACGGAAGGUUAUACUCUAUAA (SEQ ID NO: 601) | 7066b-AS | UUAUAGAGUAUAACCUUCCGU (SEQ ID NO: 602) |
| 7067a | 7067a-SS | GCCUCAAAAUGGAAGGUUA (SEQ ID NO: 603) | 7067a-AS | UAACCUUCCAUUUUGAGGCUU (SEQ ID NO: 604) |
| 7067b | 7067b-SS | GAGUCUCAAAAUGGAAGGUUA (SEQ ID NO: 605) | 7067b-AS | UAACCUUCCAUUUUGAGACUC (SEQ ID NO: 606) |
| 7068a | 7068a-SS | GAGUCUCAAAAUGGAAGGUUA (SEQ ID NO: 607) | 7068a-AS | UAACCUUCCAUUUUGAGACUUUU (SEQ ID NO: 608) |
| 7068b | 7068b-SS | GAGUCUCAAAAUGGAAGGUUA (SEQ ID NO: 609) | 7068b-AS | UAACCUUCCAUUUUGAGACUC (SEQ ID NO: 610) |
| 7069a | 7069a-SS | ACGGAAGGUUAUACUCUAUAA (SEQ ID NO: 611) | 7069a-AS | UUAUAGAGUAUAACCUUCCGUUU (SEQ ID NO: 612) |
| 7069b | 7069b-SS | ACGGAAGGUUAUACUCUAUAA (SEQ ID NO: 613) | 7069b-AS | UUAUAGAGUAUAACCUUCCGU (SEQ ID NO: 614) |
| 7070a | 7070a-SS | GCAAAAUGUUGAUCCAUCA (SEQ ID NO: 615) | 7070a-AS | UGAUGGAUCAACAUUUUGCUU (SEQ ID NO: 616) |
| 7070b | 7070b-SS | GACCAAAAUGUUGAUCCAUCA (SEQ ID NO: 617) | 7070b-AS | UGAUGGAUCAACAUUUUGGUC (SEQ ID NO: 618) |
| 7071a | 7071a-SS | ACGUUGAUCCAUCCAACAGAA (SEQ ID NO: 619) | 7071a-AS | UUCUGUUGGAUGGAUCAACGUUU (SEQ ID NO: 620) |
| 7071b | 7071b-SS | ACGUUGAUCCAUCCAACAGAA (SEQ ID NO: 621) | 7071b-AS | UUCUGUUGGAUGGAUCAACGU (SEQ ID NO: 622) |
| 7072a | 7072a-SS | GCUAUACUCUAUAAAAUCAAA (SEQ ID NO: 623) | 7072a-AS | UUUGAUUUUAUAGAGUAUAGCCU (SEQ ID NO: 624) |
| 7072b | 7072b-SS | GCUAUACUCUAUAAAAUCAAA (SEQ ID NO: 625) | 7072b-AS | UUUGAUUUUAUAGAGUAUAGC (SEQ ID NO: 626) |
| 7072c | 7072c-SS | GCCAUACUCUAUAAAAUCAAA (SEQ ID NO: 627) | 7072c-AS | UUUGAUUUUAUAGAGUAUGGC (SEQ ID NO: 628) |
| 7072d | 7072d-SS | GCCAUACUCUAUAAAAUCAAA (SEQ ID NO: 629) | 7072d-AS | UUUGAUUUUAUAGAGUAUGGCCU (SEQ ID NO: 630) |
| 7073a | 7073a-SS | GGGUUAUACUCUAUAAAAUCA (SEQ ID NO: 631) | 7073a-AS | UGAUUUUAUAGAGUAUAACCUUU (SEQ ID NO: 632) |
| 7073b | 7073b-SS | GGGUUAUACUCUAUAAAAUCA (SEQ ID NO: 633) | 7073b-AS | UGAUUUUAUAGAGUAUAACCC (SEQ ID NO: 634) |
| 7074a | 7074a-SS | CGACCAAAAUGUUGAUCCA (SEQ ID NO: 635) | 7074a-AS | UGGAUCAACAUUUUGGUUGGU (SEQ ID NO: 636) |
| 7074b | 7074b-SS | GCCAACCAAAAUGUUGAUCCA (SEQ ID NO: 637) | 7074b-AS | UGGAUCAACAUUUUGGUUGGC (SEQ ID NO: 638) |
| 7075a | 7075a-SS | GAAUGUUGAUCCAUCCAAA (SEQ ID NO: 639) | 7075a-AS | UUUGGAUGGAUCAACAUUCUG (SEQ ID NO: 640) |
| 7075b | 7075b-SS | CGAAAUGUUGAUCCAUCCAAA (SEQ ID NO: 641) | 7075b-AS | UUUGGAUGGAUCAACAUUUCG (SEQ ID NO: 642) |
| 7077a | 7077a-SS | CGACCAAAAUGUUGAUCCAUA (SEQ ID NO: 643) | 7077a-AS | UAUGGAUCAACAUUUUGGUUGGU (SEQ ID NO: 644) |
| 7077b | 7077b-SS | CGACCAAAAUGUUGAUCCAUA (SEQ ID NO: 645) | 7077b-AS | UAUGGAUCAACAUUUUGGUCG (SEQ ID NO: 646) |
| 7077c | 7077c-SS | CGACCAAAAUGUUGAUCCAUA (SEQ ID NO: 647) | 7077c-AS | UAUGGAUCAACAUUUUGGUCGAU (SEQ ID NO: 648) |
| 7078a | 7078a-SS | GGGAAGAACUACAUAUAAA (SEQ ID NO: 649) | 7078a-AS | UUUAUAUGUAGUUCUUCUCGG (SEQ ID NO: 650) |
| 7078b | 7078b-SS | CCGAGAAGAACUACAUAUAAA (SEQ ID NO: 651) | 7078b-AS | UUUAUAUGUAGUUCUUCUCGG (SEQ ID NO: 652) |
| 7079a | 7079a-SS | GUCACAAAACUUCAAUGAAAA (SEQ ID NO: 653) | 7079a-AS | UUUUCAUUGAAGUUUUGUGAUUU (SEQ ID NO: 654) |
| 7079b | 7079b-SS | ACCACAAAACUUCAAUGAAAA (SEQ ID NO: 655) | 7079b-AS | UUUUCAUUGAAGUUUUGUGGU (SEQ ID NO: 656) |
| 7079c | 7079c-SS | CGCAAAACUUCAAUGAAAA (SEQ ID NO: 657) | 7079c-AS | UUUUCAUUGAAGUUUUGCGAU (SEQ ID NO: 658) |
| 7079d | 7079d-SS | CGCAAAACUUCAAUGAAAA (SEQ ID NO: 659) | 7079d-AS | UUUUCAUUGAAGUUUUGUGUU (SEQ ID NO: 660) |
| 7080a | 7080a-SS | GAACUUCAAUGAAACGUGA (SEQ ID NO: 661) | 7080a-AS | UCACGUUUCAUUGAAGUUCUG (SEQ ID NO: 662) |
| 7080b | 7080b-SS | CGAAACUUCAAUGAAACGUGA (SEQ ID NO: 663) | 7080b-AS | UCACGUUUCAUUGAAGUUUCG (SEQ ID NO: 664) |
| 7081a | 7081a-SS | GGACUCAACUCAAAACUUA (SEQ ID NO: 665) | 7081a-AS | UAAGUUUUGAGUUGAGUUCGG (SEQ ID NO: 666) |
| 7081b | 7081b-SS | GGACUCAACUCAAAACUUA (SEQ ID NO: 667) | 7081b-AS | UAAGUUUUGAGUUGAGUUC (SEQ ID NO: 668) |
| 7082a | 7082a-SS | GAGAAUAUGUCACUUGAAA (SEQ ID NO: 669) | 7082a-AS | UUUCAAGUGACAUAUUCUCUA (SEQ ID NO: 670) |
| 7083a | 7083a-SS | GGCAAAGAAUAUGUCACUA (SEQ ID NO: 671) | 7083a-AS | UAGUGACAUAUUCUUUGCCUC (SEQ ID NO: 672) |
| 7083b | 7083b-SS | GGCAAAGAAUAUGUCACUA (SEQ ID NO: 673) | 7083b-AS | UAGUGACAUAUUCUUUGCC (SEQ ID NO: 674) |
| 7083c | 7083c-SS | GGCAAAGAAUAUGUCACUA (SEQ ID NO: 675) | 7083c-AS | UAGUGACAUAUUCUUUGCCUU (SEQ ID NO: 676) |
| 7083d | 7083d-SS | GGCAAAGAAUAUGUCACUA (SEQ ID NO: 677) | 7083d-AS | UAGUGACAUAUUCUUUGCCGG (SEQ ID NO: 678) |
| 7083e | 7083e-SS | CAGGUAAAGAAUAUGUCACUA (SEQ ID NO: 679) | 7083e-AS | UAGUGACAUAUUCUUUACCUG (SEQ ID NO: 680) |
| 7084a | 7084a-SS | CAAAGAAUAUGUCACUUGA (SEQ ID NO: 681) | 7084a-AS | UCAAGUGACAUAUUCUUUGCC (SEQ ID NO: 682) |
| 7084b | 7084b-SS | CAAAGAAUAUGUCACUUGA (SEQ ID NO: 683) | 7084b-AS | UCAAGUGACAUAUUCUUUG (SEQ ID NO: 684) |
| 7085a | 7085a-SS | GUAGAUGGAUCACAAAACA (SEQ ID NO: 685) | 7085a-AS | UGUUUUGUGAUCCAUCUACUU (SEQ ID NO: 686) |
| 7086a | 7086a-SS | CCGAAUAGAUGGAUCACAA (SEQ ID NO: 687) | 7086a-AS | UUGUGAUCCAUCUAUUCGGGG (SEQ ID NO: 688) |
| 7087a | 7087a-SS | CUGGAAGUCUCAAAAUGGA (SEQ ID NO: 689) | 7087a-AS | UCCAUUUUGAGACUUCCAGGG (SEQ ID NO: 690) |
| 7088a | 7088a-SS | CACUUGGGAUCACAAAGCA (SEQ ID NO: 691) | 7088a-AS | UGCUUUGUGAUCCCAAGUGGG (SEQ ID NO: 692) |
| 7089a | 7089a-SS | GCCAUACUCUAUAAAAUCA (SEQ ID NO: 693) | 7089a-AS | UGAUUUUAUAGAGUAUGGCUU (SEQ ID NO: 694) |
| 7090a | 7090a-SS | CGUACUCUAUAAAAUCAAA (SEQ ID NO: 695) | 7090a-AS | UUUGAUUUUAUAGAGUACGAC (SEQ ID NO: 696) |
| 7091a | 7091a-SS | GAAAGGAACUGAGAAGAAA (SEQ ID NO: 697) | 7091a-AS | UUUCUUCUCAGUUCCUUUUUU (SEQ ID NO: 698) |
| 7091b | 7091b-SS | GAAAGGAACUGAGAAGAAA (SEQ ID NO: 699) | 7091b-AS | UUUCUUCUCAGUUCCUUUU (SEQ ID NO: 700) |
| 7092a | 7092a-SS | CCCAAAACUUGAAAGCCUA (SEQ ID NO: 701) | 7092a-AS | UAGGCUUUCAAGUUUUGGGUU (SEQ ID NO: 702) |

Next, we modified the siRNA to enhance its stability both *in vivo* and *in vitro,* increase its activity at the target site, and reduce its activity at non-target sites. Unless otherwise specified, L96 delivery was employed for both *in vivo* and *in vitro* screening of single-target sequences to more accurately reflect the efficacy of liver-targeted siRNA. The siRNA was connected to L96 via the 3'-terminus of the sense strand.

7000PM (AD-1331212) is the optimal sequence in Patent US11613751B2. ARO-ANG3 is the optimal sequence in Patent US10995335B2, serving as the positive control sequence. Sequence information is provided in Table 34. To examine sequence activity, the same GalNAc was used for delivery.

**Table 35: Sequences of Modified siRNA Targeting ANGPTL3**

| siRNA Name | Modified Sense Strand Name | Modified Sense Strand Sequence 5'-3' | Modified Antisense Strand Name | Modified Antisense Strand Sequence 5'-3' |
|---|---|---|---|---|
| 7000PM | 7000PM-SM | mA*mA*mGmCmUmCmC mUfUfCfUmUmUmUmUm AmUmUmGmUmU | 7000PM-AM | mA*dA*mCmAdAmUdAmA mAmAmAdGmAfAmGmGm AmGmCmUmU*mG*mG |
| ARO-ANG3 | ARO-ANG3-SM | Invab*mG*mCmUmCmAm AmCmAfUfAfUmUmUmG mAmUmCmAmGmUmAIn vab* | ARO-ANG3-AM | mU*fA*mC*fUmGfAmUfCm AfAmAfUmAfUmGfUmUfG mAfG*mC |
| 7001.1-1 | 7001SM1 | mU*mC*mCmAmGmAfA mUfUfGdAmUmCmAmA mGmAmCmAmAmA | 7001AM1 | VPU*fU*mU*mGmUmCisoG NA- TmUmGmAmUdCmAfAmUf UmCmUmGmGmAmG*mG |
| 7002.1-1 | 7002SM1 | mU*mU*mCmCmUmCfC mAfGfAdAmUmUmGmA mUmCmAmAmGmA | 7002AM1 | VPU*fC*mU*mUmGisoGNA- AmUmCmAmAmUdTmCfUm GfGmAmGmGmAmAmA*m U |
| 7003.1-1 | 7003SM1 | mC*mA*mGmAmGmCfC mAfAfAdAmUmCmAmA mGmAmUmUmUmA | 7003AM1 | VPU*fA*mA*mAmUmCisoG NA- TmUmGmAmUdTmUfUmGf GmCmUmCmUmGmG*mA |
| 7003.2-1 | 7003SM1 | mC*mA*mGmAmGmCfC mAfAfAdAmUmCmAmA mGmAmUmUmUmA | 7003AM2 | VPU*fA*mA*mAmUmCmU mUmGmAmUdTmUfUmGfG mCmUmCmUmGmG*mA |
| 7003.3-1 | 7003SM1 | mC*mA*mGmAmGmCfC mAfAfAdAmUmCmAmA mGmAmUmUmUmA | 7003AM3 | mU*fA*mA*mAmUmCmUm UmGmAmUdTmUfUmGfGm CmUmCmUmGmG*mA |
| 7003.4-2 | 7003SM2 | mC*mG*mGmAmGmCfC mAfAfAdAmUmCmAmA mGmAmUmUmUmA | 7003AM4 | VPU*fA*mA*mAmUmCmU mUmGmAmUdTmUfUmGfG mCmUmUmUmGmG*mA |
| 7003.4-3 | 7003SM3 | Invab*mC*mGmGmAmGm CfCmAfAfAdAmUmCmA mAmGmAmUmUmUmA | 7003AM4 | VPU*fA*mA*mAmUmCmU mUmGmAmUdTmUfUmGfG mCmUmUmUmGmG*mA |
| 7004.1-1 | 7004SM1 | mG*mA*mUmCmAmAfG mAfCfAdAmUmUmCmAm UmCmAmUmUmA | 7004AM1 | VPU*fA*mA*mUmGisoGNA - AmUmGmAmAmUdTmGfUm CfUmUmGmAmUmCmA*mA |
| 7005.1-1 | 7005SM1 | mU*mA*mGmUmUmAfU mUfUfCdCmUmCmCmAm GmAmAmUmUmA | 7005AM1 | VPU*fA*mA*mUmUmCisoG NA- TmGmGmAmGdGmAfAmAf UmAmAmCmUmAmG*mA |
| 7006.1-1 | 7006SM1 | mA*mA*mGmGmGmCfC mAfAfAdTmUmAmAmUm GmAmCmAmUmA | 7006AM1 | VPU*fA*mU*mGmUmCisoG NA- AmUmUmAmAdTmUfUmGf GmCmCmCmUmUmC*mG |
| 7007.1-1 | 7007SM1 | mA*mA*mGmAmUmUfU mGfCfUdAmUmGmUmU mAmGmAmCmGmA | 7007AM1 | VPU*fC*mG*mUmCisoGNA- TmAmAmCmAmUdAmGfCm AfAmAmUmCmUmUmG*m A |
| 7008.1-1 | 7008SM1 | mA*mU*mGmUmUmAfG mAfCfGdAmUmGmUmA mAmAmAmAmUmA | 7008AM1 | VPU*fA*mU*mUmUisoGNA - TmUmAmCmAmUdCmGfUm CfUmAmAmCmAmUmA*mG |
| 7009.1-1 | 7009SM 1 | mG*mA*mCmUmUmUfG mUfCfCdAmUmAmAmGm AmCmGmAmAmA | 7009AM1 | VPU*fU*mU*mCmGisoGNA- TmCmUmUmAmUdGmGfAm CfAmAmAmGmUmCmU*mU |
| 7010.1-1 | 7010SM1 | mU*mU*mGmGmGmAfC mAfUfGdGmUmCmUmU mAmAmAmGmAmA | 7010AM1 | VPU*fU*mC*mUmUisoGNA- TmAmAmGmAmCdCmAfUm GfUmCmCmCmAmAmC*mU |
| 7011.1-1 | 7011SM1 | mC*mU*mUmAmAmAfA mCfUfUdTmUmGmUmAm GmAmAmAmAmA | 7011AM1 | VPU*fU*mU*mUmUmCisoG NA- TmAmCmAmAdAmAfGmUf UmUmUmAmAmGmU*mG |
| 7012.1-1 | 7012SM1 | mC*mU*mGmCmAmAfA mCfCfAdGmUmGmAmAm AmUmCmAmAmA | 7012AM1 | VPU*fU*mU*mGmAisoGNA - TmUmUmCmAmCdTmGfGm UfUmUmGmCmAmGmC*mG |
| 7013.1-1 | 7013SM1 | mU*mU*mUmUmUmAfU mGfAfUdCmUmAmUmCm GmCmUmGmCmA | 7013AM1 | VPU*fG*mC*mAmGmCmG mAmUmAmGdAmUfCmAfU mAmAmAmAmAmG*mA |
| 7014.1-1 | 7014SM1 | mA*mA*mCmCmAmGfU mGfAfAdAmUmCmAmA mAmGmAmAmGmA | 7014AM1 | VPU*fC*mU*mUmCisoGNA- TmUmUmGmAmUdTmUfCm AfCmUmGmGmUmUmU*m G |
| 7015.1-1 | 7015SM1 | mA*mG*mAmAmAmAfG mGfAfAdCmUmGmAmG mAmAmGmAmAmA | 7015AM1 | VPU*fU*mU*mCmUisoGNA- TmCmUmCmAmGdTmUfCm CfUmUmUmUmCmUmU*mC |
| 7016.1-1 | 7016SM1 | mGmAmGmGmUmA mC*mA*mAmGmUmCfA mAfAfAdAmUmGmAmA | 7016AM1 | VPU*fA*mC*mCmUmCisoG NA- TmUmCmAmUdTmUfUmUf GmAmCmUmUmGmU*mA |
| 7017.1-1 | 7017SM1 | mU*mA*mAmAmCmUfA mCfAfAdGmUmCmAmAm AmAmAmUmGmA | 7017AM1 | VPU*fC*mA*mUmUisoGNA- TmUmUmGmAmCdTmUfGm UfAmGmUmUmUmAmU*m A |
| 7018.1-1 | 7018SM1 | mG*mA*mGmGmUmAfA mAfGfAdAmUmAmUmG mUmCmAmCmUmA | 7018AM1 | VPU*fA*mG*mUmGisoGNA - AmCmAmUmAmUdTmCfUm UfUmAmCmCmUmCmU*mU |
| 7019.1-1 | 7019SM1 | mG*mG*mUmAmAmAfG mAfAfUdAmUmGmUmC mAmCmUmUmGmA | 7019AM1 | VPU*fC*mA*mAmGisoGNA- TmGmAmCmAmUdAmUfUm CfUmUmUmAmCmCmU*mC |
| 7020.1-1 | 7020SM1 | mU*mA*mAmAmGmAfA mUfAfUdGmUmCmAmCm UmUmGmAmAmA | 7020AM1 | VPU*fU*mU*mCmAisoGNA- AmGmUmGmAmCdAmUfA mUfUmCmUmUmUmAmC* mC |
| 7021.1-1 | 7021SM1 | mU*mU*mGmAmAmCfU mCfAfAdCmUmCmAmAm AmAmCmUmUmA | 7021AM1 | VPU*fA*mA*mGmUisoGNA - TmUmUmGmAmGdTmUfGm AfGmUmUmCmAmAmG*m U |
| 7022.1-1 | 7022SM1 | mA*mG*mAmAmGmAfA mAfAfAdAmUmUmCmU mAmCmUmUmCmA | 7022AM1 | VPU*fG*mA*mAmGisoGNA - TmAmGmAmAmUdTmUfUm UfUmCmUmUmCmUmA*mG |
| 7023.1-1 | 7023SM1 | mG*mA*mAmGmAmAfA mAfAfAdTmUmCmUmAm CmUmUmCmAmA | 7023AM1 | VPU*fU*mG*mAmAmGisoG NA- TmAmGmAmAdTmUfUmUf UmUmCmUmUmCmU*mA |
| 7024.1-1 | 7024SM1 | mC*mA*mAmAmAmUfC mAfAfCdCmUmGmAmAm AmCmUmCmCmA | 7024AM1 | VPU*fG*mG*mAmGisoGNA - TmUmUmCmAmGdGmUfUm GfAmUmUmUmUmGmA*m A |
| 7025.1-1 | 7025SM1 | mC*mA*mAmCmCmUfG mAfAfAdCmUmCmCmAm GmAmAmCmAmA | 7025AM1 | VPU*fU*mG*mUmUmCisoG NA- TmGmGmAmGdTmUfUmCf AmGmGmUmUmGmA*mU |
| 7026.1-1 | 7026SM1 | mG*mA*mAmAmAmAfC mAfAfGdAmUmAmAmU mAmGmCmAmUmA | 7026AM1 | VPU*fA*mU*mGmCisoGNA- TmAmUmUmAmUdCmUfUm GfUmUmUmUmCmU*m A |
| 7027.1-1 | 7027SM1 | mG*mA*mAmGmAmCfC mAfAfUdAmUmAmAmA mCmAmAmUmUmA | 7027AM1 | VPU*fA*mA*mUmUmGisoG NA- TmUmUmAmUdAmUfUmGf GmUmCmUmUmCmC*mA |
| 7028.1-1 | 7028SM1 | mU*mG*mGmAmAmGfA mCfCfAdAmUmAmUmAm AmAmCmAmAmA | 7028AM1 | VPU*fU*mU*mGmUisoGNA - TmUmAmUmAmUdTmGfGm UfCmUmUmCmCmAmC*mG |
| 7029.1-1 | 7029SM1 | mGfGfAdCmUmAmGmU mAmUmUmCmAmA mC*mU*mCmAmGmAfA | 7029AM1 | VPU*fU*mG*mAmAisoGNA - TmAmCmUmAmGdTmCfCm UfUmCmUmGmAmGmC*mU |
| 7030.1-1 | 7030SM1 | mA*mG*mAmAmGmGfA mCfUfAdGmUmAmUmU mCmAmAmGmAmA | 7030AM1 | VPU*fU*mC*mUmUmGisoG NA- AmAmUmAmCdTmAfGmUf CmCmUmUmCmUmG*mA |
| 7031.1-1 | 7031SM1 | mA*mU*mUmUmCmUfC mUfAfUdCmUmUmCmCm AmAmGmCmCmA | 7031AM1 | VPU*fG*mG*mCmUisoGNA- TmGmGmAmAmGdAmUfAm GfAmGmAmAmAmUmU*m U |
| 7032.1-1 | 7032SM1 | mU*mU*mUmCmUmCfU mAfUfCdTmUmCmCmAm AmGmCmCmAmA | 7032AM1 | VPU*fU*mG*mGmCisoGNA- TmUmGmGmAmAdGmAfUm AfGmAmGmAmAmAmU*m U |
| 7033.1-1 | 7033SM1 | mC*mC*mUmUmUmCfU mUfCfAdGmUmUmGmA mAmUmGmAmAmA | 7033AM1 | VPU*fU*mU*mCmAisoGNA- TmUmCmAmAmCdTmGfAm AfGmAmAmAmGmGmG*m A |
| 7034.1-1 | 7034SM1 | mG*mU*mUmGmAmAfU mGfAfAdAmUmAmAmG mAmAmAmUmGmA | 7034AM1 | VPU*fC*mA*mUmUisoGNA- TmCmUmUmAmUdTmUfCm AfUmUmCmAmAmCmU*mG |
| 7035.1-1 | 7035SM1 | mA*mU*mGmUmAmCfC mAfCfCdAmUmUmUmAm UmAmAmCmAmA | 7035AM1 | VPU*fU*mG*mUmUisoGNA - AmUmAmAmAmUdGmGfU mGfGmUmAmCmAmUmU* mC |
| 7036.1-1 | 7036SM1 | mU*mG*mUmAmCmCfA mCfCfAdTmUmUmAmUm AmAmCmAmGmA | 7036AM1 | VPU*fC*mU*mGmUisoGNA- TmAmUmAmAmAdTmGfGm UfGmGmUmAmCmAmU*m U |
| 7037.1-1 | 7037SM1 | mG*mU*mCmCmAmUfG mGfAfCdAmUmUmAmA mUmUmCmAmAmA | 7037AM1 | VPU*fU*mU*mGmAisoGNA - AmUmUmAmAmUdGmUfC mCfAmUmGmGmAmCmU* mA |
| 7038.1-1 | 7038SM1 | mC*mG*mUmGmGmGfA mGfAfAdCmUmAmCmAm AmAmUmAmUmA | 7038AM1 | VPU*fA*mU*mAmUisoGNA - TmUmGmUmAmGdTmUfCm UfCmCmCmAmCmGmU*mU |
| 7039.1-1 | 7039SM1 | mG*mU*mUmUmUmAfC mGfAfAdTmUmGmAmGm UmUmGmGmAmA | 7039AM1 | VPU*fU*mC*mCmAisoGNA- AmCmUmCmAmAdTmUfCm GfUmAmAmAmAmCmA*m U |
| 7040.1-1 | 7040SM1 | mC*mA*mAmCmUmAfU mAfCfGdCmUmAmCmAm UmCmUmAmGmA | 7040AM1 | VPU*fC*mU*mAmGisoGNA- AmUmGmUmAmGdCmGfU mAfUmAmGmUmUmGmG* mU |
| 7041.1-1 | 7041SM1 | mU*mA*mCmGmCmUfA mCfAfUdCmUmAmGmUm UmGmCmGmAmA | 7041AM1 | VPU*fU*mC*mGmCisoGNA- AmAmCmUmAmGdAmUfG mUfAmGmCmGmUmAmU* mA |
| 7042.1-1 | 7042SM1 | mU*mC*mUmAmGmUfU mGfCfGdAmUmUmAmCm UmGmGmCmAmA | 7042AM1 | VPU*fU*mG*mCmCisoGNA- AmGmUmAmAmUdCmGfCm AfAmCmUmAmGmAmU*m G |
| 7043.1-1 | 7043SM1 | mC*mU*mAmGmUmUfG mCfGfAdTmUmAmCmUm GmGmCmAmAmA | 7043AM1 | VPU*fU*mU*mGmCmCisoG NA- AmGmUmAmAdTmCfGmCf AmAmCmUmAmGmA*mU |
| 7044.1-1 | 7044SM1 | mU*mC*mUmAmCmUfU mGfGfGdAmUmCmAmCm AmAmAmGmCmA | 7044AM1 | VPU*fG*mC*mUmUisoGNA- TmGmUmGmAmUdCmCfCm AfAmGmUmAmGmAmA*m A |
| 7045.1-1 | 7045SM1 | mU*mC*mUmUmGmGfA mAfGfUdCmUmCmAmAm AmAmUmGmGmA | 7045AM1 | VPU*fC*mC*mAmUisoGNA- TmUmUmGmAmGdAmCfUm UfCmCmAmAmGmAmU*mA |
| 7046.1-1 | 7046SM1 | mA*mC*mUmCmUmAfU mAfAfAdAmUmCmAmA mCmCmAmAmAmA | 7046AM1 | VPU*fU*mU*mUmGmGisoG NA- TmUmGmAmUdTmUfUmAf UmAmGmAmGmUmA*mU |
| 7047.1-1 | 7047SM1 | mU*mA*mUmCmUmUfG mGfAfAdGmUmCmUmCm AmAmAmAmUmA | 7047AM1 | VPU*fA*mU*mUmUisoGNA - TmGmAmGmAmCdTmUfCm CfAmAmGmAmUmAmA*m U |
| 7048.1-1 | 7048SM1 | mC*mA*mUmAfUmUfUf GdAmUmCmAmGmUmC mUmUmUmA | 7048AM1 | VPU*fA*mA*mAmGmAmC mUmGmAmUdCmAfAmAfU mAmUmGmU*mU |
| 7049.1-1 | 7049SM1 | mA*mU*mCmAfAmGfAf UdTmUmGmCmUmAmU mGmUmUmA | 7049AM1 | VPU*fA*mA*mCmAmUmA mGmCmAmAdAmUfCmUfU mGmAmUmU*mU |
| 7049.3-3 | 7049SM3 | Invab*mG*mUmCmAfAm GfAfUdTmUmGmCmUmA mUmGmUmUmA | 7049AM3 | VPU*fA*mA*mCmAmUmA mGmCmAmAdAmUfCmUfU mGmAmCmU*mU |
| 7050.1-1 | 7050SM1 | mA*mA*mUmCfAmAfGf AdTmUmUmGmCmUmA mUmGmUmA | 7050AM1 | VPU*fA*mC*mAmUmAmG mCmAmAmAdTmCfUmUfG mAmUmUmU*mU |
| 7051.1-1 | 7051SM1 | mA*mA*mAmUfCmAfAf GdAmUmUmUmGmCmU mAmUmGmA | 7051AM1 | VPU*fC*mA*mUmAmGmC mAmAmAmUdCmUfUmGfA mUmUmUmU*mG |
| 7051.2-3 | 7051SM3 | Invab*mG*mAmAmUfCm AfAfGdAmUmUmUmGmC mUmAmUmGmA | 7051AM2 | VPU*fC*mA*mUmAmGmC mAmAmAmUdCmUfUmGfA mUmUmCmU*mG |
| 7052.1-1 | 7052SM1 | mA*mA*mCmUfCmAfAfC dAmUmAmUmUmUmGm AmUmCmA | 7052AM1 | VPU*fG*mA*mUmCmAmA mAmUmAmUdGmUfUmGfA mGmUmUmU*mU |
| 7053.1-1 | 7053SM1 | mU*mU*mUmGfAmUfCf AdGmUmCmUmUmUmU mUmAmUmA | 7053AM1 | VPU*fA*mU*mAmAmAmA mAmGmAmCdTmGfAmUfC mAmAmAmU*mA |
| 7054.1-1 | 7054SM1 | mC*mC*mAmGfUmGfAf AdAmUmCmAmAmAmG mAmAmGmA | 7054AM1 | VPU*fC*mU*mUmCmUmU mUmGmAmUdTmUfCmAfC mUmGmGmU*mU |
| 7055.1-1 | 7055SM1 | UdAmUmGmUmUmAmG mAmCmGmA mG*mA*mUmUfUmGfCf | 7055AM1 | VPU*fC*mG*mUmCmUmA mAmCmAmUdAmGfCmAfA mAmUmCmU*mU |
| 7056.1-1 | 7056SM1 | mU*mC*mAmAmCmAfU mAfUfUdTmGmAmUmCm AmGmUmCmUmA | 7056AM1 | VPU*fA*mG*mAmCmUmG mAmUmCmAdAmAfUmAfU mGmUmUmGmAmG*mU |
| 7057.1-1 | 7057SM1 | mG*mA*mGmCmCmAfA mAfAfUdCmAmAmGmA mUmUmUmGmCmA | 7057AM1 | VPU*fG*mC*mAmAmAmU mCmUmUmGdAmUfUmUfU mGmGmCmUmCmU*mG |
| 7058.1-1 | 7058SM1 | Invab*mG*mGmAmUmA mGmAmUfGfGfAmUfCm AmCmAmAmAmAmCmA | 7058AM1 | VPU*fG*mUmUmUmUmGm UmGmAmUdCmCfAmUfCm UmGmUmUmC*mG*mA |
| 7059.1-1 | 7059SM1 | Invab*mC*mCmUmAmAm AmGmAfCfUfUmUfGmU mCmCmAmUmAmAmA | 7059AM1 | VPU*fU*mUmAmUmGmGm AmCmAmAdAmGfUmCfUm UmUmAmAmG*mA*mC |
| 7060.1-1 | 7060SM1 | Invab*mA*mCmAmUmCm AmGmGfUfAfGmUfCmC mAmUmGmGmAmCmA | 7060AM1 | VPU*fG*mUmCmCmAmUm GmGmAmCdTmAfCmCfUm GmAmUmAmU*mA*mA |
| 7061.1-1 | 7061SM1 | Invab*mC*mGmCmUmUm GfAfAfCmUfCmAmAmC mUmCmAmAmA | 7061AM1 | VPU*fU*mUmGmAmGmUm UmGmAmGdTmUfCmAfAm GmUmG*mG*mC |
| 7061.1-2 | 7061SM2 | mG*mC*mCmAmCmUfU mGfAfAdCmUmCmAmAm CmUmCmAmAmA | 7061AM1 | VPU*fU*mUmGmAmGmUm UmGmAmGdTmUfCmAfAm GmUmG*mG*mC |
| 7061.1-3 | 7061SM3 | Invab*mG*mCmCmAmCm UfUmGfAfAdCmUmCmA mAmCmUmCmAmAmAIn vab | 7061AM1 | VPU*fU*mUmGmAmGmUm UmGmAmGdTmUfCmAfAm GmUmG*mG*mC |
| 7061.1-4 | 7061SM4 | mC*mG*mCmUmUmGfA mAfCmUfCmAmAmCmU mCmAmAmA | 7061AM1 | VPU*fU*mUmGmAmGmUm UmGmAmGdTmUfCmAfAm GmUmG*mG*mC |
| 7061.1-5 | 7061SM5 | Invab*mC*mGmCmUfUm GfAfAdCmUmCmAmAmC mUmCmAmAmAInvab | 7061AM1 | VPU*fU*mUmGmAmGmUm UmGmAmGdTmUfCmAfAm GmUmG*mG*mC |
| 7061.1-6 | 7061SM6 | Invab*mC*mGmCmUmUm GfAfAfCmUfCmAmAmC mUmCmAmAmAInvab | 7061AM1 | VPU*fU*mUmGmAmGmUm UmGmAmGdTmUfCmAfAm GmUmG*mG*mC |
| 7061.1-7 | 7061SM7 | Invab*mC*mGmCmUmUm GfAmAfCmUfCmAmAmC mUmCmAmAmAInvab | 7061AM1 | VPU*fU*mUmGmAmGmUm UmGmAmGdTmUfCmAfAm GmUmG*mG*mC |
| 7061.2-6 | 7061SM6 | Invab*mC*mGmCmUmUm GfAfAfCmUfCmAmAmC mUmCmAmAmAInvab | 7061AM2 | VPU*fU*mUmGmAmGmUfU mGmAmGdTmUfCmAfAmG mUmG*mG*mC |
| 7061.2-7 | 7061SM7 | Invab*mC*mGmCmUmUm GfAmAfCmUfCmAmAmC mUmCmAmAmAInvab | 7061AM2 | VPU*fU*mUmGmAmGmUfU mGmAmGdTmUfCmAfAmG mUmG*mG*mC |
| 7061.2-5 | 7061SM5 | Invab*mC*mGmCmUfUm GfAfAdCmUmCmAmAmC mUmCmAmAmAInvab | 7061AM2 | VPU*fU*mUmGmAmGmUfU mGmAmGdTmUfCmAfAmG mUmG*mG*mC |
| 7061.1-8 | 7061SM8 | Invab*mG*mCmCmAmCm UmUmGfAfAfCmUfCmA mAmCmUmCmAmAmAIn vab | 7061AM1 | VPU*fU*mUmGmAmGmUm UmGmAmGdTmUfCmAfAm GmUmG*mG*mC |
| 7061.1-9 | 7061SM9 | Invab*mG*mCmCmAmCm UmUmGfAmAfCmUfCmA mAmCmUmCmAmAmAIn vab | 7061AM1 | VPU*fU*mUmGmAmGmUm UmGmAmGdTmUfCmAfAm GmUmG*mG*mC |
| 7061.2-8 | 7061SM8 | Invab*mG*mCmCmAmCm UmUmGfAfAfCmUfCmA mAmCmUmCmAmAmAIn vab | 7061AM2 | VPU*fU*mUmGmAmGmUfU mGmAmGdTmUfCmAfAmG mUmG*mG*mC |
| 7061.2-9 | 7061SM9 | Invab*mG*mCmCmAmCm UmUmGfAmAfCmUfCmA mAmCmUmCmAmAmAIn vab | 7061AM2 | VPU*fU*mUmGmAmGmUfU mGmAmGdTmUfCmAfAmG mUmG*mG*mC |
| 7061.2-3 | 7061SM3 | Invab*mG*mCmCmAmCm UfUmGfAfAdCmUmCmA mAmCmUmCmAmAmAIn vab | 7061AM2 | VPU*fU*mUmGmAmGmUfU mGmAmGdTmUfCmAfAmG mUmG*mG*mC |
| 7061.1-10 | 7061SM10 | Invab*mC*mGmCmUmUm GfAfAdCmUfCmAmAmC mUmCmAmAmAInvab | 7061AM1 | VPU*fU*mUmGmAmGmUm UmGmAmGdTmUfCmAfAm GmUmG*mG*mC |
| 7061.2-10 | 7061SM10 | Invab*mC*mGmCmUmUm GfAfAdCmUfCmAmAmC mUmCmAmAmAInvab | 7061AM2 | VPU*fU*mUmGmAmGmUfU mGmAmGdTmUfCmAfAmG mUmG*mG*mC |
| 7061.1-11 | 7061SM11 | Invab*mG*mCmCmAmCm UmUmGfAfAdCmUfCmA mAmCmUmCmAmAmAIn vab | 7061AM1 | VPU*fU*mUmGmAmGmUm UmGmAmGdTmUfCmAfAm GmUmG*mG*mC |
| 7061.2-11 | 7061SM11 | Invab*mG*mCmCmAmCm UmUmGfAfAdCmUfCmA mAmCmUmCmAmAmAIn vab | 7061AM2 | VPU*fU*mUmGmAmGmUfU mGmAmGdTmUfCmAfAmG mUmG*mG*mC |
| 7061.4-11 | 7061SM11 | Invab*mG*mCmCmAmCm UmUmGfAfAdCmUfCmA mAmCmUmCmAmAmAIn vab | 7061AM4 | VPU- S*fU*mUmGmAmGmUmUm GmAmGdTmUfCmAfAmGm UmG*mG*mC |
| 7061.5-11 | 7061SM11 | Invab*mG*mCmCmAmCm UmUmGfAfAdCmUfCmA mAmCmUmCmAmAmAIn vab | 7061AM5 | VPU*fU*mUmGmAmGmUfU mGmAmGfUmUfCmAfAmG mUmG*mG*mC |
| 7061.6-11 | 7061SM11 | Invab*mG*mCmCmAmCm UmUmGfAfAdCmUfCmA mAmCmUmCmAmAmAIn vab | 7061AM6 | VPU*fU*mUmGmAmGmUfU mGmAmGdTmUfCmAfAmGf UmG*mG*mC |
| 7061.1-12 | 7061SM12 | Invab*mG*mCmCmAmCm UmUmGfAfAfCfUmCfAm AmCmUmCmAmAmAInva b | 7061AM1 | VPU*fU*mUmGmAmGmUm UmGmAmGdTmUfCmAfAm GmUmG*mG*mC |
| 7061.8-11 | 7061SM11 | Invab*mG*mCmCmAmCm UmUmGfAfAdCmUfCmA mAmCmUmCmAmAmAIn vab | 7061AM8 | mU*fU*mUmGmAmGmUmU mGmAmGdTmUfCmAfAmG mUmG*mG*mC |
| 7061.9-13 | 7061SM13 | mUmCmAmAmAInvab Invab*mC*mGmCmUfUm GfAfAdCmUmCmAmAmC | 7061AM9 | VPU*fU*mUmGmAmGmUm UmGmAmGdTmUfCmAfAm GmCmG*mA*mC |
| 7061.10-13 | 7061SM13 | Invab*mC*mGmCmUfUm GfAfAdCmUmCmAmAmC mUmCmAmAmAInvab | 7061AM10 | mU*fU*mUmGmAmGmUmU mGmAmGdTmUfCmAfAmG mCmG*mA*mC |
| 7061.11-13 | 7061SM13 | Invab*mC*mGmCmUfUm GfAfAdCmUmCmAmAmC mUmCmAmAmAInvab | 7061AM11 | VPU- S*fU*mUmGmAmGmUmUm GmAmGdTmUfCmAfAmGm CmG*mA*mC |
| 7061.9-14 | 7061SM14 | Invab*mC*mGmCmUmUm GfAfAfCmUfCmAmAmC mUmCmAmAmAInvab | 7061AM9 | VPU*fU*mUmGmAmGmUm UmGmAmGdTmUfCmAfAm GmCmG*mA*mC |
| 7061.15-13 | 7061SM13 | Invab*mC*mGmCmUfUm GfAfAdCmUmCmAmAmC mUmCmAmAmAInvab | 7061AM15 | VPU*fU*mUmGmAmGmUm UmGmAmGdTmUfCmAfAm G*mC*mG |
| 7061.16-13 | 7061SM13 | Invab*mC*mGmCmUfUm GfAfAdCmUmCmAmAmC mUmCmAmAmAInvab | 7061AM16 | VPU*fU*mUmGmAmGmUm UmGmAmGdTmUfCmAfAm GmCmG*mG*mG |
| 7061.17-14 | 7061SM14 | Invab*mU*mAmCmUmUm GfAfAfCfUmCfAmAmCm UmCmAmAmAInvab | 7061AM17 | VPU*fU*mUmGmAmGmUm UmGmAmGdTmUfCmAfAm GmUmG*mG*mG |
| 7061.17-15 | 7061SM15 | Invab*mU*mAmCmUfUm GfAfAdCmUmCmAmAmC mUmCmAmAmAInvab | 7061AM17 | VPU*fU*mUmGmAmGmUm UmGmAmGdTmUfCmAfAm GmUmG*mG*mG |
| 7061.18-14 | 7061SM14 | Invab*mU*mAmCmUmUm GfAfAfCfUmCfAmAmCm UmCmAmAmAInvab | 7061AM18 | VPU- S*fU*mUmGmAmGmUmUm GmAmGdTmUfCmAfAmGm UmG*mG*mG |
| 7061.4-16 | 7061SM16 | Invab*mG*mCmCmAmCm UmUmGfAfAfCfUmCfAm AmCmUmCmAmAmAInva b | 7061AM4 | VPU- S*fU*mUmGmAmGmUmUm GmAmGdTmUfCmAfAmGm UmG*mG*mC |
| 7062.1-1 | 7062SM1 | Invab*mC*mAmCmUmAm CfAfUfAmUfAmAmAmC mUmAmCmAmA | 7062AM1 | VPU*fU*mGmUmAmGmUm UmUmAmUdAmUfGmUfAm GmUmG*mC*mU |
| 7063.1-1 | 7063SM1 | Invab*mC*mGmUmAmUm AfAfAfCmUfAmCmAmA mGmUmCmAmA | 7063AM1 | VPU*fU*mGmAmCmUmUm GmUmAmGdTmUfUmAfUm GmUmG*mU*mA |
| 7063.1-2 | 7063SM2 | mC*mG*mUmAmUmAfAf AfCmUfAmCmAmAmGm UmCmAmA | 7063AM1 | VPU*fU*mGmAmCmUmUm GmUmAmGdTmUfUmAfUm GmUmG*mU*mA |
| 7063.2-3 | 7063SM3 | Invab*mC*mGmCmAmUm AfUmAfAfAdCmUmAmC mAmAmGmUmCmAmAIn vab | 7063AM2 | VPU*fU*mGmAmCmUmUm GmUmAmGdTmUfUmAfUm AmUmG*mC*mG |
| 7063.1-4 | 7063SM4 | mC*mG*mUmAmUmAfA mAfCmUfAmCmAmAmG mUmCmAmA | 7063AM1 | VPU*fU*mGmAmCmUmUm GmUmAmGdTmUfUmAfUm GmUmG*mU*mA |
| 7064.1-1 | 7064SM1 | Invab*mC*mCmAmCmAm AfAfAfCmUfUmCmAmA mUmGmAmAmA | 7064AM1 | VPU*fU*mUmCmAmUmUm GmAmAmGdTmUfUmUfGm UmGmG*mU*mC |
| 7064.5-3 | 7064SM3 | mUmGmAmAmAInvab Invab*mC*mCmAmCfAm AfAfAdCmUmUmCmAmA | 7064AM5 | VPU*fU*mUmCmAmUmUm GmAmAmGdTmUfUmUfGm UmGmG*mU*mU |
| 7065.1-1 | 7065SM1 | Invab*mG*mGmGmUmU mAfUfAfCmUfCmUmAm UmAmAmAmAmA | 7065AM1 | VPU*fU*mUmUmUmAmUm AmGmAmGdTmAfUmAfAm CmUmU*mU*mC |
| 7066.1-1 | 7066SM1 | Invab*mG*mGmGmAmG mGfUfUfAmUfAmCmUm CmUmAmUmAmA | 7066AM1 | VPU*fU*mAmUmAmGmAm GmUmAmUdAmAfCmCfUm UmUmC*mA*mU |
| 7067.1-1 | 7067SM1 | Invab*mG*mCmCmUmCm AfAfAfAmUfGmGmAmA mGmGmUmUmA | 7067AM1 | VPU*fA*mAmCmCmUmUm CmCmAmUdTmUfUmGfAm GmGmC*mU*mU |
| 7067.1-2 | 7067SM2 | Invab*mG*mCmCmUfCm AfAfAdAmUmGmGmAm AmGmGmUmUmAInvab | 7067AM1 | VPU*fA*mAmCmCmUmUm CmCmAmUdTmUfUmGfAm GmGmC*mU*mU |
| 7068.1-1 | 7068SM1 | Invab*mG*mAmGmUmCm UmCmAfAfAfAmUfGmG mAmAmGmGmUmUmA | 7068AM1 | VPU*fA*mAmCmCmUmUm CmCmAmUdTmUfUmGfAm GmAmCmUmU*mU*mU |
| 7069.1-1 | 7069SM1 | Invab*mA*mCmGmGmAm AmGmGfUfUfAmUfAmC mUmCmUmAmUmAmA | 7069AM1 | VPU*fU*mAmUmAmGmAm GmUmAmUdAmAfCmCfUm UmCmCmGmU*mU*mU |
| 7070.1-1 | 7070SM1 | Invab*mG*mCmAmAmAm AfUfGfUmUfGmAmUmC mCmAmUmCmA | 7070AM1 | VPU*fG*mAmUdGmGmAm UmCmAmAdCmAfUmUfUm UmGmC*mU*mU |
| 7071.1-1 | 7071SM1 | Invab*mA*mCmGmUmUm GmAmUfCfCfAmUfCmCm AmAmCmAmGmAmA | 7071AM1 | VPU*fU*mCmUdGmUmUm GmGmAmUdGmGfAmUfCm AmAmCmGmU*mU*mU |
| 7071.2-1 | 7071SM1 | Invab*mA*mCmGmUmUm GmAmUfCfCfAmUfCmCm AmAmCmAmGmAmA | 7071AM2 | VPU*fU*mCmUmGmUmUm GmGmAmUdGmGfAmUfCm AmAmCmGmU*mU*mU |
| 7071.2-2 | 7071SM2 | mA*mC*mGmUmUmGmA mUfCmCfAmUfCmCmAm AmCmAmGmAmA | 7071AM2 | VPU*fU*mCmUmGmUmUm GmGmAmUdGmGfAmUfCm AmAmCmGmU*mU*mU |
| 7071.2-6 | 7071SM6 | Invab*mA*mCmGmUmUm GmAmUfCfCdAmUfCmC mAmAmCmAmGmAmAIn vab | 7071AM2 | VPU*fU*mCmUmGmUmUm GmGmAmUdGmGfAmUfCm AmAmCmGmU*mU*mU |
| 7072.1-1 | 7072SM1 | Invab*mG*mCmUmAmUm AmCmUfCfUfAmUfAmA mAmAmUmCmAmAmA | 7072AM1 | VPU*fU*mUmGdAmUmUm UmUmAmUdAmGfAmGfUm AmUmAmGmC*mC*mU |
| 7072.2-1 | 7072SM1 | Invab*mG*mCmUmAmUm AmCmUfCfUfAmUfAmA mAmAmUmCmAmAmA | 7072AM2 | VPU*fU*mUmGmAmUmUm UmUmAmUdAmGfAmGfUm AmUmAmGmC*mC*mU |
| 7072.2-2 | 7072SM2 | Invab*mG*mCmUmAmUm AfCmUfCfUdAmUmAmA mAmAmUmCmAmAmA | 7072AM2 | VPU*fU*mUmGmAmUmUm UmUmAmUdAmGfAmGfUm AmUmAmGmC*mC*mU |
| 7072.3-3 | 7072SM3 | Invab*mG*mCmUmAmUm AfCmUfCfUdAmUmAmA mAmAmUmCmAmAmAIn vab | 7072AM3 | VPU*fU*mUmGmAmUmUm UmUmAmUdAmGfAmGfUm AmUmA*mG*mC |
| 7072.4-4 | 7072SM4 | Invab*mG*mCmCmAmUm AfCmUfCfUdAmUmAmA mAmAmUmCmAmAmAIn vab | 7072AM4 | VPU*fU*mUmGmAmUmUm UmUmAmUdAmGfAmGfUm AmUmG*mG*mC |
| 7072.2-5 | 7072SM5 | mG*mC*mUmAmUmAmC mUfCmUfAmUfAmAmAm AmUmCmAmAmA | 7072AM2 | VPU*fU*mUmGmAmUmUm UmUmAmUdAmGfAmGfUm AmUmAmGmC*mC*mU |
| 7072.2-10 | 7072SM10 | Invab*mG*mCmUmAmUm AmCmUfCfUdAmUfAmA mAmAmUmCmAmAmAIn vab | 7072AM2 | VPU*fU*mUmGmAmUmUm UmUmAmUdAmGfAmGfUm AmUmAmGmC*mC*mU |
| 7073.1-1 | 7073SM1 | Invab*mG*mGmGmUmU mAmUmAfCfUfCmUfAm UmAmAmAmAmUmCmA | 7073AM1 | VPU*fG*mAmUdTmUmUmA mUmAmGdAmGfUmAfUmA mAmCmCmU*mU*mU |
| 7073.2-1 | 7073SM1 | Invab*mG*mGmGmUmU mAmUmAfCfUfCmUfAm UmAmAmAmAmUmCmA | 7073AM2 | VPU*fG*mAmUmUmUmUm AmUmAmGdAmGfUmAfUm AmAmCmCmU*mU*mU |
| 7073.3-2 | 7073SM2 | Invab*mG*mGmGmUmU mAfUmAfCfUdCmUmAm UmAmAmAmAmUmCmAI nvab | 7073AM3 | VPU*fG*mAmUmUmUmUm AmUmAmGdAmGfUmAfUm AmAmC*mC*mC |
| 7073.2-3 | 7073SM3 | mG*mG*mGmUmUmAmU mAfCmUfCmUfAmUmAm AmAmAmUmCmA | 7073AM2 | VPU*fG*mAmUmUmUmUm AmUmAmGdAmGfUmAfUm AmAmCmCmU*mU*mU |
| 7074.1-1 | 7074SM1 | Invab*mC*mGmAmCmCm AfAfAfAmUfGmUmUmG mAmUmCmCmA | 7074AM1 | VPU*fG*mGmAdTmCmAmA mCmAmUdTmUfUmGfGmU mUmG*mG*mU |
| 7074.2-1 | 7074SM1 | Invab*mC*mGmAmCmCm AfAfAfAmUfGmUmUmG mAmUmCmCmA | 7074AM2 | VPU*fG*mGmAmUmCmAm AmCmAmUdTmUfUmGfGm UmUmG*mG*mU |
| 7074.3-2 | 7074SM2 | Invab*mG*mCmCmAmAm CfCmAfAfAdAmUmGmU mUmGmAmUmCmCmAIn vab | 7074AM3 | VPU*fG*mGmAmUmCmAm AmCmAmUdTmUfUmGfGm UmUmG*mG*mC |
| 7074.2-3 | 7074SM3 | mC*mG*mAmCmCmAfA mAfAmUfGmUmUmGmA mUmCmCmA | 7074AM2 | VPU*fG*mGmAmUmCmAm AmCmAmUdTmUfUmGfGm UmUmG*mG*mU |
| 7075.1-1 | 7075SM1 | Invab*mG*mAmAmUmG mUfUfGfAmUfCmCmAm UmCmCmAmAmA | 7075AM1 | VPU*fU*mUmGdGmAmUm GmGmAmUdCmAfAmCfAm UmUmC*mU*mG |
| 7075.2-1 | 7075SM1 | Invab*mG*mAmAmUmG mUfUfGfAmUfCmCmAm UmCmCmAmAmA | 7075AM2 | VPU*fU*mUmGmGmAmUm GmGmAmUdCmAfAmCfAm UmUmC*mU*mG |
| 7075.3-2 | 7075SM2 | mC*mG*mAmAmAmUfG mUfUfGdAmUmCmCmAm UmCmCmAmAmA | 7075AM3 | VPU*fU*mUmGmGmAmUm GmGmAmUdCmAfAmCfAm UmUmU*mC*mG |
| 7075.3-3 | 7075SM3 | Invab*mC*mGmAmAmAm UfGmUfUfGdAmUmCmC mAmUmCmCmAmAmAIn vab | 7075AM3 | VPU*fU*mUmGmGmAmUm GmGmAmUdCmAfAmCfAm UmUmU*mC*mG |
| 7075.2-4 | 7075SM4 | mG*mA*mAmUmGmUfU mGfAmUfCmCmAmUmC mCmAmAmA | 7075AM2 | VPU*fU*mUmGmGmAmUm GmGmAmUdCmAfAmCfAm UmUmC*mU*mG |
| 7077.1-1 | 7077SM1 | Invab*mC*mGmAmCmCm AmAmAfAfUfGmUfUmG mAmUmCmCmAmUmA | 7077AM1 | VPU*fA*mUmGdGmAmUm CmAmAmCdAmUfUmUfUm GmGmUmUmG*mG*mU |
| 7077.2-1 | 7077SM1 | Invab*mC*mGmAmCmCm AmAmAfAfUfGmUfUmG mAmUmCmCmAmUmA | 7077AM2 | VPU*fA*mUmGmGmAmUm CmAmAmCdAmUfUmUfUm GmGmUmUmG*mG*mU |
| 7077.3-2 | 7077SM2 | Invab*mC*mGmAmCmCm AfAmAfAfUdGmUmUmG mAmUmCmCmAmUmAIn vab | 7077AM3 | VPU*fA*mUmGmGmAmUm CmAmAmCdAmUfUmUfUm GmGmU*mC*mG |
| 7077.4-2 | 7077SM2 | Invab*mC*mGmAmCmCm AfAmAfAfUdGmUmUmG mAmUmCmCmAmUmAIn vab | 7077AM4 | VPU*fA*mUmGmGmAmUm CmAmAmCdAmUfUmUfUm GmGmUmCmG*mA*mU |
| 7077.2-3 | 7077SM3 | mC*mG*mAmCmCmAmA mAfAmUfGmUfUmGmAm UmCmCmAmUmA | 7077AM2 | VPU*fA*mUmGmGmAmUm CmAmAmCdAmUfUmUfUm GmGmUmUmG*mG*mU |
| 7078.1-1 | 7078SM1 | Invab*mG*mGmGmAmA mGfAfAfCmUfAmCmAm UmAmUmAmAmA | 7078AM1 | VPU*fU*mUmAmUmAmUm GmUmAmGdTmUfCmUfUm CmUmC*mG*mG |
| 7078.1-2 | 7078SM2 | mC*mC*mGmAmGmAfA mGfAfAdCmUmAmCmAm UmAmUmAmAmA | 7078AM1 | VPU*fU*mUmAmUmAmUm GmUmAmGdTmUfCmUfUm CmUmC*mG*mG |
| 7078.1-3 | 7078SM3 | Invab*mC*mCmGmAmGm AfAmGfAfAdCmUmAmC mAmUmAmUmAmAmAIn vab | 7078AM1 | VPU*fU*mUmAmUmAmUm GmUmAmGdTmUfCmUfUm CmUmC*mG*mG |
| 7078.1-4 | 7078SM4 | mG*mG*mGmAmAmGfA mAfCmUfAmCmAmUmA mUmAmAmA | 7078AM1 | VPU*fU*mUmAmUmAmUm GmUmAmGdTmUfCmUfUm CmUmC*mG*mG |
| 7079.1-1 | 7079SM1 | Invab*mG*mUmCmAmCm AmAmAfAfCfUmUfCmA mAmUmGmAmAmAmA | 7079AM1 | VPU*fU*mUmUmCmAmUm UmGmAmAdGmUfUmUfUm GmUmGmAmU*mU*mU |
| 7079.2-2 | 7079SM2 | mA*mC*mCmAmCmAfA mAfAfCdTmUmCmAmAm UmGmAmAmAmA | 7079AM2 | VPU*fU*mUmUmCmAmUm UmGmAmAdGmUfUmUfUm GmUmG*mG*mU |
| 7079.2-3 | 7079SM3 | Invab*mA*mCmCmAmCm AfAmAfAfCdTmUmCmA mAmUmGmAmAmAmAIn vab | 7079AM2 | VPU*fU*mUmUmCmAmUm UmGmAmAdGmUfUmUfUm GmUmG*mG*mU |
| 7079.1-4 | 7079SM4 | mG*mU*mCmAmCmAmA mAfAmCfUmUfCmAmAm UmGmAmAmAmA | 7079AM1 | VPU*fU*mUmUmCmAmUm UmGmAmAdGmUfUmUfUm GmUmGmAmU*mU*mU |
| 7079.2-10 | 7079SM10 | Invab*mA*mCmCmAmCm AmAmAfAfCdTmUfCmA mAmUmGmAmAmAmAIn vab | 7079AM2 | VPU*fU*mUmUmCmAmUm UmGmAmAdGmUfUmUfUm GmUmG*mG*mU |
| 7080.1-1 | 7080SM1 | Invab*mG*mAmAmCmUm UfCfAfAmUfGmAmAmA mCmGmUmGmA | 7080AM1 | VPU*fC*mAmCmGmUmUm UmCmAmUdTmGfAmAfGm UmUmC*mU*mG |
| 7080.2-2 | 7080SM2 | mC*mG*mAmAmAmCfU mUfCfAdAmUmGmAmA mAmCmGmUmGmA | 7080AM2 | VPU*fC*mAmCmGmUmUm UmCmAmUdTmGfAmAfGm UmUmU*mC*mG |
| 7080.2-3 | 7080SM3 | mAmAmCmGmUmGmAIn vab Invab*mC*mGmAmAmAm CfUmUfCfAdAmUmGmA | 7080AM2 | VPU*fC*mAmCmGmUmUm UmCmAmUdTmGfAmAfGm UmUmU*mC*mG |
| 7080.1-4 | 7080SM4 | mG*mA*mAmCmUmUfC mAfAmUfGmAmAmAmC mGmUmGmA | 7080AM1 | VPU*fC*mAmCmGmUmUm UmCmAmUdTmGfAmAfGm UmUmC*mU*mG |
| 7080.2-10 | 7080SM10 | Invab*mC*mGmAmAmAm CmUmUfCfAdAmUfGmA mAmAmCmGmUmGmAIn vab | 7080AM2 | VPU*fC*mAmCmGmUmUm UmCmAmUdTmGfAmAfGm UmUmU*mC*mG |
| 7081.1-1 | 7081SM1 | Invab*mG*mGmAmCfUm CfAfAdCmUmCmAmAmA mAmCmUmUmAInvab | 7081AM1 | VPU*fA*mAmGmUmUmUm UmGmAmGdTmUfGmAfGm UmUmC*mG*mG |
| 7082.1-1 | 7082SM1 | Invab*mG*mAmGmAfAm UfAfUdGmUmCmAmCmU mUmGmAmAmAInvab | 7082AM1 | VPU*fU*mUmCmAmAmGm UmGmAmCdAmUfAmUfUm CmUmC*mU*mA |
| 7083.1-1 | 7083SM1 | Invab*mG*mGmCmAfAm AfGfAdAmUmAmUmGm UmCmAmCmUmAInvab | 7083AM1 | VPU*fA*mGmUmGmAmCm AmUmAmUdTmCfUmUfUm GmCmC*mU*mC |
| 7083.2-1 | 7083SM1 | Invab*mG*mGmCmAfAm AfGfAdAmUmAmUmGm UmCmAmCmUmAInvab | 7083AM2 | mU*fA*mGmUmGmAmCmA mUmAmUdTmCfUmUfUmG mCmC*mU*mC |
| 7083.5-1 | 7083SM1 | Invab*mG*mGmCmAfAm AfGfAdAmUmAmUmGm UmCmAmCmUmAInvab | 7083AM5 | VPU*fA*mGmUmGmAmCm AmUmAmUdTmCfUmUfUm G*mC*mC |
| 7083.6-1 | 7083SM1 | Invab*mG*mGmCmAfAm AfGfAdAmUmAmUmGm UmCmAmCmUmAInvab | 7083AM6 | VPU*fA*mGmUmGmAmCm AmUmAmUdTmCfUmUfUm GmCmC*mU*mU |
| 7083.7-1 | 7083SM1 | Invab*mG*mGmCmAfAm AfGfAdAmUmAmUmGm UmCmAmCmUmAInvab | 7083AM7 | VPU*fA*mGmUmGmAmCm AmUmAmUdTmCfUmUfUm GmCmC*mG*mG |
| 7083.8-1 | 7083SM1 | Invab*mG*mGmCmAfAm AfGfAdAmUmAmUmGm UmCmAmCmUmAInvab | 7083AM8 | VPU*fA*mGmUmGmAmCm AmUmAmUdTmCfUmUfUm GmCmC*dT*dT |
| 7083.7-2 | 7083SM2 | Invab*mG*mGmCmAmAm AfGfAfAfUmAfUmGmUm CmAmCmUmAInvab | 7083AM7 | VPU*fA*mGmUmGmAmCm AmUmAmUdTmCfUmUfUm GmCmC*mG*mG |
| 7083.9-2 | 7083SM2 | Invab*mG*mGmCmAmAm AfGfAfAfUmAfUmGmUm CmAmCmUmAInvab | 7083AM9 | VPU- S*fA*mGmUmGmAmCmAm UmAmUdTmCfUmUfUmGm CmC*mG*mG |
| 7083.10-3 | 7083SM3 | Invab*mC*mAmGmGmUm AmAmAfGfAfAfUmAfUm GmUmCmAmCmUmAInva b | 7083AM10 | VPU- S*fA*mGmUmGmAmCmAm UmAmUdTmCfUmUfUmAm CmC*mU*mG |
| 7084.1-1 | 7084SM1 | Invab*mC*mAmAmAfGm AfAfUdAmUmGmUmCmA mCmUmUmGmAInvab | 7084AM1 | VPU*fC*mAmAmGmUmGm AmCmAmUdAmUfUmCfUm UmUmG*mC*mC |
| 7085.1-1 | 7085SM1 | Invab*mG*mUmAmGfAm UfGfGdAmUmCmAmCmA mAmAmAmCmAInvab | 7085AM1 | VPU*fG*mUmUmUmUmGm UmGmAmUdCmCfAmUfCm UmAmC*mU*mU |
| 7086.1-1 | 7086SM1 | UmCmAmCmAmAInvab Invab*mC*mCmGmAfAm UfAfGdAmUmGmGmAm | 7086AM1 | VPU*fU*mGmUmGmAmUm CmCmAmUdCmUfAmUfUm CmGmG*mG*mG |
| 7087.1-1 | 7087SM1 | Invab*mC*mUmGmGfAm AfGfUdCmUmCmAmAmA mAmUmGmGmAInvab | 7087AM1 | VPU*fC*mCmAmUmUmUm UmGmAmGdAmCfUmUfCm CmAmG*mG*mG |
| 7088.1-1 | 7088SM1 | Invab*mC*mAmCmUfUm GfGfGdAmUmCmAmCmA mAmAmGmCmAInvab | 7088AM1 | VPU*fG*mCmUmUmUmGm UmGmAmUdCmCfCmAfAm GmUmG*mG*mG |
| 7089.1-1 | 7089SM1 | Invab*mG*mCmCmAfUm AfCfUdCmUmAmUmAmA mAmAmUmCmAInvab | 7089AM1 | VPU*fG*mAmUmUmUmUm AmUmAmGdAmGfUmAfUm GmGmC*mU*mU |
| 7090.1-1 | 7090SM1 | Invab*mC*mGmUmAfCm UfCfUdAmUmAmAmAmA mUmCmAmAmAInvab | 7090AM1 | VPU*fU*mUmGmAmUmUm UmUmAmUdAmGfAmGfUm AmCmG*mA*mC |
| 7091.1-1 | 7091SM1 | Invab*mG*mAmAmAfGm GfAfAdCmUmGmAmGmA mAmGmAmAmAInvab | 7091AM1 | VPU*fU*mUmCmUmUmCm UmCmAmGdTmUfCmCfUm UmUmU*mU*mU |
| 7092.1-1 | 7092SM1 | Invab*mC*mCmCmAfAm AfAfCdTmUmGmAmAmA mGmCmCmUmAInvab | 7092AM1 | VPU*fA*mGmGmCmUmUm UmCmAmAdGmUfUmUfUm GmGmG*mU*mU |
| 7061.19-14 | 7061SM1 4 | Invab*mU*mAmCmUmUm GfAfAfCfUmCfAmAmCm UmCmAmAmAInvab | 7061AM1 9 | VPU- S*fU*mUmGmAmGmUmUm GmAmGdTmUfCmAfAmGm UmG*mA*mC |
| 7061.20-14 | 7061SM1 4 | Invab*mU*mAmCmUmUm GfAfAfCfUmCfAmAmCm UmCmAmAmAInvab | 7061AM2 0 | VPU- S*fU*mUmGmAmGmUmUm GmAmGdTmUfCmAfAmGm UmG*mA*mA |
| 7061.21-14 | 7061SM1 4 | Invab*mU*mAmCmUmUm GfAfAfCfUmCfAmAmCm UmCmAmAmAInvab | 7061AM2 1 | VPU- S*fU*mUmGmAmGmUmUm GmAmGdTmUfCmAfAmGm UmG*mC*mC |
| 7061.22-14 | 7061SM1 4 | Invab*mU*mAmCmUmUm GfAfAfCfUmCfAmAmCm UmCmAmAmAInvab | 7061AM2 2 | VPU- S*fU*mUmGmAmGmUmUm GmAmGdTmUfCmAfAmGm UmG*dT*dT |
| 7061.23-14 | 7061SM1 4 | Invab*mU*mAmCmUmUm GfAfAfCfUmCfAmAmCm UmCmAmAmAInvab | 7061AM2 3 | mU*fU*mUmGmAmGmUmU mGmAmGdTmUfCmAfAmG mUmG*mG*mG |
| 7061.8-16 | 7061SM1 6 | Invab*mG*mCmCmAmCm UmUmGfAfAfCfUmCfAm AmCmUmCmAmAmAInva b | 7061AM8 | mU*fU*mUmGmAmGmUmU mGmAmGdTmUfCmAfAmG mUmG*mG*mC |
| 7061.24-14 | 7061SM14 | Invab*mU*mAmCmUmUm GfAfAfCfUmCfAmAmCm UmCmAmAmAInvab | 7061AM24 | VPU- S*fU*mUmGmAmGfUmUmG mAmGdTmUfCmAfAmGmU mG*mG*mG |
| 7061.25-16 | 7061SM16 | Invab*mG*mCmCmAmCm UmUmGfAfAfCfUmCfAm AmCmUmCmAmAmAInva b | 7061AM25 | VPU- S*fU*mUmGmAmGfUmUmG mAmGdTmUfCmAfAmGmU mG*mG*mC |
| 7082.3-2 | 7082SM2 | mUmGmAmAmAInvab Invab*mG*mAmGmAmA mUfAfUfGfUmCfAmCmU | 7082AM3 | VPU- S*fU*mUmCmAmAmGmUm GmAmCdAmUfAmUfUmCm UmC*mU*mA |
| 7083.11-2 | 7083SM2 | Invab*mG*mGmCmAmAm AfGfAfAfUmAfUmGmUm CmAmCmUmAInvab | 7083AM11 | VPU- S*fA*mGmUmGmAmCmAm UmAmUdTmCfUmUfUmGm CmC*mC*mC |
| 7083.12-2 | 7083SM2 | Invab*mG*mGmCmAmAm AfGfAfAfUmAfUmGmUm CmAmCmUmAInvab | 7083AM12 | VPU- S*fA*mGmUmGmAmCmAm UmAmUdTmCfUmUfUmGm CmC*mA*mA |
| 7083.13-2 | 7083SM2 | Invab*mG*mGmCmAmAm AfGfAfAfUmAfUmGmUm CmAmCmUmAInvab | 7083AM13 | VPU- S*fA*mGmUmGmAmCmAm UmAmUdTmCfUmUfUmGm CmC*dT*dT |
| 7083.14-2 | 7083SM2 | Invab*mG*mGmCmAmAm AfGfAfAfUmAfUmGmUm CmAmCmUmAInvab | 7083AM14 | mU*fA*mGmUmGmAmCmA mUmAmUdTmCfUmUfUmG mCmC*mG*mG |
| 7083.15-2 | 7083SM2 | Invab*mG*mGmCmAmAm AfGfAfAfUmAfUmGmUm CmAmCmUmAInvab | 7083AM15 | VPU- S*fA*mGmUmGmAmCmAm UmAmUdTmCfUmUfUmGm CmC*mU*mC |
| 7083.16-2 | 7083SM2 | Invab*mG*mGmCmAmAm AfGfAfAfUmAfUmGmUm CmAmCmUmAInvab | 7083AM16 | VPU- S*fA*mGmUmGmAfCmAmU mAmUdTmCfUmUfUmGmC mC*mG*mG |
| 7083.17-3 | 7083SM3 | Invab*mC*mAmGmGmUm AmAmAfGfAfAfUmAfUm GmUmCmAmCmUmAInva b | 7083AM17 | VPU- S*fA*mGmUmGmAfCmAmU mAmUdTmCfUmUfUmAmC mC*mU*mG |
| 7084.6-2 | 7084SM2 | Invab*mC*mAmAmAmGm AfAfUfAfUmGfUmCmAm CmUmUmGmAInvab | 7084AM6 | VPU- S*fC*mAmAmGmUmGmAm CmAmUdAmUfUmCfUmUm UmG*mC*mC |
| 7093.1-1 | 7093SM1 | Invab*mU*mCmAmCmUm UfGfAfAfCmUfCmAmAm CmUmCmAmAInvab | 7093AM1 | VPU- S*fU*mGmAmGmUmUmGm AmGmUdTmCfAmAfGmUm GmA*mC*mA |
| 7094.1-1 | 7094SM1 | Invab*mG*mUmCmAmCm UfUfGfAfAmCfUmCmAm AmCmUmCmAInvab | 7094AM1 | VPU- S*fG*mAmGmUmUmGmAm GmUmUdCmAfAmGfUmGm AmC*mA*mU |
| 7095.1-1 | 7095SM1 | Invab*mA*mCmUmUmGm AfAfCfUfCmAfAmCmUm CmAmAmAmAInvab | 7095AM1 | VPU- S*fU*mUmUmGmAmGmUm UmGmAdGmUfUmCfAmAm GmU*mG*mA |
| 7096.1-1 | 7096SM1 | Invab*mC*mUmUmGmAm AfCfUfCfAmAfCmUmCm AmAmAmAmAInvab | 7096AM1 | VPU- S*fU*mUmUmUmGmAmGm UmUmGdAmGfUmUfCmAm AmG*mU*mG |
| 7097.1-1 | 7097SM1 | Invab*mA*mGmGmUmA mAfAfGfAfAmUfAmUmG mUmCmAmCmAInvab | 7097AM1 | VPU- S*fG*mUmGmAmCmAmUm AmUmUdCmUfUmUfAmCm CmU*mC*mU |
| 7098.1-1 | 7098SM1 | Invab*mG*mAmGmGmU mAfAfAfGfAmAfUmAmU mGmUmCmAmAInvab | 7098AM1 | VPU- S*fU*mGmAmCmAmUmAm UmUmCdTmUfUmAfCmCm UmC*mU*mU |
| 7099.1-1 | 7099SM1 | Invab*mG*mUmAmAmA mGfAfAfUfAmUfGmUmC mAmCmUmUmAInvab | 7099AM1 | VPU- S*fA*mAmGmUmGmAmCm AmUmAdTmUfCmUfUmUm AmC*mC*mU |

### Embodiment 12 In Vitro Activity Evaluation of siRNA Targeting ANGPTL3

The *in vitro* activity evaluation method of Hep3B cells was based on Embodiment 2. For *in vitro* activity evaluation using primary hepatocytes, refer to Embodiment 2 for the method of generating transgenic mice stably expressing hANGPTL3 and for the primary hepatocyte extraction method.

The primers for detecting ANGPTL3 are as follows:
Forward primer: ACATGTGGCTGAGATTGCTGG (SEQ ID NO: 711)
Reverse primer: CCTTTGCTCTGTGATTCCATGTAG (SEQ ID NO: 712)
Probe: CCTCCCAGAGCACACAGACCTGATGTTT (SEQ ID NO: 713) (Reporter gene 5'NED, Quencher group 3'MGB)

**Table 36: Experimental Results of Different Concentrations of Modified siRNA in Hep3B and hANGPTL3 Mouse Primary Hepatocytes Transfected by Liposome or Delivered via L96**

| siRNA | Residual ANGPTL3 mRNA Level (%) | | | |
|---|---|---|---|---|
| | Hep3B | | hANGPTL3 Mouse Primary Hepatocytes | |
| | Mean at 0.1 nM/Liposome Transfection | SD | Mean at 1 nM/Free Uptake | SD |
| 7000PM | 31.2 | 2.5 | 90.7 | 2.1 |
| 7001.1-1 | 25.8 | 6.3 | NA | NA |
| 7002.1-1 | 19.2 | 5.1 | NA | NA |
| 7003.4-3 | 13.4 | 1.6 | 20.4 | 3.6 |
| 7004.1-1 | 22.9 | 1 | NA | NA |
| 7005.1-1 | 31.5 | 3.2 | NA | NA |
| 7006.1-1 | 49.1 | 1.6 | NA | NA |
| 7007.1-1 | 17.4 | 3.2 | NA | NA |
| 7008.1-1 | 18.6 | 1 | NA | NA |
| 7009.1-1 | 78.6 | 5 | NA | NA |
| 7011.1-1 | 55.2 | 0.9 | NA | NA |
| 7012.1-1 | 21.5 | 1 | NA | NA |
| 7014.1-1 | 69.4 | 3.4 | 37 | 4.8 |
| 7016.1-1 | 20.7 | 1.6 | NA | NA |
| 7017.1-1 | 26.1 | 3.6 | NA | NA |
| 7018.1-1 | 17.2 | 1.7 | NA | NA |
| 7019.1-1 | 29.5 | 1.3 | NA | NA |
| 7020.1-1 | 55.1 | 1 | NA | NA |
| 7021.1-1 | 23.3 | 3.3 | NA | NA |
| 7022.1-1 | 42.7 | 3.7 | NA | NA |
| 7023.1-1 | 23.5 | 5.4 | NA | NA |
| 7031.1-1 | 21.5 | 3.7 | NA | NA |
| 7034.1-1 | 21.2 | 1.3 | NA | NA |
| 7037.1-1 | 74.5 | 3.5 | NA | NA |
| 7041.1-1 | 90.8 | 5.7 | NA | NA |
| 7042.1-1 | 19.8 | 5.1 | NA | NA |
| 7043.1-1 | 52.2 | 8.5 | NA | NA |
| 7048.1-1 | 70.6 | 0.7 | 39.6 | 2.7 |
| 7049.3-3 | 21.2 | 2 | 53.2 | 10.5 |
| 7050.1-1 | 66.5 | 5.7 | 32.1 | 3.2 |
| 7051.1-1 | 10.9 | 1.4 | 19.8 | 4.1 |
| 7051.2-3 | 13.8 | 1.3 | 21.8 | 0.8 |
| 7052.1-1 | 71.8 | 5.7 | NA | NA |
| 7053.1-1 | 47.8 | 20 | NA | NA |
| 7054.1-1 | 90.1 | 2.3 | 35 | 7.1 |
| 7055.1-1 | 82.6 | 3.8 | NA | NA |
| 7056.1-1 | 78.8 | 1.3 | NA | NA |
| 7057.1-1 | 70.6 | 4.1 | 43 | 10.9 |
| 7058.1-1 | NA | NA | 33.1 | 8.2 |
| 7059.1-1 | NA | NA | 72.7 | 10.9 |
| 7061.1-1 | NA | NA | 19.4 | 0.1 |
| 7063.1-1 | NA | NA | 36.5 | 3.6 |
| 7065.1-1 | NA | NA | 74.4 | 6.1 |
| 7067.1-1 | NA | NA | 40.2 | 5.5 |
| 7068.1-1 | NA | NA | 26 | 4.2 |
| 7069.1-1 | NA | NA | 26.4 | 7.2 |
| 7070.1-1 | NA | NA | 59.1 | 4.8 |
| 7071.1-1 | NA | NA | 50.5 | 6.6 |
| 7072.3-3 | NA | NA | 25.9 | 5.4 |
| 7073.1-1 | NA | NA | 47.2 | 4.5 |
| 7074.1-1 | NA | NA | 47.2 | 5.9 |
| 7075.1-1 | NA | NA | 53.8 | 8.1 |
| 7077.1-1 | NA | NA | 39.9 | 7.6 |
| 7078.1-1 | NA | NA | 30.5 | 8.6 |
| 7079.1-1 | NA | NA | 29.1 | 4.8 |
| 7080.1-1 | NA | NA | 37 | 5.6 |

| | | | | |
|---|---|---|---|---|
| NA means not tested | | | | |

The activity of modified siRNAs was evaluated in Hep3B cells and hANGPTL3 mouse primary hepatocytes via liposome transfection and free uptake, respectively. As shown in the table, multiple sequences demonstrated superior activity compared to the positive control. We then proceeded with *in vivo* activity evaluation. The results are shown in Table 37 below.

### Embodiment 13 Evaluation of the Effect of In Vitro Preferred Sequences on hANGPTL3 Expression in the Liver of AAV8-hANGPTL3 Mice

Evaluation of the Effect of *In Vitro* Preferred Sequences on hANGPTL3 Expression in the Liver of Mice Expressing hANGPTL3.

### Experimental Method:

### 1. Adenovirus integration of hANGPTL3

Transgenic mice stably expressing hANGPTL3 were generated by infecting mice with 1-10×10¹¹ titer of purified recombinant AAV8 viral particles.

### 2. Administration

The mice were equally divided into groups, with 4 mice per group, 14 days after virus injection. siRNA was dissolved in normal saline and administered subcutaneously at a dose of 1 mg/kg or 3 mg/kg.

### 3. Liver Collection and Testing

Livers were collected at different time points after siRNA injection. RNA was extracted from liver tissue using TRI REAGENT (MRC, Cat. No.: TR118). The extracted RNA was reverse transcribed into cDNA using a PrimeScript RT Reagent Kit (Takara, Cat. No.: RR047A). The prepared QPCR system was added to a 96-well PCR plate, the plate was sealed with sealing film, and QPCR was conducted on a StepOnePlus real-time PCR System (Applied Biosystems) to detect hANGPTL3 expression.

**Table 37: Experimental Results of ANGPTL3-targeted siRNA Inhibiting hANGPTL3 Gene Expression Level in the Liver of AAV8-hANGPTL3 Mice at 1 mg/kg**

| siRNA | Mean ANGPTL3 Gene Expression Level (%) (relative to the normal saline group) | | | |
|---|---|---|---|---|
| | Day 29/Mean | SD | Day 56/Mean | SD |
| ARO-ANG3 | 18.8 | 2.7 | 64.9 | 12.5 |
| 7003.4-3 | 32.7 | 6.3 | 50.1 | 6.9 |
| 7051.2-3 | 25.5 | 2.5 | 58.3 | 10.8 |
| 7061.1-11 | 14.3 | 2.2 | 30.5 | 2.3 |
| 7072.2-10 | 23.1 | 4.3 | NA | NA |
| 7079.2-10 | 14.3 | 2.7 | 36.0 | 6.3 |
| 7080.2-10 | 24.4 | 8.3 | NA | NA |

| | | | | |
|---|---|---|---|---|
| NA means not tested | | | | |

siRNA with relatively good *in vitro* activity was screened for *in vivo* activity at a dose of 1 mg/kg. The experimental results are shown in Table 37. 7061.1-11 exhibited optimal activity, significantly outperforming the positive control ARO-ANG3.

**Table 38: Experimental Results of 0.3 nM Modified siRNA Delivered via L96 in AAV8-hAGT/hANGPTL3 Mouse Primary Hepatocytes**

| siRNA | Residual ANGPTL3 mRNA Level (%) |
|---|---|
| | Mean SD |
| ARO-ANG3 | 57.6 6.3 |
| 7061.9-13 | 14.8 2.8 |
| 7061.1-11 | 18.9 4.5 |

Table 38 showed that both 7061.9-13 and 7061.1-11 exhibited significantly higher activity than the positive control ARO-ANG3

**Table 39: Experimental Results of 0.3 nM Modified siRNA Transfection via Liposome in Hep3B Cells**

| siRNA | Residual ANGPTL3 mRNA Level (%) | |
|---|---|---|
| | Mean | SD |
| 7000PM | 15.1 | 2.6 |
| ARO-ANG3 | 17.9 | 4.7 |
| 7061.9-13 | 8.9 | 3.4 |
| 7064.5-3 | 13.4 | 4.5 |
| 7079.2-10 | 17.1 | 5.2 |
| 7081.1-1 | 12.4 | 3.9 |
| 7082.1-1 | 23.0 | 1.9 |
| 7083.1-1 | 12.3 | 0.4 |
| 7084.1-1 | 12.0 | 1.4 |
| 7085.1-1 | 19.9 | 5.5 |
| 7086.1-1 | 23.4 | 2.6 |
| 7087.1-1 | 12.7 | 3.5 |
| 7088.1-1 | 25.8 | 3.1 |
| 7089.1-1 | 29.7 | 1.9 |
| 7090.1-1 | 16.7 | 1.5 |
| 7091.1-1 | 13.1 | 1.8 |
| 7092.1-1 | 18.7 | 1.3 |

Table 39 showed that multiple sequences exhibited *in vitro* activity superior to or equivalent to that of the positive control. Among them, the 7061.9-13 sequence exhibited optimal activity. We selected the sequences with relatively high activity from the table and conducted activity evaluation in AAV8-hANGPTL3 mice.

**Table 40: In Vivo Experimental Data of AAV8-hANGPTL3 Mice**

| siRNA | Mean ANGPTL3 Gene Expression Level (%) (relative to the normal saline group) | |
|---|---|---|
| | Day 28/Mean | SD |
| ARO-ANG3 | 45.7 | 13.1 |
| 7061.9-13 | 26.3 | 2.8 |
| 7064.5-3 | 43.7 | 14.5 |
| 7081.1-1 | 44.8 | 7.4 |
| 7083.1-1 | 31.1 | 4.8 |
| 7084.1-1 | 57.9 | 7.2 |

*In vivo* activity screening was conducted at a dose of 1 mg/kg. Experimental results are shown in Table 40. On Day 28, the activity of sequences 7061.9-13 and 7083.1-1 was superior to that of the positive control ARO-ANG3. Sequence 7061 was further optimized while the activity of the adjacent sequences of 7061 was tested simultaneously.

**Table 41: Experimental Results of 1 nM Modified siRNA Delivered via L96 in hANGPTL3 Mouse Primary Hepatocytes**

| siRNA | Residual ANGPTL3 mRNA Level (%) | |
|---|---|---|
| | Mean | SD |
| 7061.9-13 | 14.6 | 0.7 |
| 7061.18-14 | 20.1 | 0.4 |
| 7061.23-14 | 30.9 | 0.3 |
| 7061.4-16 | 11 | 2 |
| 7061.1-12 | 13.4 | 0.5 |
| 7061.8-16 | 21.1 | 0.4 |
| 7061.4-11 | 13.6 | 0.6 |
| 7094.1-1 | 38 | 4.3 |
| 7095.1-1 | 57.4 | 3.7 |
| 7096.1-1 | 82.1 | 1.7 |

Sequence 7061 was further optimized based on its modified siRNA 7061.9-13 and evaluated for *in vitro* activity in hANGPTL3 mouse primary hepatocytes. Table 41 showed that different modifications of 7061 exhibited significant variations in activity. Among them, 7061.4-16 exhibited the highest activity. We also compared the activity of sequences 7094.1-1, 7095.1-1, and 7096.1-1 adjacent to sequence 7061. It can be seen that under identical modification conditions, the activity of 7061 is significantly superior to that of its neighboring sequences.

We further evaluated the activity of 7061.18-14 and 7061.4-16 in hANGPTL3 mouse primary hepatocytes, with results shown in Table 42.

**Table 42: Experimental Results of 1 nM Modified siRNA Delivered via L96 in hANGPTL3 Mouse Primary Hepatocytes**

| siRNA | Residual ANGPTL3 mRNA Level (%) | |
|---|---|---|
| | Mean | SD |
| ARO-ANG3 | 20.9 | 2.3 |
| 7061.18-14 | 7.4 | 0.5 |
| 7061.4-16 | 4.4 | 0.8 |

Table 42 showed that both 7061.18-14 and 7061.4-16 exhibited significantly higher activity than the positive control ARO-ANG3.

Next, we tested the safety of sequences 7061 and 7083. The experimental results are shown in Table 43

**Table 43: Safety Experimental Results for ANGPTL3-Targeting siRNA**

| siRNA | ALT (U/L) | AST (U/L) |
|---|---|---|
| Normal saline | 31 | 121 |
| 7061.1-11 | 38 | 133 |
| 7083.9-2 | 484 | 639 |

Table 43 showed that 7061.1-11 exhibited excellent safety profiles in mice, while 7083.9-2 significantly impacted mouse liver function.

Next, we conducted further *in vivo* activity evaluations.

### Embodiment 14 Activity Evaluation of Optimized siRNA Sequence 7061 in AAV8-hANGPTL3 Mice

**Table 44: Results of In Vivo Activity Evaluation for Preferred Sequence**

| siRNA | Mean ANGPTL3 Gene Expression Level (%) (relative to the normal saline group) | | | |
|---|---|---|---|---|
| | Day 14/Mean | SD | Day 42/Mean | SD |
| ARO-ANG3 | 22.9 | 9 | 27.2 | 3.4 |
| 7061.4-16-SL01 | 15.4 | 3.4 | 17.1 | 7.3 |

In AAV8-hANGPTL3 mice, the activity of sequence 7061.4-16 was evaluated *in vivo* at a dose of 1 mg/kg under SL01 delivery conditions. Table 44 showed that 7061.4-16 exhibited superior activity compared to the positive control.

Through the above single-target screening, we identified promising sequences for each target. Next, we linked these highly active single-target sequences into a single construct using the following structure, enabling simultaneous inhibition of the expression of two genes.

### Embodiment 15 Preparation of SL01-CPG

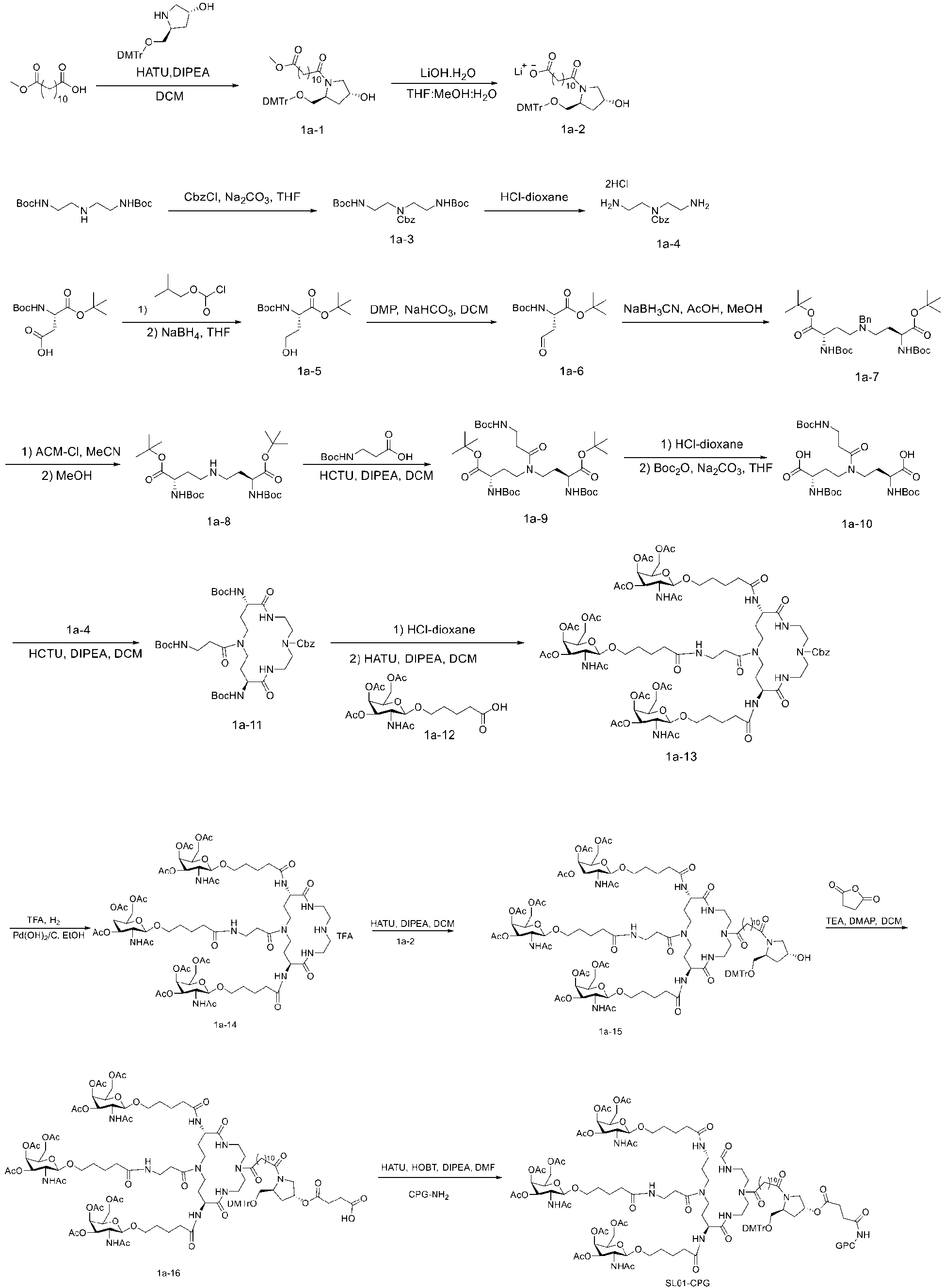

Preparation of compound 1a-1: The mixture of (3R,5S)-5-((bis(4-methoxyphenyl)(phenyl)methoxy)methyl)pyrrolidin-3-ol (10 g, 23.84 mmol, 1.0 eq), 12-methoxy-12-oxodecanoic acid (5.8 g, 23.84 mmol, 1.0 eq), and N,N-diisopropylethylamine (12.3 g, 95.36 mmol, 4.0 eq) in dichloromethane (100 mL) was stirred at room temperature for 2.0 h. The mixture was washed with sodium carbonate solution, and the aqueous phase was extracted with dichloromethane (50 mL × 3). The organic phase was washed with saturated brine, dried over anhydrous sodium sulfate, and the solvent was removed under reduced pressure. The residue was purified by silica gel column chromatography (eluent: dichloromethane/methanol = 100/1 to 20/1) to give 1a-1 as a yellow oil (16.7 g, yield: 79.3%).

Preparation of compound 1a-2: Lithium hydroxide monohydrate (3.1 g, 64.73 mmol, 2.5 eq) was added to the mixture of compound 1a-1 (16.7 g, 25.89 mmol, 1.0 eq) and tetrahydrofuran/methanol/water (80 mL/16 mL/16 mL). The mixture was reacted for 4 h at 40°C. The reaction mixture was subject to reduced pressure to remove the solvent. The aqueous solution of residues was freeze-dried. The residue was dissolved in dichloromethane and filtered. The filtrate was concentrated under reduced pressure to give 1a-2 as a white solid (14.3 g, yield: 85.3%). LCMS (ESI): m/z = 630 [M-1]⁻.

Preparation of compound 1a-3: Benzyl chloroformate (2.03 g, 11.88 mmol, 1.2 eq) was added to the mixture of the compound tert-butyl (azadiylbis(ethane-2,1-diyl))carbamate (3.0 g, 9.9 mmol, 1.0 eq) and sodium carbonate (1.57 g, 14.85 mmol, 1.5 eq) in tetrahydrofuran (20 mL) and water (10 mL). The mixture was stirred at room temperature overnight. The mixture was diluted with water (50 mL) and then extracted with ethyl acetate (20 mL × 3). The combined organic layer was washed with saturated brine (20 mL × 1), dried over anhydrous sodium sulfate, and concentrated. The residue was separated by silica gel column chromatography (petroleum ether:ethyl acetate = 3:1) to give 1a-3 as a white solid (3.80 g, yield: 88%). LCMS (ESI): m/z = 438 [M+1]⁺.

Preparation of compound 1a-4: Hydrogen chloride in dioxane (4 mol/L, 15 mL) was added to the mixture of compound 1a-3 (3.74 g, 8.55 mmol, 1.0 eq) in methanol (10 mL). The mixture was stirred at room temperature for 1.5 h. The solvent was removed under reduced pressure. The residue was dried under vacuum to give 1a-4 as a white solid (2.92 g, crude). LCMS (ESI): m/z = 238 [M+1]⁺.

Preparation of compound 1a-5: At -20°C, isobutyl chloroformate (20.17 mL, 155.54 mmol, 1.0 eq) was added dropwise to a mixture of compound (S)-4-(tert-butoxy)-3-((tert-butoxycarbonyl)amino)-4-oxobutanoic acid (45 g, 155.54 mmol, 1.0 eq) and N-methylmorpholine (15.7 g, 155.54 mmol, 1.0 eq) in tetrahydrofuran (220 mL). The mixture was stirred for 10 min and then filtered. At -30°C, aqueous solution (70 mL) of sodium borohydride (11.8 g, 311.08 mmol, 2.0 eq) was slowly added to the filtrate. The mixture was heated to 0°C and stirred for 0.5 h. The mixture was diluted with water (200 mL) and then extracted with ethyl acetate (100 mL × 3). The combined organic layer was washed with saturated brine (100 mL × 1), dried over anhydrous sodium sulfate, and concentrated to give 1a-5 as a colorless oil (43.67 g, crude). LCMS (ESI): m/z = 276 [M+1]⁺.

Preparation of compound 1a-6: At 0°C, Dess-Martin reagent (79.2 g, 186.65 mmol, 1.2 eq) was added to the mixture of compound 1a-5 (42.8 g, 155.54 mmol, 1.0 eq) and sodium bicarbonate (39.2 g, 466.62 mmol, 3.0 eq) in dichloromethane (300 mL). The mixture was brought to room temperature, stirred for 2 h, and filtered. The filtrate was concentrated under reduced pressure. The residue was diluted with ethyl acetate (200 mL) and successively washed with saturated sodium thiosulfate solution (100 mL × 1), saturated sodium bicarbonate solution (100 mL × 1), and saturated brine (100 mL × 1). Then, it was dried over anhydrous sodium sulfate and concentrated to give 1a-6 as a pale yellow oil (40 g, crude). LCMS (ESI): m/z = 274 [M+1]⁺.

Preparation of compound 1a-7: The mixture of benzylamine (6.1 g, 57.01 mmol, 1.0 eq), compound 1a-6 (38 g, 139.2 mmol, 2.44 eq), and acetic acid (3.26 mL, 57.01 mmol, 1.0 eq) in methanol (200 mL) was stirred at room temperature for 10 min. The mixture was cooled to 0°C, and sodium cyanoborohydride (12.53 g, 199.54 mmol, 3.5 eq) was slowly added. The mixture was brought to room temperature and stirred overnight. The solvent was removed under reduced pressure, and the residue was diluted with water (150 mL). Solid sodium bicarbonate was added to adjust the pH to 9, then the aqueous layer was extracted with ethyl acetate (60 mL × 3). The combined organic layer was washed with saturated brine (50 mL × 1), dried over anhydrous sodium sulfate, and concentrated. The residue was separated by silica gel column chromatography (petroleum ether:ethyl acetate = 50:1 to 5:1) to give 1a-7 as a colorless oil (24.1 g, yield: 68%). LCMS (ESI): m/z = 622 [M+1]⁺.

Preparation of compound 1a-8: At 0°C, 1-chloroethyl chloroformate (7.16 mL, 66.34 mmol, 1.5 eq) was added dropwise to the mixture of compound 1a-7 (27.5 g, 44.23 mmol, 1.0 eq) and N,N-diisopropylethylamine (1.54 mL, 8.85 mmol, 0.2 eq) in acetonitrile (125 mL). The mixture was brought to room temperature, stirred for 2 h, The solvent was removed under reduced pressure. The residue was diluted with methanol (20 mL). The mixture was heated to 63°C and stirred for 1.5 h. The solvent was removed under reduced pressure, and the residue was diluted with dichloromethane (150 mL). The organic layer was washed with saturated sodium bicarbonate solution (30 mL × 1) and saturated brine (50 mL × 1), dried over anhydrous sodium sulfate, and concentrated. The residue was separated by silica gel column chromatography (dichloromethane:methanol = 200:1 to 20:1) to give 1a-8 as a pale yellow oil (11.5 g, yield: 49%). LCMS (ESI): m/z = 532 [M+1]⁺.

Preparation of compound 1a-9: The mixture of compound 1a-8 (4.3 g, 22.7 mmol, 1.05 eq), 3-(tert-butoxycarbonyl)amino)propionic acid (4.3 g, 22.7 mmol, 1.05 eq), and N,N-diisopropylethylamine (5.58 g, 43.24 mmol, 2.0 eq) in dichloromethane (70 mL) was added with 6-chlorobenzotriazole-1,1,3,3-tetramethyluronium hexafluorophosphate (9.84 g, 23.78 mmol, 1.1 eq). The mixture was stirred at room temperature for 2.5 h. The solvent was removed under reduced pressure. The residue was separated by silica gel column chromatography (petroleum ether:ethyl acetate = 4:1 to 2:1) to give 1a-9 as a pale yellow solid (14.98 g, yield: 99%). LCMS (ESI): m/z = 703 [M+1]⁺.

Preparation of compound 1a-10: A mixture of compound 1a-9 (14.9 g, 21.19 mmol, 1.0 eq) and hydrogen chloride in dioxane (4 mol/L, 90 mL) was stirred overnight at room temperature. The solvent was removed under reduced pressure. The residue was dissolved in tetrahydrofuran (100 mL) and water (100 mL). Sodium carbonate (13.48 g, 127.14 mmol, 6.0 eq) and di-tert-butyl carbonate (24.36 mL, 105.95 mmol, 5.0 eq) were added. The mixture was stirred at room temperature for 48 h. Tetrahydrofuran was removed under reduced pressure. The residue was diluted with water (200 mL), then washed with dichloromethane (60 mL × 4). Dilute hydrochloric acid solution (2 mol/L) was added to the aqueous layer to adjust the pH to 3, then the reaction mixture was extracted with ethyl acetate (60 mL × 4). The combined organic layer was washed with saturated brine (50 mL × 1), dried over anhydrous sodium sulfate, and concentrated to give 1a-10 as a pale yellow solid (10.5 g, yield: 84%). LCMS (ESI): m/z = 592 [M+1]⁺.

Preparation of compound 1a-11: At 0°C, the solution of compound 1a-10 (3.76 g, 6.36 mmol, 1.0 eq), compound 1a-4 (1.97 g, 6.36 mmol, 1.0 eq), and N,N-diisopropylethylamine (4.92 g, 38.16 mmol, 6.0 eq) in dichloromethane (60 mL) was added dropwise to a mixture of 6-chlorobenzotriazole-1,1,3,3-tetramethyluronium hexafluorophosphate (6.58 g, 15.9 mmol, 2.5 eq) in dichloromethane (570 mL) within 30 min. The mixture was brought to room temperature and stirred for 20 min. The solvent was removed under reduced pressure. The residue was diluted with saturated sodium bicarbonate solution (50 mL) and then extracted with ethyl acetate (30 mL × 3).

The combined organic layer was washed with saturated brine (50 mL × 1), dried over anhydrous sodium sulfate, and concentrated. The residue was separated by silica gel column chromatography (ethyl acetate:methanol = 50:1 to 30:1) to give 1a-11 as a white solid (1.97 g, yield: 39%). LCMS (ESI): *m*/*z* = 792 [M+1]⁺.

Preparation of compound 1a-13: A mixture of 1a-11 (1.97 g, 2.49 mmol, 1.0 eq) and hydrogen chloride in dioxane (4 mol/L, 13 mL) was stirred at room temperature for 1.5 h. The solvent was removed under reduced pressure. The residue was diluted with dichloromethane (60 mL). Compound 1a-12 (3.34 g, 7.47 mmol, 3.0 eq), N,N-diisopropylethylamine (2.57 g, 19.92 mmol, 8.0 eq), and 2-(7-azabenzotriazole)-N,N,N',N'-tetramethylurea hexafluorophosphate (3.03 g, 7.97 mmol, 3.2 eq) were added. The mixture was stirred at room temperature for 1.5 h. The mixture was washed with saturated sodium bicarbonate solution (30 mL × 2) and saturated brine (30 mL × 1), dried over anhydrous sodium sulfate, and concentrated. The residue was separated by silica gel column chromatography (dichloromethane:methanol = 30:1, with 1% triethylamine added) to give la-13 as a pale yellow solid (3.43 g, yield: 77%). LCMS (ESI): *m*/*z* = 1780 [M+1]⁺.

Preparation of compound 1a-14: Palladium hydroxide/carbon (10%, 0.69 g) was added to a mixture of compound 1a-13 (3.47 g, 1.95 mmol, 1.0 eq) and trifluoroacetic acid (0.15 mL, 1.95 mmol, 1.0 eq) in isopropanol (50 mL) and ethanol (25 mL). The mixture was stirred at room temperature under hydrogen balloon pressure for 20 h. and filtered. The filtrate was concentrated under reduced pressure to give 1a-14 as a white solid (3.45 g, crude). LCMS (ESI): *m*/*z* = 1646 [M+1]⁺.

Preparation of compound 1a-15 (compound 31): To a mixture of compound 1a-14 (1.0 g, 0.57 mmol, 1.0 eq), compound 1a-2 (0.396 g, 0.63 mmol, 1.1 eq) and N,N-diisopropylethylamine (0.441 g, 3.42 mmol, 6.0 eq) in dichloromethane (15 mL) was added 2-(7-azabenzotriazole)-N,N,N',N'-tetramethylurea hexafluorophosphate (0.26 g, 0.68 mmol, 1.2 eq). The mixture was stirred at room temperature for 2.5 h. The solvent was removed under reduced pressure. The residue was purified by preparative high-performance liquid chromatography (HPLC) (A: phosphate buffer at pH 7, B: acetonitrile, 50-70% acetonitrile concentration over 30 min) to give 1a-15 (compound 31) as a white solid (0.45 g, yield: 35%). LCMS (ESI): m/z = 980[(M-302+2)/2]⁺.

Preparation of compound 1a-16: Succinic anhydride (34.5 mg, 0.348 mmol, 6.0 eq) was added to a mixture of compound 1a-15 (130 mg, 0.058 mmol, 1.0 eq), triethylamine (70 mg, 0.696 mmol, 12.0 eq), and 4-dimethylaminopyridine (3.5 mg, 0.029 mmol, 0.5 eq) in dichloromethane (1 mL). The mixture was stirred at room temperature for 2 days. The mixture was diluted with dichloromethane (10 mL), then washed with saturated sodium bicarbonate solution (5 mL × 2) and saturated brine (5 mL × 1), dried over anhydrous sodium sulfate, and concentrated to give 1a-16 as a white solid (111 mg, yield: 81%).

LCMS(ESI): m/z =1030.5[(M-302)/2+H]⁺. ¹H NMR (500 MHz, DMSO) δ 8.42 - 8.25 (m, 2H), 8.06 (t, J = 25.8 Hz, 2H), 7.89 - 7.70 (m, 4H), 7.30 (dd, J = 14.4, 6.9 Hz, 4H), 7.20 (dd, J = 10.1, 6.9 Hz, 5H), 6.93 - 6.84 (m, 4H), 6.58 (d, J = 5.9 Hz, 1H), 5.76 (s, 1H), 5.21 (s, 3H), 4.97 (d, J = 13.1 Hz, 3H), 4.50 (d, J = 8.2 Hz, 3H), 4.20 (s, 3H), 4.02 (s, 9H), 3.87 (dd, J = 18.8, 9.5 Hz, 3H), 3.80 - 3.63 (m, 11H), 3.41 (d, J = 8.7 Hz, 10H), 3.24 (s, 2H), 3.20 - 3.12 (m, 3H), 3.03 (dd, J = 26.7, 13.8 Hz, 3H), 2.47 (d, J = 9.6 Hz, 3H), 2.44 - 2.36 (m, 4H), 2.31 - 2.18 (m, 5H), 2.11 (d, J = 10.6 Hz, 13H), 2.07 (s, 2H), 2.03 (d, J = 7.3 Hz, 3H), 1.99 (s, 9H), 1.89 (s, 9H), 1.77 (s, 12H), 1.47 (s, 16H), 1.25 (s, 11H).

Preparation of compound SL01-CPG: To a mixture of compound 1a-16 (111 mg, 0.047 mmol, 1.0 eq) and N,N-diisopropylethylamine (36 mg, 0.282 mmol, 6.0 eq) in N,N-dimethylformamide (5.5 mL), 2-(7-azabenzotriazole)-N,N,N',N'-tetramethylurea hexafluorophosphate (21 mg, 0.056 mmol, 1.2 eq) and 1-hydroxybenzotriazole (9 mg, 0.066 mmol, 1.4 eq) were added. The mixture was shaken at room temperature for 5 min. CPG-NH₂ (0.167 mmol/g, 550 mg, 0.092 mmol, 1.95 eq) was added, and the mixture was shaken at room temperature for 22 h. The mixture was filtered. The filter cake was washed with dichloromethane, acetonitrile, and dichloromethane, then dried under vacuum for 1 h. The residue was added with pyridine/acetic anhydride (3 mL/1 mL), and the mixture was shaken at room temperature for 3 h. The mixture was filtered, and the filter cake was washed with dichloromethane, acetonitrile, and dichloromethane, then dried under vacuum for 1 h to give SL01-CPG as a white solid (578 mg, load: 35 µmol/g).

### Embodiment 16 Preparation of Intermediate M

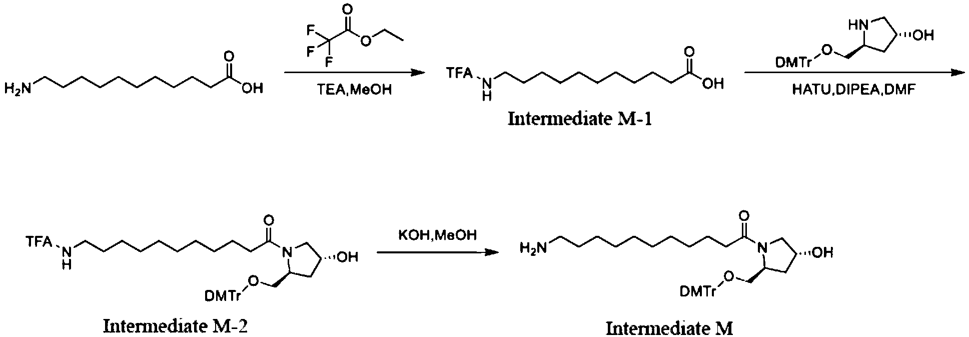

To a mixture of 11-aminoundecanoic acid (5 g, 24.8 mmol, 1.0 eq) and triethylamine (2.5 g, 24.8 mmol, 1.0 eq) in methanol (50 mL), ethyl 2,2,2-trifluoroacetate (4.4 g, 31.0 mmol, 1.25 eq) was added. The mixture was stirred at room temperature overnight. Methanol was removed under vacuum. The residue was dissolved in water, and the pH was adjusted to 1-2 using a 1 mol/L hydrochloric acid solution. The residue was extracted with ethyl acetate. The organic phase was washed with saturated brine and concentrated under vacuum to give 11-(2,2,2-trifluoroacetamido)undecanoic acid as a yellow oil (intermediate M-1, 6.16 g, yield: 83.4%). LCMS (ESI): m/z = 298 [M+H]⁺.

To a mixture of 11-(2,2,2-trifluoroacetamido)undecanoic acid (6.06 g, 20.4 mmol, 1.0 eq), (3R,5S)-5-((bis(4-methoxyphenyl)(phenyl)methoxy)methyl)pyrrolidin-3-ol (8.55 g, 20.4 mmol, 1.0 eq) and N,N-diisopropylethylamine (7.8 g, 61.2 mmol, 3.0 eq) in N,N-dimethylformamide (60 mL), 2-(7-azabenzotriazole)-N,N,N',N'-tetramethylurea hexafluorophosphate (8.1 g, 21.4 mmol, 1.05 eq) was added. The mixture was stirred at room temperature for 1 h. The mixture was diluted with water and extracted with ethyl acetate. The organic phase was washed with saturated brine, and concentrated in vacuo. The residue was purified by silica gel column chromatography (eluent: petroleum ether/ethyl acetate = 2/1 to 3/1, with 0.5% triethylamine added) to give N-(11-((2S,4R)-2-((bis(4-methoxyphenyl)(phenyl)methoxy)methyl)-4-hydroxypyrrolidin-1-yl)-11-oxoundecyl)-2,2,2-trifluoroacetamide as a white solid (intermediate M-2, 11.6 g, yield: 80.7%). LCMS (ESI): m/z = 699 [M+H]⁺.

The mixture of N-(11-((2S,4R)-2-((bis(4-methoxyphenyl)(phenyl)methoxy)methyl)-4-hydroxypyrrolidin-1-yl)-11-oxoundecyl)-2,2,2-trifluoroacetamide (11.6 g, 16.5 mmol, 1.0 eq) and potassium hydroxide (9.3 g, 165 mmol, 10.0 eq) in methanol (110 mL) was stirred overnight at room temperature. The mixture was diluted with water and extracted with ethyl acetate. The organic phase was washed with saturated brine and concentrated in vacuo. The residue was purified by silica gel column chromatography (eluent: dichloromethane/methanol = 50/1, with 0.5% triethylamine added) to give intermediate M as a yellow oil (9.24 g, yield: 92.4%). LCMS (ESI): m/z = 603 [M+H]⁺.

### Embodiment 17 Preparation of DL07-CPG

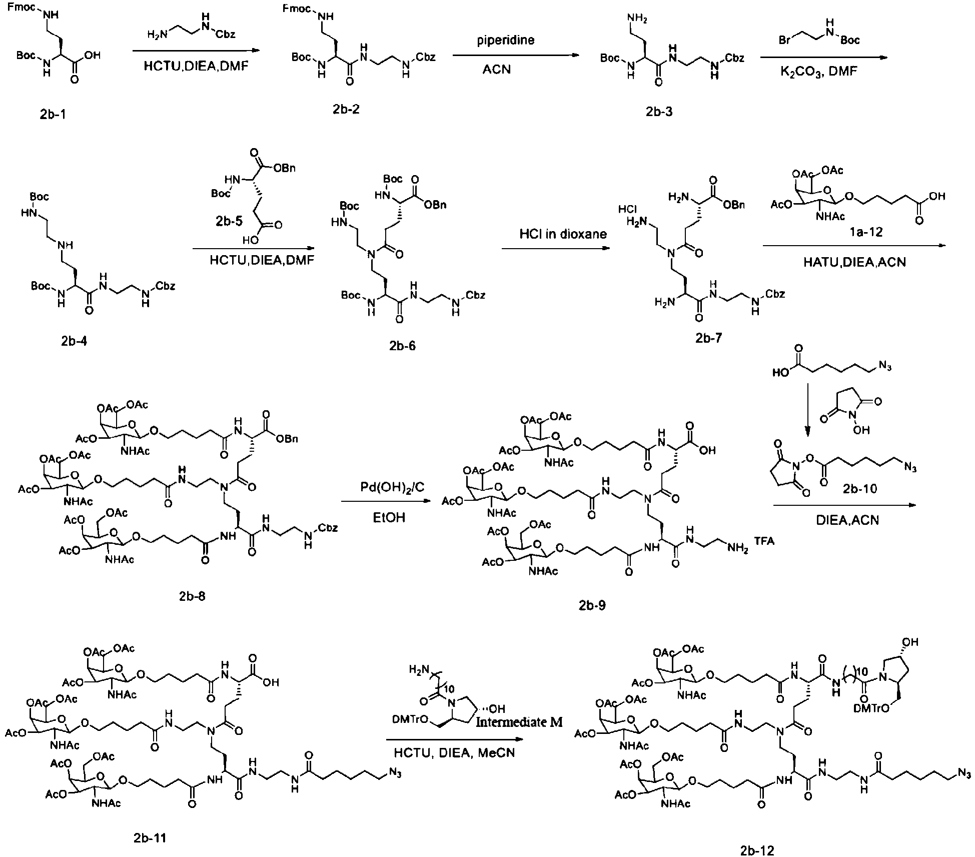

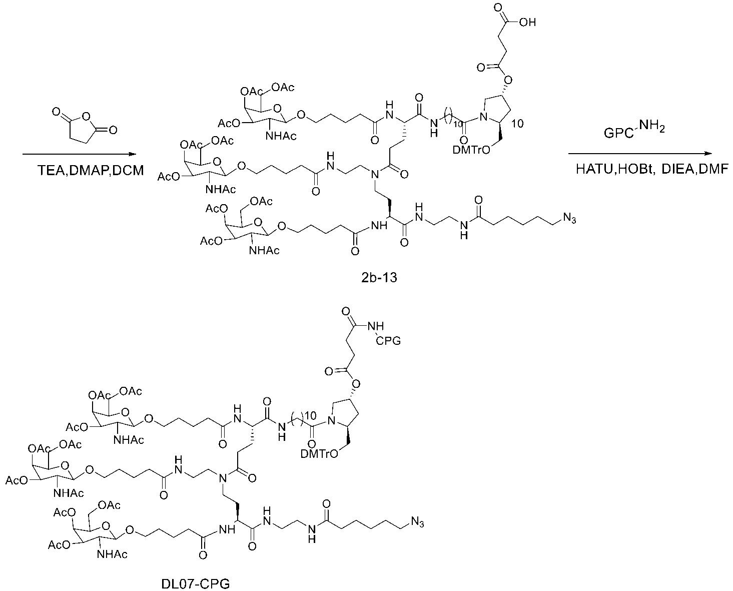

Preparation of compound 2b-2: The mixture of compound 2b-1 (22.02 g, 50.00 mmol, 1.0 eq), benzyl (2-aminoethyl)carbamate (9.71 g, 50.00 mmol, 1.0 eq), N,N-diisopropylethylamine (12.90 g, 10.00 mmol, 2.0 eq), and 6-chlorobenzotriazole-1,1,3,3-tetramethyluronium hexafluorophosphate (21.72 g, 52.50 mmol, 1.05 eq) in N,N-dimethylformamide (250 mL) was stirred for 30 min at room temperature. The mixture was diluted with water and then filtered. The filter cake was washed with water and dried to give 2b-2 as a white solid (crude).

LCMS(ESI): m/z =617[M+H]⁺. ¹H NMR (500 MHz, DMSO) δ 7.87 (t, *J =* 8.1 Hz, 3H), 7.67 (d, *J=* 6.9 Hz, 2H), 7.42 (d, *J =* 7.4 Hz, 2H), 7.37 - 7.26 (m, 7H), 7.19 (d, *J =* 4.7 Hz, 2H), 6.84 (d, *J =* 7.2 Hz, 1H), 5.00 (s, 2H), 4.34 - 4.15 (m, 3H), 3.90 (d, *J =* 5.0 Hz, 1H), 3.19 - 2.94 (m, 6H), 1.78 (d, *J* = 6.6 Hz, 1H), 1.62 (dd, *J =* 13.1, 6.6 Hz, 1H), 1.37 (s, 9H).

Preparation of compound 2b-3: The mixture of compound 2b-2 (crude) in piperidine/acetonitrile (50 mL/200 mL) was stirred at room temperature for 1 h. The mixture was filtered, the filtrate concentrated, and the residue purified by silica gel column chromatography (eluent: dichloromethane to dichloromethane/methanol = 20/1) to give 2b-3 as a yellow solid (15.0 g, yield: 76.14%). LCMS (ESI): m/z = 395 [M+H]⁺.

Preparation of compound 2b-4: The mixture of compound 2b-3 (7.80 g, 19.80 mmol, 1.5 eq), tert-butyl (2-bromoethyl)carbamate (2.96 g, 13.20 mmol, 1.0 eq), and potassium carbonate (3.64 g, 26.40 mmol, 2.0 eq) in N,N-dimethylformamide (80 mL) was stirred overnight at room temperature. The mixture was diluted with water and extracted with ethyl acetate. The organic phase was washed with saturated brine, dried over anhydrous sodium sulfate, and concentrated. The residue was purified by silica gel column chromatography (eluent: dichloromethane/methanol = 100/1 to 50/1) to give 2b-4 as a colorless oil (4.0 g, yield: 56.43%).

LCMS(ESI): m/z =538[M+H]⁺. ¹H NMR (500 MHz, DMSO) δ 7.92 (s, 1H), 7.42 - 7.28 (m, 5H), 7.19 (s, 1H), 6.93 (d, *J =* 7.3 Hz, 1H), 6.75 (s, 1H), 5.01 (s, 2H), 3.95 (d, *J =* 5.4 Hz, 1H), 3.14 (ddd, *J* = 18.3, 12.5, 6.1 Hz, 2H), 3.05 (d, *J* = 5.5 Hz, 4H), 2.68 - 2.56 (m, 4H), 1.79 (s, 1H), 1.72 - 1.60 (m, 1H), 1.37 (s, 18H).

Preparation of compound 2b-6: The mixture of compound 2b-4 (4.0 g, 7.45 mmol, 1.0 eq), compound 2b-5 (2.39 g, 7.08 mmol, 0.95 eq), N,N-diisopropylethylamine (1.92 g, 14.90 mmol, 2.0 eq), and 6-chlorobenzotriazole-1,1,3,3-tetramethyluronium hexafluorophosphate (3.08 g, 7.45 mmol, 1.0 eq) in N,N-dimethylformamide (50 mL) was stirred for 1 h at room temperature. The mixture was diluted with water and extracted with ethyl acetate. The organic phase was washed with saturated brine, dried over anhydrous sodium sulfate, and concentrated. The residue was purified by silica gel column chromatography (eluent: dichloromethane/methanol = 60/1) to give 2b-6 as a white solid (4.4 g, yield: 72.52%).

LCMS(ESI): m/z =858[M+H]⁺. ¹H NMR (500 MHz, DMSO) δ 7.88 (d, *J =* 12.6 Hz, 1H), 7.32 (d, *J* = 22.7 Hz, 10H), 7.26 - 7.13 (m, 2H), 7.01 - 6.65 (m, 2H), 5.25 - 5.03 (m, 2H), 5.00 (s, 2H), 4.10 - 3.96 (m, 1H), 3.82 (s, 1H), 3.17 (d, *J* = 43.7 Hz, 6H), 3.03 (d, *J* = 22.8 Hz, 4H), 2.43 - 2.21 (m, 2H), 2.03 - 1.86 (m, 1H), 1.79 (s, 2H), 1.63 (s, 1H), 1.44 - 1.29 (m, 27H).

Preparation of compound 2b-7: A solution of compound 2b-6 (4.4 g, 5.13 mmol, 1.0 eq) and hydrogen chloride in dioxane (40 mL) was stirred for 1 h at room temperature. The solvent was removed under vacuum. The residue was used directly in the next step without purification (crude). LCMS (ESI): m/z = 557 [M+H]⁺.

Preparation of compound 2b-8: The mixture of 2b-7 (crude), N,N-diisopropylethylamine (6.60 g, 51.16 mmol, 10.0 eq), compound 1a-12 (7.10 g, 15.87 mmol, 3.1 eq), and 2-(7-azabenzotriazole)-N,N,N',N'-tetramethylurea hexafluorophosphate (6.20 g, 16.31 mmol, 3.2 eq) in acetonitrile (70 mL) was stirred at room temperature for 1 h. The mixture was diluted with water and extracted with ethyl acetate. The organic phase was washed with saturated brine, dried over anhydrous sodium sulfate, and concentrated. The residue was purified by silica gel column chromatography (eluent: dichloromethane/methanol = 50/1 to 25/1) to give 2b-8 as a yellow solid (6.0 g, yield: 63.39%). LCMS (ESI): m/z = 923 [M/2+H]⁺.

Preparation of compound 2b-9: Under a hydrogen atmosphere, a mixture of compound 2b-8 (6.0 g, 3.25 mmol, 1.0 eq), palladium hydroxide/carbon (1.5 g, 25% mass fraction), and trifluoroacetic acid (371 mg, 3.25 mmol, 1.0 eq) in ethanol (100 mL) was stirred overnight at room temperature. The mixture was filtered through celite. The filter cake was washed with ethanol, and the filtrate was concentrated. The residue was used directly in the next step without purification (5.0 g, yield: 94.93%). LCMS (ESI): m/z = 811 [M/2+H]⁺.

Preparation of compound 2b-10: Under a nitrogen atmosphere, 1-(3-dimethylaminopropyl)-3-ethylcarbodiimide hydrochloride (40.2 g, 210 mmol, 1.1 eq) was added to the mixture of 6-azido-hexanoic acid (30 g, 190.8 mmol, 1.0 eq) and 1-hydroxypyrrolidine-2,5-dione (24 g, 210 mmol, 1.1 eq) in dichloromethane/N,N-dimethylformamide (270/30 mL). The mixture was stirred at room temperature overnight. Dichloromethane was removed under vacuum; 1 mol/L hydrochloric acid solution and tert-butyl methyl ether were added. The layers were separated. The organic layer was washed with sodium bicarbonate solution and saturated brine. The organic phase was dried and concentrated under vacuum to give 2b-10 as a yellow oil (46.8 g, yield: 96.8%). LCMS (ESI): m/z = 255 [M+1]⁺.

Preparation of compound 2b-11: The mixture of compound 2b-9 (5.0 g, 3.09 mmol, 1.0 eq), compound 2b-10 (824 mg, 3.24 mmol, 1.05 eq), and N,N-diisopropylethylamine (1.19 g, 9.22 mmol, 3.0 eq) in acetonitrile (60 mL) was stirred at room temperature for 1 h. The reaction proceeded directly to the next step without requiring post-treatment. LCMS (ESI): m/z = 881 [M/2+H]⁺.

Preparation of compound 2b-12 (compound 54): The mixture of compound 2b-11 (crude), intermediate M (2.04 g, 3.39 mmol, 1.1 eq), N,N-diisopropylethylamine (796 mg, 6.17 mmol, 2.0 eq), and 6-chlorobenzotriazole-1,1,3,3-tetramethyluronium hexafluorophosphate (1.4 g, 3.39 mmol, 1.1 eq) in acetonitrile (60 mL) was stirred for 1 h. The mixture was purified directly by HPLC (column: UniSil 10-120 C18, 30 × 250 mm) to give 2b-12 (compound 54) as a white solid (3.1 g, yield: 42.79%).

LCMS(ESI): m/z =1022 [(M-302)/2+H]⁺. ¹H NMR (500 MHz, DMSO) δ 8.04 - 7.72 (m, 9H), 7.30 (dd, *J =* 17.9, 8.1 Hz, 4H), 7.23 - 7.10 (m, 5H), 6.93 - 6.81 (m, 4H), 5.76 (s, 1H), 5.21 (d, *J* = 2.3 Hz, 3H), 5.02 - 4.83 (m, 4H), 4.49 (d, *J =* 8.2 Hz, 3H), 4.15 (s, 3H), 4.02 (s, 9H), 3.87 (q, *J =* 9.5 Hz, 3H), 3.72 (d, *J =* 12.9 Hz, 9H), 3.41 (s, 3H), 3.29 (d, *J =* 6.8 Hz, 2H), 3.07 (ddd, *J =* 23.5, 22.3, 17.2 Hz, 14H), 2.22 (dd, *J =* 22.2, 15.1 Hz, 4H), 2.11 (d, *J =* 13.0 Hz, 13H), 2.04 (dd, *J =* 14.3, 6.9 Hz, 5H), 1.99 (s, 10H), 1.89 (s, 9H), 1.84 (d, *J =* 13.3 Hz, 3H), 1.77 (s, 9H), 1.68 (d, *J =* 35.4 Hz, 2H), 1.50 (dd, *J =* 14.8, 7.4 Hz, 19H), 1.35 (s, 3H), 1.30 - 1.18 (m, 13H).

Preparation of compound 2b-13: The mixture of compound 2b-12 (3.1 g, 1.32 mmol, 1.0 eq), succinic anhydride (793 mg, 7.93 mmol, 6.0 eq), triethylamine (1.60 g, 15.84 mmol, 12.0 eq), and 4-dimethylaminopyridine (16 mg, 0.13 mmol, 0.1 eq) in dichloromethane (25 mL) was stirred at room temperature for 6 h. The mixture was washed with 10% sodium bicarbonate solution and acetonitrile. The organic phase was concentrated to give 2b-13 as a pale purple solid (3.4 g, crude). LCMS (ESI): m/z = 1072 [(M-302)/2+H]⁺.

Preparation of compound DL07-CPG: To a solution of compound 2b-13 (300 mg, 0.12 mmol, 1.0 eq) in N,N-dimethylformamide was added 2-(7-azabenzotriazole)-N,N,N',N'-tetramethylurea hexafluorophosphate (56 mg, 0.15 mmol, 1.2 eq), 1-hydroxybenzotriazole (23 mg, 0.17 mmol, 1.4 eq) and N,N-diisopropylethylamine (63 mg, 0.49 mmol, 4.0 eq), and the mixture was shaken at room temperature for 5 min. The mixture was added with 1.5 g of CPG-NH₂ and shaken overnight at room temperature. The mixture was filtered. The filter cake was washed with dichloromethane, acetonitrile, and dichloromethane, then dried under vacuum for 1 h. The residue was added with pyridine/acetic anhydride (12 mL/4 mL), and the mixture was shaken at room temperature for 3 h. The mixture was filtered, and the filter cake was washed with dichloromethane, acetonitrile, and dichloromethane, then dried under vacuum for 1 h to give DL07-CPG as a pale yellow solid (1.61 g, load: 41.1 µmol/g).

### Embodiment 18 Preparation of DL09-CPG

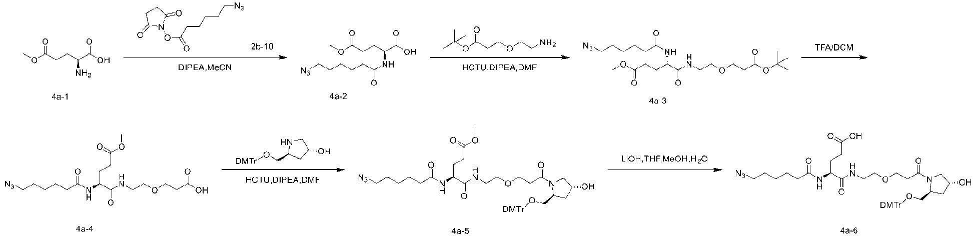

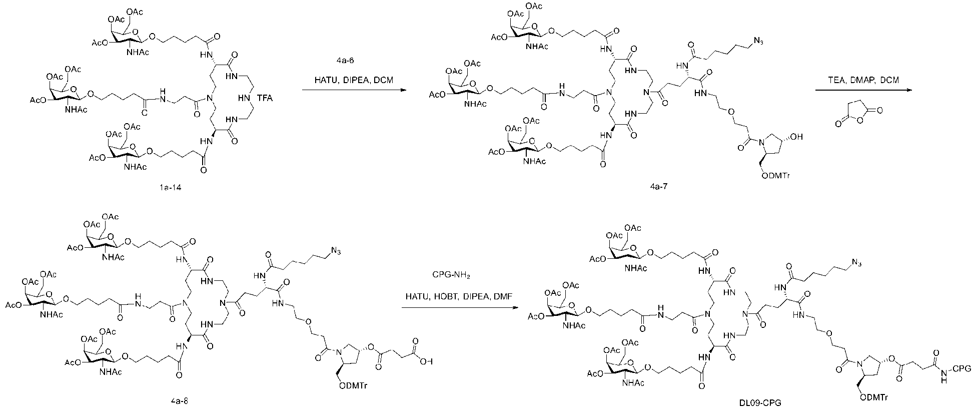

Preparation of compound 4a-2: Under a nitrogen atmosphere, the mixture of compound 4a-1 (10 g, 62 mmol, 1.0 eq), compound 2b-10 (17 g, 68 mmol, 1.1 eq), and N,N-diisopropylethylamine (18 g, 136 mmol, 2.2 eq) in acetonitrile (100 mL) was stirred overnight at room temperature. Acetonitrile was removed under vacuum; 1 mol/L hydrochloric acid solution and tert-butyl methyl ether were added. The layers were separated. The organic layer was washed with water and saturated brine. The organic phase was dried and concentrated under vacuum to give 4a-2 as a yellow oil (18.5 g, yield: 99.4%). LCMS (ESI): m/z = 301 [M+1]⁺.

Preparation of compound 4a-3: The mixture of compound 4a-2 (18.5 g, 61.5 mmol, 1.0 eq), tert-butyl 3-(2-aminoethoxy)propanoate (11.6 g, 61.5 mmol, 1.0 eq), and N,N-diisopropylethylamine (17.4 g, 135.2 mmol, 2.0 eq) in N,N-dimethylformamide (180 mL) was added with 6-chlorobenzotriazole-1,1,3,3-tetramethyluronium hexafluorophosphate (28 g, 67.6 mmol, 1.1 eq). The mixture was stirred at room temperature for 1 h. The mixture was diluted with water and extracted with ethyl acetate. The organic phase was washed with saturated brine, and concentrated in vacuo. The residue was purified by silica gel column chromatography (eluent: petroleum ether/ethyl acetate = 2/1 to dichloromethane/methanol = 20/1) to give 4a-3 as a white solid (14.8 g, yield: 51.4%). LCMS (ESI): m/z = 472 [M+1]⁺.

Preparation of compound 4a-4: The mixture of compound 4a-3 (8.86 g, 20.7 mmol, 1.0 eq) in trifluoroacetic acid/dichloromethane (10/50 mL) was stirred at room temperature for 5.0 h. The mixture was concentrated under vacuum to give 4a-4 as a yellow oil (8 g, crude). LCMS (ESI): m/z = 416 [M+1]⁺.

Preparation of compound 4a-5: The mixture of compound 4a-4 (8.86 g, 18.7 mmol, 1.0 eq), (3R,5S)-5-((bis(4-methoxyphenyl)(phenyl)methoxy)methyl)pyrrolidin-3-ol (7.8 g, 18.7 mmol, 1.0 eq), and N,N-diisopropylethylamine (9.6 g, 75 mmol, 4.0 eq) in N,N-dimethylformamide (90 mL) was added with 6-chlorobenzotriazole-1,1,3,3-tetramethyluronium hexafluorophosphate (8.1 g, 19.7 mmol, 1.1 eq). The mixture was stirred at room temperature for 1 h. The mixture was diluted with water and extracted with ethyl acetate. The organic phase was washed with saturated brine, and concentrated in vacuo. The residue was purified by silica gel column chromatography (eluent: petroleum ether/dichloromethane/methanol = 50/50/1 to 30/30/1) to give 4a-5 as a white solid (7.5 g, yield: 49%). LCMS (ESI): m/z = 817 [M+1]⁺.

Preparation of compound 4a-6: The mixture of compound 4a-5 (7.5 g, 9.19 mmol, 1.0 eq) and lithium hydroxide monohydrate (1.1 g, 27.5 mmol, 3.0 eq) in tetrahydrofuran/methanol/water (60/30/30 mL) was stirred overnight at room temperature. The mixture was diluted with water and washed with tert-butyl methyl ether. The aqueous phase was extracted with dichloromethane. The organic phase was concentrated under vacuum to give 4a-6 as a pale white solid (4.68 g, yield: 63.4%). LC-MS (ESI): m/z = 801 [M-1]⁻.

Preparation of compound 4a-7 (compound 53): The mixture of compound 1a-14 (570 mg, 0.32 mmol, 1.0 eq), compound 4a-6 (260 mg, 0.32 mmol, 1.0 eq), and N,N-diisopropylethylamine (146 mg, 1.13 mmol, 3.5 eq) in N,N-dimethylformamide (2.5 mL) was added with 2-(7-azabenzotriazole)-N,N,N',N'-tetramethylurea hexafluorophosphate (148 mg, 0.39 mmol, 1.2 eq). The mixture was stirred at room temperature overnight. The mixture was purified by preparative HPLC (A: phosphate buffer at pH 7, B: acetonitrile, 30-90% acetonitrile concentration over 30 min) to give 4a-7 (compound 53) as a white solid (245 mg, yield: 31%). LCMS (ESI):m/z = 1065 [(M-302+2)/2]⁺.

Preparation of compound 4a-8: Succinic anhydride (60.5 mg, 0.60 mmol, 6.0 eq) was added to a mixture of compound 4a-7 (245 mg, 0.10 mmol, 1.0 eq), triethylamine (121 mg, 1.2 mmol, 12.0 eq), and 4-dimethylaminopyridine (12 mg, 0.10 mmol, 1.0 eq) in dichloromethane (2 mL). The mixture was stirred at room temperature for 20 h. The mixture was diluted with dichloromethane (10 mL), then washed with saturated sodium bicarbonate solution (10 mL × 2) and saturated brine (10 mL × 1), dried over anhydrous sodium sulfate, and concentrated to give 4a-8 as a white solid (275 mg, crude). LCMS (ESI): m/z = 1115 [(M-302+2)/2]⁺.

Preparation of compound DL09-CPG: To a mixture of compound 4a-8 (253 mg, 0.10 mmol, 1.0 eq) and N,N-diisopropylethylamine (77 mg, 0.60 mmol, 6.0 eq) in N,N-dimethylformamide (10 mL), 2-(7-azabenzotriazole)-N,N,N',N'-tetramethylurea hexafluorophosphate (57 mg, 0.15 mmol, 1.5 eq) and 1-hydroxybenzotriazole (20 mg, 0.15 mmol, 1.5 eq) were added. The mixture was shaken at room temperature for 5 min. CPG-NH₂ (0.167 mmol/g, 1.27 g, 0.21 mmol, 2.1 eq) was added, and the mixture was shaken at room temperature for 22 h. The mixture was filtered. The filter cake was washed with dichloromethane, acetonitrile, and dichloromethane, then dried under vacuum for 1 h. The residue was added with pyridine/acetic anhydride (12 mL/4 mL), and the mixture was shaken at room temperature for 3 h. The mixture was filtered, and the filter cake was washed with dichloromethane, acetonitrile, and dichloromethane, then dried under vacuum for 1 h to give DL09-CPG as a white solid (1.36 g, load: 30 µmol/g).

### Embodiment 19 Preparation of DL15-CPG

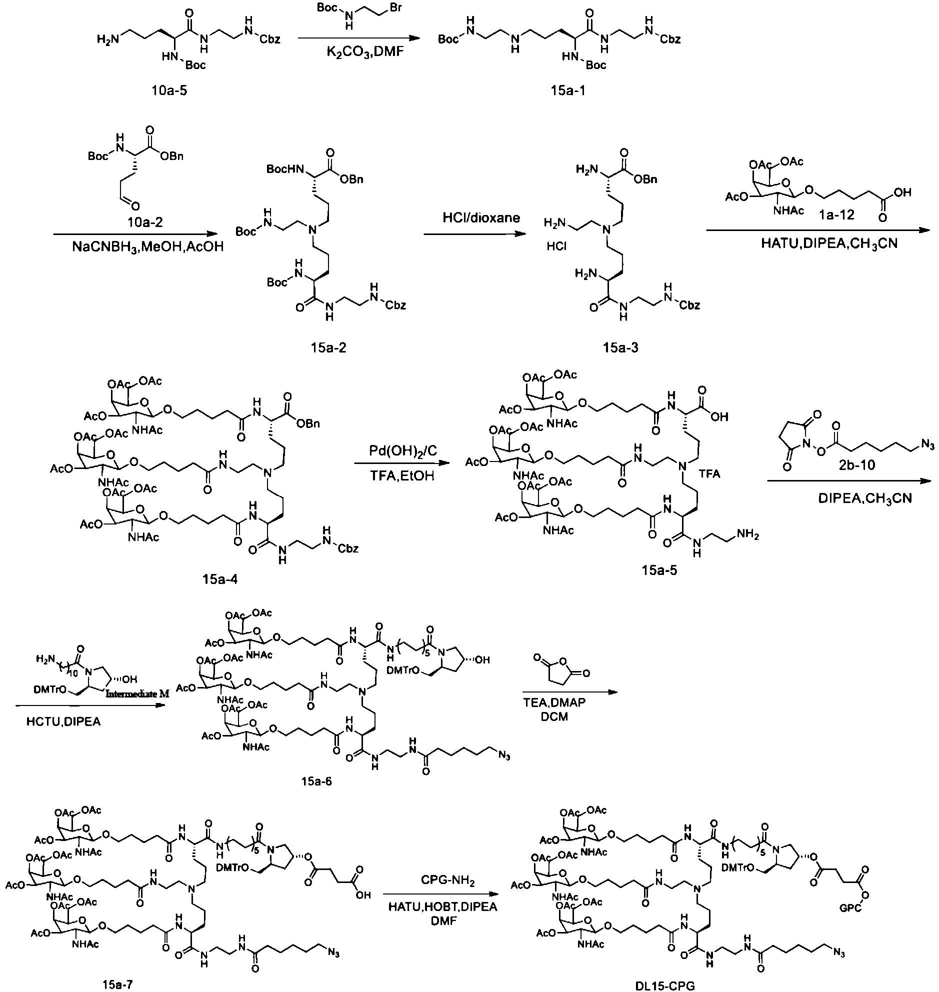

Preparation of compound 15a-1: The mixture of compound 10a-5 (6.0 g, 14.7 mmol, 1.5 eq) and tert-butyl (2-bromoethyl)carbamate (2.2 g, 9.8 mmol, 1.0 eq) in 30 mL of dimethylformamide was added with potassium carbonate (5.07 g, 36.75 mmol, 2.5 eq) at room temperature, and the mixture was stirred overnight. The mixture was diluted with ethyl acetate and washed with water and saturated brine. The organic phase was concentrated to dryness under reduced pressure. The residue was purified by silica gel column chromatography (eluent: dichloromethane/methanol = 30/1 to 10/1, with ammonia water added) to give 15a-1 as a white solid compound (3.29 g, yield: 60.8%). LCMS (ESI): m/z = 552 (M+H)⁺.

Preparation of compound 15a-2: Compound 15a-1 (1.7 g, 3.08 mmol, 1.0 eq), compound 10a-2 (3.2 g, 9.97 mmol, 3.2 eq), acetic acid (185 mg, 3.08 mmol, 1.0 eq), and sodium cyanoborohydride (626 mg, 9.97 mmol, 3.2 eq) were added to 24 mL of methanol. The mixture was stirred at room temperature for 96 h, concentrated under reduced pressure, treated with sodium carbonate solution, and extracted with ethyl acetate. The organic phase was washed with water and saturated brine, then concentrated to dryness under reduced pressure. The residue was purified by silica gel column chromatography (eluent: dichloromethane/methanol = 40/1 to 10/1, with ammonia water added) to give 15a-2 as a white solid compound (1.72 g, yield: 54.0%). LCMS (ESI): *m*/*z* = 857.5 (M+H)⁺. Preparation of compound 15a-3: Compound 15a-2 (1.72 g, 2.01 mmol, 1.0 eq) was added to 20 mL of 4M hydrogen chloride in dioxane. The mixture was stirred at room temperature for 1 h. The mixture was concentrated under reduced pressure to give 15a-3 as a compound (1.57 g, crude). LCMS (ESI): m/z = 557 (M+H)⁺.

Preparation of compound 15a-4: The crude compound 15a-3 (1.57 g, 2.01 mmol, 1.0 eq), compound 1a-12 (2.87 g, 6.41 mmol, 3.2 eq), diisopropylethylamine (2.58 g, 20.1 mmol, 10.0 eq), and 2-(7-azobenzotriazol)-N,N,N',N'-tetramethyluronium hexafluorophosphate (2.87 g, 6.41 mmol, 3.2 eq) were added to 35 mL of acetonitrile. The mixture was stirred at room temperature for 1 h. Ethyl acetate was added. The organic phase was washed with water and saturated brine, then concentrated to dryness under reduced pressure. The residue was purified by silica gel column chromatography (eluent: dichloromethane/methanol = 40/1 to 15/1, with triethylamine added) to give 15a-4 as a white solid compound (2.01 g, yield: 54.1%). LCMS (ESI): m/z = 1845 (M+H)⁺.

Preparation of compound 15a-5: Compound 15a-4 (2.0 g, 1.08 mmol, 1.0 eq) was dissolved in 45 mL of ethanol. Trifluoroacetic acid (160 mg, 1.41 mmol, 1.3 eq) and 300 mg of 10% palladium hydroxide/carbon were added. The mixture was stirred overnight at room temperature under a hydrogen atmosphere. The mixture was filtered through celite, and the filtrate was concentrated under reduced pressure to give 15a-5 as a white solid compound (2.0 g, crude). LCMS (ESI): m/z = 1621 (M+H)⁺.

Preparation of compound 15a-6: Compound 15a-5 (2.0 g, 1.08 mmol, 1.0 eq), diisopropylethylamine (418 mg, 3.24 mmol, 3.0 eq), and compound 2b-10 (307 mg, 1.21 mmol, 1.12 eq) were added to 15 mL of acetonitrile. The mixture was stirred at room temperature for 1.5 h. Diisopropylethylamine (209 mg, 1.62 mmol, 1.5 eq), 6-chlorobenzotriazole-1,1,3,3-tetramethyluronium hexafluorophosphate (538 mg, 1.30 mmol, 1.2 eq), and intermediate M (783 mg, 1.30 mmol, 1.2 eq) were added. The mixture was stirred at room temperature for 1 h. Ethyl acetate was added. The organic phase was washed with water and saturated brine, then concentrated to dryness under reduced pressure. The residue was purified by preparative liquid chromatography (eluent: pH 7.0 potassium dihydrogen phosphate-potassium hydroxide buffer/acetonitrile) to give 15a-6 as a white solid compound (1.81 g, yield: 37.1%). LCMS (ESI): m/z = 1022 ((M-302)/2+H)⁺.

Preparation of compound 15a-7: Under a nitrogen atmosphere, compound 15a-6 (300 mg, 0.128 mmol, 1.0 eq), triethylamine (155 mg, 1.54 mmol, 12.0 eq), succinic anhydride (77 mg, 0.768 mmol, 6.0 eq), and p-dimethylaminopyridine (1.6 mg, 0.013 mmol, 0.1 eq) were added to 1.5 mL of dichloromethane. The mixture was stirred overnight at room temperature. The mixture was diluted with dichloromethane/acetonitrile and washed with 10% sodium bicarbonate solution and saturated brine. The organic phase was dried over anhydrous sodium sulfate and concentrated under reduced pressure to give 15a-7 as a white solid compound (375 mg, crude). LCMS (ESI): m/z = 1072 ((M-302)/2+H)⁺.

Preparation of compound DL15-CPG: Compound 15a-7 (375 mg, 0.128 mmol, 1.0 eq), 2-(7-azobenzotriazol)-*N*,*N*,*N*',*N*'-tetramethyluronium hexafluorophosphate (68 mg, 0.179 mmol, 1.4 eq), 1-hydroxybenzotriazole (28 mg, 0.205 mmol, 1.6 eq), and diisopropylethylamine (76 mg, 0.59 mmol, 4.6 eq) were added to 13 mL of dimethylformamide. The mixture was stirred at room temperature for 5 min. The mixture was added with 1.735 g of CPG-NH₂, and shaken overnight at room temperature. The mixture was filtered, the solid phase was washed with dichloromethane and acetonitrile, and the solid phase was dried under reduced pressure. The mixture was added with 10 mL of pyridine and 3.4 mL of acetic anhydride, then shaken for 3 h. The mixture was filtered, the solid phase was washed with dichloromethane and acetonitrile, and the solid phase was dried under reduced pressure to give DL15-CPG as a yellow solid compound (1.39 g, 37.5 µmol/g).

### Embodiment 20 Preparation of DL16-CPG

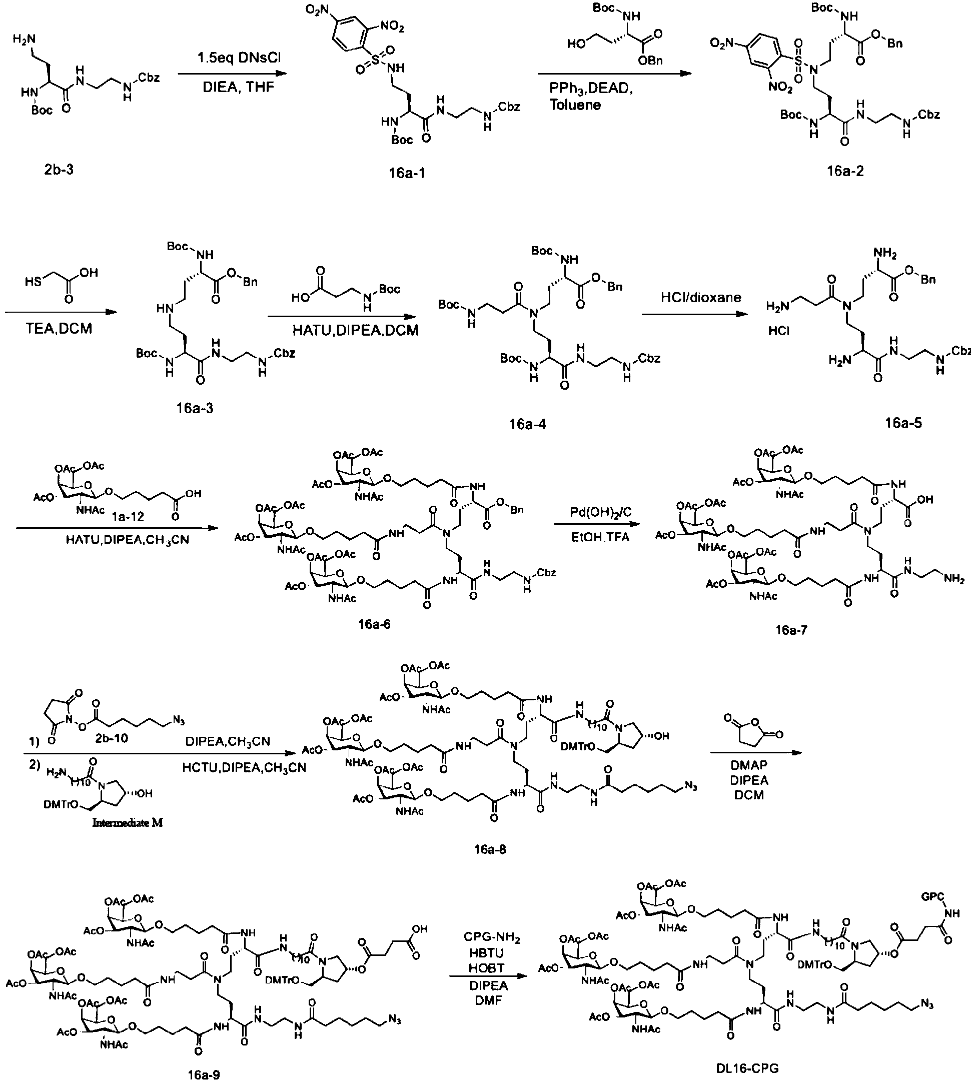

Preparation of compound 16a-1: Under a nitrogen atmosphere and ice bath conditions, 2,4-dinitrobenzenesulfonyl chloride (2.6 g, 9.7 mmol, 1.2 eq) in tetrahydrofuran (10 mL) was added dropwise to the mixture of compound 2b-3 (3.2 g, 8.1 mmol, 1.0 eq) and N,N-diisopropylethylamine (2.0 g, 16.2 mmol, 2.0 eq) in tetrahydrofuran (40 mL). The mixture was stirred in an ice bath for 1 h. Ethyl acetate was added to the mixture, followed by washing with water, dilute hydrochloric acid, aqueous sodium bicarbonate solution, and brine. The mixture was dried over anhydrous sodium sulfate and concentrated under reduced pressure to give 16a-1 as a brown solid (6.0 g, crude). LCMS (ESI): m/z=582 [M+H]⁺.

Preparation of compound 16a-2: Compound 16a-1 (1 g, 1.6 mmol, 1.0 eq), (tert-butoxycarbonyl)-L-homoserine benzyl ester (1.24 g, 4.0 mmol, 2.5 eq), and triphenylphosphine (1.26 g, 4.8 mmol, 3.0 eq) were added to 30 mL of toluene and stirred at room temperature for 5 min. Diethyl azodicarboxylate (0.835 g, 4.8 mmol, 3.0 eq) was added dropwise under ice bath conditions. The mixture was stirred at room temperature for 2 h. and filtered. The residue was dissolved in 30 mL of dichloromethane, then 30 mL of petroleum ether was added. The mixture was stirred at room temperature for 1 h. and filtered. The residue was washed with dichloromethane/petroleum ether (1:1) and dried to give 16a-2 as a yellow solid compound (1.42 g, yield: 96.59%). LCMS (ESI): m/z = 916 (M+H)⁺.

Preparation of compound 16a-3: Compound 16a-2 (1.6 g, 1.75 mmol, 1.0 eq) and triethylamine (530 mg, 5.25 mmol, 3.0 eq) were added to 10 mL of dichloromethane. The mixture was added with 2-mercaptoacetic acid (322 mg, 3.5 mmol, 2.0 eq) and stirred at room temperature for 10 min. Ethyl acetate was added for extraction. The organic phase was washed with aqueous sodium carbonate solution and saturated brine. The organic phase was concentrated under reduced pressure to give 16a-3 as a yellow oily compound (1.2 g, crude). LCMS (ESI): m/z = 686 (M+H)⁺.

Preparation of compound 16a-4: Compound 16a-3 (1.2 g, 1.75 mmol, 1.0 eq), 3-(tert-butoxycarbonyl)amino)propionic acid (331 mg, 1.75 mmol, 1.0 eq), diisopropylethylamine (451 mg, 3.5 mmol, 2.0 eq), and 2-(7-azobenzotriazol)-*N*,*N*,*N'*,*N'*-tetramethyluronium hexafluorophosphate (798 mg, 2.1 mmol, 1.2 eq) were added to 30 mL of dichloromethane. The mixture was stirred at room temperature for 1 h. Ethyl acetate was added for extraction. The organic phase was washed with water and saturated brine, then concentrated to dryness under reduced pressure. The residue was purified by silica gel column chromatography (eluent: dichloromethane/methanol = 25/1 to 10/1, with triethylamine added) to give 16a-4 as a white solid compound (740 mg, yield: 49.33%). LCMS (ESI): m/z = 858 (M+H)⁺.

Preparation of compound 16a-5: Compound 16a-4 (740 mg, 0.86 mmol, 1.0 eq) was added to 5 mL of 4M hydrogen chloride in dioxane. The mixture was stirred at room temperature for 1 h. The mixture was concentrated under reduced pressure to give 16a-5 as a yellow solid compound (482 mg, crude). LCMS (ESI): m/z = 557 (M+H)⁺.

Preparation of compound 16a-6: Compound 16a-5 (482 mg, 0.86 mmol, 1.0 eq), compound 1a-12 (1.2 g, 2.68 mmol, 3.1 eq), diisopropylethylamine (1.12 g, 8.64 mmol, 10.0 eq), and 2-(7-azobenzotriazol)-*N*,*N*,*N*',*N*'-tetramethyluronium hexafluorophosphate (1.05 g, 2.76 mmol, 3.2 eq) were added to 10 mL of acetonitrile. The mixture was stirred at room temperature for 1 h. Ethyl acetate was added for extraction. The organic phase was washed with water and saturated brine, then concentrated to dryness under reduced pressure. The residue was purified by silica gel column chromatography (eluent: dichloromethane/methanol = 25/1 to 10/1, with triethylamine added) to give 16a-6 as a white solid compound (879 mg, yield: 55.28%). LCMS (ESI): m/z = 1844 (M+H)⁺. Preparation of compound 16a-7: Compound 16a-6 (879 mg, 0.476 mmol, 1.0 eq) was dissolved in 5 mL of ethanol. Trifluoroacetic acid (54 mg, 0.476 mmol, 1.0 eq) and 88 mg of 10% palladium hydroxide/carbon were added. The mixture was stirred overnight at room temperature under a hydrogen atmosphere. The mixture was filtered through celite, and the filtrate was concentrated under reduced pressure to give 16a-7 as a white solid compound (800 mg, crude). LCMS (ESI): m/z = 1621 (M+H)⁺.

Preparation of compound 16a-8 (compound 65): Compound 16a-7 (800 mg, 0.476 mmol, 1.0 eq), diisopropylethylamine (184 mg, 1.428 mmol, 3 eq), and compound 2b-10 (133 mg, 0.523 mmol, 1.1 eq) were added to 10 mL of acetonitrile. The mixture was stirred at room temperature for 1.5 h. Diisopropylethylamine (184 mg, 1.428 mmol, 3 eq), 6-chlorobenzotriazole-1,1,3,3-tetramethyluronium hexafluorophosphate (236 mg, 0.57 mmol, 1.2 eq), and intermediate M (343 mg, 0.57 mmol, 1.2 eq) were added. The mixture was stirred at room temperature for 1 h. Ethyl acetate was added for extraction. The organic phase was washed with water and saturated brine, then concentrated to dryness under reduced pressure. The residue was purified by preparative liquid chromatography (eluent: pH 7.0 potassium dihydrogen phosphate-potassium hydroxide buffer/acetonitrile) to give 16a-8 as a yellow solid compound (343 mg, yield: 30.90%). LCMS (ESI): m/z = 1022 ((M-302)/2+H)⁺.

Preparation of compound 16a-9: Compound 16a-8 (343 mg, 0.146 mmol, 1.0 eq), triethylamine (177 mg, 1.752 mmol, 12.0 eq), succinic anhydride (88 mg, 0.878 mmol, 6.0 eq), and p-dimethylaminopyridine (2 mg, 0.014 mmol, 0.1 eq) were added to 5 mL of dichloromethane. The mixture was stirred overnight at room temperature. The mixture was diluted with dichloromethane and washed with 10% sodium bicarbonate solution and saturated brine. The organic phase was dried over anhydrous sodium sulfate and concentrated under reduced pressure to give 16a-9 as a yellow solid compound (396 mg, crude). LCMS (ESI): m/z = 1072 ((M-302)/2+H)⁺.

Preparation of compound DL16-CPG: Compound 16a-9 (396 mg, 0.162 mmol, 1.0 eq), 2-(7-azobenzotriazol)-N,N,N',N'-tetramethyluronium hexafluorophosphate (74 mg, 0.194 mmol, 1.2 eq), 1-hydroxybenzotriazole (30 mg, 0.226 mmol, 1.4 eq), and diisopropylethylamine (84 mg, 0.648 mmol, 4.0 eq) were added to 10 mL of dimethylformamide. The mixture was stirred at room temperature for 5 min. The mixture was added with 1.58 g of CPG-NH₂, and shaken overnight at room temperature. The mixture was filtered, the solid phase was washed with dichloromethane and acetonitrile, and the solid phase was dried under reduced pressure. The mixture was added with 9 mL of pyridine and 3 mL of acetic anhydride, then shaken for 3 h. The mixture was filtered, the solid phase was washed with dichloromethane and acetonitrile, and the solid phase was dried under reduced pressure to give DL16-CPG as a yellow solid compound (1.68 g, load: 45.7 µmol/g).

### Embodiment 21 Preparation of DL18-CPG

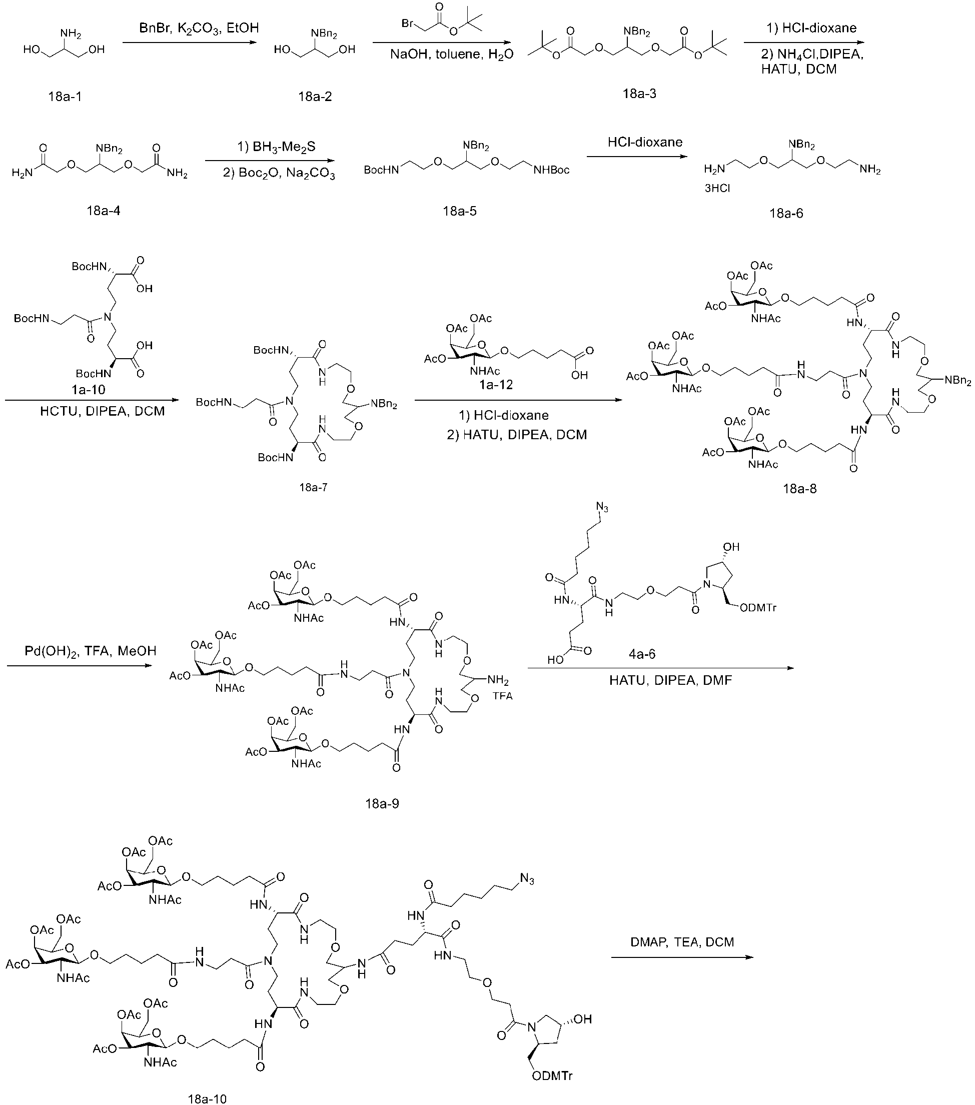

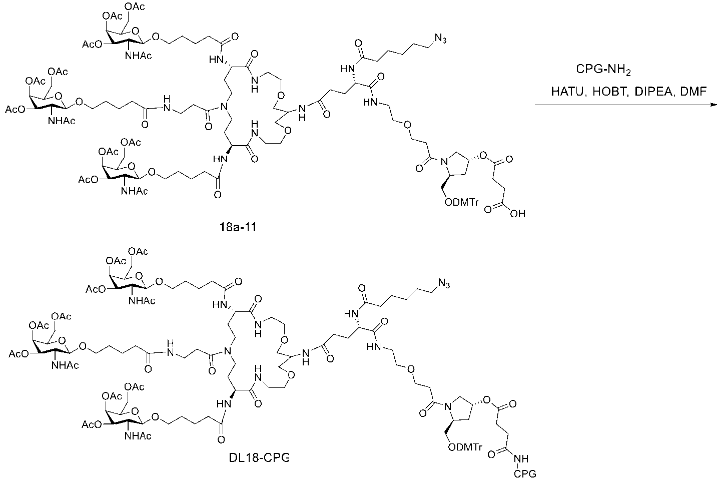

Preparation of compound 18a-2: The mixture of compound 18a-1 (4.0 g, 43.96 mmol, 1.0 eq) and potassium carbonate (18.2 g, 131.88 mmol, 3.0 eq) in ethanol (40 mL) was added with benzyl bromide (13.1 mL, 109.89 mmol, 2.5 eq). The mixture was heated to reflux for 3 h. The solvent was removed under reduced pressure. The residue was diluted with water (60 mL) and extracted with dichloromethane (50 mL × 2). The combined organic layer was washed with saturated brine (50 mL × 1), dried over anhydrous sodium sulfate, and concentrated. The residue was separated by silica gel column chromatography (dichloromethane:methanol = 80:1) to give 18a-2 as a white solid compound (9.89 g, yield: 83%). LCMS (ESI): m/z 272 [M+1]⁺; TLC: R_{f} 0.5 (dichloromethane:methanol = 50:1).

Preparation of compound 18a-3: The mixture of compound 18a-2 (6.7 g, 24.72 mmol, 1.0 eq), tert-butyl bromoacetate (28.78 g, 147.55 mmol, 6.0 eq), and tetrabutylammonium hydrogen sulfate (1.26 g, 3.71 mmol, 0.15 eq) in water (50 mL) and toluene (65 mL) was added with sodium hydroxide (15 g, 375 mmol, 15 eq). The mixture was heated to 50°C and reacted for 40 h. The mixture was diluted with water (50 mL) and extracted with dichloromethane (50 mL × 2). The combined organic layer was washed with saturated brine (50 mL × 1), dried over anhydrous sodium sulfate, and concentrated. The residue was separated by silica gel column chromatography (petroleum ether:ethyl acetate = 50:1) to give 18a-3 as a colorless oily compound (3.61 g, yield: 29%). LCMS (ESI): m/z 500 [M+1]⁺; TLC: R_{f} 0.5 (petroleum ether:ethyl acetate = 30:1).

Preparation of compound 18a-4: The mixture of compound 18a-3 (3.73 g, 7.46 mmol, 1.0 eq) and hydrogen chloride in dioxane (4M, 20 mL) was stirred overnight at room temperature. The solvent was removed under reduced pressure. The residue was diluted with dichloromethane (40 mL). Ammonium chloride (2.39 g, 44.76 mmol, 6.0 eq), N,N-diisopropylethylamine (7.7 g, 59.68 mmol, 8.0 eq), and 2-(7-azabenzotriazole)-N,N,N',N'-tetramethylurea hexafluorophosphate (8.51 g, 22.38 mmol, 3.0 eq) were added. The mixture was stirred at room temperature for 7 h. The solvent was removed under reduced pressure. The residue was diluted with saturated sodium carbonate solution (50 mL) and then extracted with ethyl acetate (20 mL × 3). The combined organic layer was washed with saturated brine (30 mL × 1), dried over anhydrous sodium sulfate, and concentrated to give 18a-4 as a pale yellow oily compound (3.5 g, crude). LCMS (ESI): m/z 386 [M+1]+; TLC: R_{f} 0.5 (dichloromethane:methanol = 20:1).

Preparation of compound 18a-5: Borane-methyl sulfide complex (10M, 5.97 mL, 59.7 mmol, 8.0 eq) was added to the mixture of compound 18a-4 (2.87 g, 7.46 mmol, 1.0 eq) in tetrahydrofuran (40 mL). The mixture was heated to 65°C and reacted for 5 h. The mixture was quenched with water (25 mL). Sodium carbonate (2.37 g, 22.38 mmol, 3.0 eq) and di-tert-butyl dicarbonate (4.88 g, 22.38 mmol, 3.0 eq) were added. The mixture was stirred at room temperature for 3 h. The mixture was diluted with water (50 mL) and then extracted with ethyl acetate (30 mL × 2). The combined organic layer was washed with saturated brine (30 mL × 1), dried over anhydrous sodium sulfate, and concentrated. The residue was separated by silica gel column chromatography (petroleum ether:ethyl acetate = 8:1 to 3:1) to give 18a-5 as a pale yellow oily compound (741 mg, yield: 18%). LCMS (ESI): m/z 558 [M+1]⁺; TLC: R_{f} 0.5 (petroleum ether:ethyl acetate = 5:1).

Preparation of compound 18a-6: The mixture of compound 18a-5 (741 mg, 1.33 mmol, 1.0 eq) in methanol (2 mL) was added with hydrogen chloride in dioxane (4M, 5 mL). The mixture was stirred at room temperature for 1 h. The solvent was removed under reduced pressure. The residue was dried under vacuum to give 18a-6 as a white solid crude compound. LCMS (ESI): m/z 358 [M+1]⁺; TLC: R_{f} 0.2 (dichloromethane:methanol = 10:1).

Preparation of compound 18a-7: At 0°C, the solution of compound 1a-10 (786 mg, 1.33 mmol, 1.0 eq), compound 18a-6 (616 mg, 1.33 mmol, 1.0 eq), and N,N-diisopropylethylamine (1.03 g, 7.98 mmol, 6.0 eq) in dichloromethane (15 mL) was added dropwise to a mixture of 6-chlorobenzotriazole-1,1,3,3-tetramethyluronium hexafluorophosphate (1.38 g, 3.33 mmol, 2.5 eq) in dichloromethane (85 mL) within 30 min. The mixture was brought to room temperature and stirred for 20 min. The solvent was removed under reduced pressure. The residue was diluted with saturated sodium carbonate solution (30 mL) and then extracted with ethyl acetate (20 mL × 3). The combined organic layer was washed with saturated brine (20 mL × 1), dried over anhydrous sodium sulfate, and concentrated. The residue was separated by silica gel column chromatography (ethyl acetate:methanol = 50:1 to 30:1) to give 18a-7 as a white solid compound (0.42 g, yield: 35%). LCMS (ESI): *m*/*z* 911 [M+1]⁺; TLC: R_{f} 0.3 (ethyl acetate:dichloromethane = 1:1).

Preparation of compound 18a-8: The mixture of compound 18a-7 (0.42 g, 0.46 mmol, 1.0 eq) and hydrogen chloride in dioxane (4M, 4 mL) was stirred for 1 h at room temperature. The solvent was removed under reduced pressure. The residue was diluted with dichloromethane (10 mL). Compound 1a-12 (618 mg, 1.38 mmol, 3.0 eq), N,N-diisopropylethylamine (475 mg, 3.68 mmol, 8.0 eq), and 2-(7-azabenzotriazole)-N,N,N',N'-tetramethylurea hexafluorophosphate (525 mg, 1.38 mmol, 3.0 eq) were added. The mixture was stirred at room temperature for 1 h. The mixture was diluted with dichloromethane (20 mL), washed with saturated sodium bicarbonate solution (20 mL × 2) and saturated brine (20 mL × 1), dried over anhydrous sodium sulfate, and concentrated. The residue was separated by silica gel column chromatography (dichloromethane:methanol:triethylamine = 30:1:0.3) to give 18a-8 as a white solid compound (778 mg, yield: 89%). LCMS (ESI): *m*/*z* 1900[M+1]⁺; TLC: R_{f} 0.5 (dichloromethane:methanol = 10:1).

Preparation of compound 18a-9: Palladium hydroxide/carbon (10%, 0.5 g) was added to a mixture of compound 18a-8 (778 mg, 0.409 mmol, 1.0 eq) and trifluoroacetic acid (47 mg, 0.409 mmol, 1.0 eq) in methanol (10 mL) and ethanol (5 mL). The mixture was stirred at room temperature overnight under hydrogen balloon pressure. and filtered. The filtrate was concentrated under reduced pressure to give 18a-9 as a pale yellow solid compound (600 mg, crude). LCMS (ESI): m/z 1720[M+1]⁺; TLC: R_{f} 0.3 (dichloromethane:methanol = 10:1).

Preparation of compound 18a-10: The mixture of compound 18a-9 (600 mg, 0.33 mmol, 1.0 eq), compound 4a-6 (265 mg, 0.33 mmol, 1.0 eq), and N,N-diisopropylethylamine (255 mg, 1.98 mmol, 6.0 eq) in N,N-dimethylformamide (2 mL) was added with 2-(7-azabenzotriazole)-N,N,N',N'-tetramethylurea hexafluorophosphate (188 mg, 0.495 mmol, 1.5 eq). The mixture was stirred at room temperature for 0.5 h. The mixture was purified by preparative liquid chromatography (A: phosphate buffer at pH 7, B: acetonitrile, 30%-90% acetonitrile concentration over 30 min) to give 18a-10 as a white solid compound (180 mg, yield: 22%). LCMS (ESI): m/z 1102 [(M-302+2)/2]⁺; TLC: R_{f} 0.5 (dichloromethane:methanol = 10:1).

Preparation of compound 18a-11: Succinic anhydride (43 mg, 0.43 mmol, 6.0 eq) was added to a mixture of compound 18a-10 (180 mg, 0.072 mmol, 1.0 eq), triethylamine (87 mg, 0.862 mmol, 12.0 eq), and 4-dimethylaminopyridine (4.4 mg, 0.036 mmol, 0.5 eq) in dichloromethane (2 mL). The mixture was stirred at room temperature for 20 h. The mixture was diluted with dichloromethane (10 mL), then washed with saturated sodium bicarbonate solution (10 mL × 2) and saturated brine (10 mL × 1), dried over anhydrous sodium sulfate, and concentrated to give 18a-11 as a white solid compound (155 mg, yield: 83%). LCMS (ESI): m/z 1152 [(M-302+2)/2]⁺; TLC: R_{f} 0.3 (dichloromethane:methanol = 10:1).

Preparation of compound DL18-CPG: To a mixture of compound 18a-11 (155 mg, 0.06 mmol, 1.0 eq) and N,N-diisopropylethylamine (46 mg, 0.36 mmol, 6.0 eq) in N,N-dimethylformamide (8 mL), 2-(7-azabenzotriazole)-N,N,N',N'-tetramethylurea hexafluorophosphate (34 mg, 0.09 mmol, 1.5 eq) and 1-hydroxybenzotriazole (12 mg, 0.09 mmol, 1.5 eq) were added. The mixture was stirred at room temperature for 5 min. CPG-NH₂ (0.167 mmol/g, 775 mg, 0.13 mmol, 2.2 eq) was added. The mixture was stirred at room temperature overnight. and filtered. The solid was washed with dichloromethane (8 mL × 5). Pyridine (6 mL) and acetic anhydride (2 mL) were added. The mixture was stirred at room temperature for 3 h. and filtered. The solid was washed with dichloromethane (8 mL × 5) and dried under vacuum to give DL18-CPG as a white solid compound (785 mg, 21.5 µmol/g, yield: 29%).

### Embodiment 22 Preparation of DL20-CPG

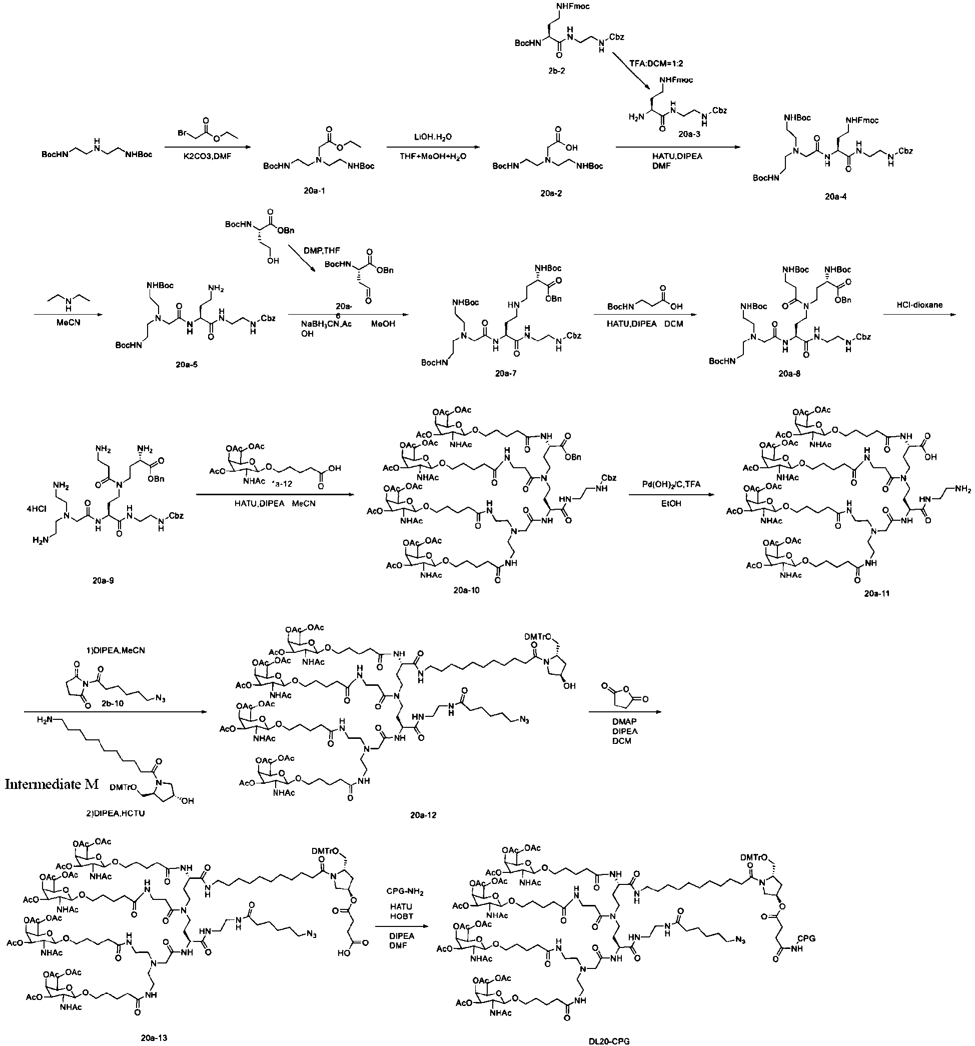

Preparation of compound 20a-1: The mixture of compound di-tert-butyl diyldicarbamate (5.0 g, 16.48 mmol, 1.00 eq), ethyl 2-bromoacetate (3.1 g, 18.13 mmol, 1.1 eq), and potassium carbonate (4.5 g, 32.96 mmol, 2.0 eq) in N,N-dimethylformamide (40 mL) was reacted overnight at room temperature. The mixture was diluted with water and extracted with ethyl acetate. The organic phase was washed with brine, dried over anhydrous sodium sulfate, and concentrated to give 20a-1 as a yellow oil (6.4 g, crude). LCMS (ESI): m/z = 390 [M+H]⁺.

Preparation of compound 20a-2: The solution of compound 20a-1 (6.2 g, 15.94 mmol, 1.0 eq) and lithium hydroxide monohydrate (1.6 g, 39.85 mmol, 2.5 eq) in tetrahydrofuran + methanol + water (40 mL + 10 mL + 10 mL) was stirred at 40°C for 2.5 h. The mixture was added with water, then 2M dilute hydrochloric acid to adjust the pH to 5. The aqueous phase was extracted with ethyl acetate. The organic phase was washed with brine, dried over anhydrous sodium sulfate, and concentrated to give 20a-2 as a compound (5.1 g, crude). LCMS (ESI): m/z = 362 [M+H]⁺.

Preparation of compound 20a-3: The mixture of compound 2b-2 (13.6 g, 22.05 mmol, 1.00 eq) in a trifluoroacetic acid/dichloromethane solution (50 mL/100 mL) was reacted for 1 h at room temperature. The mixture was spin-dried. The residue was adjusted to pH 8 with water. The organic phase was washed with brine, dried over anhydrous sodium sulfate, and concentrated to give 20a-3 as a white solid (6.1 g, yield: 53.6%). LCMS (ESI): m/z=517 [M+H]⁺.

Preparation of compound 20a-4: The mixture of compound 20a-2 (4.3 g, 11.82 mmol, 1.0 eq), compound 20a-3 (6.1 g, 11.82 mmol, 1.0 eq), 2-(7-azabenzotriazole)-N,N,N',N'-tetramethylurea hexafluorophosphate (5.4 g, 14.18 mmol, 1.2 eq), and diisopropylethylamine (3.1 g, 23.04 mmol, 2.0 eq) in N,N-dimethylformamide (50 mL) was stirred at room temperature for 1 h. The mixture was diluted with water and extracted with ethyl acetate. The organic phase was washed with brine, dried over anhydrous sodium sulfate, and concentrated. The residue was stirred with dichloromethane/petroleum ether = 1:1. The mixture was filtered to give 20a-4 as a yellow solid compound (10 g, crude). LCMS (ESI): m/z = 861 [M+H]⁺.

Preparation of compound 20a-5: A solution of compound 20a-4 (9.5 g, 11.05 mmol, 1.0 eq) in diethylamine/acetonitrile (20/80 mL) was stirred at room temperature for 1 h. The mixture was purified by silica gel column chromatography (eluent: dichloromethane to dichloromethane/methanol = 10/1) to give 20a-5 as a yellow solid (5.1 g, yield: 72.5%). LCMS (ESI): m/z = 638 [M+H]⁺.

Preparation of compound 20a-6: At 0°C, Dess-Martin reagent (4.7 g, 11.17 mmol, 1.5 eq) was added to a solution of (tert-butoxycarbonyl)-L-homoserine benzyl ester (2.3 g, 7.44 mmol, 1.0 eq) in tetrahydrofuran (30 mL). The mixture was stirred at room temperature for 2 h. The mixture was quenched with saturated sodium thiosulfate solution and then extracted with ethyl acetate. The organic phase was washed with brine, dried over anhydrous sodium sulfate, and concentrated to give 20a-6 as a yellow oil (3.5 g, crude). LCMS (ESI): m/z = 308 [M+H]⁺.

Preparation of compound 20a-7: Compound 20a-5 (3.65 g, 5.72 mmol, 1.0 eq), compound 20a-6 (1.8 g, 6.01 mmol, 1.05 eq), and acetic acid (343 mg, 5.72 mmol, 1.0 eq) were dissolved in methanol (50 mL), and the solution was stirred at room temperature for 30 min. Sodium cyanoborohydride was added at 0°C. The mixture was stirred at room temperature for 1.5 h. The mixture was quenched with water and extracted with ethyl acetate. The organic phase was washed with saturated sodium carbonate solution and brine, dried over anhydrous sodium sulfate, and concentrated. The residue was purified by silica gel column chromatography (eluent: dichloromethane to dichloromethane/methanol = 100/1 to 30/1) to give 20a-7 as a white solid (2.65 g, yield: 49.9%). LCMS (ESI): m/z = 930 [M+H]⁺.

Preparation of compound 20a-8: Compound 20a-7 (3 g, 3.23 mmol, 1.0 eq), 3-(tert-butoxycarbonyl)amino)propionic acid (611 mg, 3.23 mmol, 1.0 eq), diisopropylethylamine (835 mg, 6.46 mmol, 2.0 eq), and 2-(7-azobenzotriazol)-N,N,N',N'-tetramethyluronium hexafluorophosphate (1.5 mg, 3.88 mmol, 1.2 eq) were added to dichloromethane (36 mL). The mixture was stirred at room temperature for 1 h. The mixture was diluted with water and extracted with ethyl acetate. The organic phase was washed with brine, dried over anhydrous sodium sulfate, and concentrated. The residue was purified by silica gel column chromatography (eluent: dichloromethane/methanol = 100/1 to 20/1, with 1% triethylamine added) to give 20a-8 as a white solid compound (1.5 g, yield: 42.1%). LCMS (ESI): m/z = 1101 [M+H]⁺.

Preparation of compound 20a-9: Compound 20a-8 (1.5 g, 1.36 mmol, 1.0 eq) was added to 20 mL of 4M hydrogen chloride in dioxane. The mixture was stirred at room temperature for 1 h. The mixture was concentrated under reduced pressure to give 20a-9 as a yellow solid (1.1 g, crude). LCMS (ESI): m/z = 700 [M+H]⁺.

Preparation of compound 20a-10: Compound 20a-9 (1.1 g, 1.27 mmol, 1.0 eq), compound 1a-12 (2.3 g, 5.21 mmol, 4.1 eq), diisopropylethylamine (2 g, 15.24 mmol, 12.0 eq), and 2-(7-azobenzotriazol)-N,N,N',N'-tetramethyluronium hexafluorophosphate (2 g, 5.33 mmol, 4.2 eq) were added to 15 mL of acetonitrile. The mixture was stirred at room temperature for 1 h. The mixture was diluted with water and extracted with ethyl acetate. The organic phase was washed with brine, dried over anhydrous sodium sulfate, and concentrated. The residue was purified by silica gel column chromatography (eluent: dichloromethane/methanol = 100/1 to 10/1, with triethylamine added) to give DL20-8 as a white solid compound (1.8 g, yield: 58.6%). LCMS (ESI): m/z = 1209 [M/2+H]⁺.

Preparation of compound 20a-11: Compound 20a-10 (800 mg, 0.331 mmol, 1.0 eq) was dissolved in 8 mL of ethanol. Trifluoroacetic acid (38 mg, 0.331 mmol, 1.0 eq) and 80 mg of 10% palladium hydroxide/carbon were added. The mixture was stirred for two days at room temperature under a hydrogen atmosphere. The mixture was filtered, and the filtrate was concentrated under reduced pressure to give 20a-11 as a white solid compound (890 mg, crude). LCMS (ESI): m/z = 1097 [M/2+H]⁺.

Preparation of compound 20a-12: Compound 20a-11 (760 mg, 0.346 mmol, 1.0 eq), diisopropylethylamine (134 mg, 1.038 mmol, 3.0 eq), and compound 2b-10 (97 mg, 0.381 mmol, 1.1 eq) were added to 10 mL of acetonitrile. The mixture was stirred at room temperature for 1.5 h. Diisopropylethylamine (134 mg, 1.038 mmol, 3.0 eq), 6-chlorobenzotriazole-1,1,3,3-tetramethyluronium hexafluorophosphate (171 mg, 0.415 mmol, 1.2 eq), and intermediate M (249 mg, 0.415 mmol, 1.2 eq) were added. The mixture was stirred at room temperature for 1 h. The mixture was subject to reduced pressure to remove the solvent. The residue was purified by preparative liquid chromatography (eluent: pH 7.0 potassium dihydrogen phosphate-potassium hydroxide buffer/acetonitrile) to give DL20-10 as a yellow solid compound (272 mg, yield: 27.2%). LCMS (ESI): m/z = 1308 [(M-302)/2+H]⁺.

Preparation of compound 20a-13: Compound 20a-12 (172 mg, 0.0589 mmol, 1.0 eq), triethylamine (71 mg, 0.707 mmol, 12.0 eq), succinic anhydride (35 mg, 0.354 mmol, 6.0 eq), and p-dimethylaminopyridine (1 mg, 0.00589 mmol, 0.1 eq) were added to 1 mL of dichloromethane. The mixture was stirred overnight at room temperature. The mixture was diluted with dichloromethane and washed with 10% sodium bicarbonate solution and saturated brine. The organic phase was dried over anhydrous sodium sulfate and concentrated under reduced pressure to give 20a-13 as a yellow solid compound (167 mg, crude). LCMS (ESI): m/z = 1358 [(M-302)/2+H]⁺.

Preparation of compound DL20-CPG: Compound 20a-13 (167 mg, 0.0554 mmol, 1.0 eq), 2-(7-azobenzotriazol)-N,N,N',N'-tetramethyluronium hexafluorophosphate (29 mg, 0.0775 mmol, 1.2 eq), 1-hydroxybenzotriazole (12 mg, 0.0886 mmol, 1.6 eq), and diisopropylethylamine (29 mg, 0.222 mmol, 4.0 eq) were added to 2 mL of dimethylformamide. The mixture was stirred at room temperature for 5 min. The mixture was added with 501 mg of CPG-NH₂, and shaken overnight at room temperature. The mixture was filtered, the solid phase was washed with dichloromethane and acetonitrile, and the solid phase was dried under reduced pressure. The mixture was added with 6 mL of pyridine and 2 mL of acetic anhydride, then shaken for 3 h. The mixture was filtered, the solid phase was washed with dichloromethane and acetonitrile, and the solid phase was dried under reduced pressure to give DL20-CPG as a yellow solid compound (532 mg, load: 28.6 µmol/g).

### Embodiment 23 Preparation of Q20-CPG

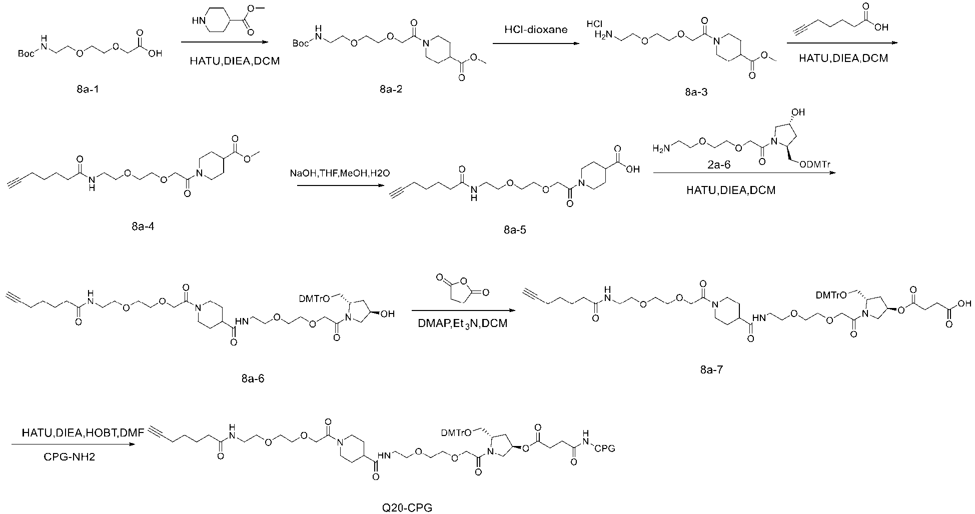

Preparation of compound 8a-2: The mixture of compound 8a-1 (2.72 g, 18.99 mmol, 1.0 eq), methyl piperidine-4-carboxylate (2.72 g, 18.99 mmol, 1.0 eq), N,N-diisopropylethylamine (4.90 g, 37.98 mmol, 2.0 eq), and 2-(7-azabenzotriazole)-N,N,N',N'-tetramethylurea hexafluorophosphate (8.47 g, 22.28 mmol, 1.2 eq) in dichloromethane (50 mL) was stirred at room temperature for 30 min. The mixture was concentrated under reduced pressure. The residue was diluted with water and extracted with ethyl acetate. The organic phase was washed with saturated brine, dried over anhydrous sodium sulfate, and concentrated to give 8a-2 as a yellow oil (12.7 g, crude). LCMS (ESI): m/z = 389 [M+H]⁺.

Preparation of compound 8a-3: The solution of compound 8a-2 (crude) and hydrogen chloride in dioxane (40 mL) was stirred at room temperature for 1 h. The solvent was removed under vacuum. The residue was used directly in the next step without purification (crude). LCMS (ESI): m/z = 289 [M+H]⁺.

Preparation of compound 8a-4: The mixture of compound 8a-3 (crude), hept-6-ynoic acid (2.35 g, 18.69 mmol, 1.0 eq), N,N-diisopropylethylamine (7.23 g, 56.06 mmol, 3.0 eq), and 2-(7-azabenzotriazole)-N,N,N',N'-tetramethylurea hexafluorophosphate (8.52 g, 22.42 mmol, 1.2 eq) in dichloromethane (50 mL) was stirred at room temperature for 30 min. The mixture was concentrated under reduced pressure. The residue was diluted with water and extracted with ethyl acetate. The organic phase was washed with saturated brine, dried over anhydrous sodium sulfate, and concentrated. The residue was purified by silica gel column chromatography (eluent: ethyl acetate to dichloromethane/methanol = 20/1) to give 8a-4 as a yellow oil (8.19 g, crude). LCMS (ESI): m/z = 397 [M+H]⁺.

Preparation of compound 8a-5: The mixture of compound 8a-4 (8.19 g, 18.69 mmol, 1.0 eq) and sodium hydroxide (1.24 g, 30.94 mmol, 1.5 eq) in tetrahydrofuran/methanol/water (20 mL/10 mL/10 mL) was stirred at room temperature for 1 h. The mixture was concentrated under reduced pressure, diluted with water, adjusted to pH 3 with dilute hydrochloric acid, and extracted with dichloromethane. The organic phase was dried over anhydrous sodium sulfate and concentrated to give 8a-5 as a yellow oil (6.0 g, crude). LCMS (ESI): m/z = 383 [M+H]⁺.

Preparation of compound 8a-6: The mixture of compound 8a-5 (1.1 g, 2.87 mmol, 1.0 eq), compound 2a-6 (1.22 g, 2.15 mmol, 0.75 eq), N,N-diisopropylethylamine (1.11 g, 8.62 mmol, 3.0 eq), and 2-(7-azabenzotriazole)-N,N,N',N'-tetramethylurea hexafluorophosphate (1.31 g, 3.45 mmol, 1.2 eq) in N,N-dimethylformamide (10 mL) was stirred at room temperature for 1 h. The mixture was purified by preparative HPLC (A: phosphate buffer at pH 7, B: acetonitrile, 30%-90% acetonitrile concentration over 30 min) to give 8a-6 as a white solid (320 mg, yield: 16%). LCMS (ESI): m/z = 627 [(M-302)+H]⁺.

Preparation of compound 8a-7: The mixture of compound 8a-6 (320 mg, 0.34 mmol, 1.0 eq), succinic anhydride (207 mg, 2.07 mmol, 6.0 eq), triethylamine (418 mg, 4.14 mmol, 12.0 eq), and 4-dimethylaminopyridine (5 mg, 0.034 mmol, 0.1 eq) in dichloromethane (5 mL) was stirred at room temperature overnight. The mixture was diluted with dichloromethane and washed with 10% sodium bicarbonate solution and saturated brine. The organic phase was dried over anhydrous sodium sulfate and concentrated under vacuum to give 8a-7 as a yellow solid (380 mg, crude). LCMS(ESI): m/z =727[(M-302)+H]⁺. ¹H NMR (500 MHz, MeOD) δ 7.42 - 7.12 (m, 9H), 6.93 - 6.71 (m, 4H), 5.55 - 5.28 (m, 1H), 4.40 (dd, *J =* 44.2, 7.2 Hz, 2H), 4.32 - 4.16 (m, 3H), 3.91 - 3.82 (m, 2H), 3.77 (s, 6H), 3.72 - 3.60 (m, 8H), 3.54 (td, *J =* 13.4, 6.2 Hz, 6H), 3.35 (dd, *J =* 14.8, 9.4 Hz, 4H), 3.18 - 3.05 (m, 6H), 3.02 - 2.82 (m, 1H), 2.64 - 2.41 (m, 6H), 2.39 - 2.24 (m, 1H), 2.17 (dtd, J= 17.2, 12.8, 5.6 Hz, 5H), 1.81 - 1.60 (m, 5H), 1.51 (dt, *J=* 14.4, 7.0 Hz, 3H).

Preparation of compound Q20-CPG: To a solution of compound 8a-7 (380 mg, 0.37 mmol, 1.0 eq) in N,N-dimethylformamide (10 mL) was added 2-(7-azabenzotriazole)-N,N,N',N'-tetramethylurea hexafluorophosphate (169 mg, 0.44 mmol, 1.2 eq), 1-hydroxybenzotriazole (70 mg, 0.52 mmol, 1.4 eq) and N,N-diisopropylethylamine (191 mg, 1.48 mmol, 4.0 eq), and the mixture was shaken at room temperature for 5 min. Then, CPG-NH₂ (1.9 g) was added to the reaction mixture and shaken overnight at room temperature. The mixture was filtered. The filter cake was washed with dichloromethane, acetonitrile, and dichloromethane, then dried under vacuum for 1 h. The residue was added with pyridine/acetic anhydride (7.5 mL/2.5 mL), and the mixture was shaken at room temperature for 3 h. The mixture was filtered, and the filter cake was washed with dichloromethane, acetonitrile, and dichloromethane, then dried under vacuum for 1 h to give Q20-CPG as a pale yellow solid (2.08 g, load: 101 µmol/g).

### Embodiment 24 Synthesis of Dual-Targeting siRNA

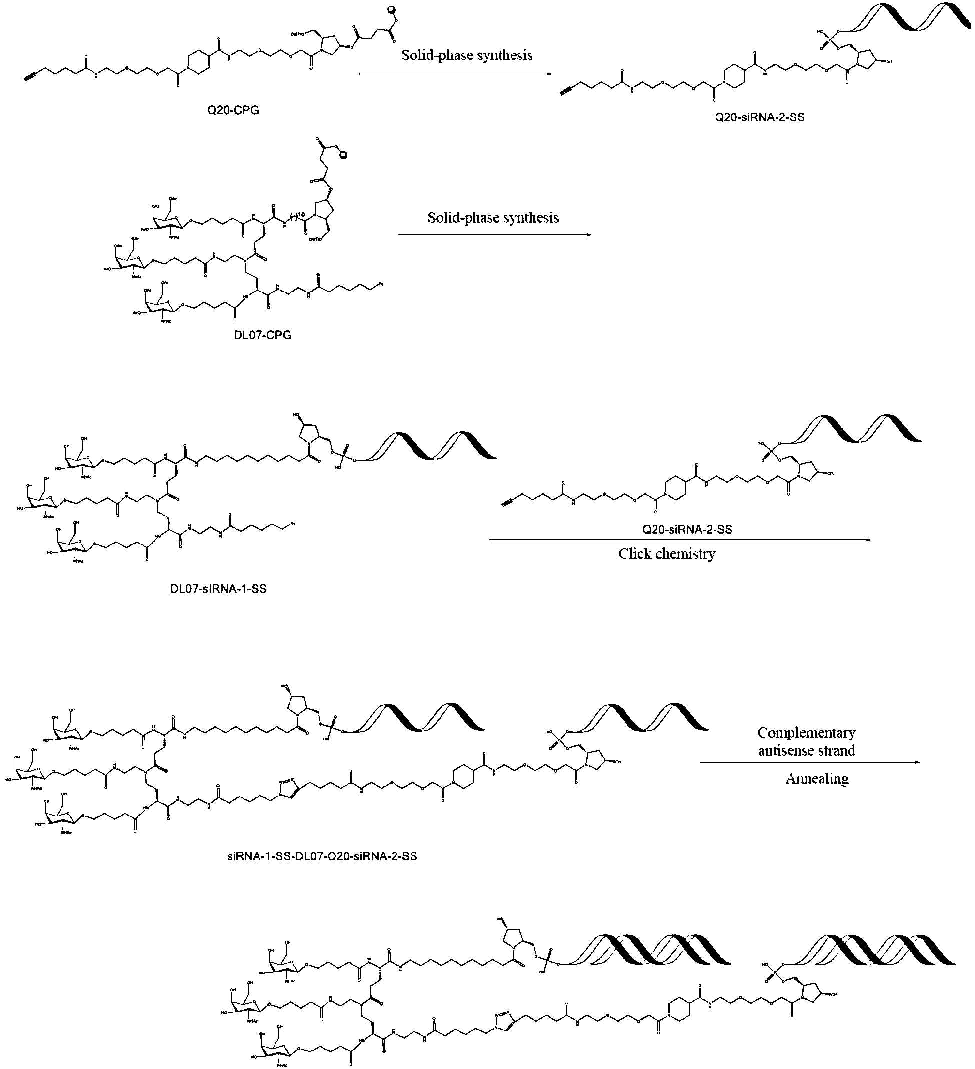

### 1. Synthesis of Two siRNA Sense Strands for Dual-Targeting siRNA Conjugation

First, with the solid-phase oligonucleotide synthesis method from Embodiment 1, the solid-phase carrier was replaced with DL07-CPG and Q20-CPG to synthesize DL07-siRNA-1-SS containing an azido group and Q20-siRNA-2-SS containing an alkyne group, respectively. Concurrently, universal CPG was used to synthesize the corresponding antisense strands.

### 2. Synthesis of Dual-Targeting siRNA-SS Chain

Specific quantities of DL07-siRNA-1-SS and Q20-siRNA-2-SS were taken and dissolved separately in DEPC-treated water, mixed in a 1:1.5 equivalent ratio, and added with CuSO₄·5H₂O (45 equiv.), THPTA (225 equiv.), NaVc (205 equiv.), MgCl₂ aqueous solution (100 mM, 50 equiv.), TEAA Buffer (2M, 1000 equiv.), and DMSO (1/2 volume of DEPC-treated water). The mixture was reacted overnight at room temperature. The reaction was monitored by LC-MS until completion. Three volumes of anhydrous ethanol and one-third volume of 3M NaCl solution were added to the reaction mixture. Then, the mixture was vigorously shaken and incubated at -20°C for approximately 30 min and centrifuged at 4°C and 15,000 rpm for 3 min. The supernatant was removed, then DEPC-treated water was added for dissolution. Following concentration determination, the mixture was purified by HPLC to obtain the dual-targeting siRNA-SS chain (siRNA-1-SS-DL07-Q20-siRNA-2-SS).

### 3. Synthesis of Dual-Targeting siRNA

An appropriate amount of the double-targeting siRNA-SS (siRNA-1-SS-DL07-Q20-siRNA-2-SS) strand was dissolved in DEPC-treated water. An equivalent amount of the two antisense strands, siRNA-1-AS and siRNA-2-AS, was added. The mixture was heated at 95°C for 5 min, then allowed to cool naturally to room temperature to obtain the dual-targeting siRNA (siRNA-1-DL07-Q20-siRNA-2).

The synthesis of dual-targeting siRNA may be adapted to other linkers and delivery systems, as illustrated in this Embodiment.

**Table 45: Dual-Target Sequence and Delivery System Structural Information**

| Target Genes | Dual-Target ID | Antisense/Sense Strand ID | Modified Sequence 5'->3' |
|---|---|---|---|
| AGT& ANGPTL3 | DT02042 | 1167AM25 | VPU- S*fU*mCmAmCmAmAmAmCmAmAdGmCfUmGfGmUm CmG*mG*mG |
| | | 7061AM18 | VPU- S*fU*mUmGmAmGmUmUmGmAmGdTmUfCmAfAmGm UmG*mG*mG |
| | | DT02042SM (7061SM14-DL07-1167SM19-Q20) | Invab*mU*mAmCmUmUmGfAfAfCfUmCfAmAmCmUmC mAmAmAInvabDL07- Invab*mU*mGmAmCmCmAfGfCfUfUmGfUmUmUmGmU mGmAmAInvabQ20 |
| AGT& ANGPTL3 | DT02043 | 1167AM27 | VPU- S*fU*mCmAmCmAmAmAmCmAmAdGmCfUmGfGmUm CmG*mG*mC |
| | | 7061AM4 | VPU- S*fU*mUmGmAmGmUmUmGmAmGdTmUfCmAfAmGm UmG*mG*mC |
| | | DT02043SM (7061SM16-DL07-1167SM21-Q20) | Invab*mG*mCmCmAmCmUmUmGfAfAfCfUmCfAmAmC mUmCmAmAmAInvabDL07- Invab*mG*mCmCmGmAmCmCmAfGfCfUfUmGfUmUmU mGmUmGmAmAInvabQ20 |
| AGT& ANGPTL3 | DT02044 | 1167AM25 | VPU- S*fU*mCmAmCmAmAmAmCmAmAdGmCfUmGfGmUm CmG*mG*mG |
| | | 7083AM9 | VPU- S*fA*mGmUmGmAmCmAmUmAmUdTmCfUmUfUmGm CmC*mG*mG |
| | | DT02044SM (7083SM2-DL07-1167SM19-Q20) | Invab*mG*mGmCmAmAmAfGfAfAfUmAfUmGmUmCmA mCmUmAInvabDL07- Invab*mU*mGmAmCmCmAfGfCfUfUmGfUmUmUmGmU mGmAmAInvabQ20 |
| AGT& ANGPTL3 | DT02045 | 1167AM27 | VPU- S*fU*mCmAmCmAmAmAmCmAmAdGmCfUmGfGmUm CmG*mG*mC |
| | | 7083AM10 | VPU- S*fA*mGmUmGmAmCmAmUmAmUdTmCfUmUfUmAm CmC*mU*mG |
| | | DT02045SM (7083SM3-DL07-1167SM21-Q20) | Invab*mC*mAmGmGmUmAmAmAfGfAfAfUmAfUmGm UmCmAmCmUmAInvabDL07- Invab*mG*mCmCmGmAmCmCmAfGfCfUfUmGfUmUmU mGmUmGmAmAInvabO20 |
| AGT& ANGPTL3 | DT02046 | 1165AM5 | VPU- S*fC*mGmCmUmAmAmAmCmAmCdTmGfGmUfUmCm UmU*mG*mG |
| | | 7061AM18 | VPU- S*fU*mUmGmAmGmUmUmGmAmGdTmUfCmAfAmGm UmG*mG*mG |
| | | DT02046SM (7061SM14-DL07-1165SM14-Q20) | Invab*mU*mAmCmUmUmGfAfAfCfUmCfAmAmCmUmC mAmAmAInvabDL07- Invab*mG*mAmGmAmAmCfCfAfGfUmGfUmUmUmAmG mCmGmAInvabQ20 |
| AGT& ANGPTL3 | DT02047 | 1165AM5 | VPU- S*fC*mGmCmUmAmAmAmCmAmCdTmGfGmUfUmCm UmU*mG*mG |
| | | 7061AM4 | VPU- S*fU*mUmGmAmGmUmUmGmAmGdTmUfCmAfAmGm UmG*mG*mC |
| | | DT02047SM (7061SM16-DL07-1165SM16-Q20) | Invab*mG*mCmCmAmCmUmUmGfAfAfCfUmCfAmAmC mUmCmAmAmAInvabDL07- Invab*mC*mCmAmAmGmAmAmCfCfAfGfUmGfUmUmU mAmGmCmGmAInvabQ20 |
| AGT& ANGPTL3 | DT02048 | 1165AM5 | VPU- S*fC*mGmCmUmAmAmAmCmAmCdTmGfGmUfUmCm UmU*mG*mG |
| | | 7083AM9 | VPU- S*fA*mGmUmGmAmCmAmUmAmUdTmCfUmUfUmGm CmC*mG*mG |
| | | DT02048SM (7083SM2-DL07-1165SM14-Q20) | Invab*mG*mGmCmAmAmAfGfAfAfUmAfUmGmUmCmA mCmUmAInvabDL07- Invab*mG*mAmGmAmAmCfCfAfGfUmGfUmUmUmAmG mCmGmAInvabQ20 |
| AGT& ANGPTL3 | DT02049 | 1165AM5 | VPU- S*fC*mGmCmUmAmAmAmCmAmCdTmGfGmUfUmCm UmU*mG*mG |
| | | 7083AM10 | VPU- S*fA*mGmUmGmAmCmAmUmAmUdTmCfUmUfUmAm CmC*mU*mG |
| | | DT02049SM (7083SM3-DL07-1165SM16-Q20) | Invab*mC*mAmGmGmUmAmAmAfGfAfAfUmAfUmGm UmCmAmCmUmAInvabDL07- Invab*mC*mCmAmAmGmAmAmCfCfAfGfUmGfUmUmU mAmGmCmGmAInvabQ20 |
| AGT& ANGPTL3 | DT02050 | 1167AM25 | VPU- S*fU*mCmAmCmAmAmAmCmAmAdGmCfUmGfGmUm CmG*mG*mG |
| | | 7061AM18 | VPU- S*fU*mUmGmAmGmUmUmGmAmGdTmUfCmAfAmGm UmG*mG*mG |
| | | DT02050SM (7061SM14-DL09-1167SM19-Q20) | Invab*mU*mAmCmUmUmGfAfAfCfUmCfAmAmCmUmC mAmAmAInvabDL09- Invab*mU*mGmAmCmCmAfGfCfUfUmGfUmUmUmGmU mGmAmAInvabQ20 |
| AGT& ANGPTL3 | DT02051 | 1167AM27 | VPU- S*fU*mCmAmCmAmAmAmCmAmAdGmCfUmGfGmUm CmG*mG*mC |
| | | 7061AM4 | VPU- S*fU*mUmGmAmGmUmUmGmAmGdTmUfCmAfAmGm UmG*mG*mC |
| | | DT02051SM (7061SM16-DL09-1167SM21-Q20) | Invab*mG*mCmCmAmCmUmUmGfAfAfCfUmCfAmAmC mUmCmAmAmAInvabDL09- Invab*mG*mCmCmGmAmCmCmAfGfCfUfUmGfUmUmU mGmUmGmAmAInvabQ20 |
| AGT& ANGPTL3 | DT02052 | 1167AM25 | VPU- S*fU*mCmAmCmAmAmAmCmAmAdGmCfUmGfGmUm CmG*mG*mG |
| | | 7083AM9 | VPU- S*fA*mGmUmGmAmCmAmUmAmUdTmCfUmUfUmGm CmC*mG*mG |
| | | DT02052SM (7083SM2-DL09-1167SM19-Q20) | Invab*mG*mGmCmAmAmAfGfAfAfUmAfUmGmUmCmA mCmUmAInvabDL09- Invab*mU*mGmAmCmCmAfGfCfUfUmGfUmUmUmGmU mGmAmAInvabQ20 |
| AGT& ANGPTL3 | DT02053 | 1167AM27 | VPU- S*fU*mCmAmCmAmAmAmCmAmAdGmCfUmGfGmUm CmG*mG*mC |
| | | 7083AM10 | VPU- S*fA*mGmUmGmAmCmAmUmAmUdTmCfUmUfUmAm CmC*mU*mG |
| | | DT02053SM (7083SM3-DL09-1167SM21-Q20) | Invab*mC*mAmGmGmUmAmAmAfGfAfAfUmAfUmGm UmCmAmCmUmAInvabDL09- Invab*mG*mCmCmGmAmCmCmAfGfCfUfUmGfUmUmU mGmUmGmAmAInvabQ20 |
| AGT& ANGPTL3 | DT02054 | 1165AM5 | VPU- S*fC*mGmCmUmAmAmAmCmAmCdTmGfGmUfUmCm UmU*mG*mG |
| | | 7061AM18 | VPU- S*fU*mUmGmAmGmUmUmGmAmGdTmUfCmAfAmGm UmG*mG*mG |
| | | DT02054SM (7061SM14-DL09-1165SM14-Q20) | Invab*mU*mAmCmUmUmGfAfAfCfUmCfAmAmCmUmC mAmAmAInvabDL09- Invab*mG*mAmGmAmAmCfCfAfGfUmGfUmUmUmAmG mCmGmAInvabQ20 |
| AGT& ANGPTL3 | DT02055 | 1165AM5 | VPU- S*fC*mGmCmUmAmAmAmCmAmCdTmGfGmUfUmCm UmU*mG*mG |
| | | 7061AM4 | VPU- S*fU*mUmGmAmGmUmUmGmAmGdTmUfCmAfAmGm UmG*mG*mC |
| | | DT02055SM (7061SM16-DL09-1165SM16-Q20) | Invab*mG*mCmCmAmCmUmUmGfAfAfCfUmCfAmAmC mUmCmAmAmAInvabDL09- Invab*mC*mCmAmAmGmAmAmCfCfAfGfUmGfUmUmU mAmGmCmGmAInvabQ20 |
| AGT& ANGPTL3 | DT02056 | 1165AM5 | VPU- S*fC*mGmCmUmAmAmAmCmAmCdTmGfGmUfUmCm UmU*mG*mG |
| | | 7083AM9 | VPU- S*fA*mGmUmGmAmCmAmUmAmUdTmCfUmUfUmGm CmC*mG*mG |
| | | DT02056SM (7083SM2-DL09-1165SM14-Q20) | Invab*mG*mGmCmAmAmAfGfAfAfUmAfUmGmUmCmA mCmUmAInvabDL09- Invab*mG*mAmGmAmAmCfCfAfGfUmGfUmUmUmAmG mCmGmAInvabQ20 |
| AGT& ANGPTL3 | DT02057 | 1165AM5 | VPU- S*fC*mGmCmUmAmAmAmCmAmCdTmGfGmUfUmCm UmU*mG*mG |
| | | 7083AM10 | VPU- S*fA*mGmUmGmAmCmAmUmAmUdTmCfUmUfUmAm CmC*mU*mG |
| | | DT02057SM (7083SM3-DL09-1165SM16-Q20) | Invab*mC*mAmGmGmUmAmAmAfGfAfAfUmAfUmGm UmCmAmCmUmAInvabDL09- Invab*mC*mCmAmAmGmAmAmCfCfAfGfUmGfUmUmU mAmGmCmGmAInvabQ20 |
| AGT& ANGPTL3 | DT02058 | 1161AM7 | VPU- S*fU*mCmCmAmGmUmUmGmAmGdGmGfAmGfUmUm UmU*mG*mG |
| | | 7061AM4 | VPU- S*fU*mUmGmAmGmUmUmGmAmGdTmUfCmAfAmGm UmG*mG*mC |
| | | DT02058SM (7061SM16-DL07-1161SM17-Q20) | Invab*mG*mCmCmAmCmUmUmGfAfAfCfUmCfAmAmC mUmCmAmAmAInvabDL07- Invab*mC*mCmAmAmAmAmCmUfCfCfCfUmCfAmAmC mUmGmGmAmAInvabQ20 |
| AGT& ANGPTL3 | DT02059 | 1167AM25 | VPU- S*fU*mCmAmCmAmAmAmCmAmAdGmCfUmGfGmUm CmG*mG*mG |
| | | 7061AM4 | VPU- S*fU*mUmGmAmGmUmUmGmAmGdTmUfCmAfAmGm UmG*mG*mC |
| | | DT02059SM (7061SM16-DL07-1167SM19-Q20) | Invab*mG*mCmCmAmCmUmUmGfAfAfCfUmCfAmAmC mUmCmAmAmAInvabDL07- Invab*mU*mGmAmCmCmAfGfCfUfUmGfUmUmUmGmU mGmAmAInvabQ20 |
| AGT& ANGPTL3 | DT02060 | 1167AM25 | VPU- S*fU*mCmAmCmAmAmAmCmAmAdGmCfUmGfGmUm CmG*mG*mG |
| | | 7061AM18 | VPU- S*fU*mUmGmAmGmUmUmGmAmGdTmUfCmAfAmGm UmG*mG*mG |
| | | DT02060SM (1167SM19-DL07-7061SM14-Q20) | Invab*mU*mGmAmCmCmAfGfCfUfUmGfUmUmUmGmU mGmAmAInvabDL07- Invab*mU*mAmCmUmUmGfAfAfCfUmCfAmAmCmUmC mAmAmAInvabO20 |
| AGT& ANGPTL3 | DT02061 | 1167AM27 | VPU- S*fU*mCmAmCmAmAmAmCmAmAdGmCfUmGfGmUm CmG*mG*mC |
| | | 7061AM4 | VPU- S*fU*mUmGmAmGmUmUmGmAmGdTmUfCmAfAmGm UmG*mG*mC |
| | | DT02061SM (1167SM21-DL07-7061SM16-Q20) | Invab*mG*mCmCmGmAmCmCmAfGfCfUfUmGfUmUmU mGmUmGmAmAInvabDL07- Invab*mG*mCmCmAmCmUmUmGfAfAfCfUmCfAmAmC mUmCmAmAmAInvabQ20 |
| AGT& ANGPTL3 | DT02062 | 1167AM27 | VPU- S*fU*mCmAmCmAmAmAmCmAmAdGmCfUmGfGmUm CmG*mG*mC |
| | | 7083AM10 | VPU- S*fA*mGmUmGmAmCmAmUmAmUdTmCfUmUfUmAm CmC*mU*mG |
| | | DT02062SM (1167SM21-DL07-7083SM3-Q20) | Invab*mG*mCmCmGmAmCmCmAfGfCfUfUmGfUmUmU mGmUmGmAmAInvabDL07- Invab*mC*mAmGmGmUmAmAmAfGfAfAfUmAfUmGm UmCmAmCmUmAInvabQ20 |
| AGT& ANGPTL3 | DT02063 | 1165AM5 | VPU- S*fC*mGmCmUmAmAmAmCmAmCdTmGfGmUfUmCm UmU*mG*mG |
| | | 7061AM18 | VPU- S*fU*mUmGmAmGmUmUmGmAmGdTmUfCmAfAmGm UmG*mG*mG |
| | | DT02063SM (1165SM14-DL07-7061SM14-Q20) | Invab*mG*mAmGmAmAmCfCfAfGfUmGfUmUmUmAmG mCmGmAInvabDL07- Invab*mU*mAmCmUmUmGfAfAfCfUmCfAmAmCmUmC mAmAmAInvabQ20 |
| AGT& ANGPTL3 | DT02064 | 1165AM5 | VPU- S*fC*mGmCmUmAmAmAmCmAmCdTmGfGmUfUmCm UmU*mG*mG |
| | | 7061AM4 | VPU- S*fU*mUmGmAmGmUmUmGmAmGdTmUfCmAfAmGm UmG*mG*mC |
| | | DT02064SM (1165SM16-DL07-7061SM16-Q20) | Invab*mC*mCmAmAmGmAmAmCfCfAfGfUmGfUmUmU mAmGmCmGmAInvabDL07- Invab*mG*mCmCmAmCmUmUmGfAfAfCfUmCfAmAmC mUmCmAmAmAInvabQ20 |
| AGT& ANGPTL3 | DT02065 | 1165AM5 | VPU- S*fC*mGmCmUmAmAmAmCmAmCdTmGfGmUfUmCm UmU*mG*mG |
| | | 7083AM10 | VPU- S*fA*mGmUmGmAmCmAmUmAmUdTmCfUmUfUmAm CmC*mU*mG |
| | | DT02065SM (1165SM16-DL07-7083SM3-Q20) | Invab*mC*mCmAmAmGmAmAmCfCfAfGfUmGfUmUmU mAmGmCmGmAInvabDL07- Invab*mC*mAmGmGmUmAmAmAfGfAfAfUmAfUmGm UmCmAmCmUmAInvabQ20 |
| AGT& ANGPTL3 | DT02066 | 1167AM25 | VPU- S*fU*mCmAmCmAmAmAmCmAmAdGmCfUmGfGmUm CmG*mG*mG |
| | | 7061AM18 | VPU- S*fU*mUmGmAmGmUmUmGmAmGdTmUfCmAfAmGm UmG*mG*mG |
| | | DT02066SM (1167SM19-DL09-7061SM14-Q20) | Invab*mU*mGmAmCmCmAfGfCfUfUmGfUmUmUmGmU mGmAmAInvabDL09- Invab*mU*mAmCmUmUmGfAfAfCfUmCfAmAmCmUmC mAmAmAInvabQ20 |
| AGT& ANGPTL3 | DT02067 | 1167AM27 | VPU- S*fU*mCmAmCmAmAmAmCmAmAdGmCfUmGfGmUm CmG*mG*mC |
| | | 7061AM4 | VPU- S*fU*mUmGmAmGmUmUmGmAmGdTmUfCmAfAmGm UmG*mG*mC |
| | | DT02067SM (1167SM21-DL09-7061SM16-Q20) | Invab*mG*mCmCmGmAmCmCmAfGfCfUfUmGfUmUmU mGmUmGmAmAInvabDL09- Invab*mG*mCmCmAmCmUmUmGfAfAfCfUmCfAmAmC mUmCmAmAmAInvabQ20 |
| AGT& ANGPTL3 | DT02068 | 1167AM27 | VPU- S*fU*mCmAmCmAmAmAmCmAmAdGmCfUmGfGmUm CmG*mG*mC |
| | | 7083AM10 | VPU- S*fA*mGmUmGmAmCmAmUmAmUdTmCfUmUfUmAm CmC*mU*mG |
| | | DT02068SM (1167SM21-DL09-7083SM3-Q20) | Invab*mG*mCmCmGmAmCmCmAfGfCfUfUmGfUmUmU mGmUmGmAmAInvabDL09- Invab*mC*mAmGmGmUmAmAmAfGfAfAfUmAfUmGm UmCmAmCmUmAInvabQ20 |
| AGT& ANGPTL3 | DT02069 | 1165AM5 | VPU- S*fC*mGmCmUmAmAmAmCmAmCdTmGfGmUfUmCm UmU*mG*mG |
| | | 7061AM18 | VPU- S*fU*mUmGmAmGmUmUmGmAmGdTmUfCmAfAmGm UmG*mG*mG |
| | | DT02069SM (1165SM14-DL09-7061SM14-Q20) | Invab*mG*mAmGmAmAmCfCfAfGfUmGfUmUmUmAmG mCmGmAInvabDL09- Invab*mU*mAmCmUmUmGfAfAfCfUmCfAmAmCmUmC mAmAmAInvabQ20 |
| AGT& ANGPTL3 | DT02070 | 1165AM5 | VPU- S*fC*mGmCmUmAmAmAmCmAmCdTmGfGmUfUmCm UmU*mG*mG |
| | | 7061AM4 | VPU- S*fU*mUmGmAmGmUmUmGmAmGdTmUfCmAfAmGm UmG*mG*mC |
| | | DT02070SM (1165SM16-DL09-7061SM16-Q20) | Invab*mC*mCmAmAmGmAmAmCfCfAfGfUmGfUmUmU mAmGmCmGmAInvabDL09- Invab*mG*mCmCmAmCmUmUmGfAfAfCfUmCfAmAmC mUmCmAmAmAInvabQ20 |
| AGT& ANGPTL3 | DT02071 | 1165AM5 | VPU- S*fC*mGmCmUmAmAmAmCmAmCdTmGfGmUfUmCm UmU*mG*mG |
| | | 7083AM10 | VPU- S*fA*mGmUmGmAmCmAmUmAmUdTmCfUmUfUmAm CmC*mU*mG |
| | | DT02071SM (1165SM16-DL09-7083SM3-Q20) | Invab*mC*mCmAmAmGmAmAmCfCfAfGfUmGfUmUmU mAmGmCmGmAInvabDL09- Invab*mC*mAmGmGmUmAmAmAfGfAfAfUmAfUmGm UmCmAmCmUmAInvabQ20 |
| AGT& PCSK9 | DT03081 | 1167AM25 | VPU- S*fU*mCmAmCmAmAmAmCmAmAdGmCfUmGfGmUm CmG*mG*mG |
| | | 11040AM10 | VPU- S*fG*mCmUmAmCmAmAmAmAmCdCmCfAmGfAmAm UmA*mA*mG |
| | | DT03081SM (11040SM23-DL07-1167SM19-Q20) | Invab*mC*mUmUmAmUmUmCmUfGfGfGfUmUfUmUmG mUmAmGmCmAInvabDL07- Invab*mU*mGmAmCmCmAfGfCfUfUmGfUmUmUmGmU mGmAmAInvabQ20 |
| AGT& PCSK9 | DT03082 | 1167AM25 | VPU- S*fU*mCmAmCmAmAmAmCmAmAdGmCfUmGfGmUm CmG*mG*mG |
| | | 11040AM10 | VPU- S*fG*mCmUmAmCmAmAmAmAmCdCmCfAmGfAmAm UmA*mA*mG |
| | | DT03082SM (11040SM23-DL09-1167SM19-Q20) | Invab*mC*mUmUmAmUmUmCmUfGfGfGfUmUfUmUmG mUmAmGmCmAInvabDL09- Invab*mU*mGmAmCmCmAfGfCfUfUmGfUmUmUmGmU mGmAmAInvabQ20 |
| AGT& PCSK9 | DT03085 | 1167AM27 | VPU- S*fU*mCmAmCmAmAmAmCmAmAdGmCfUmGfGmUm CmG*mG*mC |
| | | 11040AM10 | VPU- S*fG*mCmUmAmCmAmAmAmAmCdCmCfAmGfAmAm UmA*mA*mG |
| | | DT03085SM (11040SM23-DL07-1167SM21-Q20) | Invab*mC*mUmUmAmUmUmCmUfGfGfGfUmUfUmUmG mUmAmGmCmAInvabDL07- Invab*mG*mCmCmGmAmCmCmAfGfCfUfUmGfUmUmU mGmUmGmAmAInvabQ20 |
| AGT& PCSK9 | DT03086 | 1165AM5 | VPU- S*fC*mGmCmUmAmAmAmCmAmCdTmGfGmUfUmCm UmU*mG*mG |
| | | 11040AM10 | VPU- S*fG*mCmUmAmCmAmAmAmAmCdCmCfAmGfAmAm UmA*mA*mG |
| | | DT03086SM (11040SM23-DL07-1165SM16-Q20) | Invab*mC*mUmUmAmUmUmCmUfGfGfGfUmUfUmUmG mUmAmGmCmAInvabDL07- Invab*mC*mCmAmAmGmAmAmCfCfAfGfUmGfUmUmU mAmGmCmGmAInvabQ20 |
| AGT& PCSK9 | DT03089 | 1165AM5 | VPU- S*fC*mGmCmUmAmAmAmCmAmCdTmGfGmUfUmCm UmU*mG*mG |
| | | 11002AM17 | VPU- S*fU*mCmUmAmGmAmAmAmAmGdTmUfGmGfCmUm GmU*mG*mG |
| | | DT03089SM (11002SM21-DL07-1165SM14-Q20) | Invab*mG*mCmAmGmCmCfAfAfCfUmUfUmUmCmUmA mGmAmAInvabDL07- Invab*mG*mAmGmAmAmCfCfAfGfUmGfUmUmUmAmG mCmGmAInvabQ20 |
| AGT& PCSK9 | DT03090 | 1161AM7 | VPU- S*fU*mCmCmAmGmUmUmGmAmGdGmGfAmGfUmUm UmU*mG*mG |
| | | 11040AM10 | VPU- S*fG*mCmUmAmCmAmAmAmAmCdCmCfAmGfAmAm UmA*mA*mG |
| | | DT03090SM (11040SM23-DL07-1161SM17-Q20) | Invab*mC*mUmUmAmUmUmCmUfGfGfGfUmUfUmUmG mUmAmGmCmAInvabDL07- Invab*mC*mCmAmAmAmAmCmUfCfCfCfUmCfAmAmC mUmGmGmAmAInvabQ20 |
| AGT& PCSK9 | DT03092 | 1167AM25 | VPU- S*fU*mCmAmCmAmAmAmCmAmAdGmCfUmGfGmUm CmG*mG*mG |
| | | 11040AM10 | VPU- S*fG*mCmUmAmCmAmAmAmAmCdCmCfAmGfAmAm UmA*mA*mG |
| | | DT03092SM (11040SM23-DL15-1167SM19-Q20) | Invab*mC*mUmUmAmUmUmCmUfGfGfGfUmUfUmUmG mUmAmGmCmAInvabDL15- Invab*mU*mGmAmCmCmAfGfCfUfUmGfUmUmUmGmU mGmAmAInvabQ20 |
| AGT& PCSK9 | DT03093 | 1167AM25 | VPU- S*fU*mCmAmCmAmAmAmCmAmAdGmCfUmGfGmUm CmG*mG*mG |
| | | 11040AM10 | VPU- S*fG*mCmUmAmCmAmAmAmAmCdCmCfAmGfAmAm UmA*mA*mG |
| | | DT03093SM (11040SM23-DL16-1167SM19-Q20) | Invab*mC*mUmUmAmUmUmCmUfGfGfGfUmUfUmUmG mUmAmGmCmAInvabDL16- Invab*mU*mGmAmCmCmAfGfCfUfUmGfUmUmUmGmU mGmAmAInvabQ20 |
| AGT& PCSK9 | DT03095 | 1167AM25 | VPU- S*fU*mCmAmCmAmAmAmCmAmAdGmCfUmGfGmUm CmG*mG*mG |
| | | 11040AM10 | VPU- S*fG*mCmUmAmCmAmAmAmAmCdCmCfAmGfAmAm UmA*mA*mG |
| | | DT03095SM (11040SM23-DL18-1167SM19-Q20) | Invab*mC*mUmUmAmUmUmCmUfGfGfGfUmUfUmUmG mUmAmGmCmAInvabDL18- Invab*mU*mGmAmCmCmAfGfCfUfUmGfUmUmUmGmU mGmAmAInvabQ20 |
| AGT& PCSK9 | DT03097 | 1167AM25 | VPU- S*fU*mCmAmCmAmAmAmCmAmAdGmCfUmGfGmUm CmG*mG*mG |
| | | 11040AM10 | VPU- S*fG*mCmUmAmCmAmAmAmAmCdCmCfAmGfAmAm UmA*mA*mG |
| | | DT03097SM (11040SM23-DL20-1167SM19-Q20) | Invab*mC*mUmUmAmUmUmCmUfGfGfGfUmUfUmUmG mUmAmGmCmAInvabDL20- Invab*mU*mGmAmCmCmAfGfCfUfUmGfUmUmUmGmU mGmAmAInvabQ20 |
| AGT& PCSK9 | DT03099 | 1167AM25 | VPU- S*fU*mCmAmCmAmAmAmCmAmAdGmCfUmGfGmUm CmG*mG*mG |
| | | 11002AM17 | VPU- S*fU*mCmUmAmGmAmAmAmAmGdTmUfGmGfCmUm GmU*mG*mG |
| | | DT03099SM (11002SM21-DL07-1167SM19-Q20) | Invab*mG*mCmAmGmCmCfAfAfCfUmUfUmUmCmUmA mGmAmAInvabDL07- Invab*mU*mGmAmCmCmAfGfCfUfUmGfUmUmUmGmU mGmAmAInvabQ20 |
| AGT& PCSK9 | DT03100 | 1167AM27 | VPU- S*fU*mCmAmCmAmAmAmCmAmAdGmCfUmGfGmUm CmG*mG*mC |
| | | 11002AM10 | VPU- S*fU*mCmUmAmGmAmAmAmAmGdTmUfGmGfCmUm GmU*mA*mG |
| | | DT03100SM (11002SM23-DL07-1167SM21-Q20) | Invab*mC*mUmAmCmAmGmCmCfAfAfCfUmUfUmUmC mUmAmGmAmAInvabDL07- Invab*mG*mCmCmGmAmCmCmAfGfCfUfUmGfUmUmU mGmUmGmAmAInvabQ20 |
| AGT& PCSK9 | DT03101 | 1167AM25 | VPU- S*fU*mCmAmCmAmAmAmCmAmAdGmCfUmGfGmUm CmG*mG*mG |
| | | 11040AM10 | VPU- S*fG*mCmUmAmCmAmAmAmAmCdCmCfAmGfAmAm UmA*mA*mG |
| | | DT03101SM (1167SM19-DL07-11040SM23-Q20) | Invab*mU*mGmAmCmCmAfGfCfUfUmGfUmUmUmGmU mGmAmAInvabDL07- Invab*mC*mUmUmAmUmUmCmUfGfGfGfUmUfUmUmG mUmAmGmCmAInvabQ20 |
| AGT& PCSK9 | DT03102 | 1165AM5 | VPU- S*fC*mGmCmUmAmAmAmCmAmCdTmGfGmUfUmCm UmU*mG*mG |
| | | 11002AM17 | VPU- S*fU*mCmUmAmGmAmAmAmAmGdTmUfGmGfCmUm GmU*mG*mG |
| | | DT03102SM (11002SM21-DL07-1165SM16-Q20) | Invab*mG*mCmAmGmCmCfAfAfCfUmUfUmUmCmUmA mGmAmAInvabDL07- Invab*mC*mCmAmAmGmAmAmCfCfAfGfUmGfUmUmU mAmGmCmGmAInvabQ20 |
| AGT& PCSK9 | DT03103 | 1165AM5 | VPU- S*fC*mGmCmUmAmAmAmCmAmCdTmGfGmUfUmCm UmU*mG*mG |
| | | 11002AM10 | VPU- S*fU*mCmUmAmGmAmAmAmAmGdTmUfGmGfCmUm GmU*mA*mG |
| | | DT03103SM (11002SM23-DL07-1165SM16-Q20) | Invab*mC*mUmAmCmAmGmCmCfAfAfCfUmUfUmUmC mUmAmGmAmAInvabDL07- Invab*mC*mCmAmAmGmAmAmCfCfAfGfUmGfUmUmU mAmGmCmGmAInvabQ20 |
| AGT& PCSK9 | DT03104 | 1167AM27 | VPU- S*fU*mCmAmCmAmAmAmCmAmAdGmCfUmGfGmUm CmG*mG*mC |
| | | 11040AM10 | VPU- S*fG*mCmUmAmCmAmAmAmAmCdCmCfAmGfAmAm UmA*mA*mG |
| | | DT03104SM (1167SM21-DL07-11040SM23-Q20) | Invab*mG*mCmCmGmAmCmCmAfGfCfUfUmGfUmUmU mGmUmGmAmAInvabDL07- Invab*mC*mUmUmAmUmUmCmUfGfGfGfUmUfUmUmG mUmAmGmCmAInvabQ20 |
| AGT& PCSK9 | DT03105 | 1167AM25 | VPU- S*fU*mCmAmCmAmAmAmCmAmAdGmCfUmGfGmUm CmG*mG*mG |
| | | 11002AM17 | VPU- S*fU*mCmUmAmGmAmAmAmAmGdTmUfGmGfCmUm GmU*mG*mG |
| | | DT03105SM (1167SM19-DL07-11002SM21-Q20) | Invab*mU*mGmAmCmCmAfGfCfUfUmGfUmUmUmGmU mGmAmAInvabDL07- Invab*mG*mCmAmGmCmCfAfAfCfUmUfUmUmCmUmA mGmAmAInvabQ20 |
| AGT& PCSK9 | DT03106 | 1167AM27 | VPU- S*fU*mCmAmCmAmAmAmCmAmAdGmCfUmGfGmUm CmG*mG*mC |
| | | 11002AM10 | VPU- S*fU*mCmUmAmGmAmAmAmAmGdTmUfGmGfCmUm GmU*mA*mG |
| | | DT03106SM (1167SM21-DL07-11002SM23-Q20) | Invab*mG*mCmCmGmAmCmCmAfGfCfUfUmGfUmUmU mGmUmGmAmAInvabDL07- Invab*mC*mUmAmCmAmGmCmCfAfAfCfUmUfUmUmC mUmAmGmAmAInvabQ20 |
| AGT& PCSK9 | DT03107 | 1165AM5 | VPU- S*fC*mGmCmUmAmAmAmCmAmCdTmGfGmUfUmCm UmU*mG*mG |
| | | 11040AM10 | VPU- S*fG*mCmUmAmCmAmAmAmAmCdCmCfAmGfAmAm UmA*mA*mG |
| | | DT03107SM (1165SM16-DL07-11040SM23-Q20) | Invab*mC*mCmAmAmGmAmAmCfCfAfGfUmGfUmUmU mAmGmCmGmAInvabDL07- Invab*mC*mUmUmAmUmUmCmUfGfGfGfUmUfUmUmG mUmAmGmCmAInvabQ20 |
| AGT& PCSK9 | DT03108 | 1165AM5 | VPU- S*fC*mGmCmUmAmAmAmCmAmCdTmGfGmUfUmCm UmU*mG*mG |
| | | 11002AM17 | VPU- S*fU*mCmUmAmGmAmAmAmAmGdTmUfGmGfCmUm GmU*mG*mG |
| | | DT03108SM (1165SM14-DL07-11002SM21-Q20) | Invab*mG*mAmGmAmAmCfCfAfGfUmGfUmUmUmAmG mCmGmAInvabDL07- Invab*mG*mCmAmGmCmCfAfAfCfUmUfUmUmCmUmA mGmAmAInvabQ20 |
| AGT& PCSK9 | DT03109 | 1165AM5 | VPU- S*fC*mGmCmUmAmAmAmCmAmCdTmGfGmUfUmCm UmU*mG*mG |
| | | 11002AM17 | VPU- S*fU*mCmUmAmGmAmAmAmAmGdTmUfGmGfCmUm GmU*mG*mG |
| | | DT03109SM (1165SM16-DL07-11002SM21-Q20) | Invab*mC*mCmAmAmGmAmAmCfCfAfGfUmGfUmUmU mAmGmCmGmAInvabDL07- Invab*mG*mCmAmGmCmCfAfAfCfUmUfUmUmCmUmA mGmAmAInvabQ20 |
| AGT& PCSK9 | DT03110 | 1165AM5 | VPU- S*fC*mGmCmUmAmAmAmCmAmCdTmGfGmUfUmCm UmU*mG*mG |
| | | 11002AM10 | VPU- S*fU*mCmUmAmGmAmAmAmAmGdTmUfGmGfCmUm GmU*mA*mG |
| | | DT03110SM (1165SM16-DL07-11002SM23-Q20) | Invab*mC*mCmAmAmGmAmAmCfCfAfGfUmGfUmUmU mAmGmCmGmAInvabDL07- Invab*mC*mUmAmCmAmGmCmCfAfAfCfUmUfUmUmC mUmAmGmAmAInvabQ20 |
| AGT& PCSK9 | DT03111 | 1167AM25 | VPU- S*fU*mCmAmCmAmAmAmCmAmAdGmCfUmGfGmUm CmG*mG*mG |
| | | 11040AM10 | VPU- S*fG*mCmUmAmCmAmAmAmAmCdCmCfAmGfAmAm UmA*mA*mG |
| | | DT03111SM (1167SM19-DL09-11040SM23-Q20) | Invab*mU*mGmAmCmCmAfGfCfUfUmGfUmUmUmGmU mGmAmAInvabDL09- Invab*mC*mUmUmAmUmUmCmUfGfGfGfUmUfUmUmG mUmAmGmCmAInvabQ20 |
| AGT& PCSK9 | DT03112 | 1167AM27 | VPU- S*fU*mCmAmCmAmAmAmCmAmAdGmCfUmGfGmUm CmG*mG*mC |
| | | 11040AM10 | VPU- S*fG*mCmUmAmCmAmAmAmAmCdCmCfAmGfAmAm UmA*mA*mG |
| | | DT03112SM (1167SM21-DL09-11040SM23-Q20) | Invab*mG*mCmCmGmAmCmCmAfGfCfUfUmGfUmUmU mGmUmGmAmAInvabDL09- Invab*mC*mUmUmAmUmUmCmUfGfGfGfUmUfUmUmG mUmAmGmCmAInvabQ20 |
| AGT& PCSK9 | DT03113 | 1167AM25 | VPU- S*fU*mCmAmCmAmAmAmCmAmAdGmCfUmGfGmUm CmG*mG*mG |
| | | 11002AM17 | VPU- S*fU*mCmUmAmGmAmAmAmAmGdTmUfGmGfCmUm GmU*mG*mG |
| | | DT03113SM (1167SM19-DL09-11002SM21-Q20) | Invab*mU*mGmAmCmCmAfGfCfUfUmGfUmUmUmGmU mGmAmAInvabDL09- Invab*mG*mCmAmGmCmCfAfAfCfUmUfUmUmCmUmA mGmAmAInvabQ20 |
| AGT& PCSK9 | DT03114 | 1167AM27 | VPU- S*fU*mCmAmCmAmAmAmCmAmAdGmCfUmGfGmUm CmG*mG*mC |
| | | 11002AM10 | VPU- S*fU*mCmUmAmGmAmAmAmAmGdTmUfGmGfCmUm GmU*mA*mG |
| | | DT03114SM (1167SM21-DL09-11002SM23-Q20) | Invab*mG*mCmCmGmAmCmCmAfGfCfUfUmGfUmUmU mGmUmGmAmAInvabDL09- Invab*mC*mUmAmCmAmGmCmCfAfAfCfUmUfUmUmC mUmAmGmAmAInvabQ20 |
| AGT& PCSK9 | DT03115 | 1165AM5 | VPU- S*fC*mGmCmUmAmAmAmCmAmCdTmGfGmUfUmCm UmU*mG*mG |
| | | 11040AM10 | VPU- S*fG*mCmUmAmCmAmAmAmAmCdCmCfAmGfAmAm UmA*mA*mG |
| | | DT03115SM (1165SM16-DL09-11040SM23-Q20) | Invab*mC*mCmAmAmGmAmAmCfCfAfGfUmGfUmUmU mAmGmCmGmAInvabDL09- Invab*mC*mUmUmAmUmUmCmUfGfGfGfUmUfUmUmG mUmAmGmCmAInvabQ20 |
| AGT& PCSK9 | DT03116 | 1165AM5 | VPU- S*fC*mGmCmUmAmAmAmCmAmCdTmGfGmUfUmCm UmU*mG*mG |
| | | 11002AM17 | VPU- S*fU*mCmUmAmGmAmAmAmAmGdTmUfGmGfCmUm GmU*mG*mG |
| | | DT03116SM (1165SM14-DL09-11002SM21-Q20) | Invab*mG*mAmGmAmAmCfCfAfGfUmGfUmUmUmAmG mCmGmAInvabDL09- Invab*mG*mCmAmGmCmCfAfAfCfUmUfUmUmCmUmA mGmAmAInvabQ20 |
| AGT& PCSK9 | DT03117 | 1165AM5 | VPU- S*fC*mGmCmUmAmAmAmCmAmCdTmGfGmUfUmCm UmU*mG*mG |
| | | 11002AM17 | VPU- S*fU*mCmUmAmGmAmAmAmAmGdTmUfGmGfCmUm GmU*mG*mG |
| | | DT03117SM (1165SM16-DL09-11002SM21-Q20) | Invab*mC*mCmAmAmGmAmAmCfCfAfGfUmGfUmUmU mAmGmCmGmAInvabDL09- Invab*mG*mCmAmGmCmCfAfAfCfUmUfUmUmCmUmA mGmAmAInvabQ20 |
| AGT& PCSK9 | DT03118 | 1165AM5 | VPU- S*fC*mGmCmUmAmAmAmCmAmCdTmGfGmUfUmCm UmU*mG*mG |
| | | 11002AM10 | VPU- S*fU*mCmUmAmGmAmAmAmAmGdTmUfGmGfCmUm GmU*mA*mG |
| | | DT03118SM (1165SM16-DL09-11002SM23-Q20) | Invab*mC*mCmAmAmGmAmAmCfCfAfGfUmGfUmUmU mAmGmCmGmAInvabDL09- Invab*mC*mUmAmCmAmGmCmCfAfAfCfUmUfUmUmC mUmAmGmAmAInvabQ20 |
| ANGPTL3& PCSK9 | DT09001 | 7061AM4 | VPU- S*fU*mUmGmAmGmUmUmGmAmGdTmUfCmAfAmGm UmG*mG*mC |
| | | 11040AM10 | VPU- S*fG*mCmUmAmCmAmAmAmAmCdCmCfAmGfAmAm UmA*mA*mG |
| | | DT09001SM (7061SM16-DL07-11040SM23-Q20) | Invab*mG*mCmCmAmCmUmUmGfAfAfCfUmCfAmAmC mUmCmAmAmAInvabDL07- Invab*mC*mUmUmAmUmUmCmUfGfGfGfUmUfUmUmG mUmAmGmCmAInvabQ20 |
| ANGPTL3& PCSK9 | DT09002 | 7083AM10 | VPU- S*fA*mGmUmGmAmCmAmUmAmUdTmCfUmUfUmAm CmC*mU*mG |
| | | 11040AM10 | VPU- S*fG*mCmUmAmCmAmAmAmAmCdCmCfAmGfAmAm UmA*mA*mG |
| | | DT09002SM (7083SM3-DL07-11040SM23-Q20) | Invab*mC*mAmGmGmUmAmAmAfGfAfAfUmAfUmGm UmCmAmCmUmAInvabDL07- Invab*mC*mUmUmAmUmUmCmUfGfGfGfUmUfUmUmG mUmAmGmCmAInvabQ20 |
| ANGPTL3& PCSK9 | DT09003 | 7061AM18 | VPU- S*fU*mUmGmAmGmUmUmGmAmGdTmUfCmAfAmGm UmG*mG*mG |
| | | 11002AM17 | VPU- S*fU*mCmUmAmGmAmAmAmAmGdTmUfGmGfCmUm GmU*mG*mG |
| | | DT09003SM (7061SM14-DL07-11002SM21-Q20) | Invab*mU*mAmCmUmUmGfAfAfCfUmCfAmAmCmUmC mAmAmAInvabDL07- Invab*mG*mCmAmGmCmCfAfAfCfUmUfUmUmCmUmA mGmAmAInvabQ20 |
| ANGPTL3& PCSK9 | DT09004 | 7083AM9 | VPU- S*fA*mGmUmGmAmCmAmUmAmUdTmCfUmUfUmGm CmC*mG*mG |
| | | 11002AM17 | VPU- S*fU*mCmUmAmGmAmAmAmAmGdTmUfGmGfCmUm GmU*mG*mG |
| | | DT09004SM (7083SM2-DL07-11002SM21-Q20) | Invab*mG*mGmCmAmAmAfGfAfAfUmAfUmGmUmCmA mCmUmAInvabDL07- Invab*mG*mCmAmGmCmCfAfAfCfUmUfUmUmCmUmA mGmAmAInvabQ20 |
| ANGPTL3& PCSK9 | DT09005 | 7061AM4 | VPU- S*fU*mUmGmAmGmUmUmGmAmGdTmUfCmAfAmGm UmG*mG*mC |
| | | 11002AM17 | VPU- S*fU*mCmUmAmGmAmAmAmAmGdTmUfGmGfCmUm GmU*mG*mG |
| | | DT09005SM (7061SM16-DL07-11002SM21-Q20) | Invab*mG*mCmCmAmCmUmUmGfAfAfCfUmCfAmAmC mUmCmAmAmAInvabDL07- Invab*mG*mCmAmGmCmCfAfAfCfUmUfUmUmCmUmA mGmAmAInvabQ20 |

The structural schematic of the dual-targeting siRNA-linked ligand listed in the table above is as follows:

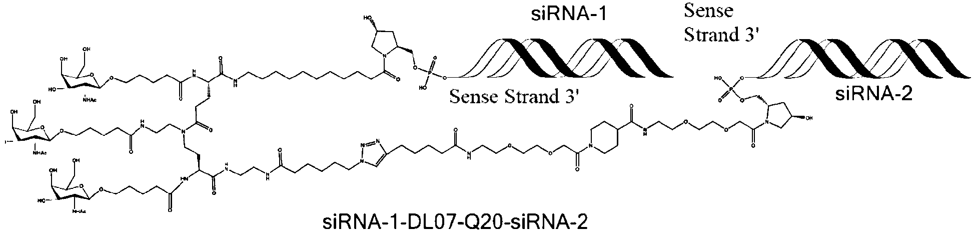

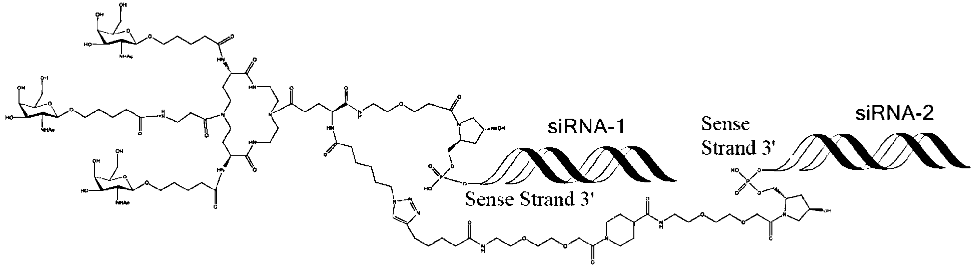

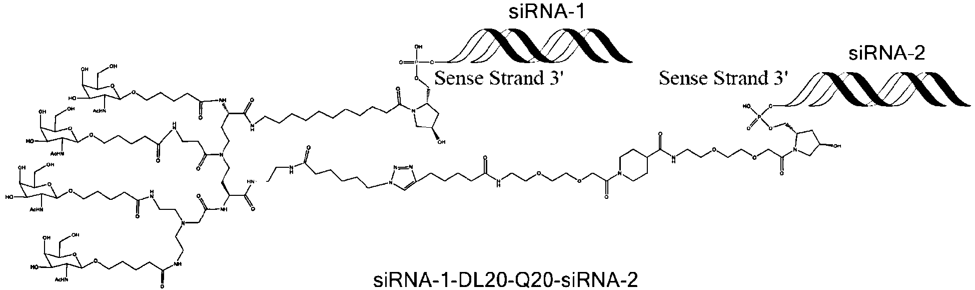

### Embodiment 25 Evaluation of the Effects of Dual-Targeting siRNAs with Different Delivery Structures on AGT and PCSK9 Expression in AAV8-hAGT/hPCSK9 Mouse Primary Hepatocytes

**Table 46: Experimental Results of Dual-targeting siRNA Delivered via Different Methods at Various Concentrations in AAV8-hAGT/hPCSK9 Mouse Primary Henatocytes**

| Dual-Targeting siRNA | Residual AGT mRNA Level (%) | | | | | |
|---|---|---|---|---|---|---|
| | 1nM/ Free Uptake | SD | 0.3nM/ Free Uptake | SD | 0.1nM/ Transfection | SD |
| 1000PM mix 11000PM | 14.7 | 1 | 37.7 | 3.7 | 7.9 | 2.1 |
| DT03081 | 3.5 | 0.7 | 13.3 | 0.6 | 0.5 | 0.1 |
| DT03082 | 5.2 | 0.6 | 17.3 | 2 | 0.6 | 0.1 |
| DT03092 | 5.5 | 0.9 | 21.6 | 0.9 | 0.9 | 0 |
| DT03093 | 7.3 | 0.1 | 23.9 | 3.5 | 0.9 | 0.1 |
| DT03095 | 8.1 | 0.6 | 24.5 | 1.2 | 0.6 | 0.1 |
| DT03097 | 1.9 | 0.2 | 9 | 0.4 | 0.4 | 0 |

| Dual-Targeting siRNA | Residual PCSK9 mRNA Level (%) | | | | | |
|---|---|---|---|---|---|---|
| | 1nM/ Free Uptake | SD | 0.3nM/ Free Uptake | SD | 0.1nM/ Transfection | SD |
| 1000PM mix 11000PM | 23.6 | 1.4 | 43.2 | 0.3 | 18.7 | 1.1 |
| DT03081 | 13.2 | 1.7 | 23.2 | 0.1 | 8.1 | 0.7 |
| DT03082 | 11.2 | 1 | 19.1 | 1 | 11.1 | 3.3 |
| DT03092 | 9.8 | 2.1 | 17.8 | 1 | 8.9 | 1.3 |
| DT03093 | 10.6 | 1.5 | 17.6 | 1.6 | 9.1 | 1.6 |
| DT03095 | 12.3 | 0.7 | 19.9 | 1.7 | 6.8 | 0.5 |
| DT03097 | 10.6 | 1.6 | 15.8 | 0.5 | 8.6 | 2 |

Experiments showed that all dual-targeting siRNA activities significantly exceeded the combined activities of the two independent positive controls. We also observed that different delivery systems exerted varying effects on the activity of both targets. Among these, siRNA delivered via DL07-Q20, DL09-Q20, and DL20-Q20 demonstrated the highest activity, significantly exceeding that of the positive control 1000PM mix 11000PM. We will next test the IC₅₀ values for knockdown of both targets in AAV8-hAGT/hPCSK9 mouse primary hepatocytes using the highly active dual-target structure DT03081.

**Table 47: IC₅₀ Experimental Results for DT03081 in AAV8-hAGT/hPCSK9 Mouse Primary Hepatocytes**

| Dual-Targeting siRNA | IC₅₀ Values (nM) in hAGT/hPCSK9 Mouse Primary Hepatocytes | |
|---|---|---|
| | hAGT | hPCSK9 |
| DT03081 | 0.04402 | 0.04461 |

As shown in Table 47, DT03081 exhibited extremely high activity, with IC₅₀ values of approximately 44 pM for both the AGT and PCSK9 targets. Demonstrating comparable inhibitory activity against both targets indicates that DT03081 can simultaneously and persistently inhibit the expression of both targets *in vivo.*

Additional combinations of preferred sequences targeting AGT and PCSK9 were evaluated for activity *in vitro* using the same delivery system, with the following results.

**Table 48: Experimental Results of Dual-targeting siRNA Delivered via Different Methods at Various Concentrations in AAV8-hAGT/hPCSK9 Mouse Primary Hepatocytes**

| Dual-Targeting siRNA | Residual AGT mRNA Level (%) | | | | | |
|---|---|---|---|---|---|---|
| | 1 nM/Free Uptake | SD | 0.3 nM/Free Uptake | SD | 0.1 nM/Transfection | SD |
| 1000PM mix 11000PM | 5.3 | 0.3 | 13.2 | 1.8 | 13 | 1.4 |
| DT03081 | 2 | 0.2 | 6.3 | 0.2 | 2.2 | 0.3 |
| DT03099 | 1.2 | 0.2 | 3.9 | 0.3 | 1.4 | 0.4 |
| DT03100 | 4.5 | 0.4 | 12.2 | 0.7 | 2.9 | 0.5 |
| DT03101 | 1.7 | 0.1 | 8 | 0.3 | 1.1 | 0.1 |
| DT03102 | 3.7 | 0.1 | 10.9 | 0.8 | 4.7 | 0.9 |
| DT03103 | 3.9 | 0.6 | 10.2 | 0.8 | 5.3 | 1.6 |

| Dual-Targeting siRNA | Residual PCSK9 mRNA Level (%) | | | | | |
|---|---|---|---|---|---|---|
| | 1 nM/Free Uptake | SD | 0.3 nM/Free Uptake | SD | 0.1 nM/Transfection | SD |
| 1000PM mix 11000PM | 21.8 | 4.9 | 29.7 | 4.8 | 28 | 2.3 |
| DT03081 | 17.8 | 7.3 | 19.5 | 2 | 14.8 | 4.7 |
| DT03099 | 13.5 | 0.7 | 16.4 | 5 | 20.4 | 5 |
| DT03100 | 14 | 0.5 | 17.3 | 1.2 | 15.9 | 2.6 |
| DT03 101 | 14 | 1 | 19.2 | 1.9 | 12.4 | 3.1 |
| DT03102 | 16.3 | 0.8 | 15.9 | 2.2 | 15 | 1 |
| DT03103 | 14.3 | 3.6 | 15.2 | 1.8 | 18.3 | 7.8 |

Data showed that the activity of dual-targeting siRNA from multiple preferred sequence combinations consistently outperformed the positive control 1000PM mix 11000PM

### Embodiment 26 Evaluation of the Effects of Different Dual-Target Sequences on hAGT and hPCSK9 Protein Expression Levels in Mice Simultaneously Expressing hAGT and hPCSK9

### Experimental Method:

### 1. Adenovirus Integration of hAGT/hPCSK9

Transgenic mice stably expressing hAGT/hPCSK9 were generated by co-infecting mice with 1.5 × 10¹¹ titer ultra-purified recombinant AAV8-hAGT and 1.5 × 10¹¹ titer ultra-purified recombinant AAV8-hPCSK9 viral particles.

### 2. Grouping and Administration

Blood was collected from the submandibular vein of mice 14 days after virus injection. Samples were allowed to stand at room temperature for 30 min, then centrifuged at 1000 × g for 10 min. The supernatant serum was collected, aliquoted, and frozen at -80°C. Serum samples were diluted 1000-fold, and hAGT and hPCSK9 expression in mouse serum was analyzed using the Human Angiotensinogen/AGT/SerpinA8 ELISA Kit (Lianke Bio, Cat. No: EK1202-96) and the Human PCSK9 ELISA Kit (Proteintech, Cat. No: KE00278), respectively. Mice were equally divided into groups based on hAGT and hPCSK9 expression levels, with 4 mice per group. siRNA was dissolved in normal saline and adjusted to a concentration of 10 µM. Mice were administered subcutaneous injections of the siRNA solution at doses of 60 nmol/kg or 180 nmol/kg.

### 3. ELISA Test

Following siRNA injection, a small amount of blood samples was collected from the submandibular vein every other week. Samples were allowed to stand at room temperature for 30 min and centrifuged at 1000 × g for 10 min. The supernatant serum was collected, diluted 1000-fold, and analyzed for hPCSK9 expression by ELISA.

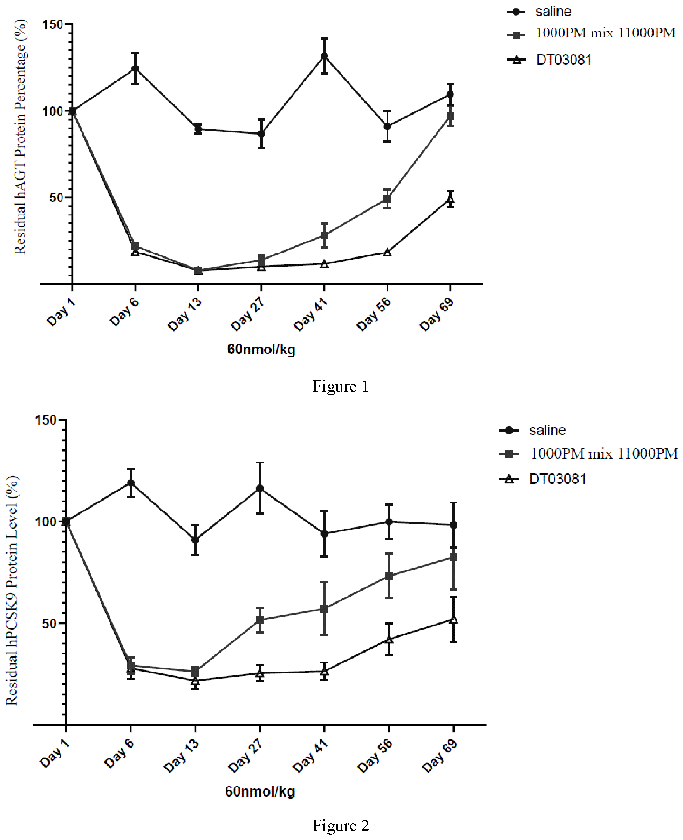

The experimental results are shown in the table and Figure 1

**Table 49: Inhibition Effects of AGT/PCSK9 Dual-targeting siRNA DT03081 on Both Targets in AAV8-hAGT/hPCSK9 Mice at a Dose of 60 nmol/kg**

| Dual-Targeting siRNA | Mean AGT Protein Expression Level (%) (relative to the predose level) | | | | | | | | | |
|---|---|---|---|---|---|---|---|---|---|---|
| | Day 13 | SD | Day 27 | SD | Day 41 | SD | Day 56 | SD | Day 69 | SD |
| 1000PM mix 11000PM | 8 | 1.7 | 13.9 | 4.8 | 28.2 | 11.8 | 49.3 | 9.3 | 97 | 10 |
| DT03081 | 7.9 | 1.1 | 10.1 | 1.8 | 11.7 | 2.3 | 18.5 | 3.5 | 49.3 | 8.2 |

| Dual-Targeting siRNA | Mean PCSK9 Protein Expression Level (%) (relative to the predose level) | | | | | | | | | |
|---|---|---|---|---|---|---|---|---|---|---|
| | Day 13 | SD | Day 27 | SD | Day 41 | SD | Day 56 | SD | Day 69 | SD |
| 1000PM mix 11000PM | 26.2 | 4 | 51.6 | 10.5 | 57.2 | 22.4 | 73.2 | 18.8 | 82.4 | 27.7 |
| DT03081 | 21.7 | 7.2 | 25.4 | 6.7 | 26.4 | 7.3 | 42.1 | 13.6 | 52 | 19.2 |

As shown in Table 49 and Figures 1 and 2, at a dose of 60 nmol/kg, DT03081 demonstrated excellent *in vivo* knockdown effects on both the AGT and PCSK9 targets, significantly outperforming the combined efficacy of Zilebesiran and Inclisiran. Furthermore, the comparison of the duration of inhibition between the two positive drug combinations showed that 1000PM maintained approximately 50% inhibition at Day 56, whereas 11000PM restored PCSK9 inhibition to around 50% by Day 27. This demonstrates inconsistent persistence of target inhibition between the two sequences. DT03081, obtained through sequence optimization and delivery structure refinement, basically exhibited consistent temporal trends in inhibition levels across both targets. This provides greater clinical control over administration time and frequency.

**Table 50: Inhibition Effects of AGT/PCSK9 Dual-targeting siRNA on Both Targets in AAV8-hAGT/hPCSK9 Mice at Different Doses**

| Dose | Dual-Targeting siRNA | Mean AGT Protein Expression Level (%) (relative to the predose level) | | | | | |
|---|---|---|---|---|---|---|---|
| | | Day 27 | SD | Day 40 | SD | Day 63 | SD |
| 60nmol/kg | DT03081 | 18.5 | 4.8 | 25.1 | 4.6 | 31 | 4.3 |
| | DT03082 | 16.9 | 1.5 | 29.9 | 3.9 | 31.4 | 2 |
| | DT03085 | 14.2 | 3.6 | 28.9 | 4.8 | 31.4 | 1.9 |
| | DT03086 | 13.4 | 2.8 | 19.2 | 4.2 | 23.6 | 3.2 |
| 180nmol/kg | DT03081 | 4.3 | 1 | 8.7 | 1.3 | 15.6 | 1.8 |
| | DT03082 | 5 | 0.4 | 9.1 | 2.9 | 16.5 | 2.5 |

| Dose | Dual-Targeting siRNA | Mean PCSK9 Protein Expression Level (%) (relative to the predose level) | | | | | |
|---|---|---|---|---|---|---|---|
| | | Day 27 | SD | Day 40 | SD | Day 63 | SD |
| 60nmol/kg | DT03081 | 21.8 | 5.1 | 33.7 | 6 | 50.3 | 13.6 |
| | DT03082 | 23.2 | 7.1 | 44.7 | 15.5 | 68.9 | 20.5 |
| | DT03085 | 18.9 | 4.9 | 36.9 | 7.9 | 65.5 | 8.5 |
| | DT03086 | 17.3 | 4.2 | 26.3 | 4.7 | 49.1 | 6.7 |
| 180nmol/kg | DT03081 | 9.5 | 0.6 | 12.7 | 2.5 | 32.7 | 4.1 |
| | DT03082 | 8.1 | 1.7 | 12.3 | 1.8 | 29 | 9.2 |

We further compared the activity of preferred sequences after forming dual targets. The data showed that DT03081, DT03082, DT03085, and DT03086 exhibited comparable activity.

The activity of other preferred sequence combinations after forming dual targets was also compared at a dose of 60 nmol/kg.

### Experimental Method:

### 1. Adenovirus Integration of hAGT/hPCSK9

Transgenic mice stably expressing hAGT/hPCSK9 were generated by infecting humanized PCSK9 mice with ultra-purified recombinant AAV8-hAGT viral particles at a titer of 1.5 × 10¹¹.

### 2. Grouping and Administration

Blood was collected from the submandibular vein of mice 14 days after virus injection. Samples were allowed to stand at room temperature for 30 min, then centrifuged at 1000 × g for 10 min. The supernatant serum was collected, aliquoted, and frozen at -80°C. Serum samples were diluted 1000-fold, and hAGT and hPCSK9 expression in mouse serum was analyzed using the Human Angiotensinogen/AGT/SerpinA8 ELISA Kit (Lianke Bio, Cat. No: EK1202-96) and the Human PCSK9 ELISA Kit (Proteintech, Cat. No: KE00278), respectively. Mice were equally divided into groups based on hAGT and hPCSK9 expression levels, with 4 mice per group. siRNA was dissolved in normal saline and adjusted to a concentration of 10 µM. Mice were administered subcutaneous injections of the siRNA solution at a dose of 60 nmol/kg.

### 3. ELISA Test

Following siRNA injection, a small amount of blood samples was collected from the submandibular vein every other week. Samples were allowed to stand at room temperature for 30 min and centrifuged at 1000 × g for 10 min. The supernatant serum was collected, diluted 1000-fold, and analyzed for hPCSK9 expression by ELISA.

The experimental results are shown in the table.

**Table 51: Inhibition Effects of AGT/PCSK9 Dual-targeting siRNA on Both Targets in Humanized PCSK9 Mice Infected with AAV8 hAGT Virus at a Dose of 60 nmol/kg**

| Dual-Targeting siRNA | Mean AGT Protein Expression Level (%) (relative to the predose level) | | | |
|---|---|---|---|---|
| | Day 7 | SD | Day 14 | SD |
| DT03081 | 1.4 | 0.4 | 0.7 | 0.2 |
| DT03101 | 1.5 | 0.5 | 0.7 | 0.4 |
| DT03099 | 1.3 | 0.2 | 0.8 | 0.2 |
| DT03100 | 1.8 | 0.5 | 1.3 | 0.4 |
| DT03102 | 1.2 | 0.2 | 0.5 | 0.1 |
| DT03103 | 1.7 | 0.2 | 0.8 | 0.1 |

| Dual-Targeting siRNA | Mean PCSK9 Protein Expression Level (%) (relative to the predose level) | | | |
|---|---|---|---|---|
| | Day 7 | SD | Day 14 | SD |
| DT03081 | 21.5 | 3.6 | 16 | 4.9 |
| DT03101 | 17.4 | 3.5 | 14.9 | 4.6 |
| DT03099 | 24.8 | 12.2 | 13 | 2.7 |
| DT03100 | 10.6 | 1.4 | 8.1 | 1.9 |
| DT03102 | 15.3 | 7.1 | 12.2 | 4.5 |
| DT03103 | 15.1 | 2.9 | 7.8 | 0.9 |

The data showed that multiple dual-targeting siRNAs exhibited activity comparable to or slightly superior to DT03081.

### Embodiment 27 Evaluation of the Effects of Different Dual-Target Sequences on the Expression of hAGT and hANGPTL3 in the Liver of Mice Simultaneously Expressing hAGT and hANGPTL3

### Experimental Method:

### 1. Adenovirus integration of hAGT and hANGPTL3

Transgenic mice stably expressing both hAGT and hANGPTL3 were generated by infecting mice with ultra-purified recombinant AAV8 hAGT virus particles at a titer of 1×10¹¹ and ultra-purified recombinant AAV8 hANGPTL3 virus particles at a titer of 1×10¹¹. 100 µL of virus was added to 5.9 mL of PBS, and 200 µL of the diluted virus solution was administered intravenously to each mouse.

### 2. Administration

The mice were equally divided into groups, with 4 mice per group, 14 days after virus injection. siRNA was dissolved in normal saline and administered subcutaneously at a dose of 60 nmol/kg.

### 3. Liver Collection and Testing

Livers were collected at different time points after siRNA injection. RNA was extracted from liver tissue using TRI REAGENT (MRC, Cat. No.: TR118). The extracted RNA was reverse transcribed into cDNA using a PrimeScript RT Reagent Kit (Takara, Cat. No.: RR047A). The prepared QPCR system was added to a 96-well PCR plate, the plate was sealed with sealing film, and QPCR was conducted on a StepOnePlus real-time PCR System (Applied Biosystems) to detect hAGT and hANGPTL3 expression.

**Table 52: Inhibition Effects of AGT/ANGPTL3 Dual-targeting siRNA on Both Targets in AAV8-hAGT/hANGPTL3 Mice at a Dose of 60 nmol/kg**

| Dual-Targeting siRNA | Mean Residual AGT Gene Expression Level (%) | | Mean Residual ANGPTL3 Gene Expression Level (%) | |
|---|---|---|---|---|
| | (Relative to the normal saline group) | | (Relative to the normal saline group) | |
| | Day 28 | SD | Day 28 | SD |
| 1000PM mix ARO-ANG3 | 30.4 | 7.9 | 36.5 | 10 |
| DT02042 | 32.6 | 6.7 | 39.5 | 7.8 |
| DT02043 | 20.7 | 6 | 21 | 8.1 |
| DT02047 | 21.9 | 8.3 | 34.4 | 6.6 |
| DT02058 | 44.8 | 10.8 | 28 | 6.7 |
| DT02059 | 27.6 | 5.4 | 20.9 | 4.5 |

The AGT-targeting preferred sequences 1167.25-19, 1167.27-21, 1165.5-16, and 1161.7-17 were combined with the ANGPTL3-targeting preferred sequences 7061.18-14 and 7061.4-16 to generate AGT/ANGPTL3 dual-targeting siRNAs DT02042, DT02043, DT02047, DT02058, and DT02059. *In vivo* activity evaluation was conducted alongside positive control sequences. Table 52 demonstrates significant variations in activity among different sequence combinations. The most potent sequence, DT02043, exhibited substantially higher activity at both targets compared to the positive control 1000PM mix ARO-ANG3. Conversely, the least effective dual-targeting siRNA, DT02042, showed activity inferior to that of the positive control. Further comparison of DT02043 and DT02047 reveals that although both dual-targeting siRNAs employ the 7061.4-16 sequence targeting ANGPTL3, the differing sequences targeting AGT result in significant variations in ANGPTL3-targeting activity.

### Embodiment 28 Evaluation of the Effects of Different Dual-Target Sequences on hANGPTL3 and hPCSK9 Expression Levels in Mice Simultaneously Expressing hANGPTL3 and hPCSK9

### Experimental Method:

### 1. Adenovirus integration of hANGPTL3/hPCSK9

Transgenic mice stably expressing hANGPTL3/hPCSK9 were generated by co-infecting mice with 1.5 × 10¹¹ titer ultra-purified recombinant AAV8-hANGPTL3 and 1.5 × 10¹¹ titer ultra-purified recombinant AAV8-hPCSK9 viral particles.

### 2. Grouping and Administration

Blood was collected from the submandibular vein of mice 14 days after virus injection. Samples were allowed to stand at room temperature for 30 min, then centrifuged at 1000 × g for 10 min. The supernatant serum was collected, aliquoted, and frozen at -80°C. Serum samples were diluted 1000-fold, and hPCSK9 expression in mouse serum was analyzed using the Human PCSK9 ELISA Kit (Proteintech, Cat. No: KE00278). Mice were equally divided into groups based on hPCSK9 expression levels, with 4 mice per group. siRNA was dissolved in normal saline and adjusted to a concentration of 10 µM. Mice were administered subcutaneous injections of the siRNA solution at a dose of 60 nmol/kg.

### 3. ELISA Test

Following siRNA injection, a small amount of blood samples was collected from the submandibular vein every other week. Samples were allowed to stand at room temperature for 30 min and centrifuged at 1000 × g for 10 min. The supernatant serum was collected, diluted 1000-fold, and analyzed for hPCSK9 expression by ELISA.

### 4. Liver Collection and Testing

Livers were collected at different time points after siRNA injection. RNA was extracted from liver tissue using TRI REAGENT (MRC, Cat. No.: TR118). The extracted RNA was reverse transcribed into cDNA using a PrimeScript RT Reagent Kit (Takara, Cat. No.: RR047A). The prepared QPCR system was added to a 96-well PCR plate, the plate was sealed with sealing film, and QPCR was conducted on a StepOnePlus real-time PCR System (Applied Biosystems) to detect hPCSK9 and hANGPTL3 expression.

**Table 53: Inhibition Effects of PCSK9/ANGPTL3 Dual-targeting siRNA on Both Targets in AAV8-hPCSK9/hANGPTL3 Mice at a Dose of 60 nmol/kg**

| Dual-Targeting siRNA | Mean Residual ANGPTL3 Expression Level (%) (relative to the normal saline group) | | Mean Residual hPCSK9 Protein Expression Level (%) (relative to the predose level) | |
|---|---|---|---|---|
| | Day 14/Mean | SD | Day 22/Mean | SD |
| 11000PM mix ARO-ANG3 | 25.5 | 4.2 | 50.1 | 6.1 |
| DT09001 | 24 | 6.9 | 34.9 | 4.4 |
| DT09003 | 16.3 | 3.5 | 50.6 | 3.6 |
| DT09005 | 21.8 | 2.4 | 29.3 | 4.2 |

Preferred ANGPTL3-targeting sequences 7061.4-16 and 7061.18-14 were combined with PCSK9-targeting sequences 11040.10-23 and 11002.17-21 to obtain ANGPTL3/PCSK9 dual-targeting siRNAs DT09001, DT09003, and DT09005, and their activities were evaluated in AAV8-hANGPTL3/hPCSK9 mice. Blood and liver samples were collected at different time points, and PCSK9 and ANGPTL3 expression levels were detected by ELISA and qPCR, respectively. The data showed that DT09001 and DT09005 exhibited the highest knockdown activity against both targets. In this experiment, we also discovered that even though dual-targeting siRNAs DT09003 and DT09005 both utilized the 11002.17-21 sequence to target PCSK9, the differing sequences targeting ANGPTL3 resulted in significant variations in their PCSK9-targeting activity.

The above embodiments only express several embodiments of the present invention. Although the description is relatively specific and detailed, it cannot be understood as limiting the patent scope of the present invention. It is to be pointed out that ordinarily skilled persons in the art may make several modifications and improvements without departing from the concept of the present invention, which all fall within the protection scope of the present invention. Therefore, the protection scope of the present invention shall be subject to the attached claims.

## Claims

1. A dual-targeting siRNA agent, **characterized in that** the dual-targeting siRNA agent comprises two different siRNAs targeting two different genes or pharmaceutically acceptable salts thereof, wherein the two different siRNAs or salts thereof are linked by a ligand for delivering nucleic acid and thereof integrated into a single entity, each of the siRNAs is a dsRNA composed of a sense strand and an antisense strand, and the two different genes are selected from any two of angiotensinogen (AGT), proprotein convertase subtilisin/kexin type 9 (PCSK9) and angiopoietin-like 3 (ANGPTL3);
wherein the base composition of the siRNA targeting the AGT gene is selected from any one of the following dsRNA sequences, or a sequence having a sense strand or antisense strand that differs by no more than 1 nucleotide therefrom:
A) 1167f: the sense strand thereof is shown in SEQ ID NO: 59, and the antisense strand thereof is shown in SEQ ID NO: 60;
B) 1167b: the sense strand thereof is shown in SEQ ID NO: 51, and the antisense strand thereof is shown in SEQ ID NO: 52;
C) 1165d: the sense strand thereof is shown in SEQ ID NO: 39, and the antisense strand thereof is shown in SEQ ID NO: 40;
D) 1165f: the sense strand thereof is shown in SEQ ID NO: 43, and the antisense strand thereof is shown in SEQ ID NO: 44;
wherein the base composition of the siRNA targeting the PCSK9 gene is selected from any one of the following dsRNA sequences, or a sequence having a sense strand or antisense strand that differs by no more than 1 nucleotide therefrom:
E) 11040a: the sense strand thereof is shown in SEQ ID NO: 375, and the antisense strand thereof is shown in SEQ ID NO: 376;
F) 11002d: the sense strand thereof is shown in SEQ ID NO: 259, and the antisense strand thereof is shown in SEQ ID NO: 260;
G) 11002a: the sense strand thereof is shown in SEQ ID NO: 253, and the antisense strand thereof is shown in SEQ ID NO: 254;
wherein the base composition of the siRNA targeting ANGPTL3 is selected from any one of the following dsRNA sequences, or a sequence having a sense strand or antisense strand that differs by no more than 1 nucleotide therefrom:
H) 7061f: the sense strand thereof is shown in SEQ ID NO: 579, and the antisense strand thereof is shown in SEQ ID NO: 580;
I) 7061b: the sense strand thereof is shown in SEQ ID NO: 571, and the antisense strand thereof is shown in SEQ ID NO: 572.

2. The dual-targeting siRNA agent according to claim 1, **characterized in that** the two target genes are AGT and any one selected from PCSK9 and ANGPTL3, preferably AGT and PCSK9.

3. The dual-targeting siRNA agent according to claim 1, **characterized in that** the nucleotides of the sense strand and the antisense strand are modified, the sense strand comprises no more than 3, 2, 1 or 0 unmodified nucleotides, the modified nucleotides in the sense strand are selected from 2'-O-methyl modified nucleotides, 2'-deoxynucleotides, 2'-fluoro modified nucleotides and inverted abasic residues, and the 5'-terminus and 3'-terminus of the sense strand each independently contains 0, 1, 2 or 3 phosphorothioate bonds; the antisense strand comprises no more than 3, 2, 1 or 0 unmodified nucleotides, the modified nucleotides in the antisense strand are selected from 2'-O-methyl modified nucleotides, 2'-deoxynucleotides, 2'-fluoro modified nucleotides, VPU (2'-O-methyluridine-5'-(E)-vinylphosphonate-3'-phosphate), VPU-S (2'-S-methyluridine-5'-(E)-vinylphosphonate-3'-phosphate) or other VPU derivatives, and the 5'- terminus and 3'-terminus of the antisense strand each independently contains 1-3 phosphorothioate bonds.

4. The dual-targeting siRNA agent according to claim 3, **characterized in that** the siRNA targeting the AGT gene is modified and selected from any one of the following sequences, or a sequence having a sense strand or antisense strand that differs by no more than 1 nucleotide therefrom:
a) 1167.25-19: the 5'-3' sense strand thereof is Invab*mU*mGmAmCmCmAfGfCfUfUmGfUmUmUmGmUmGmAmAInvab, and the 5'-3' antisense strand thereof is VPU-S*fU*mCmAmCmAmAmAmCmAmAdGmCfUmGfGmUmCmG*mG*mG;
b) 1167.27-21: the 5'-3' sense strand thereof is Invab*mG*mCmCmGmAmCmCmAfGfCfUfUmGfUmUmUmGmUmGmAmAInvab, and the 5'-3' antisense strand thereof is VPU-S*fU*mCmAmCmAmAmAmCmAmAdGmCfUmGfGmUmCmG*mG*mC;
c) 1165.5-14: the 5'-3' sense strand thereof is Invab*mG*mAmGmAmAmCfCfAfGfUmGfUmUmUmAmGmCmGmAInvab, and its 5'-3' antisense strand is VPU-S*fC*mGmCmUmAmAmAmCmAmCdTmGfGmUfUmCmUmU*mG*mG;
d) 1165.5-16: the 5'-3' sense strand thereof is Invab*mC*mCmAmAmGmAmAmCfCfAfGfUmGfUmUmUmAmGmCmGmAInvab, and the 5'-3' antisense strand thereof is VPU-S*fC*mGmCmUmAmAmAmCmAmCdTmGfGmUfUmCmUmU*mG*mG;
wherein VPU-S is 2'-S-methyluridine-5'-(E)-vinylphosphonate-3'-phosphate, mA is 2'-O-methyladenosine-3'-phosphate, mU is 2'-O-methyluridine-3'-phosphate, mC is 2'-O-methylcytidine-3'-phosphate, mG is 2'-O-methylguanosine-3'-phosphate, fA is 2'-fluoroadenosine-3'-phosphate, fU is 2'-fluorouridine-3'-phosphate, fC is 2'-fluorocytidine-3'-phosphate, fG is 2'-fluoroguanosine-3'-phosphate, dA is 2'-deoxyadenosine-3'-phosphate, dT is 2'-deoxythymidine-3'-phosphate, dC is 2'-deoxycytidine-3'-phosphate, dG is 2'-deoxyguanosine-3'-phosphate, Invab is an inverted abasic residue, and * is a phosphorothioate bond.

5. The dual-targeting siRNA agent according to claim 3 or 4, **characterized in that** the siRNA targeting the PCSK9 gene is modified and selected from any one of the following sequences, or a sequence having a sense strand or antisense strand that differs by no more than 1 nucleotide therefrom:
e) 11040.10-23: the 5'-3' sense strand thereof is Invab*mC*mUmUmAmUmUmCmUfGfGfGfUmUfUmUmGmUmAmGmCmAInvab, and the 5'-3' antisense strand thereof is VPU-S*fG*mCmUmAmCmAmAmAmAmCdCmCfAmGfAmAmUmA*mA*mG;
f) 11002.17-21: the 5'-3' sense strand thereof is Invab*mG*mCmAmGmCmCfAfAfCfUmUfUmUmCmUmAmGmAmAInvab, and the 5'-3' antisense strand thereof is VPU-S*fU*mCmUmAmGmAmAmAmAmGdTmUfGmGfCmUmGmU*mG*mG;
g) 11002.10-23: the 5'-3' sense strand thereof is Invab*mC*mUmAmCmAmGmCmCfAfAfCfUmUfUmUmCmUmAmGmAmAInvab, and the 5'-3' antisense strand thereof is VPU-S*fU*mCmUmAmGmAmAmAmAmGdTmUfGmGfCmUmGmU*mA*mG;
wherein VPU-S is 2'-S-methyluridine-5'-(E)-vinylphosphonate-3'-phosphate, mA is 2'-O-methyladenosine-3'-phosphate, mU is 2'-O-methyluridine-3'-phosphate, mC is 2'-O-methylcytidine-3'-phosphate, mG is 2'-O-methylguanosine-3'-phosphate, fA is 2'-fluoroadenosine-3'-phosphate, fU is 2'-fluorouridine-3'-phosphate, fC is 2'-fluorocytidine-3'-phosphate, fG is 2'-fluoroguanosine-3'-phosphate, dA is 2'-deoxyadenosine-3'-phosphate, dT is 2'-deoxythymidine-3'-phosphate, dC is 2'-deoxycytidine-3'-phosphate, dG is 2'-deoxyguanosine-3'-phosphate, Invab is an inverted abasic residue, and * is a phosphorothioate bond.

6. The dual-targeting siRNA agent according to claim 3 or 4, **characterized in that** the siRNA targeting the ANGPTL3 gene is modified and selected from any one of the following sequences, or a sequence having a sense strand or antisense strand that differs by no more than 1 nucleotide therefrom:
h) 7061.18-14: the 5'-3' sense strand thereof is Invab*mU*mAmCmUmUmGfAfAfCfUmCfAmAmCmUmCmAmAmAInvab, and the 5'-3' antisense strand thereof is VPU-S*fU*mUmGmAmGmUmUmGmAmGdTmUfCmAfAmGmUmG*mG*mG;
i) 7061.4-16: the 5'-3' sense strand thereof is Invab*mG*mCmCmAmCmUmUmGfAfAfCfUmCfAmAmCmUmCmAmAmAInvab, and the 5'-3' antisense strand thereof is VPU-S*fU*mUmGmAmGmUmUmGmAmGdTmUfCmAfAmGmUmG*mG*mC;
wherein VPU-S is 2'-S-methyluridine-5'-(E)-vinylphosphonate-3'-phosphate, mA is 2'-O-methyladenosine-3'-phosphate, mU is 2'-O-methyluridine-3'-phosphate, mC is 2'-O-methylcytidine-3'-phosphate, mG is 2'-O-methylguanosine-3'-phosphate, fA is 2'-fluoroadenosine-3'-phosphate, fU is 2'-fluorouridine-3'-phosphate, fC is 2'-fluorocytidine-3'-phosphate, fG is 2'-fluoroguanosine-3'-phosphate, dA is 2'-deoxyadenosine-3'-phosphate, dT is 2'-deoxythymidine-3'-phosphate, dC is 2'-deoxycytidine-3'-phosphate, dG is 2'-deoxyguanosine-3'-phosphate, Invab is an inverted abasic residue, and * is a phosphorothioate bond.

7. The dual-targeting siRNA agent according to claim 5, **characterized in that** when targeting AGT and PCSK9 genes, each siRNA of the dual-targeting siRNA agent is selected from any one of the dual-targeting siRNAs DT03081, DT03086, DT03103, DT03099, DT03085, DT03101, DT03082, DT03097, DT03100 or DT03102 shown in Table 45.

8. The dual-targeting siRNA agent according to claim 6, **characterized in that** when targeting AGT and ANGPTL3 genes, each siRNA of the dual-targeting siRNA agent is selected from any one of the dual-targeting siRNAs DT02043 or DT02047 shown in Table 45.

9. The dual-targeting siRNA agent according to claim 3, **characterized in that** when targeting ANGPTL3 and PCSK9 genes, each siRNA of the dual-targeting siRNA agent is selected from any one of the dual-targeting siRNAs DT09001, DT09003 or DT09005 shown in Table 45.

10. The dual-targeting siRNA agent according to any one of claims 1-4, **characterized in that** the pharmaceutically acceptable salt is a sodium salt or a potassium salt.

11. The dual-targeting siRNA agent according to any one of claims 1-4, **characterized in that** the ligand is N-acetylgalactosamine (GalNAc) or a derivative thereof.

12. The dual-targeting siRNA agent according to claim 11, **characterized in that** the GalNAc derivative is connected to the 3'-termini of the sense strands of two siRNAs via chemical bonds, preferably via phosphate bonds or phosphorothioate bonds, respectively.

13. The dual-targeting siRNA agent according to claim 12, **characterized in that** the GalNAc derivative has the following structure and is connected to the 3' terminus of the sense strand of each siRNA in the following mode: 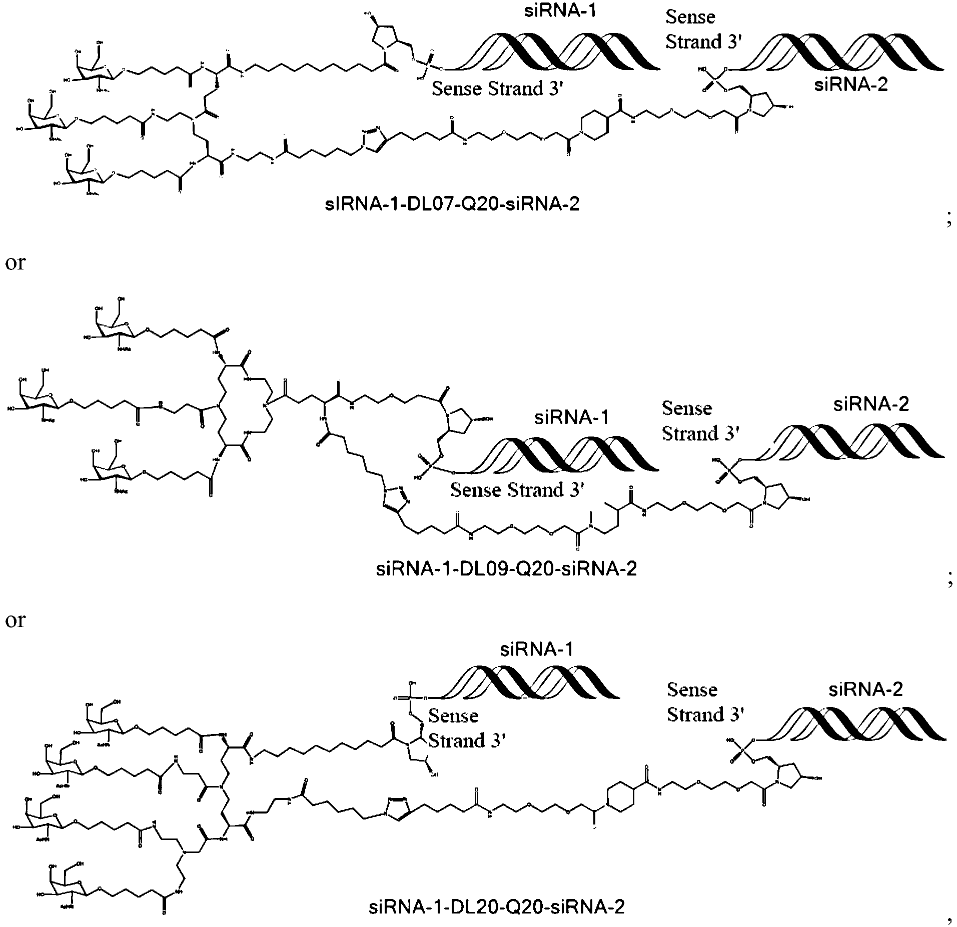 wherein 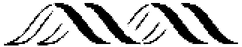 represents a modified siRNA sequence targeting the AGT gene, PCSK9 gene or ANGPTL3 gene.

14. Use of the dual-targeting siRNA agent according to any one of claims 1-13 in the preparation of a product for simultaneously inhibiting the expression of any two target genes selected from AGT, PCSK9 and ANGPTL3 in cells, preferably, wherein the product is a biological preparation or a pharmaceutical preparation.

15. A biological preparation or pharmaceutical preparation for simultaneously inhibiting the expression of any two target genes selected from AGT, PCSK9 and ANGPTL3, **characterized in that** the active ingredient thereof comprises the dual-targeting siRNA agent according to any one of claims 1-13.

16. Use of the dual-targeting siRNA agent according to any one of claims 1-13 in the preparation of a drug for preventing and/or treating hypertension and/or lipid disorder-related disease, **characterized in that** the hypertension is mediated by the AGT gene, and the lipid disorder-related disease is mediated by the PCSK9 gene or the ANGPTL3 gene.

17. The use according to claim 16, **characterized in that** the hypertension is any one of borderline hypertension, primary hypertension, secondary hypertension, hypertensive crisis, hypertensive emergency, isolated systolic and diastolic hypertension, pregnancy-related hypertension, diabetic hypertension, intractable hypertension, refractory hypertension, paroxysmal hypertension, renovascular hypertension, Goldblatt hypertension, pulmonary hypertension, portal hypertension, systemic venous hypertension, systolic hypertension and unstable hypertension, or any other disease mediated by the AGT gene.

18. The use according to claim 16, **characterized in that** the lipid disorder-related disease is mediated by the PCSK9 gene and selected from any one of hyperlipidemia, atherosclerosis, non-alcoholic steatohepatitis, and non-alcoholic fatty liver disease.

19. The use according to claim 16, **characterized in that** the lipid disorder-related disease is mediated by the ANGPTL3 gene and selected from any one of hyperlipidemia, hypertriglyceridemia, lipid and/or cholesterol metabolism disorders, homozygous and heterozygous familial hypercholesterolemia, statin-resistant hypercholesterolemia, cardiometabolic disease, obesity, atherosclerosis, type 2 diabetes, cardiovascular disease, coronary artery disease, non-alcoholic steatohepatitis, non-alcoholic fatty liver disease, and hypertriglyceridemia-induced pancreatitis.

20. A method for simultaneously inhibiting the expression of any two target genes selected from AGT, PCSK9 and ANGPTL3 in cells *in vivo* or *in vitro,* **characterized in that** the method comprises:
(a) exposing cells *in vivo* or *in vitro* to the dual-targeting siRNA agent according to (a) exposing cells in vivo or in vitro to the dual-targeting siRNA agent according to any one of claims 1 - 13 or the biological preparation or pharmaceutical preparation according to claim 15; and
(b) maintaining the cells generated in step (a) for a time sufficient to achieve degradation of mRNA transcripts expressed by any two target genes selected from AGT, PCSK9 and ANGPTL3, thereby simultaneously inhibiting the expression of any two target genes selected from AGT, PCSK9, and ANGPTL3 in cells.

21. A method for treating a disease associated with AGT and/or PCSK9 and/or ANGPTL3, **characterized in that** it comprises administering to the subject a therapeutically effective amount of the dual-targeting siRNA agent according to any one of claims 1-13, or the biological preparation or pharmaceutical preparation according to claim 15, thereby treating the subject.

22. An siRNA targeting the AGT gene or a pharmaceutically acceptable salt thereof, **characterized in that** the sequence thereof is selected from any one of the following sequences, or a sequence having a sense strand or antisense strand that differs by no more than 1 nucleotide therefrom:
A) 1167f: the sense strand thereof is shown in SEQ ID NO: 59, and the antisense strand thereof is shown in SEQ ID NO: 60;
B) 1167b: the sense strand thereof is shown in SEQ ID NO: 51, and the antisense strand thereof is shown in SEQ ID NO: 52;
C) 1165d: the sense strand thereof is shown in SEQ ID NO: 39, and the antisense strand thereof is shown in SEQ ID NO: 40;
D) 1165f: the sense strand thereof is shown in SEQ ID NO: 43, and the antisense strand thereof is shown in SEQ ID NO: 44.

23. The siRNA targeting the AGT gene or a pharmaceutically acceptable salt thereof according to claim 22, wherein the siRNA is modified and selected from any one of the following sequences, or a sense strand or antisense strand sequence that differs by no more than 1 nucleotide therefrom:
a) 1167.25-19: the 5'-3' sense strand thereof is Invab*mU*mGmAmCmCmAfGfCfUfUmGfUmUmUmGmUmGmAmAInvab, and the 5'-3' antisense strand thereof is VPU-S*fU*mCmAmCmAmAmAmCmAmAdGmCfUmGfGmUmCmG*mG*mG;
b) 1167.27-21: the 5'-3' sense strand thereof is Invab*mG*mCmCmGmAmCmCmAfGfCfUfUmGfUmUmUmGmUmGmAmAInvab, and the 5'-3' antisense strand thereof is VPU-S*fU*mCmAmCmAmAmAmCmAmAdGmCfUmGfGmUmCmG*mG*mC;
c) 1165.5-14: the 5'-3' sense strand is Invab*mG*mAmGmAmAmCfCfAfGfUmGfUmUmUmAmGmCmGmAInvab, and the 5'-3' antisense strand thereof is VPU-S*fC*mGmCmUmAmAmAmCmAmCdTmGfGmUfUmCmUmU*mG*mG;
d) 1165.5-16: the 5'-3' sense strand is Invab*mC*mCmAmAmGmAmAmCfCfAfGfUmGfUmUmUmAmGmCmGmAInvab, and the 5'-3' antisense strand thereof is VPU-S*fC*mGmCmUmAmAmAmCmAmCdTmGfGmUfUmCmUmU*mG*mG;
wherein VPU-S is 2'-S-methyluridine-5'-(E)-vinylphosphonate-3'-phosphate, mA is 2'-O-methyladenosine-3'-phosphate, mU is 2'-O-methyluridine-3'-phosphate, mC is 2'-O-methylcytidine-3'-phosphate, mG is 2'-O-methylguanosine-3'-phosphate, fA is 2'-fluoroadenosine-3'-phosphate, fU is 2'-fluorouridine-3'-phosphate, fC is 2'-fluorocytidine-3'-phosphate, fG is 2'-fluoroguanosine-3'-phosphate, dA is 2'-deoxyadenosine-3'-phosphate, dT is 2'-deoxythymidine-3'-phosphate, dC is 2'-deoxycytidine-3'-phosphate, dG is 2'-deoxyguanosine-3'-phosphate, Invab is an inverted abasic residue, and * is a phosphorothioate bond.

24. An siRNA targeting the PCSK9 gene or a pharmaceutically acceptable salt thereof, **characterized in that** the siRNA is selected from any one of the following sequences, or a sense strand or antisense strand sequence that differs by no more than 1 nucleotide therefrom:
E) 11040a: the sense strand thereof is shown in SEQ ID NO: 375, and the antisense strand thereof is shown in SEQ ID NO: 376;
F) 11002d: the sense strand thereof is shown in SEQ ID NO: 259, and the antisense strand thereof is shown in SEQ ID NO: 260;
G) 11002a: the sense strand thereof is shown in SEQ ID NO: 253, and the antisense strand thereof is shown in SEQ ID NO: 254.

25. An siRNA targeting the PCSK9 gene or a pharmaceutically acceptable salt thereof according to claim 24, **characterized in that** the siRNA is modified and selected from any one of the following sequences, or a sequence having a sense strand or antisense strand that differs by no more than 1 nucleotide therefrom:
e) 11040.10-23: the 5'-3' sense strand thereof is Invab*mC*mUmUmAmUmUmCmUfGfGfGfUmUfUmUmGmUmAmGmCmAInvab, and the 5'-3' antisense strand thereof is VPU-S*fG*mCmUmAmCmAmAmAmAmCdCmCfAmGfAmAmUmA*mA*mG;
f) 11002.17-21: the 5'-3' sense strand thereof is Invab*mG*mCmAmGmCmCfAfAfCfUmUfUmUmCmUmAmGmAmAInvab, and the 5'-3' antisense strand thereof is VPU-S*fU*mCmUmAmGmAmAmAmAmGdTmUfGmGfCmUmGmU*mG*mG;
g) 11002.10-23: the 5'-3' sense strand thereof is Invab*mC*mUmAmCmAmGmCmCfAfAfCfUmUfUmUmCmUmAmGmAmAInvab, and the 5'-3' antisense strand thereof is VPU-S*fU*mCmUmAmGmAmAmAmAmGdTmUfGmGfCmUmGmU*mA*mG;
wherein VPU-S is 2'-S-methyluridine-5'-(E)-vinylphosphonate-3'-phosphate, mA is 2'-O-methyladenosine-3'-phosphate, mU is 2'-O-methyluridine-3'-phosphate, mC is 2'-O-methylcytidine-3'-phosphate, mG is 2'-O-methylguanosine-3'-phosphate, fA is 2'-fluoroadenosine-3'-phosphate, fU is 2'-fluorouridine-3'-phosphate, fC is 2'-fluorocytidine-3'-phosphate, fG is 2'-fluoroguanosine-3'-phosphate, dA is 2'-deoxyadenosine-3'-phosphate, dT is 2'-deoxythymidine-3'-phosphate, dC is 2'-deoxycytidine-3'-phosphate, dG is 2'-deoxyguanosine-3'-phosphate, Invab is an inverted abasic residue, and * is a phosphorothioate bond.

26. An siRNA targeting the ANGPTL3 gene or a pharmaceutically acceptable salt thereof, **characterized in that** the siRNA is modified and selected from any one of the following sequences, or a sequence having a sense strand or antisense strand that differs by no more than 1 nucleotide therefrom:
H) 7061f: the sense strand thereof is shown in SEQ ID NO: 579, and the antisense strand thereof is shown in SEQ ID NO: 580;
I) 7061b: the sense strand thereof is shown in SEQ ID NO: 571, and the antisense strand thereof is shown in SEQ ID NO: 572.

27. An siRNA targeting the ANGPTL3 gene or a pharmaceutically acceptable salt thereof according to claim 26, **characterized in that** the siRNA is modified and selected from any one of the following sequences, or a sequence having a sense strand or antisense strand that differs by no more than 1 nucleotide therefrom:
h) 7061.18-14: the 5'-3' sense strand thereof is Invab*mU*mAmCmUmUmGfAfAfCfUmCfAmAmCmUmCmAmAmAInvab, and the 5'-3' antisense strand thereof is VPU-S*fU*mUmGmAmGmUmUmGmAmGdTmUfCmAfAmGmUmG*mG*mG;
i) 7061.4-16: the 5'-3' sense strand thereof is Invab*mG*mCmCmAmCmUmUmGfAfAfCfUmCfAmAmCmUmCmAmAmAInvab, and the 5'-3' antisense strand thereof is VPU-S*fU*mUmGmAmGmUmUmGmAmGdTmUfCmAfAmGmUmG*mG*mC;
wherein VPU-S is 2'-S-methyluridine-5'-(E)-vinylphosphonate-3'-phosphate, mA is 2'-O-methyladenosine-3'-phosphate, mU is 2'-O-methyluridine-3'-phosphate, mC is 2'-O-methylcytidine-3'-phosphate, mG is 2'-O-methylguanosine-3'-phosphate, fA is 2'-fluoroadenosine-3'-phosphate, fU is 2'-fluorouridine-3'-phosphate, fC is 2'-fluorocytidine-3'-phosphate, fG is 2'-fluoroguanosine-3'-phosphate, dA is 2'-deoxyadenosine-3'-phosphate, dT is 2'-deoxythymidine-3'-phosphate, dC is 2'-deoxycytidine-3'-phosphate, dG is 2'-deoxyguanosine-3'-phosphate, Invab is an inverted abasic residue, and * is a phosphorothioate bond.

28. An siRNA agent targeting AGT, **characterized in that** the siRNA agent is formed by connecting the siRNA or a pharmaceutically acceptable salt thereof according to any one of claims 22-23 to a ligand for delivering nucleic acid.

29. An siRNA agent targeting PCSK9, **characterized in that** the siRNA agent is formed by connecting the siRNA or a pharmaceutically acceptable salt thereof according to any one of claims 24-25 to a ligand for delivering nucleic acid.

30. An siRNA agent targeting ANGPTL3, **characterized in that** the siRNA agent is formed by connecting the siRNA or a pharmaceutically acceptable salt thereof according to any one of claims 26-27 to a ligand for delivering nucleic acid.

31. The siRNA agent according to any one of claims 28-30, **characterized in that** the ligand is GalNAc or a derivative thereof, preferably, wherein the ligand has the structural formula as follows:

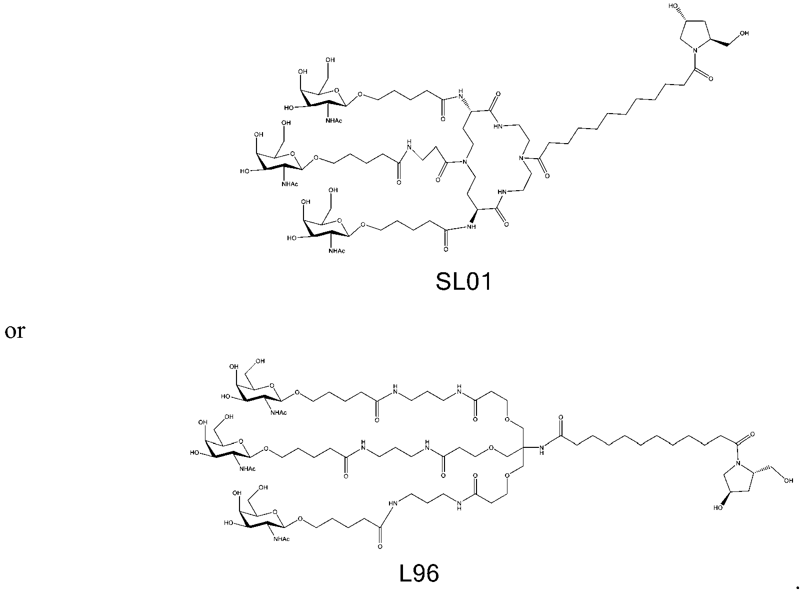

32. The siRNA agent according to claim 31, **characterized in that** the ligand is connected to the 3'-termini of sense strands via chemical bonds, preferably via phosphate bonds or phosphorothioate bonds, and the connection mode is as follows: 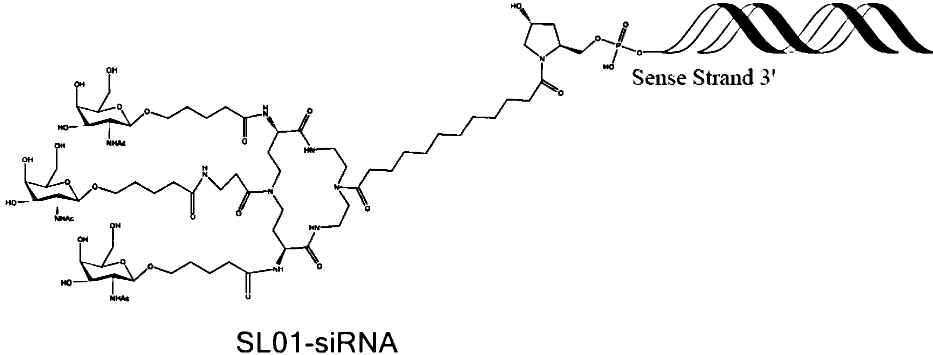 or
